# EUROPEAN PATENT SPECIFICATION

(11) **EP 3 983 385 B1**
(45) Date of publication and mention of the grant of the patent: **30.08.2023**
(21) Application number: 20736509.9
(22) Date of filing: 13.06.2020
(51) Int. Cl.: C07D 213/50, C07D 213/61, C07D 213/65, C07D 213/79, C07D 213/82, C07D 213/84, C07D 237/08, C07D 237/14, C07D 237/24, C07D 239/26, C07D 405/10, A61K 31/44, A61P 25/28, A61P 25/16

(54) **NAPHTHOQUINONE DERIVATIVES FOR TREATMENT OF OXIDATIVE STRESS DISORDERS**
NAPHTHOCHINONDERIVATE ZUR BEHANDLUNG VON ERKRANKUNGEN IN VERBINDUNG MIT OXIDATIVEM STRESS
DÉRIVÉS DE NAPHTOQUINONE POUR LE TRAITEMENT DE TROUBLES DU STRESS OXYDATIF

(30) Priority: 13.06.2019 US 201962861255 P
(43) Date of publication of application: 20.04.2022
(73) Proprietor: PTC Therapeutics, Inc., South Plainfield, New Jersey 07080 (US)
(72) Inventor: UTEULIYEV, Maulen M., South Plainfield 07080 (US); SHU, Lianhe, South Plainfield 07080 (US); RICHARDS, Steven J., South Plainfield 07080 (US); WARKENTIN, Alexander A., South Plainfield 07080 (US); DAVIS, Dana, South Plainfield 07080 (US)
(74) Representative: J A Kemp LLP
(86) International application number: PCT/US2020/037639
(87) International publication number: WO 2020/252414

(56) References cited:
- EP-A1- 2 505 583
- WO-A1-2014/074976
- MAHAL KATHARINA ET AL: "Improved anticancer and antiparasitic activity of new lawsone Mannich bases", EUROPEAN JOURNAL OF MEDICINAL CHEMISTRY, ELSEVIER, AMSTERDAM, NL, vol. 126, 22 November 2016 (2016-11-22), pages 421-431, XP029885648, ISSN: 0223-5234, DOI: 10.1016/J.EJMECH.2016.11.043

## Description

### TECHNICAL FIELD

The application discloses compounds, compositions and uses of those compounds and compositions in methods useful for treatment or suppression of diseases, developmental delays and symptoms related to oxidative stress disorders. Examples of such disorders include mitochondrial disorders, impaired energy processing disorders, neurodegenerative diseases and diseases of aging. The application further discloses compounds, compositions and methods useful for inhibition of ferroptosis. Further provided are compounds, compositions and uses of those compounds and compositions in methods useful for treatment or suppression of diseases and symptoms related to neurodegenerative disorders.

### BACKGROUND

Oxidative stress is caused by disturbances to the normal redox state within cells. An imbalance between routine production and detoxification of reactive oxygen species such as peroxides and free radicals can result in oxidative damage to the cellular structure and machinery. The most important source of reactive oxygen species under normal conditions in aerobic organisms is probably the leakage of activated oxygen from mitochondria during normal oxidative respiration. Impairments associated with this process are suspected to contribute to mitochondrial disease, neurodegenerative disease, and diseases of aging.

Mitochondria are organelles in eukaryotic cells, popularly referred to as the "powerhouse" of the cell. One of their primary functions is oxidative phosphorylation. The molecule adenosine triphosphate (ATP) functions as an energy "currency" or energy carrier in the cell, and eukaryotic cells derive the majority of their ATP from biochemical processes carried out by mitochondria. These biochemical processes include the citric acid cycle (the tricarboxylic acid cycle, or Krebs cycle), which generates reduced nicotinamide adenine dinucleotide (NADH + H+) from oxidized nicotinamide adenine dinucleotide (NAD+), and oxidative phosphorylation, during which NADH + H+ is oxidized back to NAD+. (The citric acid cycle also reduces flavin adenine dinucleotide, or FAD, to FADH2; FADH2 also participates in oxidative phosphorylation.)

The electrons released by oxidation of NADH + H+ are shuttled down a series of protein complexes (Complex I, Complex II, Complex III, and Complex IV) known as the mitochondrial respiratory chain. These complexes are embedded in the inner membrane of the mitochondrion. Complex IV, at the end of the chain, transfers the electrons to oxygen, which is reduced to water. The energy released as these electrons traverse the complexes is used to generate a proton gradient across the inner membrane of the mitochondrion, which creates an electrochemical potential across the inner membrane. Another protein complex, Complex V (which is not directly associated with Complexes I, II, III and IV) uses the energy stored by the electrochemical gradient to convert ADP into ATP.

When cells in an organism are temporarily deprived of oxygen, anaerobic respiration is utilized until oxygen again becomes available or the cell dies. The pyruvate generated during glycolysis is converted to lactate during anaerobic respiration. The buildup of lactic acid is believed to be responsible for muscle fatigue during intense periods of activity, when oxygen cannot be supplied to the muscle cells. When oxygen again becomes available, the lactate is converted back into pyruvate for use in oxidative phosphorylation.

Oxygen poisoning or toxicity is caused by high concentrations of oxygen that may be damaging to the body and increase the formation of free-radicals and other structures such as nitric oxide, peroxynitrite, and trioxidane. Normally, the body has many defense systems against such damage, but at higher concentrations of free oxygen, these systems are eventually overwhelmed with time, and the rate of damage to cell membranes exceeds the capacity of systems which control or repair it. Cell damage and cell death then results.

Qualitative and/or quantitative disruptions in the transport of oxygen to tissues results in energy disruption in the function of red cells and contribute to various diseases such as haemoglobinopathies. Haemoglobinopathy is a kind of genetic defect that results in abnormal structure of one of the globin chains of the hemoglobin molecule. Common haemoglobinopathies include thalassemia and sickle-cell disease. Thalassemia is an inherited autosomal recessive blood disease. In thalassemia, the genetic defect results in reduced rate of synthesis of one of the globin chains that make up hemoglobin. While thalassemia is a quantitative problem of too few globins synthesized, sickle-cell disease is a qualitative problem of synthesis of an incorrectly functioning globin. Sickle-cell disease is a blood disorder characterized by red blood cells that assume an abnormal, rigid, sickle shape. Sickling decreases the cells' flexibility and results in their restricted movement through blood vessels, depriving downstream tissues of oxygen.

Mitochondrial dysfunction contributes to various disease states. Some mitochondrial diseases are due to mutations or deletions in the mitochondrial genome. If a threshold proportion of mitochondria in the cell is defective, and if a threshold proportion of such cells within a tissue have defective mitochondria, symptoms of tissue or organ dysfunction can result. Practically any tissue can be affected, and a large variety of symptoms may be present, depending on the extent to which different tissues are involved. Some examples of mitochondrial diseases are Friedreich's ataxia (FRDA), Leber's Hereditary Optic Neuropathy (LHON), mitochondrial myopathy, encephalopathy, encephalomyopathy, lactacidosis, and stroke (MELAS), Myoclonus Epilepsy Associated with Ragged-Red Fibers (MERRF) syndrome, Leigh Syndrome, Leigh-like Syndrome, and respiratory chain disorders. Most mitochondrial diseases involve children who manifest the signs and symptoms of accelerated aging, including neurodegenerative diseases, stroke, blindness, hearing impairment, vision impairment, diabetes, and heart failure.

Friedreich's ataxia is an autosomal recessive neurodegenerative and cardiodegenerative disorder caused by decreased levels of the protein Frataxin. The disease causes the progressive loss of voluntary motor coordination (ataxia) and cardiac complications. Symptoms typically begin in childhood, and the disease progressively worsens as the patient grows older; patients eventually become wheelchair-bound due to motor disabilities.

Leber's Hereditary Optic Neuropathy (LHON) is a disease characterized by blindness which occurs on average between 27 and 34 years of age. Other symptoms may also occur, such as cardiac abnormalities and neurological complications.

Mitochondrial myopathy, encephalopathy, lactacidosis, and stroke (MELAS) can manifest itself in infants, children, or young adults. Strokes, accompanied by vomiting and seizures, are one of the most serious symptoms. It is postulated that the metabolic impairment of mitochondria in certain areas of the brain is responsible for cell death and neurological lesions, rather than the impairment of blood flow as occurs in ischemic stroke.

Myoclonus Epilepsy Associated with Ragged-Red Fibers (MERRF) syndrome is one of a group of rare muscular disorders that are called mitochondrial encephalomyopathies. Mitochondrial encephalomyopathies are disorders in which a defect in the genetic material arises from a part of the cell structure that releases energy (mitochondria). This can cause a dysfunction of the brain and muscles (encephalomyopathies). The mitochondrial defect as well as "ragged-red fibers" (an abnormality of tissue when viewed under a microscope) are always present. The most characteristic symptom of MERRF syndrome is myoclonic seizures that are usually sudden, brief, jerking, spasms that can affect the limbs or the entire body, difficulty speaking (dysarthria), optic atrophy, short stature, hearing loss, dementia, and involuntary jerking of the eyes (nystagmus) may also occur.

Leigh Disease or Leigh Syndrome is a rare inherited neurometabolic disorder characterized by degeneration of the central nervous system where the symptoms usually begin between the ages of 3 months to 2 years and progress rapidly. In most children, the first signs may be poor sucking ability and loss of head control and motor skills. These symptoms may be accompanied by loss of appetite, vomiting, irritability, continuous crying, and seizures. As the disorder progresses, symptoms may also include generalized weakness, lack of muscle tone, and episodes of lactic acidosis, which can lead to impairment of respiratory and kidney function. Heart problems may also occur.

Co-Enzyme Q10 Deficiency is a respiratory chain disorder, with syndromes such as myopathy with exercise intolerance and recurrent myoglobin in the urine manifested by ataxia, seizures or mental retardation and leading to renal failure (Di Mauro et al., (2005) Neuromusc. Disord., 15:311-315), childhood-onset cerebellar ataxia and cerebellar atrophy (Masumeci et al., (2001) Neurology 56:849-855 and Lamperti et al., (2003) 60:1206:1208); and infantile encephalomyopathy associated with nephrosis. Biochemical measurement of muscle homogenates of patients with CoQ10 deficiency showed severely decreased activities of respiratory chain complexes I and II + III, while complex IV (COX) was moderately decreased (Gempel et al., (2007) Brain, 130(8):2037-2044).

Complex I Deficiency or NADH dehydrogenase NADH-CoQ reductase deficiency is a respiratory chain disorder, with symptoms classified by three major forms: (1) fatal infantile multisystem disorder, characterized by developmental delay, muscle weakness, heart disease, congenital lactic acidosis, and respiratory failure; (2) myopathy beginning in childhood or in adult life, manifesting as exercise intolerance or weakness; and (3) mitochondrial encephalomyopathy (including MELAS), which may begin in childhood or adult life and consists of variable combinations of symptoms and signs, including ophthalmoplegia, seizures, dementia, ataxia, hearing loss, pigmentary retinopathy, sensory neuropathy, and uncontrollable movements.

Complex II Deficiency or Succinate dehydrogenase deficiency is a respiratory chain disorder with symptoms including encephalomyopathy and various manifestations, including failure to thrive, developmental delay, hypotonia, lethargy, respiratory failure, ataxia, myoclonus, and lactic acidosis.

Complex III Deficiency or Ubiquinone-cytochrome C oxidoreductase deficiency is a respiratory chain disorder with symptoms categorized in four major forms: (1) fatal infantile encephalomyopathy, congenital lactic acidosis, hypotonia, dystrophic posturing, seizures, and coma; (2) encephalomyopathies of later onset (childhood to adult life): various combinations of weakness, short stature, ataxia, dementia, hearing loss, sensory neuropathy, pigmentary retinopathy, and pyramidal signs; (3) myopathy, with exercise intolerance evolving into fixed weakness; and (4) infantile histiocytoid cardiomyopathy.

Complex IV Deficiency or Cytochrome C oxidase deficiency is a respiratory chain disorder with symptoms categorized in two major forms: (1) encephalomyopathy, where patients typically are normal for the first 6 to 12 months of life and then show developmental regression, ataxia, lactic acidosis, optic atrophy, ophthalmoplegia, nystagmus, dystonia, pyramidal signs, respiratory problems and frequent seizures; and (2) myopathy with two main variants: (a) Fatal infantile myopathy-may begin soon after birth and accompanied by hypotonia, weakness, lactic acidosis, ragged-red fibers, respiratory failure, and kidney problems: and (b) Benign infantile myopathy- may begin soon after birth and accompanied by hypotonia, weakness, lactic acidosis, ragged-red fibers, respiratory problems, but (if the child survives) followed by spontaneous improvement.

Complex V Deficiency or ATP synthase deficiency is a respiratory chain disorder including symptoms such as slow, progressive myopathy.

Chronic Progressive External Ophthalmoplegia Syndrome (CPEO) is a respiratory chain disorder including symptoms such as visual myopathy, retinitis pigmentosa, or dysfunction of the central nervous system.

Kearns-Sayre Syndrome (KSS) is a mitochondrial disease characterized by a triad of features including: (1) typical onset in persons younger than age 20 years; (2) chronic, progressive, external ophthalmoplegia; and (3) pigmentary degeneration of the retina. In addition, KSS may include cardiac conduction defects, cerebellar ataxia, and raised cerebrospinal fluid (CSF) protein levels (e.g., >100 mg/dL). Additional features associated with KSS may include myopathy, dystonia, endocrine abnormalities (e.g., diabetes, growth retardation or short stature, and hypoparathyroidism), bilateral sensorineural deafness, dementia, cataracts, and proximal renal tubular acidosis.

Maternally inherited diabetes and deafness (MIDD) is a mitochondrial disorder characterized by maternally transmitted diabetes and sensorineural deafness. In most cases, MIDD is caused by a point mutation in the mitochondrial gene MT-TL1, encoding the mitochondrial tRNA for leucine, and in rare cases in MT-TE and MT-TK genes, encoding the mitochondrial tRNAs for glutamic acid, and lysine, respectively.

In addition to congenital disorders involving inherited defective mitochondria, acquired mitochondrial dysfunction contributes to diseases, particularly neurodegenerative disorders associated with aging like Parkinson's, Alzheimer's, and Huntington's Diseases. The incidence of somatic mutations in mitochondrial DNA rises exponentially with age; diminished respiratory chain activity is found universally in aging people. Mitochondrial dysfunction is also implicated in excitoxic, neuronal injury, such as that associated with cerebrovascular accidents, seizures, and ischemia.

Some of the diseases disclosed herein, including the above diseases, appear to be caused by defects in Complex I of the respiratory chain. Electron transfer from Complex I to the remainder of the respiratory chain is mediated by the compound coenzyme Q (also known as Ubiquinone). Oxidized coenzyme Q (CoQox or Ubiquinone) is reduced by Complex I to reduced coenzyme Q (CoQred or Ubiquinol). The reduced coenzyme Q then transfers its electrons to Complex III of the respiratory chain, where it is re-oxidized to CoQox (Ubiquinone). CoQox can then participate in further iterations of electron transfer.

Very few treatments are available for patients suffering from these mitochondrial diseases. The compound Idebenone has been proposed for treatment of Friedreich's Ataxia. While the clinical effects of Idebenone have been relatively modest, the complications of mitochondrial diseases can be so severe that even marginally useful therapies are preferable to the untreated course of the disease. Another compound, MitoQ, has been proposed for treating mitochondrial disorders (see U.S. Patent No. 7,179,928); clinical results for MitoQ have not yet been reported. Administration of coenzyme Q10 (CoQ10) and vitamin supplements has shown only transient beneficial effects in individual cases of KSS. CoQ10 supplementation has also been used for the treatment of CoQ10 deficiency with mixed results.

Oxidative stress is suspected to be important in neurodegenerative diseases such as Motor Neuron Disease, Amyotrophic Lateral Sclerosis (ALS), Creutzfeldt-Jakob disease, Machado-Joseph disease, Spino-cerebellar ataxia, Multiple sclerosis (MS), Parkinson's disease, Alzheimer's disease, and Huntington's disease. Oxidative stress is thought to be linked to certain cardiovascular disease and also plays a role in the ischemic cascade due to oxygen reperfusion injury following hypoxia. This cascade includes both strokes and heart attacks.

Damage accumulation theory, also known as the free radical theory of aging, invokes random effects of free radicals produced during aerobic metabolism that cause damage to DNA, lipids and proteins and accumulate over time. The concept of free radicals playing a role in the aging process was first introduced by Himan D (1956), Aging - A theory based on free-radical and radiation chemistry J. Gerontol. 11, 298-300.

According to the free radical theory of aging, the process of aging begins with oxygen metabolism (Valko et al, (2004) Role of oxygen radicals in DNA damage and cancer incidence, Mol. Cell. Biochem., 266, 37-56). Even under ideal conditions some electrons "leak" from the electron transport chain. These leaking electrons interact with oxygen to produce superoxide radicals, so that under physiological conditions, about 1-3% of the oxygen molecules in the mitochondria are converted into superoxide. The primary site of radical oxygen damage from superoxide radical is mitochondrial DNA (mtDNA) (Cadenas et al., (2000) Mitochondrial free radical generation, oxidative stress and aging, Free Radic. Res, 28, 601-609). The cell repairs much of the damage done to nuclear DNA (nDNA) but mtDNA repair seems to be less efficient. Therefore, extensive mtDNA damage accumulates over time and shuts down mitochondria causing cells to die and the organism to age.

Some of the diseases associated with increasing age are cancer, diabetes mellitus, hypertension, atherosclerosis, ischemia/reperfusion injury, rheumatoid arthritis, neurodegenerative disorders such as dementia, Alzheimer's, and Parkinson's. Diseases resulting from the process of aging as a physiological decline include decreases in muscle strength, cardiopulmonary function, vision and hearing, as well as wrinkled skin and graying hair.

Ferroptosis, a non-apoptotic form of cell death, has been shown to drive neurodegeneration via increased lipid peroxidation and oxidative stress (Chen et al. J. Biol. Chem. 2015, 290, 28097-28106). Thus, ferroptosis inhibitors may be useful in treating neurodegenerative disorders.

WO 2014/074976 discloses compounds for the treatment of neurological diseases or injuries, including neurodegenerative diseases, stroke, trauma, epilepsy, acute and chronic kidney injuries, diabetes mellitus, and/or seizures, where in some embodiments, derivatives of vitamin K are provided.

What is needed are compounds useful in treating or suppressing oxidative stress disorders. What is further needed are compounds useful in inhibiting ferroptosis. In addition, what is needed are compounds useful in the treatment of neurodegenerative disorders such as Alzheimer's Disease, Parkinson's Disease, Huntington's Disease, Juvenile Huntington's Disease, amyotrophic lateral sclerosis, and frontotemporal dementia (FTD).

What is further needed are compounds useful in treating or suppressing oxidative stress disorders, inhibiting ferroptosis, and/or in the treatment or suppression of neurodegenerative disorders, wherein the compounds preferably do not interfere with the metabolism of co-administered medications via inhibition of cytochrome P450 enzymes, in particular the 3A4 isoform. Thus, compounds that do not inhibit CYP3A4 may be advantageous in the treatment or suppression of oxidative stress disorders, inhibition of ferroptosis, and/or the treatment or suppression of neurodegenerative disorders.

### BRIEF SUMMARY OF THE INVENTION

In one aspect is a compound of the formula: 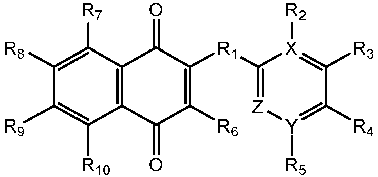 or the reduced form thereof; wherein: X and Y are C; or X is N, Y is C, and R₂ is not present; or X is C, Y is N, and R₅ is not present; Z is N or N-oxide, wherein when Z is N-oxide then X and Y are C; R₁ is -C(R₁₁)(R₁₂)-; R₂, R₃, R₄, and R₅ are each independently selected from the group consisting of hydrogen, C₁-C₆ haloalkyl, halo, C₁-C₆ alkyl, C₂-C₆ alkenyl, C₂-C₆ alkynyl, cyano, C₁-C₆ alkoxy, -C(O)OH, - C(O)O(phenyl), -C(O)NR₁₃R₁₄, -OH, and C₁-C₆ haloalkoxy; R₆ is C₁-C₆ alkyl, H, or OH; where the C₁-C₆ alkyl is optionally substituted with a 3-7 membered heterocyclic group which is optionally substituted with one C₁-C₆ alkyl or C₁-C₆ haloalkyl; R₇, R₈, R₉, and R₁₀ are each independently selected from the group consisting of: hydrogen, halo, C₁-C₆ alkyl, C₁-C₆ haloalkyl, hydroxy, C₁-C₆ alkoxy, -NHSO₂CH₃, and 5-6 membered heteroaryl where the 5-6 membered heteroaryl is optionally substituted with one C₁-C₆ alkyl; R₁₁ and R₁₂ are independently selected from the group consisting of hydrogen, methyl, and methoxy; with the proviso that R₁₁ and R₁₂ are not both methoxy; and R₁₃ and R₁₄ are each independently H or C₁-C₆ alkyl; or wherein R₁₃ and R₁₄ together with the nitrogen atom to which they are attached form a 4-6 membered saturated heterocyclic ring in which one of the carbons is optionally replaced by an additional N, and wherein the 4-6 membered saturated heterocyclic ring is optionally substituted with one or more substituents independently selected from the group consisting of halo and C₁-C₆ alkyl; or any salt(s), stereoisomer, mixture of stereoisomers, isotopologue(s), solvate(s), and/or hydrate(s) thereof. In some or any embodiments, including any of the foregoing embodiments, R₁ is -CH₂-; or any salt(s), stereoisomer, mixture of stereoisomers, isotopologue(s), solvate(s), and/or hydrate(s) thereof. In some or any embodiments, including any of the foregoing embodiments, R₁ is -CD₂-; or any salt(s), stereoisomer, mixture of stereoisomers, isotopologue(s), solvate(s), and/or hydrate(s) thereof. In some or any embodiments, including any of the foregoing embodiments, R₁ is -CH(CH₃)- ; or any salt(s), stereoisomer, mixture of stereoisomers, isotopologue(s), solvate(s), and/or hydrate(s) thereof. In some or any embodiments, including any of the foregoing embodiments, R₁ is -CH(OCH₃)-; or any salt(s), stereoisomer, mixture of stereoisomers, isotopologue(s), solvate(s), and/or hydrate(s) thereof. In some or any embodiments, including any of the foregoing embodiments, R₂, R₃, R₄, and R₅ are each independently selected from the group consisting of hydrogen, C₁-C₆ haloalkyl, halo, C₁-C₆ alkyl, C₁-C₆ alkoxy, C₁-C₆ haloalkoxy, C₂-C₆ alkynyl, -OH, cyano, -C(O)OH, and - C(O)NR₁₃R₁₄; or any salt(s), stereoisomer, mixture of stereoisomers, isotopologue(s), solvate(s), and/or hydrate(s) thereof. In some or any embodiments, including any of the foregoing embodiments, zero to two of R₂, R₃, R₄, and R₅ are independently selected from the group consisting of C₁-C₆ haloalkyl, halo, C₁-C₆ alkyl, C₁-C₆ alkoxy, C₁-C₆ haloalkoxy, C₂-C₆ alkynyl, -OH, cyano, -C(O)OH, and -C(O)NR₁₃R₁₄, and wherein the others are hydrogen; or any salt(s), stereoisomer, mixture of stereoisomers, isotopologue(s), solvate(s), and/or hydrate(s) thereof. In some or any embodiments, including any of the foregoing embodiments, R₁₃ and R₁₄ are each independently H or C₁-C₆ alkyl; or any salt(s), stereoisomer, mixture of stereoisomers, isotopologue(s), solvate(s), and/or hydrate(s) thereof. In some or any embodiments, including any of the foregoing embodiments, R₂, R₃, R₄, and R₅ are each independently selected from the group consisting of hydrogen, -CHF₂, -CF₃, -CF₂CF₃, -CF₂CH₃, -CH₂CF₃, -F, -Cl, -CH₃, -CH₂CH₃, -CH(CH₃)₂, -OCH₃, -OCF₃, and -OCHF₂, -C=CH, -C=CCH₃, -OH, -CN, -C(O)OH, -C(O)-NH₂, -C(O)-(CH₂CH₃)(CH(CH₃)₂), -C(O)-N(H)(CH₂-cyclopropyl), -C(O)-N(H)(cyclopentyl), -C(O)-N(CH₃)(CH(CH₃)₂), and 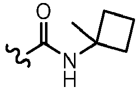 ; or any salt(s), stereoisomer, mixture of stereoisomers, isotopologue(s), solvate(s), and/or hydrate(s) thereof. In some or any embodiments, including any of the foregoing embodiments, R₂, R₃, R₄, and R₅ are each independently selected from the group consisting of hydrogen, -CF₃, -CHF₂, F, Cl, -CH₃, -OCF₃, and -CN; or any salt(s), stereoisomer, mixture of stereoisomers, isotopologue(s), solvate(s), and/or hydrate(s) thereof. In some or any embodiments, including any of the foregoing embodiments, zero to two of R₂, R₃, R₄, and R₅ are independently selected from the group consisting of -CHF₂, -CF₃, -CF₂CF₃, -CF₂CH₃, -CH₂CF₃, -F, -Cl, -CH₃, -CH₂CH₃, -CH(CH₃)₂, -OCH₃, -OCF₃, and -OCHF₂, -C=CH, -C=CCH₃, -OH, -CN, -C(O)OH, -C(O)-NH₂, -C(O)-N(CH₂CH₃)(CH(CH₃)₂), -C(O)-N(H)(CH₂-cyclopropyl), -C(O)-N(H)(cyclopentyl), -C(O)-N(CH₃)(CH(CH₃)₂), and 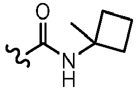 , and wherein the others are hydrogen; or any salt(s), stereoisomer, mixture of stereoisomers, isotopologue(s), solvate(s), and/or hydrate(s) thereof. In some or any embodiments, including any of the foregoing embodiments, zero to two of R₂, R₃, R₄, and R₅ are independently selected from the group consisting of -CF₃, -CHF₂, F, Cl, -CH₃, -OCF₃, and -CN, and wherein the others are hydrogen; or any salt(s), stereoisomer, mixture of stereoisomers, isotopologue(s), solvate(s), and/or hydrate(s) thereof. In some or any embodiments, including any of the foregoing embodiments, R₆ is unsubstituted C₁-C₆ alkyl; or any salt(s), stereoisomer, mixture of stereoisomers, isotopologue(s), solvate(s), and/or hydrate(s) thereof. In some or any embodiments, including any of the foregoing embodiments, R₆ is selected from the group consisting of methyl, -CD₃, ethyl, cyclopropyl, and n-propyl; or any salt(s), stereoisomer, mixture of stereoisomers, isotopologue(s), solvate(s), and/or hydrate(s) thereof. In some or any embodiments, including any of the foregoing embodiments, R₆ is unsubstituted C₂-C₆ alkyl; or any salt(s), stereoisomer, mixture of stereoisomers, isotopologue(s), solvate(s), and/or hydrate(s) thereof. In some or any embodiments, including any of the foregoing embodiments, R₆ is methyl; or any salt(s), stereoisomer, mixture of stereoisomers, isotopologue(s), solvate(s), and/or hydrate(s) thereof. In some or any embodiments, including any of the foregoing embodiments, R₆ is -CD₃; or any salt(s), stereoisomer, mixture of stereoisomers, isotopologue(s), solvate(s), and/or hydrate(s) thereof. In some or any embodiments, including any of the foregoing embodiments, R₆ is ethyl; or any salt(s), stereoisomer, mixture of stereoisomers, isotopologue(s), solvate(s), and/or hydrate(s) thereof. In some or any embodiments, including any of the foregoing embodiments, R₆ is n-propyl; or any salt(s), stereoisomer, mixture of stereoisomers, isotopologue(s), solvate(s), and/or hydrate(s) thereof. In some or any embodiments, including any of the foregoing embodiments, R₆ is cyclopropyl; or any salt(s), stereoisomer, mixture of stereoisomers, isotopologue(s), solvate(s), and/or hydrate(s) thereof. In some or any embodiments, including any of the foregoing embodiments, R₆ is C₁-C₆ alkyl substituted with a 3-7 membered heterocyclic group which is optionally substituted with one C₁-C₆ alkyl or C₁-C₆ haloalkyl; or any salt(s), stereoisomer, mixture of stereoisomers, isotopologue(s), solvate(s), and/or hydrate(s) thereof. In some or any embodiments, including any of the foregoing embodiments, R₆ is C₁-C₆ alkyl substituted with a 4-6 membered saturated heterocyclic group which is optionally substituted with one C₁-C₆ alkyl; or any salt(s), stereoisomer, mixture of stereoisomers, isotopologue(s), solvate(s), and/or hydrate(s) thereof. In some or any embodiments, including any of the foregoing embodiments, R₆ is selected from the group consisting of: -CH₃, -CD₃, -CH₂CH₃, -CH₂CH₂CH₃, -CH(CH₃)₂, - CH₂CH(CH₃)₂, -CH₂CH₂CH(CH₃)₂, -CH₂C(CH₃)₃, -cyclopropyl, 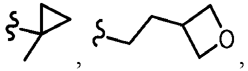 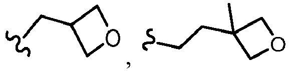 , -H, and -OH; or any salt(s), stereoisomer, mixture of stereoisomers, isotopologue(s), solvate(s), and/or hydrate(s) thereof. In some or any embodiments, including any of the foregoing embodiments, R₆ is methyl or -CD₃. In some or any embodiments, including any of the foregoing embodiments, R₆ is -H; or any salt(s), stereoisomer, mixture of stereoisomers, isotopologue(s), solvate(s), and/or hydrate(s) thereof. In some or any embodiments, including any of the foregoing embodiments, R₆ is -OH; or any salt(s), stereoisomer, mixture of stereoisomers, isotopologue(s), solvate(s), and/or hydrate(s) thereof. In some or any embodiments, including any of the foregoing embodiments, R₆ is not methyl; or any salt(s), stereoisomer, mixture of stereoisomers, isotopologue(s), solvate(s), and/or hydrate(s) thereof. In some or any embodiments, including any of the foregoing embodiments, R₇, R₈, R₉, and R₁₀ are each independently selected from the group consisting of: hydrogen, halo, C₁-C₆ alkyl, C₁-C₆ haloalkyl, and C₁-C₆ alkoxy; or any salt(s), stereoisomer, mixture of stereoisomers, isotopologue(s), solvate(s), and/or hydrate(s) thereof. In some or any embodiments, including any of the foregoing embodiments, zero to three of R₇, R₈, R₉, and R₁₀ are each independently selected from the group consisting of: halo, C₁-C₆ alkyl, C₁-C₆ haloalkyl, and C₁-C₆ alkoxy, and wherein the others are hydrogen; or any salt(s), stereoisomer, mixture of stereoisomers, isotopologue(s), solvate(s), and/or hydrate(s) thereof. In some or any embodiments, including any of the foregoing embodiments, one or two of R₇, R₈, R₉, and R₁₀ are each independently selected from the group consisting of: halo, C₁-C₆ alkyl, C₁-C₆ haloalkyl, and C₁-C₆ alkoxy, and wherein the others are hydrogen; or any salt(s), stereoisomer, mixture of stereoisomers, isotopologue(s), solvate(s), and/or hydrate(s) thereof. In some or any embodiments, including any of the foregoing embodiments, R₇, R₈, R₉, and R₁₀ are hydrogen; or any salt(s), stereoisomer, mixture of stereoisomers, isotopologue(s), solvate(s), and/or hydrate(s) thereof. In some or any embodiments, including any of the foregoing embodiments, R₇, R₈, R₉, and R₁₀ are each independently selected from the group consisting of: H, -CH₃, -OCH₃, -F, -Cl, and -CF₃; or any salt(s), stereoisomer, mixture of stereoisomers, isotopologue(s), solvate(s), and/or hydrate(s) thereof. In some or any embodiments, including any of the foregoing embodiments, R₇, R₈, R₉, and R₁₀ are each independently selected from the group consisting of: hydrogen and -F; or any salt(s), stereoisomer, mixture of stereoisomers, isotopologue(s), solvate(s), and/or hydrate(s) thereof. In some or any embodiments, including any of the foregoing embodiments, R₇, R₈, R₉, and R₁₀ are each independently selected from the group consisting of: hydrogen and -Cl; or any salt(s), stereoisomer, mixture of stereoisomers, isotopologue(s), solvate(s), and/or hydrate(s) thereof. In some or any embodiments, including any of the foregoing embodiments, zero to three of R₇, R₈, R₉, and R₁₀ are each independently selected from the group consisting of: -CH₃, -OCH₃, -F, -Cl, and -CF₃, and wherein the others are hydrogen; or any salt(s), stereoisomer, mixture of stereoisomers, isotopologue(s), solvate(s), and/or hydrate(s) thereof. In some or any embodiments, including any of the foregoing embodiments, zero to three of R₇, R₈, R₉, and R₁₀ are -F, and wherein the others are hydrogen; or any salt(s), stereoisomer, mixture of stereoisomers, isotopologue(s), solvate(s), and/or hydrate(s) thereof. In some or any embodiments, including any of the foregoing embodiments, one or two of R₇, R₈, R₉, and R₁₀ are each independently selected from the group consisting of: -CH₃, -OCH₃, -F, -Cl, and -CF₃, and wherein the others are hydrogen; or any salt(s), stereoisomer, mixture of stereoisomers, isotopologue(s), solvate(s), and/or hydrate(s) thereof. In some or any embodiments, including any of the foregoing embodiments, one or two of R₇, R₈, R₉, and R₁₀ are -F, and wherein the others are hydrogen; or any salt(s), stereoisomer, mixture of stereoisomers, isotopologue(s), solvate(s), and/or hydrate(s) thereof. In some or any embodiments, including any of the foregoing embodiments, one or two of R₇, R₈, R₉, and R₁₀ are -Cl, and wherein the others are hydrogen; or any salt(s), stereoisomer, mixture of stereoisomers, isotopologue(s), solvate(s), and/or hydrate(s) thereof. In some or any embodiments, including any of the foregoing embodiments, one or two of R₇, R₈, R₉, and R₁₀ are each independently selected from the group consisting of: -F and -Cl, and wherein the others are hydrogen; or any salt(s), stereoisomer, mixture of stereoisomers, isotopologue(s), solvate(s), and/or hydrate(s) thereof. In some or any embodiments, including any of the foregoing embodiments, Z is N; or any salt(s), stereoisomer, mixture of stereoisomers, isotopologue(s), solvate(s), and/or hydrate(s) thereof. In some or any embodiments, including any of the foregoing embodiments, Z is N-oxide; or any salt(s), stereoisomer, mixture of stereoisomers, isotopologue(s), solvate(s), and/or hydrate(s) thereof. In some or any embodiments, including any of the foregoing embodiments, X and Y are C; or any salt(s), stereoisomer, mixture of stereoisomers, isotopologue(s), solvate(s), and/or hydrate(s) thereof. In some or any embodiments, including any of the foregoing embodiments, X is N, Y is C, and R₂ is not present; or any salt(s), stereoisomer, mixture of stereoisomers, isotopologue(s), solvate(s), and/or hydrate(s) thereof. In some or any embodiments, including any of the foregoing embodiments, wherein Y is N, X is C, and R₅ is not present; or any salt(s), stereoisomer, mixture of stereoisomers, isotopologue(s), solvate(s), and/or hydrate(s) thereof. In some or any embodiments, including any of the foregoing embodiments, the compound is selected from the group consisting of: 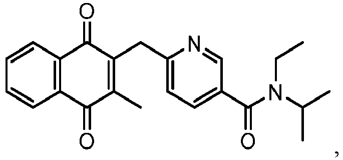 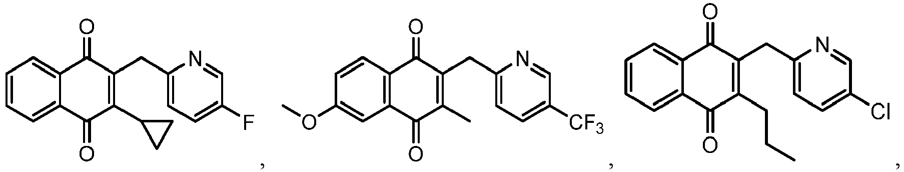 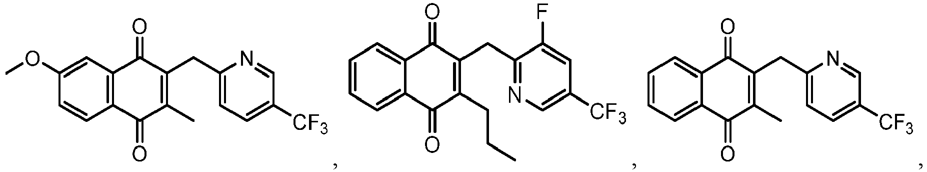 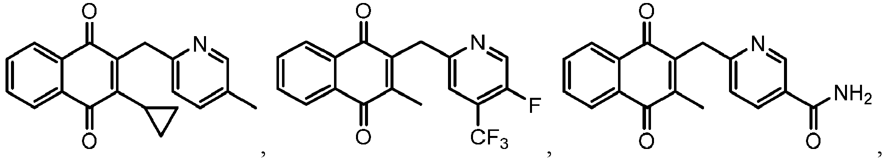 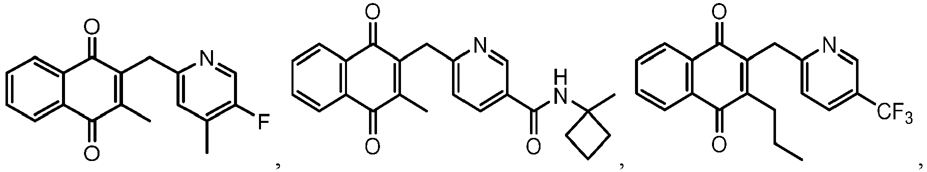 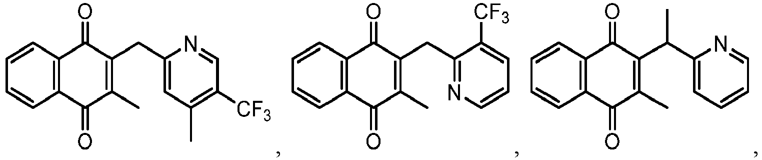 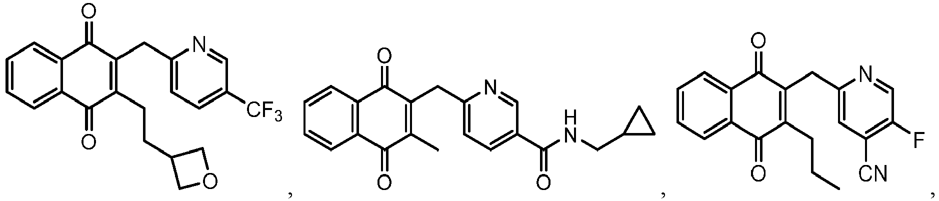 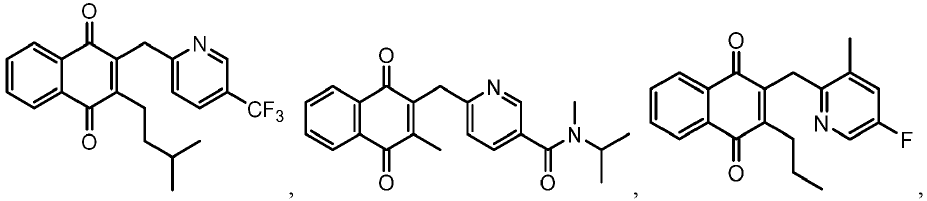 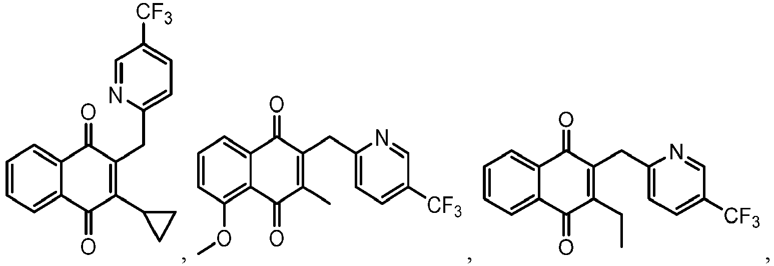 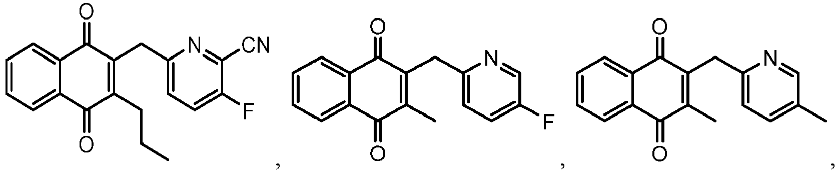 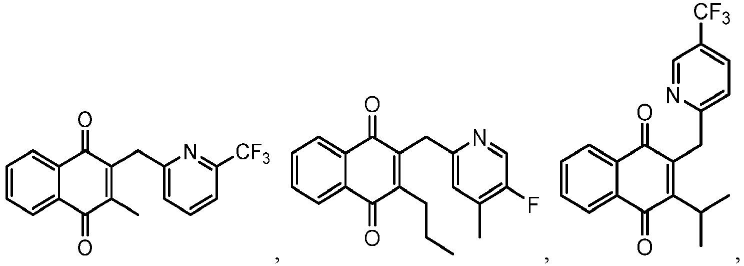 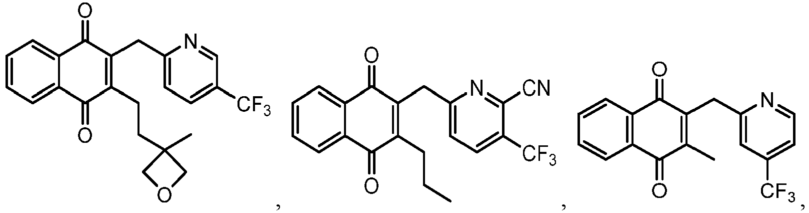 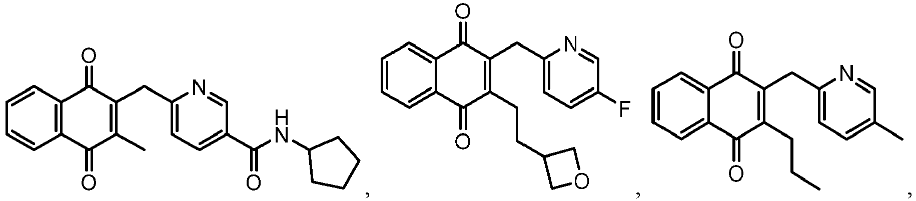 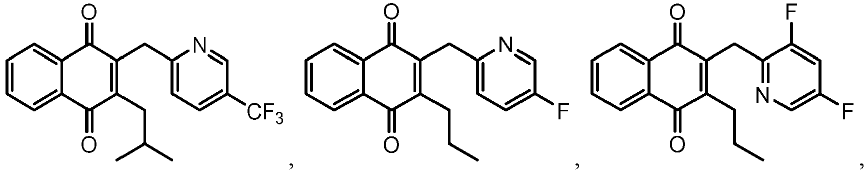 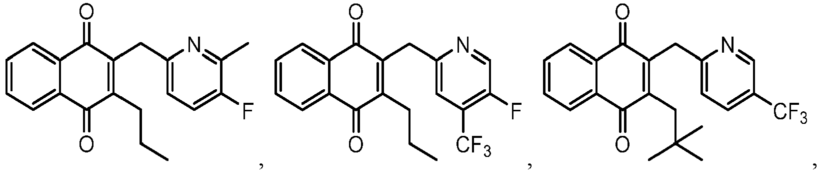 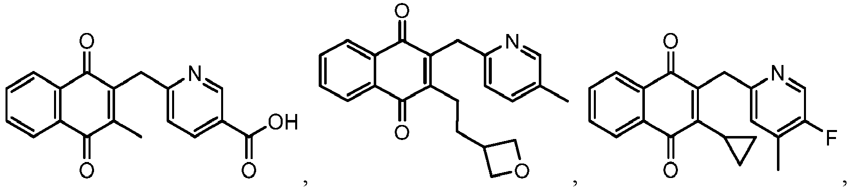 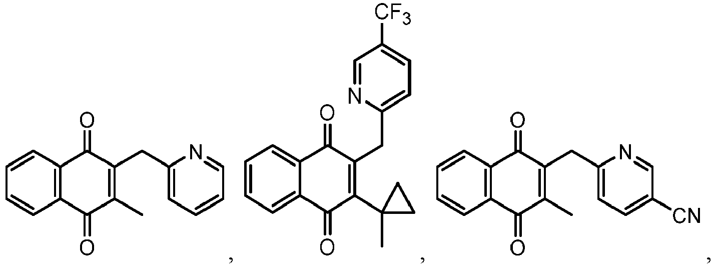 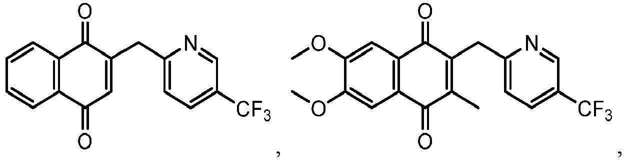 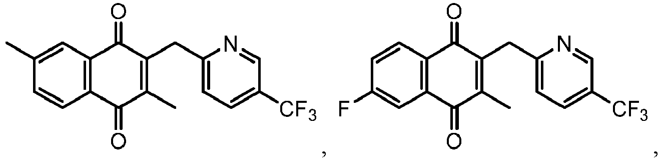 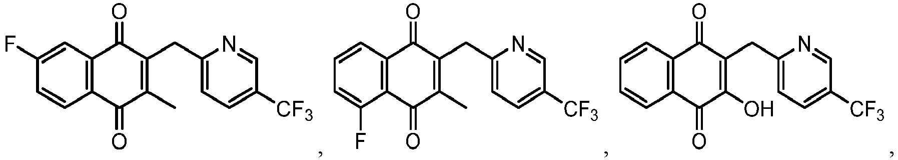 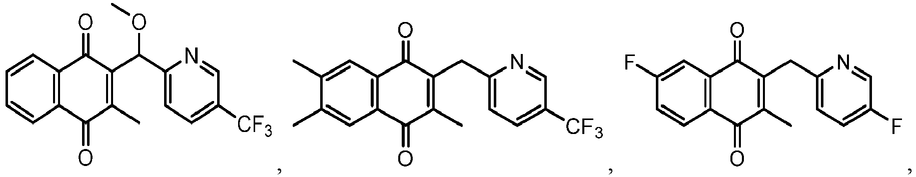 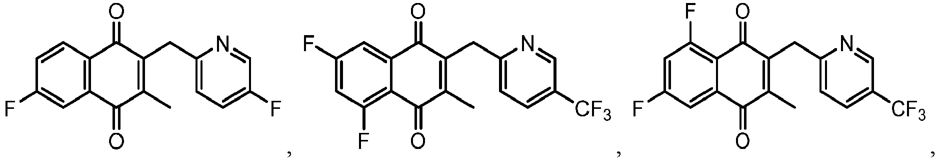 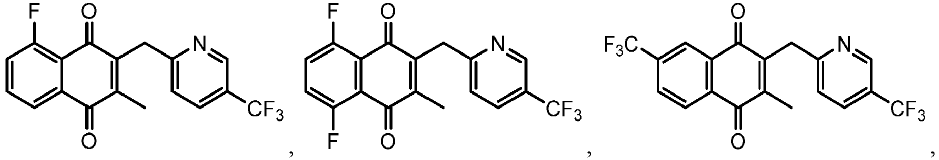 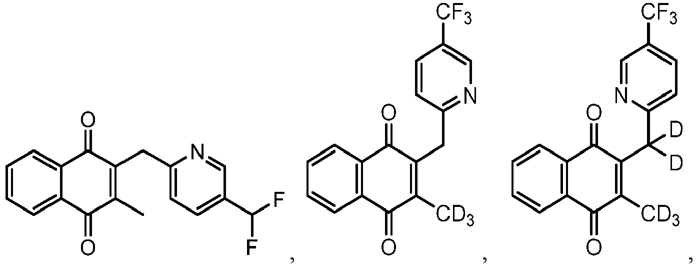 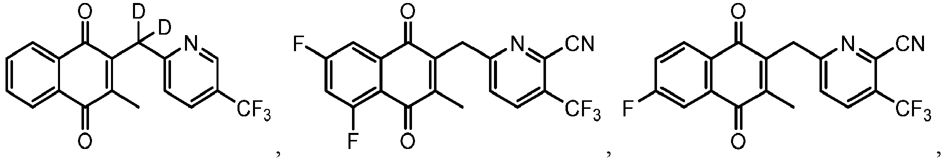 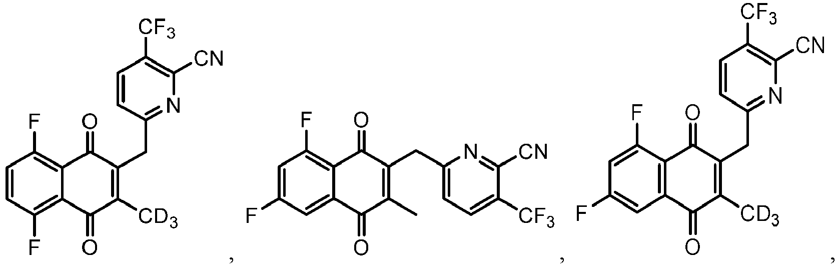 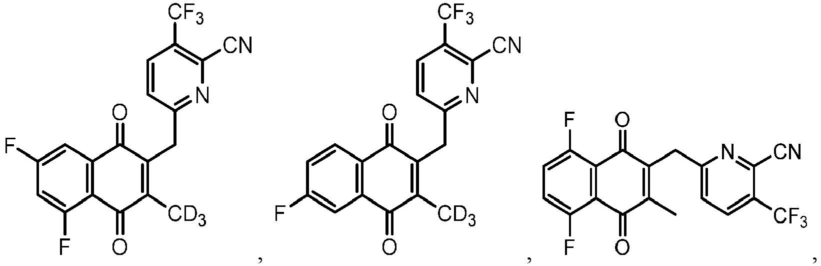 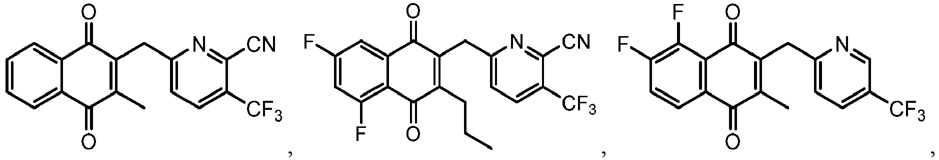 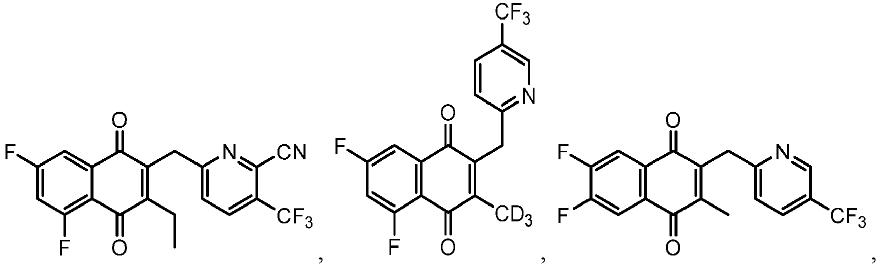 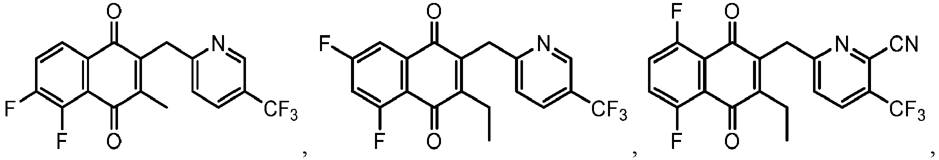 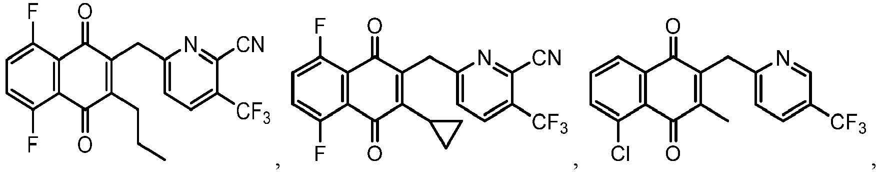 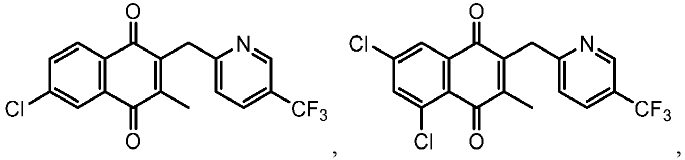 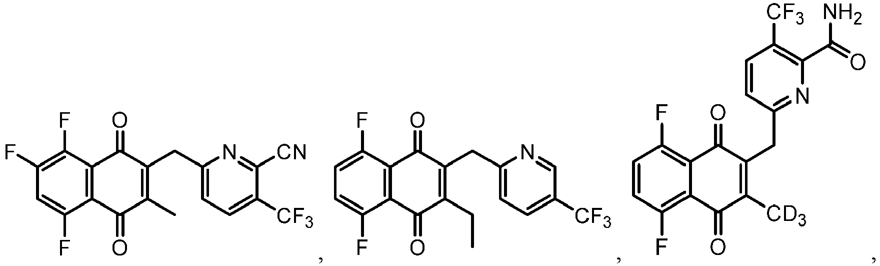 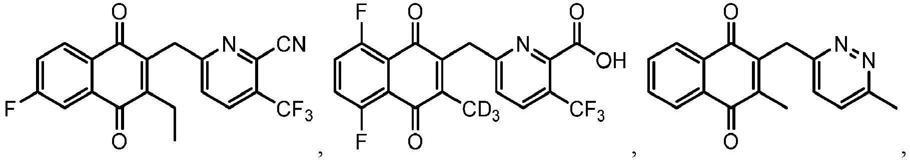 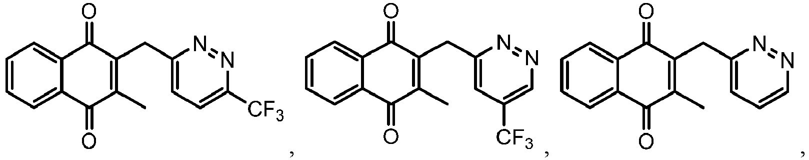 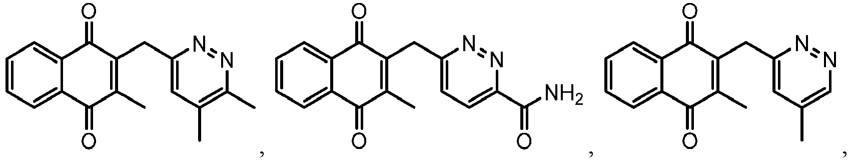 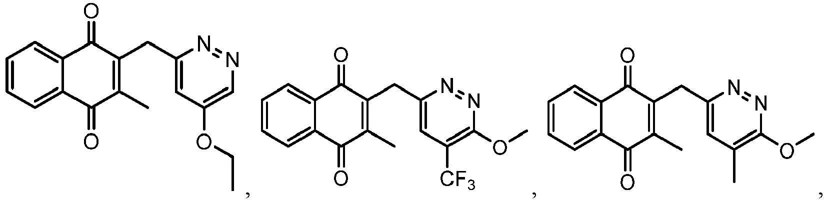 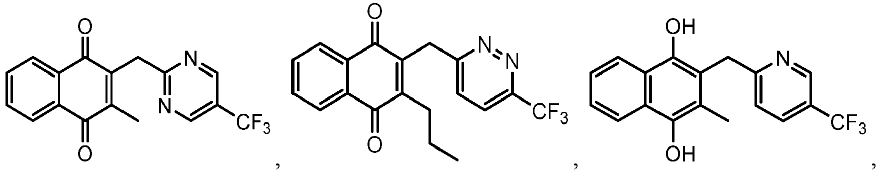 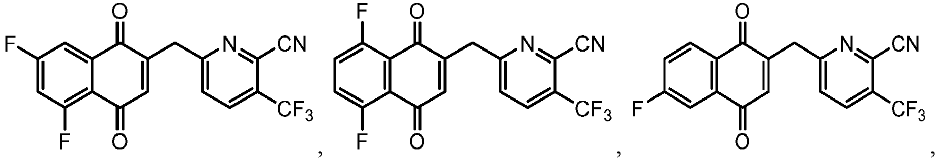 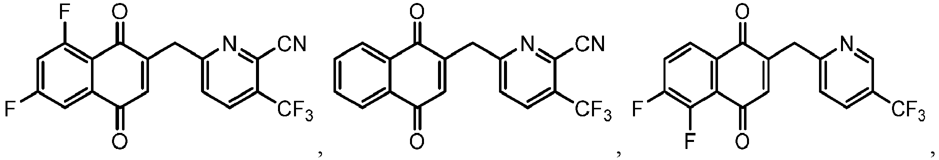 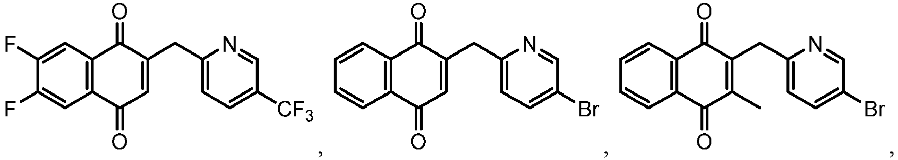 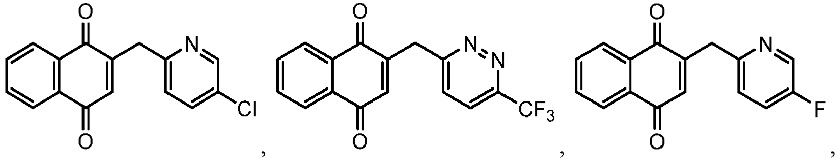 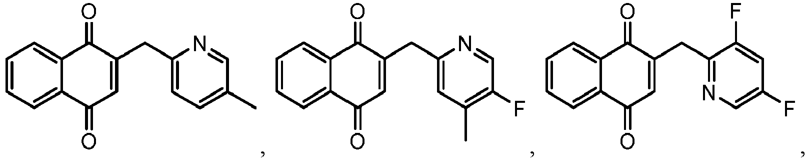 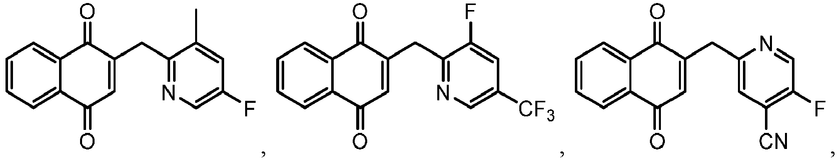 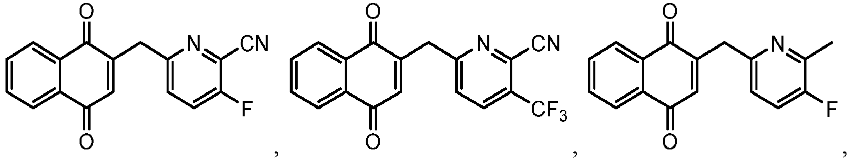 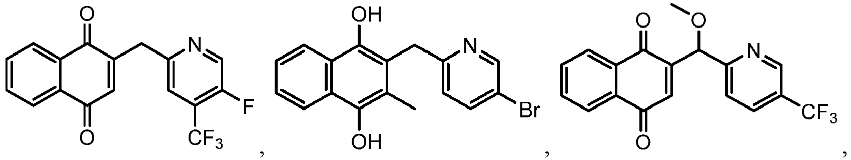 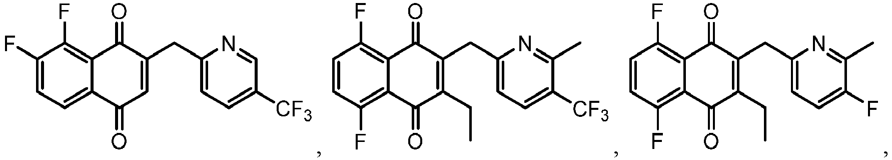 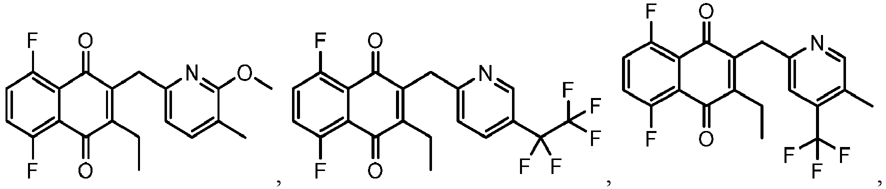 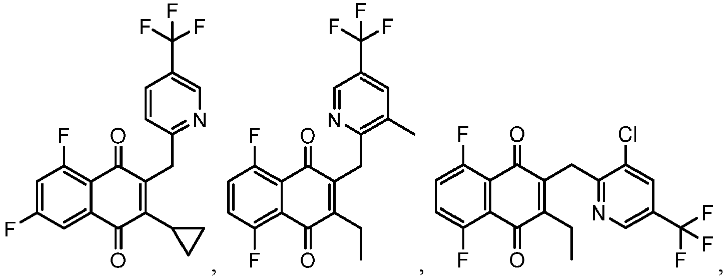 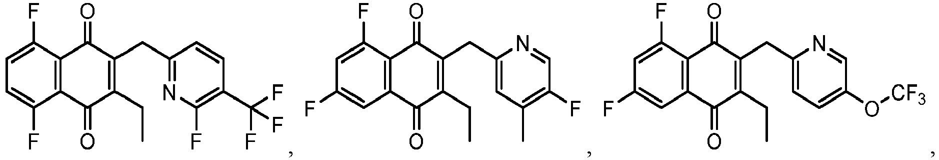 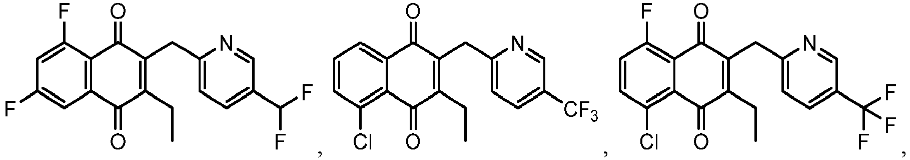 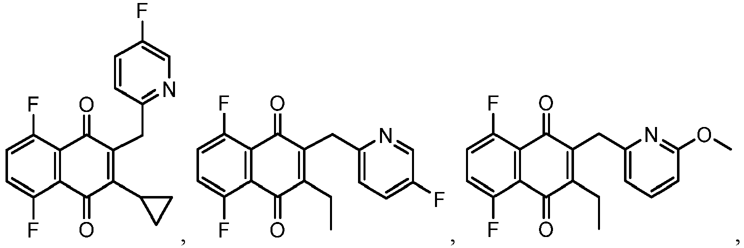 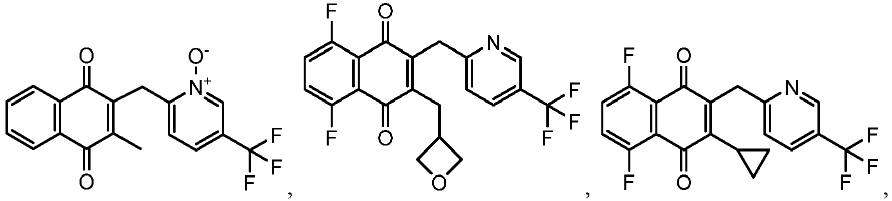 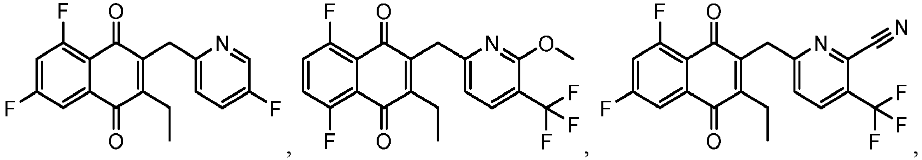 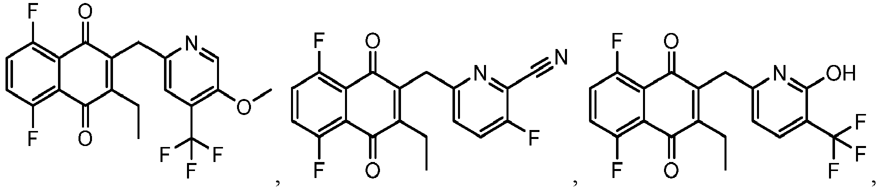 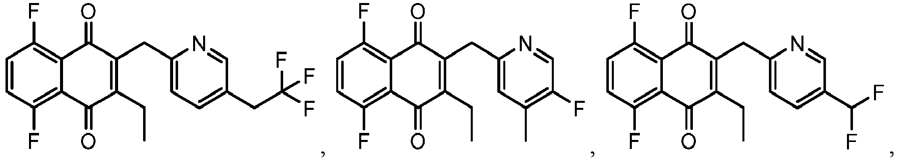 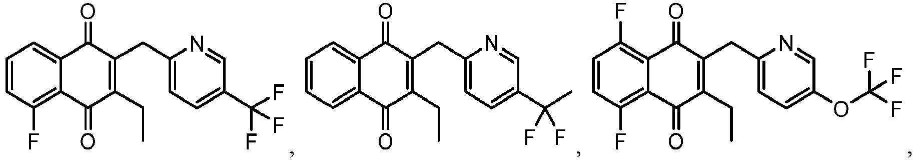 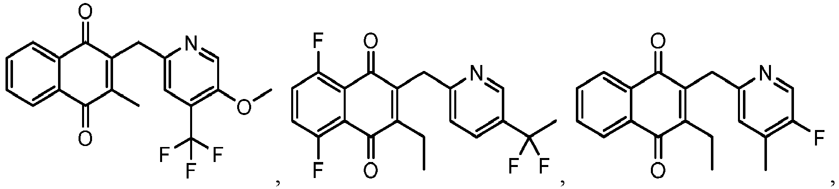 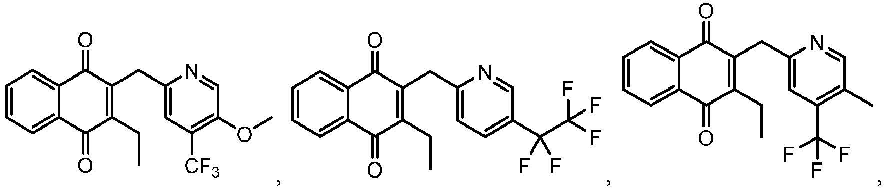 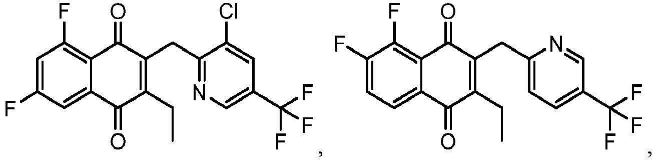 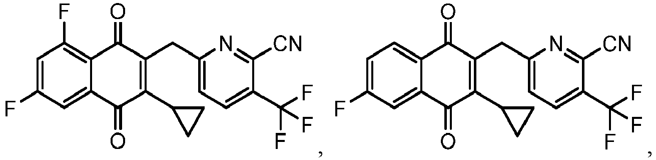 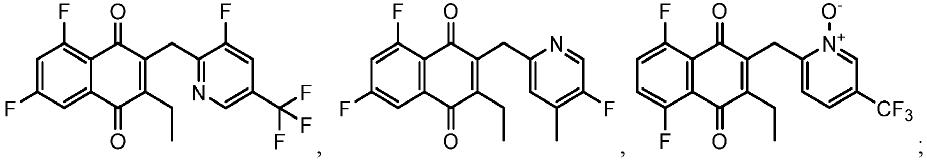 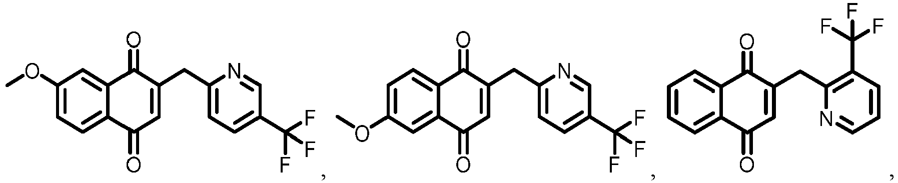 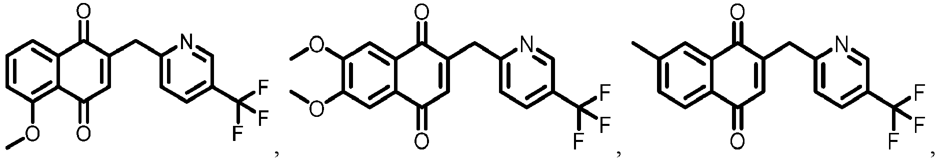 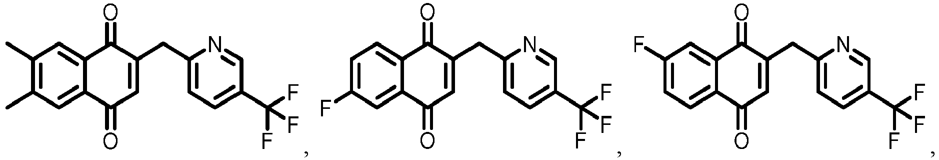 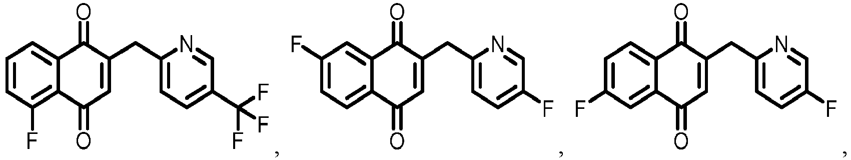 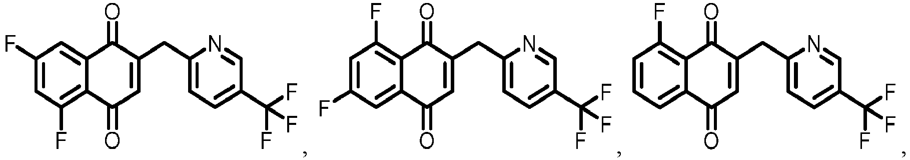 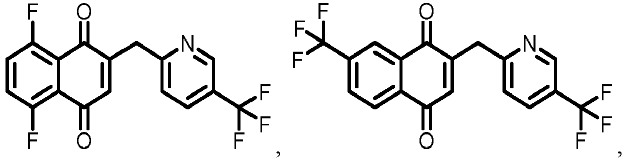 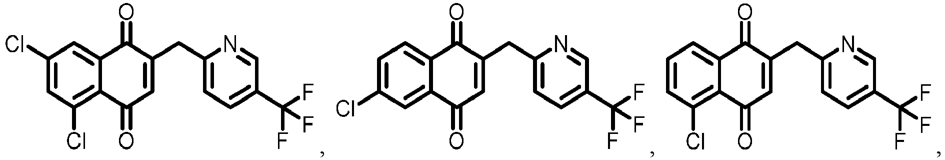 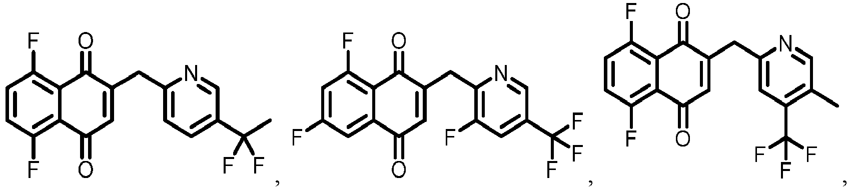 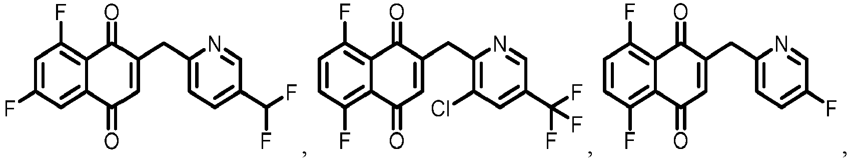 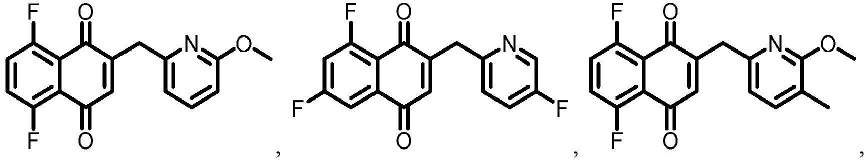 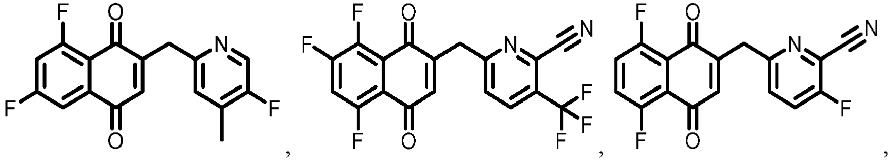 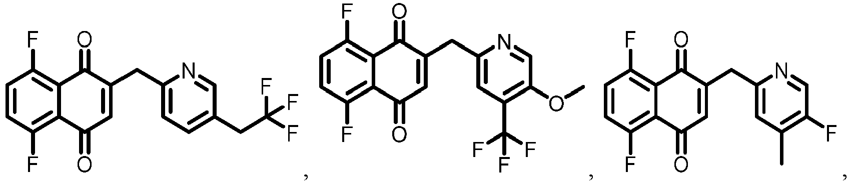 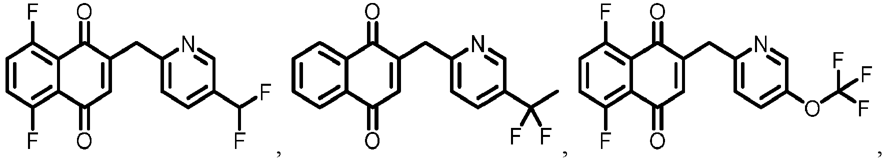 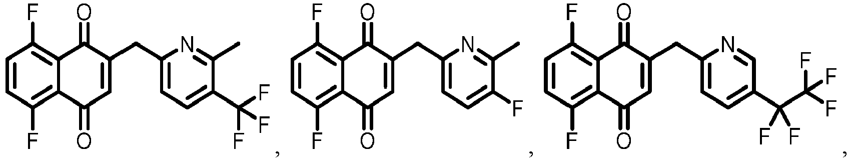 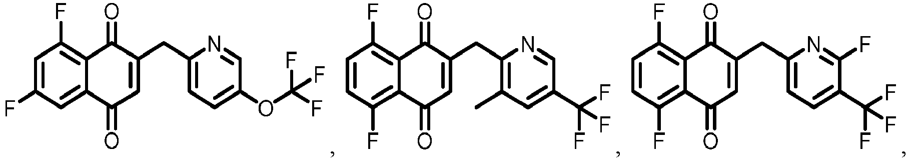 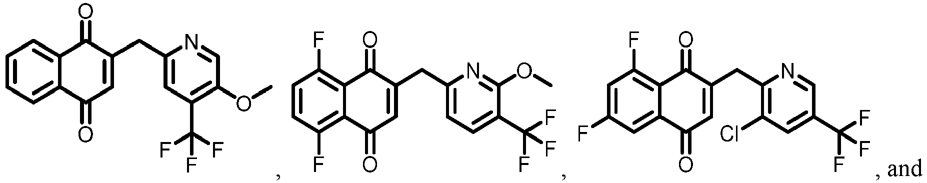 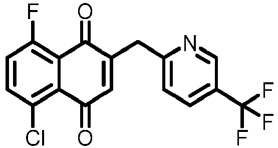 ; and the reduced forms thereof; and all salts, stereoisomers, mixtures of stereoisomers, isotopologues, solvates, and/or hydrates thereof. In some or any embodiments, including any of the foregoing embodiments, the compound is selected from the group consisting of: 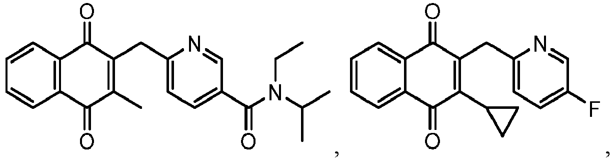 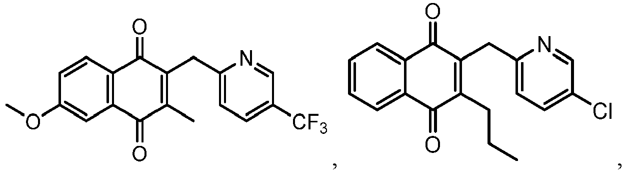 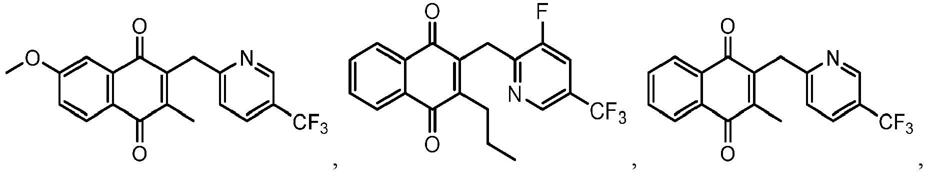 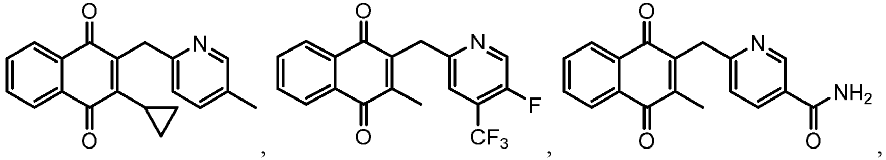 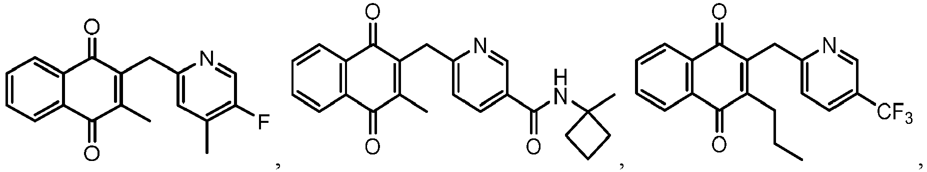 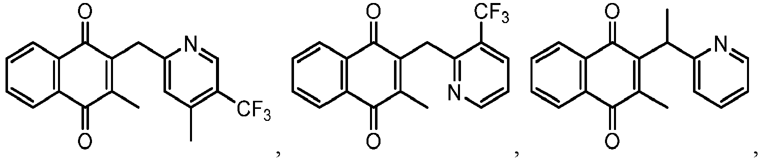 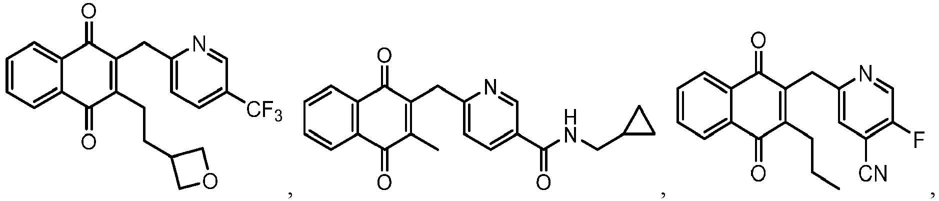 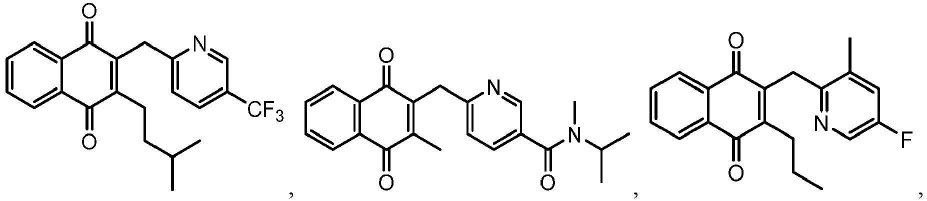 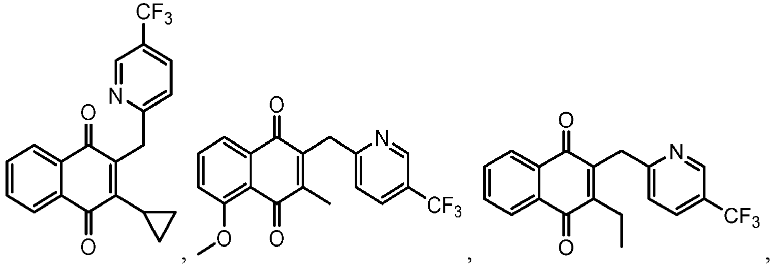 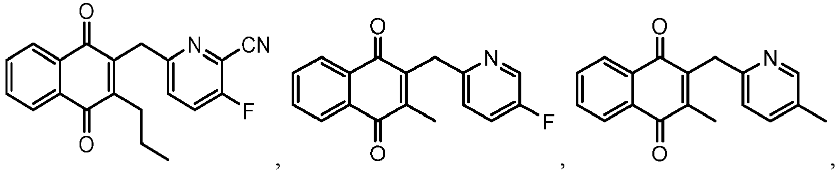 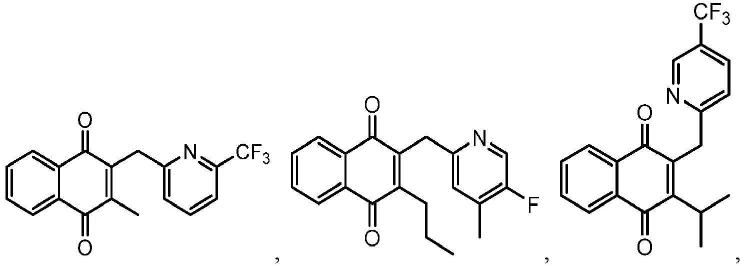 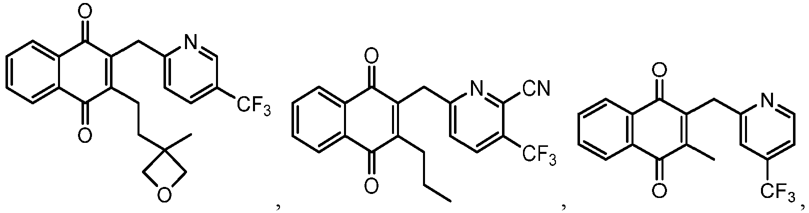 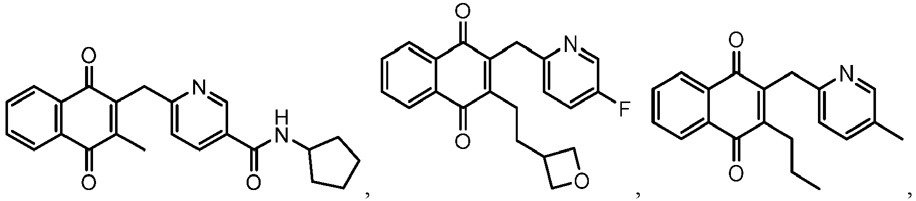 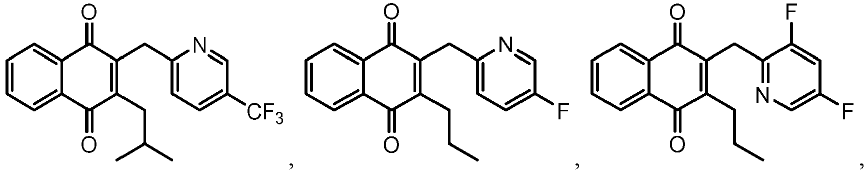 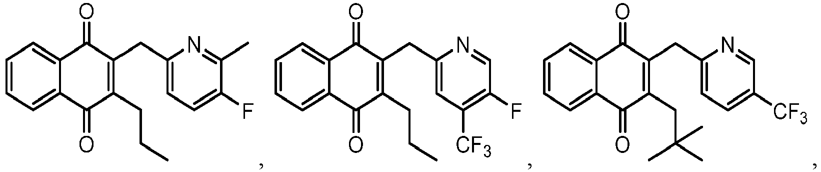 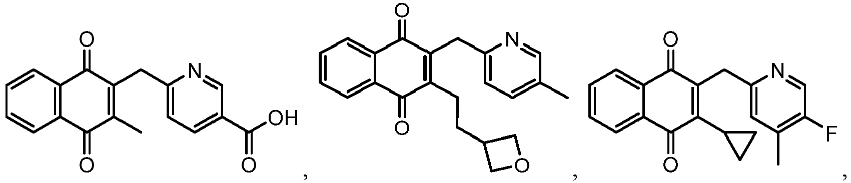 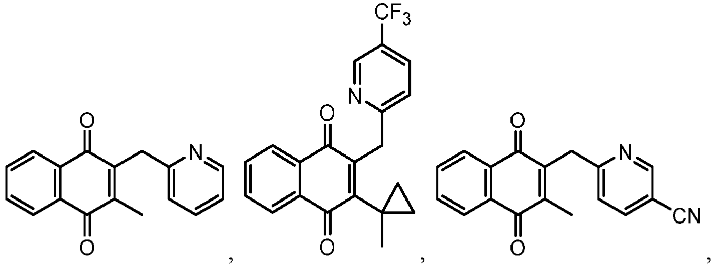 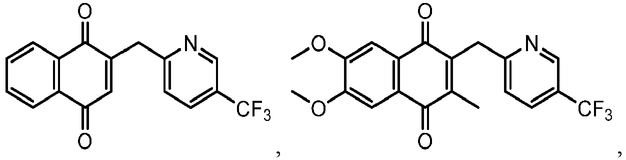 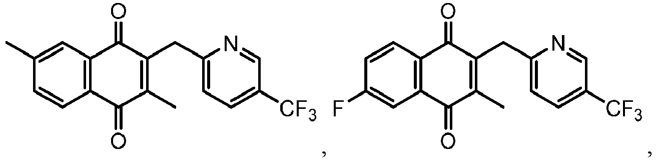 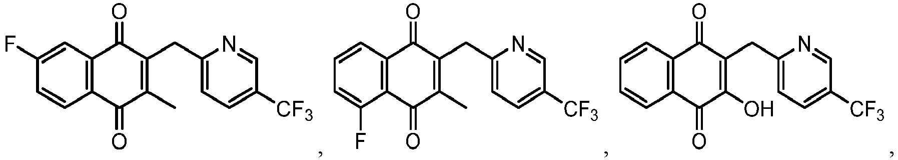 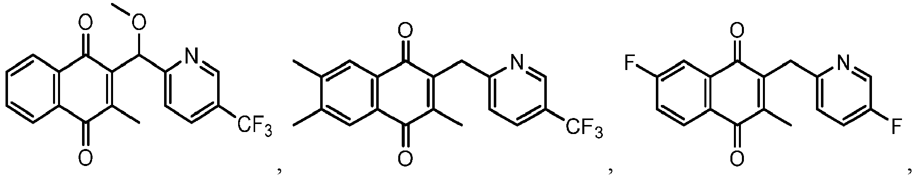 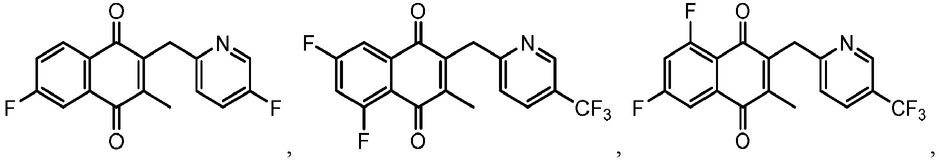 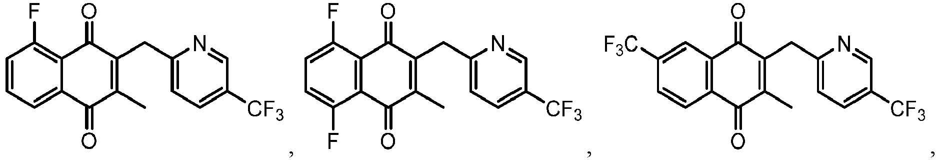 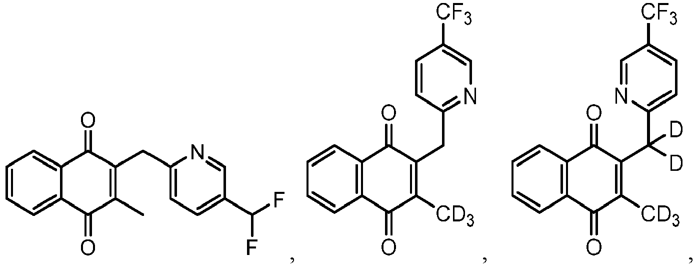 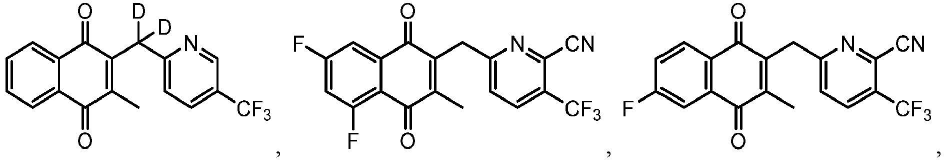 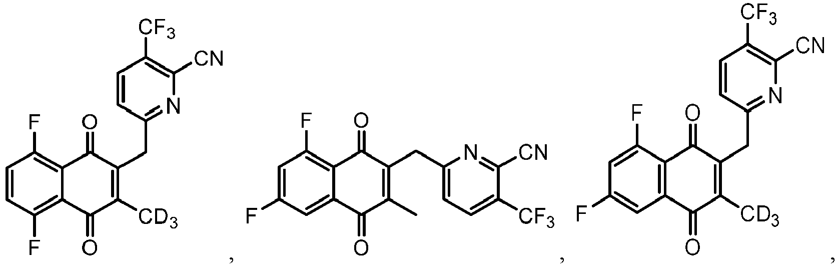 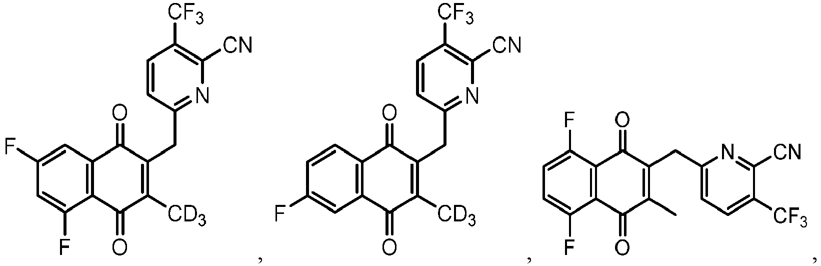 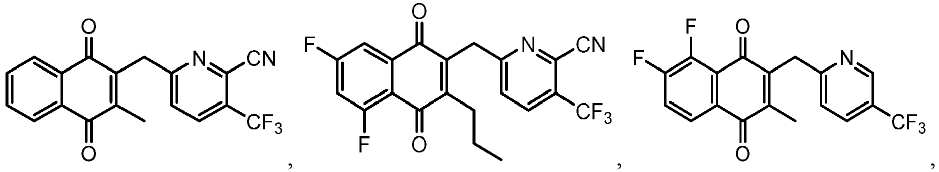 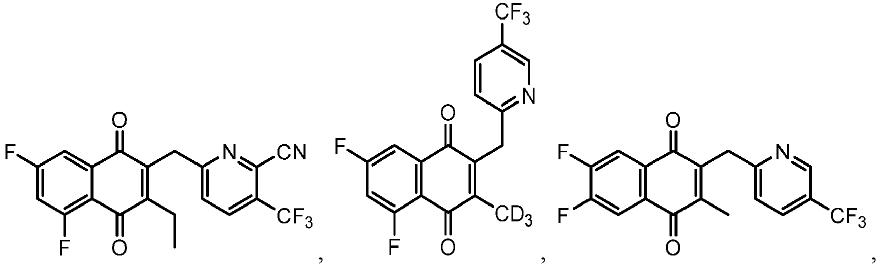 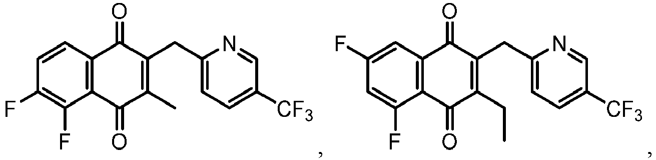 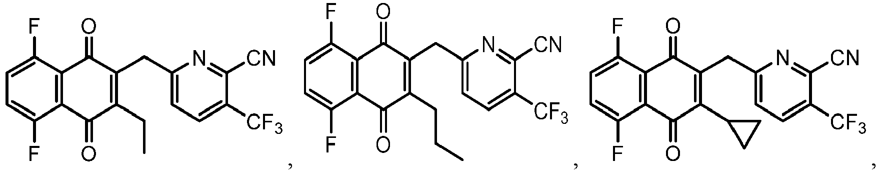 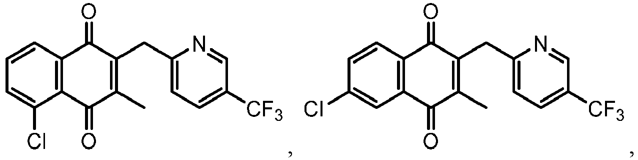 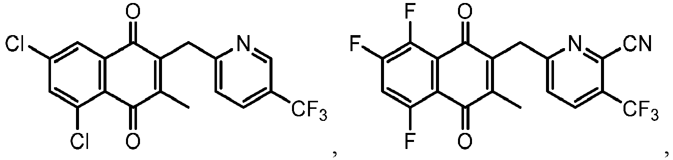 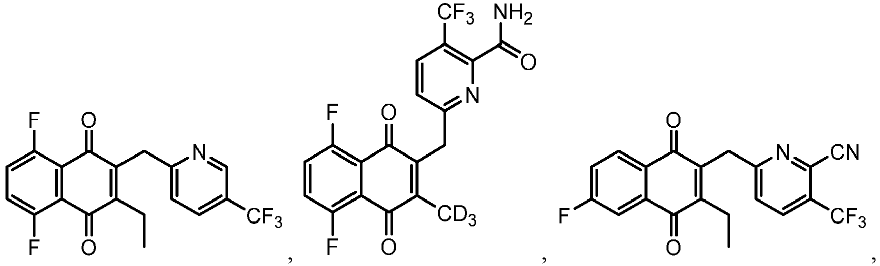 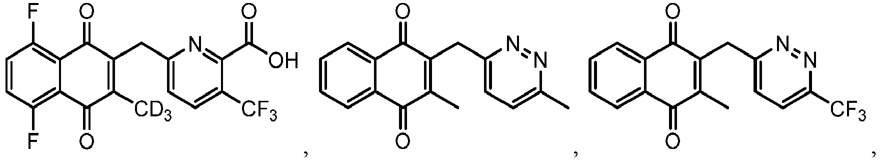 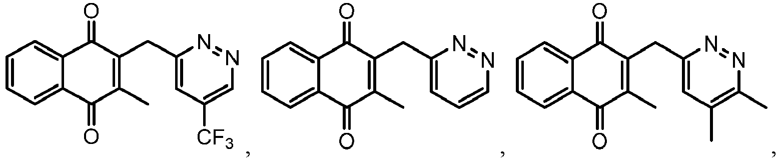 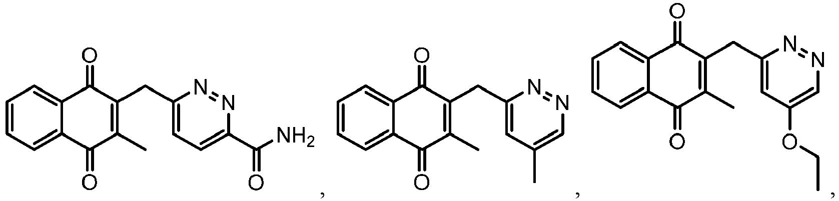 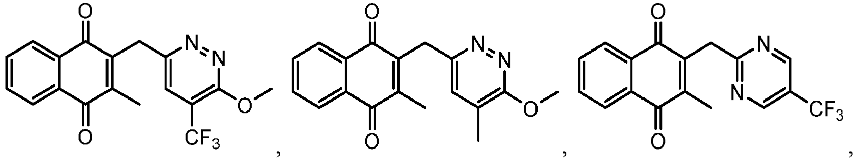 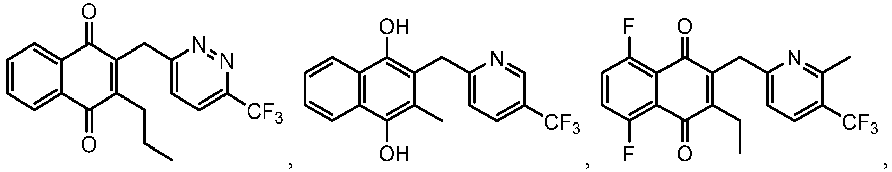 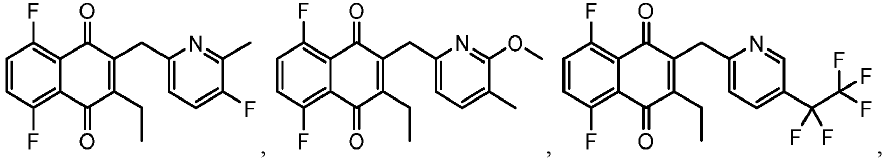 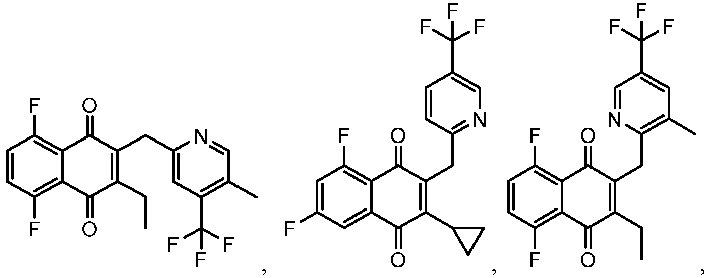 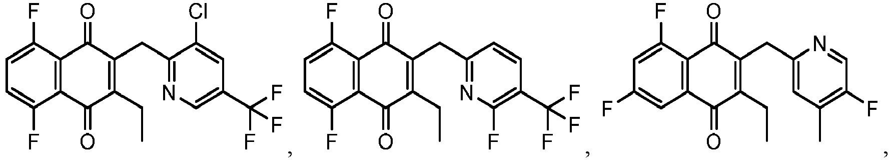 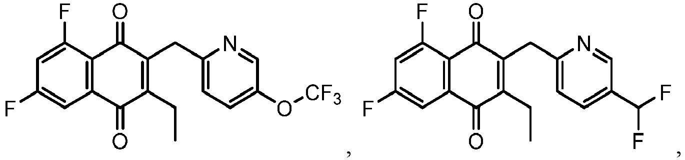 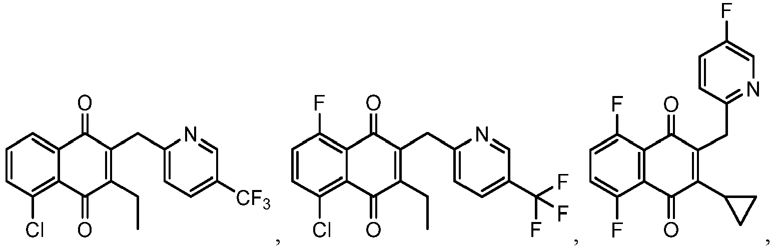 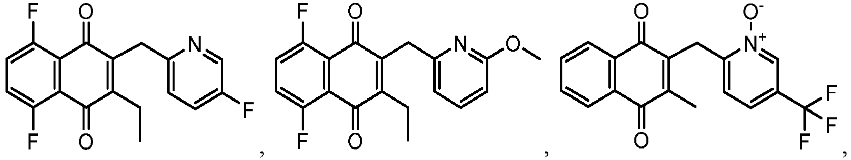 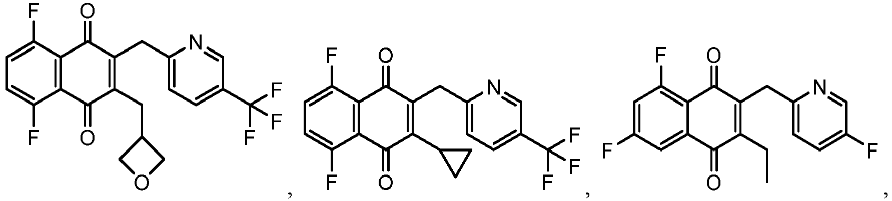 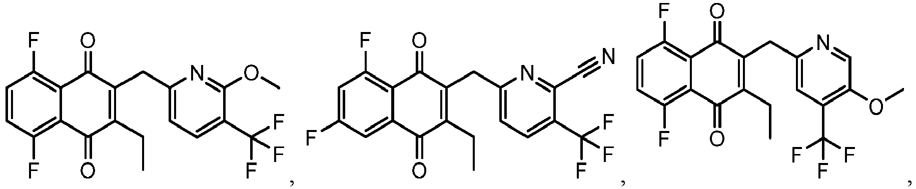 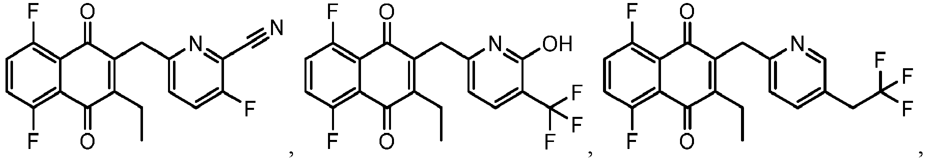 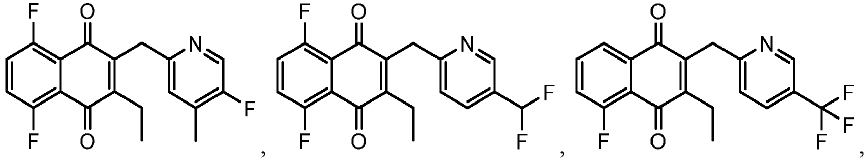 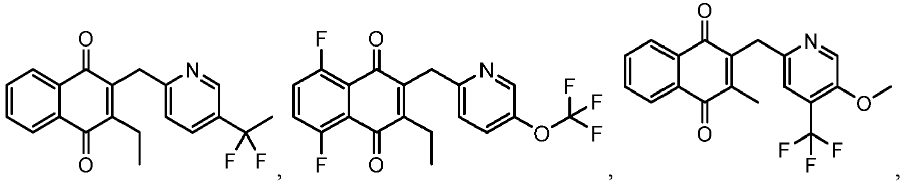 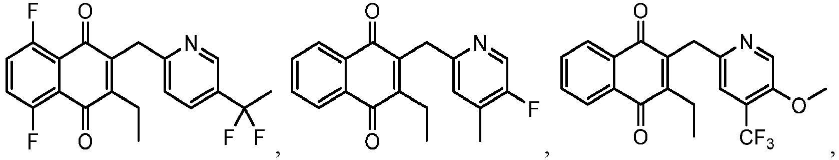 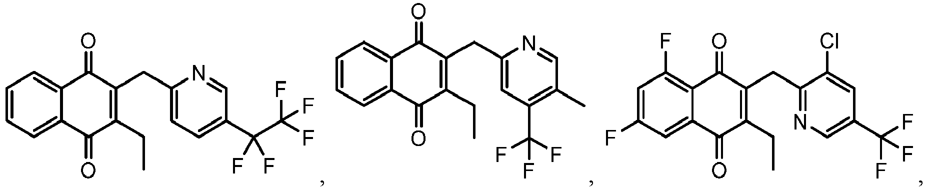 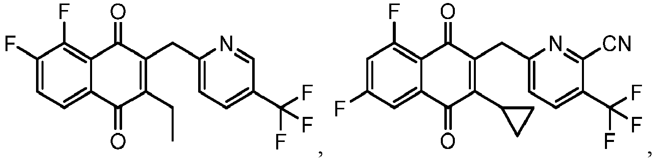 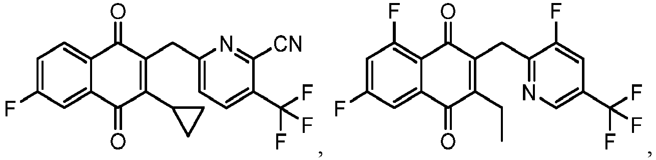 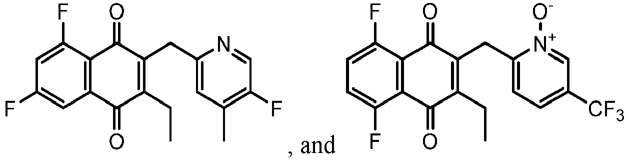 ; and the reduced forms thereof; and all salts, stereoisomers, mixtures of stereoisomers, isotopologues, solvates, and/or hydrates thereof. In some or any embodiments, including any of the foregoing embodiments, the compound is selected from the group consisting of: 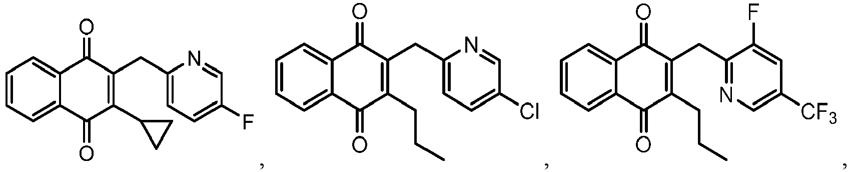 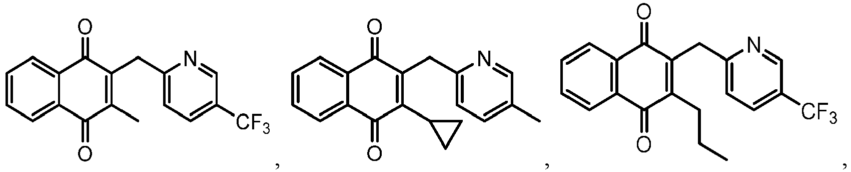 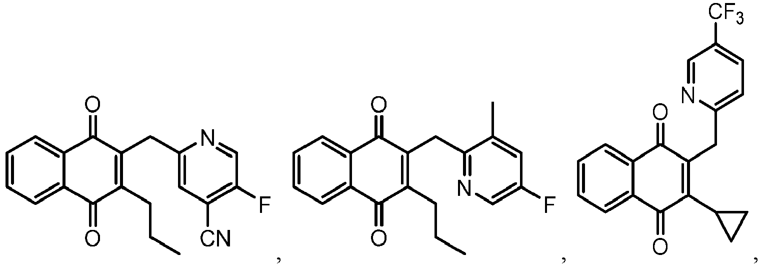 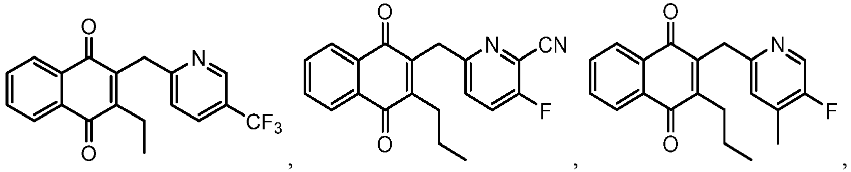 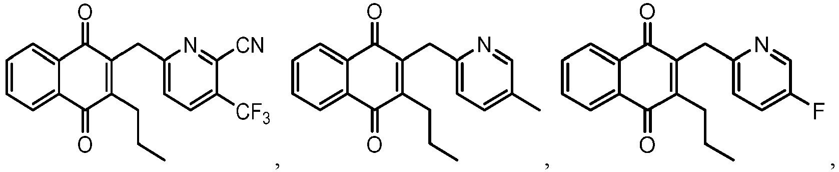 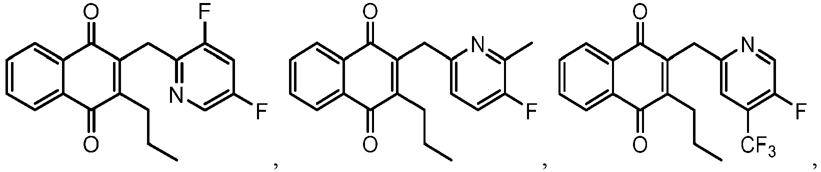 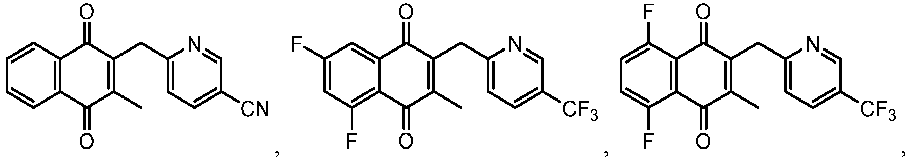 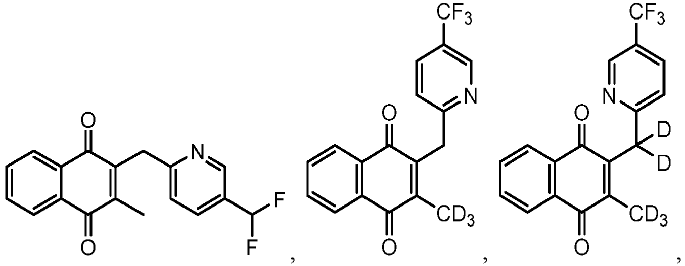 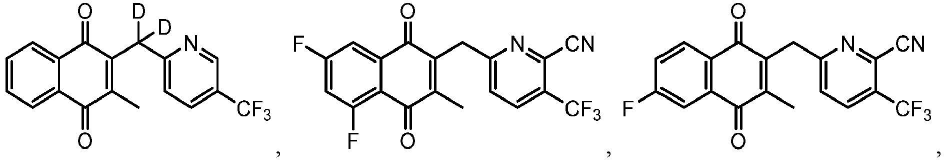 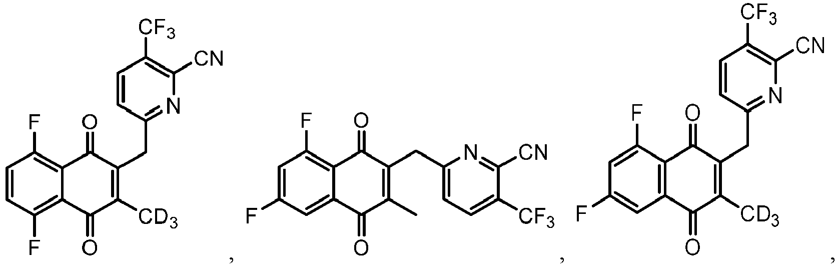 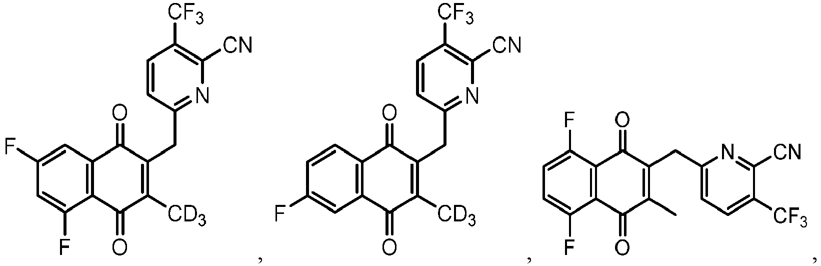 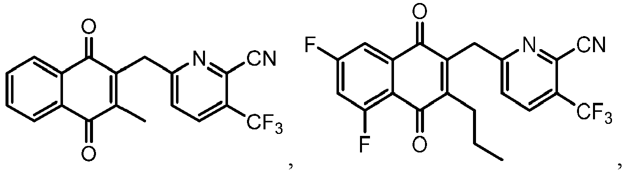 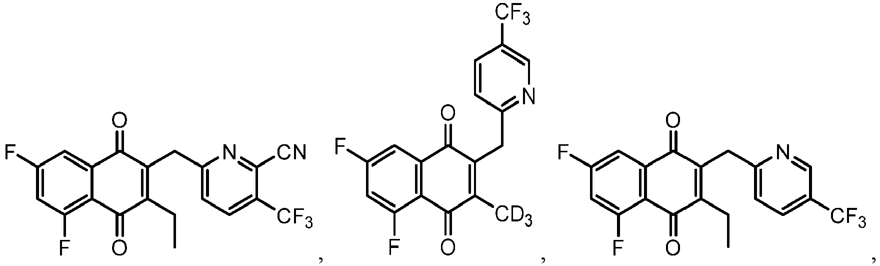 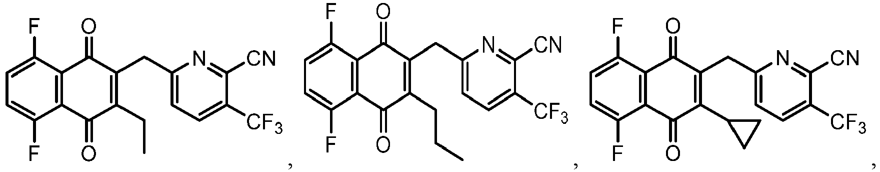 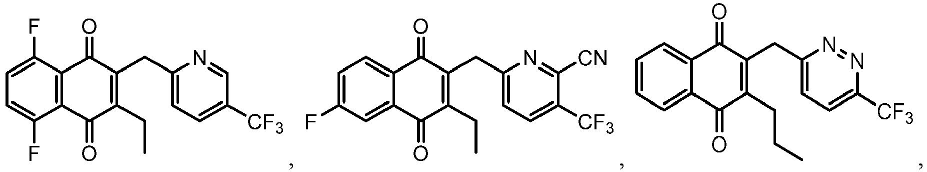 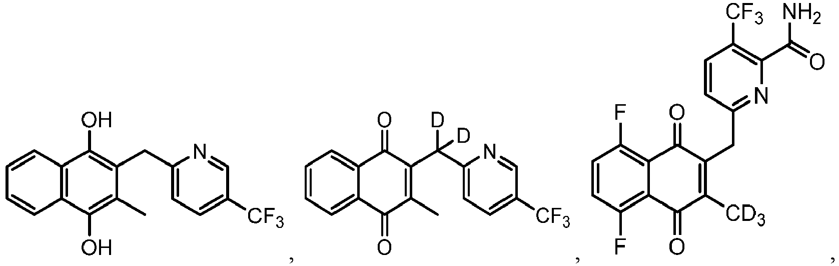 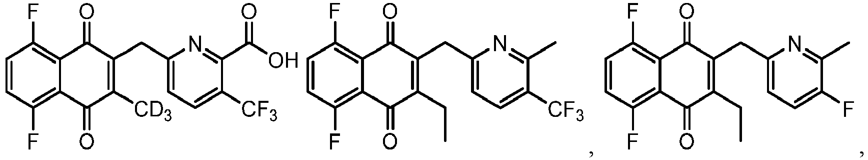 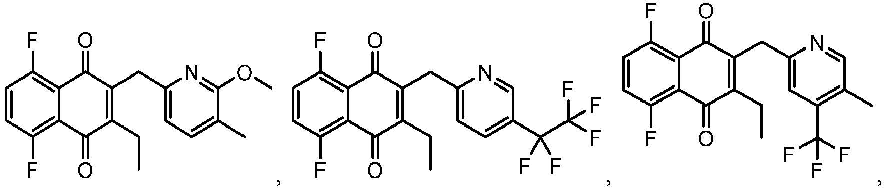 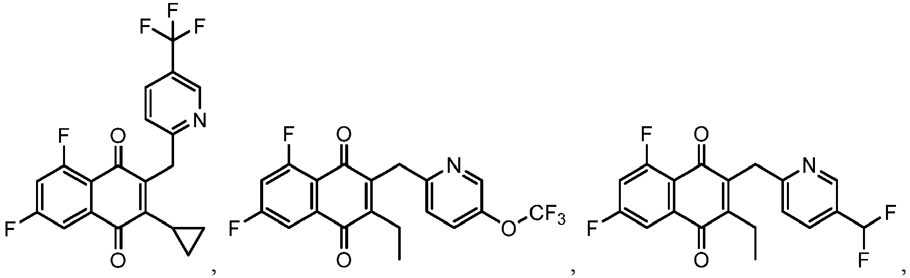 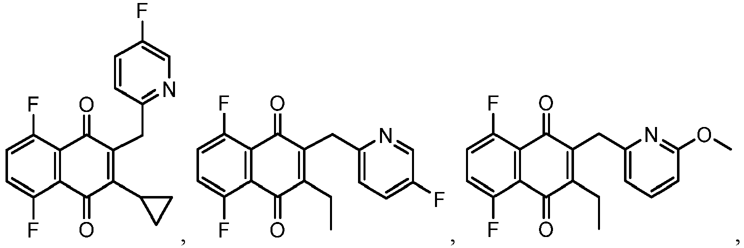 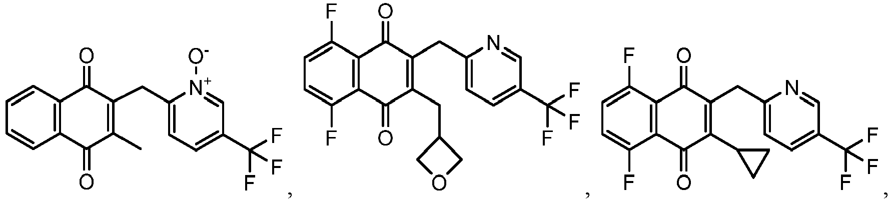 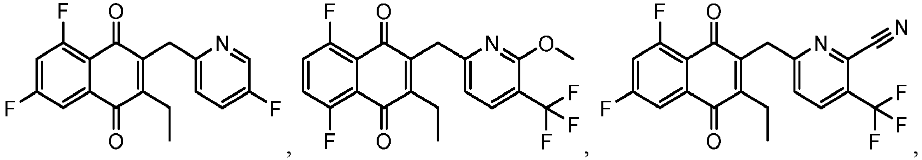 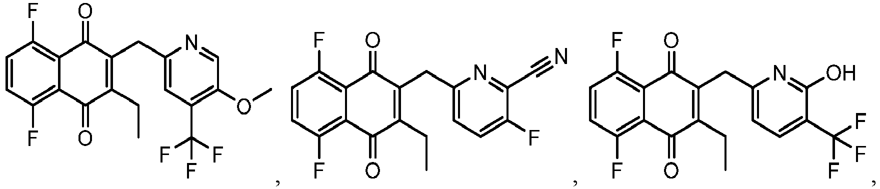 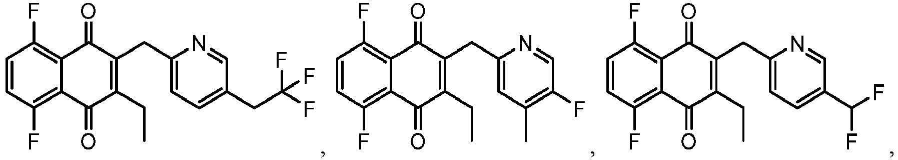 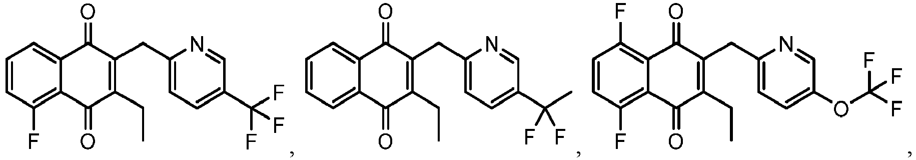 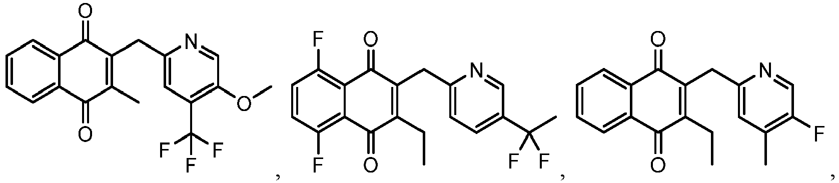 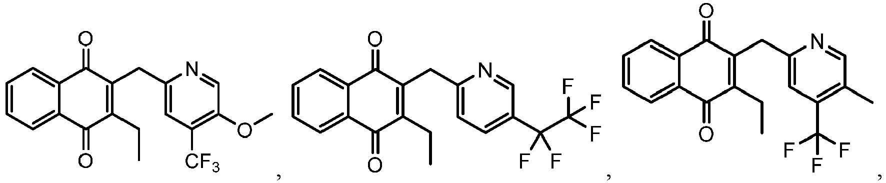 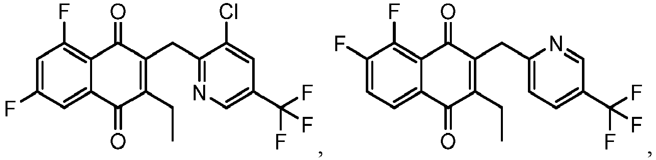 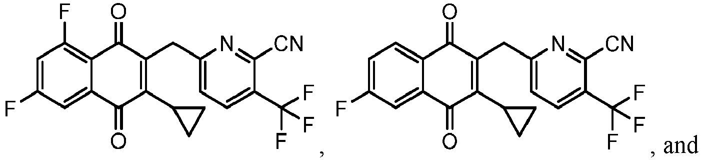 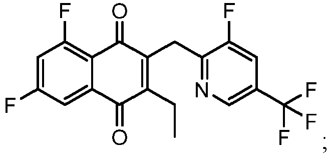 and the reduced forms thereof; and all salts, stereoisomers, mixtures of stereoisomers, isotopologues, solvates, and/or hydrates thereof. In some embodiments, including any of the foregoing embodiments, the compound is in the oxidized (quinone) form. In some embodiments, including any of the foregoing embodiments, the compound is in the reduced (hydroquinone) form. In some embodiments, including any of the foregoing embodiments, the compound is not a salt. In some embodiments, including any of the foregoing embodiments, the compound is a salt. In some embodiments, including any of the foregoing embodiments, the compound is a pharmaceutically acceptable salt. In some embodiments, including any of the foregoing embodiments, the compound is not an isotopologue. In some embodiments, including any of the foregoing embodiments, the compound is an isotopologue. In some embodiments, including any of the foregoing embodiments, the compound is not a solvate or hydrate thereof. In some embodiments, including any of the foregoing embodiments, the compound is a solvate or hydrate thereof.

In another aspect is a pharmaceutical composition comprising a compound as described herein (including but not limited to a compound described in the above paragraph) or any salt(s), stereoisomer, mixture of stereoisomers, isotopologue(s), solvate(s), and/or hydrate(s) thereof; and a pharmaceutically acceptable carrier. In another aspect is a pharmaceutical composition comprising an active agent and a pharmaceutically acceptable carrier, wherein the active agent consists of, or consists essentially of, a compound as described herein (including but not limited to a compound described in the above paragraph). Any one or more of the compounds described herein, including all of the foregoing compounds, can be formulated into a unit dose formulation.

In some or any embodiments, any one of the individual compounds described in paragraph [0034], or a pharmaceutical composition thereof as described in [0035], may be used to treat a disease as described in paragraph [0037].

The compound or any salt(s), stereoisomer, mixture of stereoisomers, isotopologue(s), solvate(s), and/or hydrate(s) thereof or composition as described herein (including but not limited to a compound or composition described in the above paragraphs) may be used in a method of inhibiting ferroptosis, or of treating or suppressing an oxidative stress disorder, the method comprising administering to a subject in need thereof a therapeutically effective amount of the compound or any salt(s), stereoisomer, mixture of stereoisomers, isotopologue(s), solvate(s), and/or hydrate(s) thereof or the composition. In another aspect, the use is in a method of inhibiting ferroptosis, or of treating or suppressing an oxidative stress disorder wherein the oxidative stress disorder is a mitochondrial disorder, an impaired energy processing disorder, a metabolic disorder, a neurodegenerative disease or disorder, or a disease of aging. In some embodiments, including any of the foregoing embodiments, the oxidative stress disorder is selected from the group consisting of: a neurodegenerative disease or disorder; an α-synucleinopathy; Parkinson's disease; familial Parkinson's Disease; idiopathic Parkinson's Disease; Parkinson's Disease wherein the patient has a mutation in one or more of the following genes: MAPT (Microtubule-associated protein tau), PRKN (parkin), PINK1 (PINK1), LRRK2 (leucine-rich repeat kinase 2), GBA (glucocerebrosidase), SNCA (alpha synuclein), PARK7 (DJ-1), and/or UCHL1 (ubiquitin carboxyl-terminal esterase L1); Parkinson's Disease with dementia (PDD); multisystem atrophy (MSA); Frontotemporal Dementia (FTD); Dementia with Lewy Bodies (DLB); Gaucher's disease (GD); Neurodegeneration with Brain Iron Accumulation (NBIA); neuroaxonal dystrophies (PLA2G6-associated neurodegeneration); a tauopathy; Alzheimer's disease; dementia pugilistica; Guam Amyotrophic lateral sclerosis-Parkinsonism-Dementia (Guam ALS/PD); Pick Disease; Argyrophilic grain dementia; Nieman-Pick type C; Subacute sclerosing panencephalitis (SSPE); Progressive supranuclear palsy (PSP); Corticobasoganglionic degeneration; Frontotemporal dementia with parkinsonism-17 (FTDP-17); Postencephalitic Parkinsonism (PEP); Autosomal recessive Parkinsonism; Huntington's Disease; Juvenile Huntington's Disease; amyotrophic lateral sclerosis (ALS); a motor neuron disease; a leukodystrophy; Adrenoleukodystrophy; Adrenomyeloneuropathy; traumatic brain injury; chronic traumatic encephalopathy (CTE); spinal muscular atrophy (SMA); a neurological disease; epilepsy; seizures; a mood disorder; schizophrenia; bipolar disorder; attention deficit/hyperactivity disorder (ADHD); Tourette syndrome; a pervasive developmental disorder; Down's syndrome; autistic disorder; Asperger's syndrome; childhood disintegrative disorder (CDD); Rett syndrome; CDKL5 deficiency disorder; PDD-not otherwise specified (PDD-NOS); NGLY1-congenital disorder of deglycosylation; a mitochondrial disorder; an inherited mitochondrial disease; Alpers Disease; Barth syndrome; a Beta-oxidation Defect; Carnitine-Acyl-Carnitine Deficiency; Carnitine Deficiency; a Creatine Deficiency Syndrome; Co-Enzyme Q10 Deficiency; Complex I Deficiency; Complex II Deficiency; Complex III Deficiency; Complex IV Deficiency; Complex V Deficiency; COX Deficiency; chronic progressive external ophthalmoplegia (CPEO); CPT I Deficiency; CPT II deficiency; Friedreich's Ataxia (FA); Glutaric Aciduria Type II; Kearns-Sayre Syndrome (KSS); Lactic Acidosis; Long-Chain Acyl-CoA Dehydrongenase Deficiency (LCAD); Long-chain 3-Hydroxyacyl-CoA Dehydrogenase Deficiency (LCHAD); Leigh Syndrome; Leigh-like Syndrome; Leber's Hereditary Optic Neuropathy (LHON); Lethal Infantile Cardiomyopathy (LIC); Luft Disease; Multiple Acyl-CoA Dehydrogenase Deficiency (MAD); Medium-Chain Acyl-CoA Dehydrongenase Deficiency (MCAD); Mitochondrial Myopathy, Encephalopathy, Lactacidosis, Stroke (MELAS); Myoclonic Epilepsy with Ragged Red Fibers (MERRF); Mitochondrial Recessive Ataxia Syndrome (MIRAS); Mitochondrial Cytopathy, Mitochondrial DNA Depletion; Mitochondrial Encephalopathy; Mitochondrial Myopathy; Myoneurogastointestinal Disorder and Encephalopathy (MNGIE); Neuropathy, Ataxia, and Retinitis Pigmentosa (NARP); Pearson Syndrome; Pyruvate Carboxylase Deficiency; Pyruvate Dehydrogenase Deficiency; disorder associated with a POLG Mutation; a Respiratory Chain Disorder; Short-Chain Acyl-CoA Dehydrogenase Deficiency (SCAD); Short-chain 3-Hydroxyacyl-CoA Dehydrogenase Deficiency (SCHAD); Very Long-Chain Acyl-CoA Dehydrongenase Deficiency (VLCAD); a myopathy; cardiomyopathy; encephalomyopathy; a mitochondrial myopathy; a primary mitochondrial myopathy; a muscular dystrophy; Duchenne muscular dystrophy (DMD); Becker muscular dystrophy; congenital muscular dystrophy; Fukuyama type congenital muscular dystrophy; limb-girdle muscular dystrophy; myotonic dystrophy; a cerebrovascular accident; stroke; a vision impairment; vision disorders; ocular disease; optic neuropathy; dominant inherited juvenile optic atrophy; optic neuropathy caused by a toxic agent; Leber's hereditary optic neuropathy (LHON); Dominant Optic Atrophy (DOA); DOA plus; glaucoma; retinitis pigmentosa; macular degeneration; age-related macular degeneration (AMD); dry AMD; wet AMD; Stargardt's macular dystrophy; diabetic retinopathy; diabetic maculopathy; retinopathy of prematurity; ischemic reperfusion related retinal injury; ischemia; ischemia reperfusion injury; ischemia reperfusion injury due to transplant; ischemia reperfusion injury due to surgery; oxygen poisoning; an age-associated disease; diabetes; metabolic syndrome; Wolfram's disease; cancer; brain cancer; glioblastoma; chronic fatigue; a genetic disease; a haemoglobionopathy; thalassemia; sickle cell anemia; G6PD deficiency; Multiple Sclerosis; a neurodegenerative disorder resulting in hearing or balance impairment; hearing loss; noise induced hearing loss; Maternally inherited diabetes and deafness (MIDD); renal tubular acidosis; acute tubular necrosis; contrast-induced kidney damage; contrast-induced retinopathy damage; Abetalipoproteinemia; cobalamin c defect; methylmalonic aciduria; and radiation damage or injury (in some embodiments, the compound is administered before, during or after the subject is exposed to radiation which causes the radiation damage or injury). In some embodiments, the use is in a method for treating or suppressing a neurodegenerative disorder. In some embodiments, the neurodegenerative disorder is selected from the group consisting of: Alzheimer's Disease, Parkinson's Disease, Huntington's Disease, Juvenile Huntington's Disease, amyotrophic lateral sclerosis, frontotemporal dementia (FTD), and Friedreich's Ataxia. In some embodiments, including any of the foregoing embodiments, the oxidative stress disorder is selected from the group consisting of Parkinson's Disease, Duchenne muscular dystrophy, Leigh Syndrome, Complex I Deficiency, and Huntington's Disease. In some embodiments, including any of the foregoing embodiments, the oxidative stress disorder is Parkinson's Disease. In some embodiments, including any of the foregoing embodiments, the oxidative stress disorder is Duchenne muscular dystrophy. In some embodiments, including any of the foregoing embodiments, the oxidative stress disorder is Leigh Syndrome. In some embodiments, including any of the foregoing embodiments, the oxidative stress disorder is Complex I Deficiency. In some embodiments, including any of the foregoing embodiments, the oxidative stress disorder is Huntington's Disease. In some embodiments, including any of the foregoing embodiments, the use is in a method for treating the oxidative stress disorder. In some embodiments, including any of the foregoing embodiments, the use is in a method for suppressing the oxidative stress disorder.

Any individual compound as described herein or a salt, stereoisomer, mixture of stereoisomers, isotopologue, solvate, and/or hydrate thereof, or a combination of compounds or salts, stereoisomers, mixture of stereoisomers, isotopologues, solvates, and/or hydrates thereof can be used in the method. In some embodiments, including any of the foregoing embodiments, the compound or a salt, stereoisomer, mixture of stereoisomers, isotopologue, solvate, and/or hydrate thereof, or a combination of compounds or salts, stereoisomers, mixture of stereoisomers, isotopologues, solvates, and/or hydrates thereof is administered as a pharmaceutical composition comprising the compound or a salt, stereoisomer, mixture of stereoisomers, isotopologue, solvate, and/or hydrate thereof, or a combination of compounds or salts, stereoisomers, mixture of stereoisomers, isotopologues, solvates, and/or hydrates thereof and a pharmaceutically acceptable carrier. In some embodiments, including any of the foregoing embodiments, the pharmaceutical composition comprises an active agent consisting essentially of the compound, or a salt, stereoisomer, mixture of stereoisomers, isotopologue, solvate, and/or hydrate thereof, or a combination of compounds or salts, stereoisomers, mixture of stereoisomers, isotopologues, solvates, and/or hydrates thereof; and a pharmaceutically acceptable carrier. In some embodiments, including any of the foregoing embodiments, the use is in a method for treating the oxidative stress disorder. In some embodiments, including any of the foregoing embodiments, the use is in a method for suppressing the oxidative stress disorder. In some embodiments, including any of the foregoing embodiments, the use is in a method for treating the neurodegenerative disorder. In some embodiments, including any of the foregoing embodiments, the use is in a method for suppressing the neurodegenerative disorder.

In another aspect is the use of a compound as described herein, including but not limited to any of the foregoing embodiments, for treating or suppressing the disorder.

For all the compounds, compositions, formulations and uses described herein, any compound in the quinone (oxidized) form can also be used in its reduced (hydroquinone) form when desired. That is, the compounds recited herein as cyclohexadienedione compounds (oxidized quinone) can also be used in their benzenediol (reduced hydroquinone) form as desired.

It is to be understood that the description of compounds, compositions, formulations, and uses in methods of treatment described herein include "comprising", "consisting of', and "consisting essentially of' embodiments. In some embodiments, for all compositions described herein, and all methods using a composition described herein, the compositions can either comprise the listed components or steps, or can "consist essentially of" the listed components or steps. When a composition is described as "consisting essentially of" the listed components, the composition contains the components listed, and may contain other components which do not substantially affect the condition being treated, but do not contain any other components which substantially affect the condition being treated other than those components expressly listed; or, if the composition does contain extra components other than those listed which substantially affect the condition being treated, the composition does not contain a sufficient concentration or amount of the extra components to substantially affect the condition being treated. When a method is described as "consisting essentially of" the listed steps, the method contains the steps listed, and may contain other steps that do not substantially affect the condition being treated, but the method does not contain any other steps which substantially affect the condition being treated other than those steps expressly listed. As a non-limiting specific example, when a composition is described as 'consisting essentially of' a a component, the composition may additionally contain any amount of pharmaceutically acceptable carriers, vehicles, or diluents and other such components which do not substantially affect the condition being treated.

### DETAILED DESCRIPTION

Provided herein are compounds useful in treating or suppressing diseases, developmental delays and symptoms related to oxidative stress such as mitochondrial disorders, impaired energy processing disorders, metabolic diseases, neurodegenerative diseases, and diseases of aging, and use of such compounds in methods for treating or suppressing an oxidative stress disorder. Further provided herein are compounds useful in inhibiting ferroptosis. Further provided herein are compounds useful in treating or suppressing neurodegenerative disorders. In some embodiments, the compounds advantageously do not inhibit, or have a reduced level of inhibition of, CYP3A4. Compounds that potently inhibit CYP3A4 may cause drug-drug interactions, and may limit therapeutic utility. However, compounds that inhibit CYP3A4 at substantially higher concentrations than is necessary to treat or suppress an oxidative stress disorder, rescue from ferroptosis, and/or to treat or suppress a neurodegenerative disorder, may still be therapeutically useful.

The abbreviations used herein have their conventional meaning within the chemical and biological arts, unless otherwise specified.

Reference to "about" a value or parameter herein includes (and describes) variations that are directed to that value or parameter per se. For example, description referring to "about X" includes description of "X". As used herein, and unless otherwise specified, the terms "about" and "approximately," when used in connection with temperatures, doses, amounts, or weight percent of ingredients of a composition or a dosage form, mean a dose, amount, or weight percent that is recognized by those of ordinary skill in the art to provide a pharmacological effect equivalent to that obtained from the specified dose, amount, or weight percent. Specifically, the terms "about" and "approximately," when used in this context, contemplate a dose, amount, or weight percent within 15%, within 10%, within 5%, within 4%, within 3%, within 2%, within 1%, or within 0.5% of the specified dose, amount, or weight percent.

The terms "a" and "an," as used in herein mean one or more, unless context clearly dictates otherwise.

The terms "subject," "individual," and "patient" mean an individual organism, preferably a vertebrate, more preferably a mammal, most preferably a human.

"Treating" a disorder with the compounds and uses discussed herein is defined as administering one or more of the compounds discussed herein, with or without additional therapeutic agents, in order to reduce or eliminate either the disorder or one or more symptoms of the disorder, or to retard the progression of the disorder or of one or more symptoms of the disorder, or to reduce the severity of the disorder or of one or more symptoms of the disorder. "Suppression" of a disorder with the compounds and uses discussed herein is defined as administering one or more of the compounds discussed herein, with or without additional therapeutic agents, in order to suppress the clinical manifestation of the disorder, or to suppress the manifestation of adverse symptoms of the disorder. The distinction between treatment and suppression is that treatment occurs after adverse symptoms of the disorder are manifest in a subject, while suppression occurs before adverse symptoms of the disorder are manifest in a subject. Suppression may be partial, substantially total, or total. In some embodiments, genetic screening can be used to identify patients at risk of the disorder. The compounds disclosed herein can then be administered to asymptomatic patients at risk of developing the clinical symptoms of the disorder, in order to suppress the appearance of any adverse symptoms.

"Therapeutic use" of the compounds discussed herein is defined as using one or more of the compounds discussed herein to treat or suppress a disorder, as defined herein. A "therapeutically effective amount" of a compound is an amount of the compound, which, when administered to a subject, is sufficient to reduce or eliminate either a disorder or one or more symptoms of a disorder, or to retard the progression of a disorder or of one or more symptoms of a disorder, or to reduce the severity of a disorder or of one or more symptoms of a disorder, or to suppress the clinical manifestation of a disorder, or to suppress the manifestation of adverse symptoms of a disorder. A therapeutically effective amount can be given in one or more administrations.

While the compounds described herein can occur and can be used as the neutral (non-salt) compound, the description is intended to embrace all salts of the compounds described herein, as well as uses of such salts of the compounds. In some embodiments, the salts of the compounds comprise pharmaceutically acceptable salts. Pharmaceutically acceptable salts are those salts which can be administered as drugs or pharmaceuticals to humans and/or animals and which, upon administration, retain at least some of the biological activity of the free compound (non-ionic compound or non-salt compound). The desired salt of a basic compound may be prepared by methods known to those of skill in the art by treating the compound with an acid. In some embodiments, inorganic acids include, but are not limited to, hydrochloric acid, hydrobromic acid, sulfuric acid, nitric acid, and phosphoric acid. In some embodiments, organic acids include, but are not limited to, formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, sulfonic acids, and salicylic acid. Salts of basic compounds with amino acids, such as aspartate salts and glutamate salts, can also be prepared. The desired salt of an acidic compound can be prepared by methods known to those of skill in the art by treating the compound with a base. In some embodiments, inorganic salts of acid compounds include, but are not limited to, alkali metal and alkaline earth salts, such as sodium salts, potassium salts, magnesium salts, and calcium salts; ammonium salts; and aluminum salts. In some embodiments, organic salts of acid compounds include, but are not limited to, procaine, dibenzylamine, N-ethylpiperidine,
N,N-dibenzylethylenediamine, and triethylamine salts. Salts of acidic compounds with amino acids, such as lysine salts, can also be prepared.

Included herein, when chemically relevant, are all stereoisomers of the compounds, including diastereomers and enantiomers. Also included are mixtures of possible stereoisomers in any ratio, including, but not limited to, racemic mixtures. Unless stereochemistry is explicitly indicated in a structure, the structure is intended to embrace all possible stereoisomers of the compound depicted. If stereochemistry is explicitly indicated for one portion or portions of a molecule, but not for another portion or portions of a molecule, the structure is intended to embrace all possible stereoisomers for the portion or portions where stereochemistry is not explicitly indicated.

"Isotopologue" refers herein to a compound which differs in its isotopic composition from its "natural" isotopic composition. "Isotopic composition" refers to the amount of each isotope present for a given atom, and "natural isotopic composition" refers to the naturally occurring isotopic composition or abundance for a given atom. Atoms containing their natural isotopic composition may also be referred to herein as "non-enriched" atoms. Unless otherwise designated, the atoms of the compounds recited herein are meant to represent any stable isotope of that atom. For example, unless otherwise stated, when a position is designated specifically as "H" or "hydrogen," the position is understood to have hydrogen at its natural isotopic composition. The description of compounds herein also includes all isotopologues, in some embodiments, partially deuterated or perdeuterated analogs of all compounds herein. "Isotopically enriched" may also refer to a compound containing at least one atom having an isotopic composition other than the natural isotopic composition of that atom. "Isotopic enrichment" refers to the percentage of incorporation of an amount of a specific isotope at a given atom in a molecule in the place of that atom's natural isotopic abundance. For example, deuterium enrichment of 1% at a given position means that 1% of the molecules in a given sample contain deuterium at the specified position. Because the naturally occurring distribution of deuterium is about 0.0156%, deuterium enrichment at any position in a compound synthesized using non-enriched starting materials is about 0.0156%. The isotopic enrichment of the compounds provided herein can be determined using conventional analytical methods known to one of ordinary skill in the art, including mass spectrometry and nuclear magnetic resonance spectroscopy. In some embodiments, the compound comprises a -CD₃ group.

The description of compounds herein also includes all salts, stereoisomers, mixture of stereoisomers, solvates, and/or hydrates thereof, unless otherwise specified or clear from the context.

"Hydroquinone form" or "reduced form" indicates the form of the compound when a two electron reduction of the quinone ring is effected, providing a net conversion of the two oxo groups to two hydroxy groups. For example, the hydroquinone (reduced) form of the compounds described herein indicates: 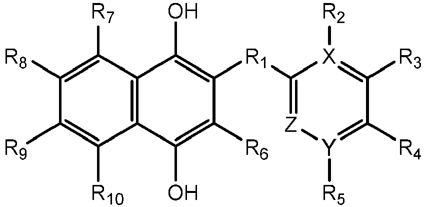 , wherein R₁, R₂, R₃, R₄, R₅, R₆, R₇, R₈, R₉, R₁₀, X, Y, and Z are as defined herein. The quinone (oxidized) form of the compounds described herein indicates the following structure: 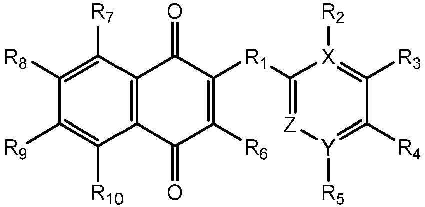 wherein R₁, R₂, R₃, R₄, R₅, R₆, R₇, R₈, R₉, R₁₀, X, Y, and Z are as defined herein.

The term "alkyl" is intended to embrace a saturated linear, branched, or cyclic hydrocarbon, or any combination thereof. The point of attachment of the alkyl group to the remainder of the molecule can be at any chemically possible location on the alkyl group. In some embodiments, an alkyl has from 1 to 6 carbon atoms ("C₁-C₆ alkyl"), from 1 to 4 carbon atoms ("C₁-C₄ alkyl"), from 1 to 3 carbon atoms ("C₁-C₃ alkyl"), or from 1 to 2 carbon atoms ("C₁-C₂ alkyl"). In some embodiments, non-limiting examples of "C₁-C₆ alkyl" include methyl, ethyl, n-propyl, isopropyl, cyclopropyl, n-butyl, isobutyl, sec-butyl, t-butyl, cyclobutyl, cyclopropyl-methyl, methyl-cyclopropyl, pentyl, cyclopentyl, hexyl, and cyclohexyl.

The term "alkenyl" is intended to embrace a linear, branched, or cyclic hydrocarbon, or any combination thereof, comprising one or more carbon-carbon double bonds. The point of attachment of the alkenyl group to the remainder of the molecule can be at any chemically possible location on the alkenyl group. In some embodiments, alkenyl includes vinyl, allyl, propenyl, butenyl, cyclohexenyl, and cyclohexenylmethyl.

The term "alkynyl" is intended to embrace a linear, branched, or cyclic hydrocarbon, or any combination thereof, comprising one or more carbon-carbon triple bonds. The point of attachment of the alkynyl group to the remainder of the molecule can be at any chemically possible location on the alkynyl group. In some embodiments, alkynyl includes ethynyl, propynyl, and butynyl.

The term "alkylene" is intended to embrace a divalent alkyl as defined herein.

The term "alkenylene" is intended to embrace a divalent alkenyl as defined herein.

The term "alkynylene" is intended to embrace a divalent alkynyl as defined herein.

The term "alkoxy" is intended to embrace -O-alkyl, wherein alkyl is defined above.

"Cycloalkyl" in intended to embrace a monocyclic, saturated hydrocarbon radical having three to six carbon atoms. In some embodiments, cycloalkyl is cyclopropyl, cyclobutyl, cyclopentyl, or cyclohexyl.

The term "halo" indicates -F, -Cl, -Br, or -I.

As used herein, "haloalkyl" refers to alkyl, as defined above, wherein the alkyl includes at least one substituent selected from the group consisting of a halogen, *e.g.,* F, Cl, Br, and/or I. In some embodiments, the alkyl in haloalkyl is substituted with 1, 2, 3, 4, 5 or 6 halo which are independently selected; in some embodiments, 1, 2, 3, 4, or 5 halo which are independently selected; in some embodiments, 1, 2, 3, or 5 halo which are independently selected. In some embodiments, haloalkyl includes, but is not limited to, -CF₃, -CHF₂, -CH₂F, -CH₂-CF₃, -CH₂-CHF₂, or -CH₂-CH₂F. When haloalkyl is a C₁ haloalkyl, then the C₁ haloalkyl is selected from -CX₃, -CHX₂, and -CH₂X, wherein X is independently in each instance selected from the group consisting of F, Cl, Br, and I.

As used herein, "haloalkoxy" refers to -O-haloalkyl.

As used herein, "heterocyclic ring" or "heterocyclic group" generally refers to a cyclic hydrocarbon in which one or more (e.g. one to four, one to three, one to two, or one) carbon atoms are replaced by a heteroatom independently selected from the group consisting of N, O, and S(O)₀₋₂. Heterocyclic groups may be saturated, partially unsaturated, or aromatic. In some embodiments, a heterocyclic group may be a "3-7 membered heterocyclic group," containing 3-7 ring atoms. In some embodiments, a heterocyclic group may be a "4-6 membered heterocyclic group," containing 4-6 ring atoms. In some embodiments, a heterocyclic group may be a "5-6 membered heterocyclic group," containing 5-6 ring atoms. In some embodiments, saturated heterocyclic rings include, but are not limited to, oxetanyl, morpholinyl, piperidinyl, piperazinyl, and pyrrolidinyl.

As used herein, "heteroaryl" refers to a monovalent moiety that is a radical of an aromatic compound wherein the ring atoms contain carbon atoms and at least one oxygen, sulfur, or nitrogen atom. Embodiments of heteroaryl moieties include those having 5 to 6 ring atoms. In some embodiments, heteroaryl is pyridinyl, thiazolyl, oxazolyl, pyrazolyl, pyrimidinyl, pyridazinyl, and pyrazinyl.

As used herein, "optionally substituted," when used to describe a radical moiety, *e.g.,* a heteroaryl group is optionally independently substituted with one or more substituents, means that such moiety is optionally bonded to e.g. one, two, three, or four or more such substituents. It is to be understood that only chemically possible substitutions are included. In certain embodiments, when a radical moiety is optionally substituted with an optional substituent(s), the optional substituent(s) is not further substituted, unless otherwise specified.

By "respiratory chain disorder" is meant a disorder which results in the decreased utilization of oxygen by a mitochondrion, cell, tissue, or individual, due to a defect or disorder in a protein or other component contained in the mitochondrial respiratory chain. By "protein or other component contained in the mitochondrial respiratory chain" is meant the components (including, but not limited to, proteins, tetrapyrroles, and cytochromes) comprising mitochondrial complex I, II, III, IV, and/or V. "Respiratory chain protein" refers to the protein components of those complexes, and "respiratory chain protein disorder" is meant a disorder which results in the decreased utilization of oxygen by a mitochondrion, cell, tissue, or individual, due to a defect or disorder in a protein contained in the mitochondrial respiratory chain.

The terms "Parkinson's," (also called "Parkinsonism" and "Parkinsonian syndrome") ("PD") is intended to include not only Parkinson's disease but also drug-induced Parkinsonism and post-encephalitic Parkinsonism. Parkinson's disease is also known as paralysis agitans or shaking palsy. It is characterized by tremor, muscular rigidity, and loss of postural reflexes. The disease usually progresses slowly with intervals of 10 to 20 years elapsing before the symptoms cause incapacity. Due to their mimicry of effects of Parkinson's disease, treatment of animals with methamphetamine, rotenone, or MPTP has been used to generate models for Parkinson's disease. These animal models have been used to evaluate the efficacy of various therapies for Parkinson's disease. In some embodiments, the patient has a mutation in one or more of the following genes: *MAPT* (Microtubule-associated protein tau), *PRKN* (parkin), *PINK1* (PINK1), LRRK2 (leucine-rich repeat kinase *2), GBA* (glucocerebrosidase), *SNCA* (alpha synuclein), *PARK7* (DJ-1), and/or *UCHL1* (ubiquitin carboxyl-terminal esterase L1). In some embodiments, Parkinson's is familial Parkinson's Disease. In some embodiments, Parkinson's is idiopathic Parkinson's Disease. In some embodiments, Parkinson's is Parkinson's Disease with dementia (PDD).

### Diseases amenable to treatment or suppression with compounds and uses of compounds in methods disclosed herein

A variety of disorders/diseases are believed to be caused or aggravated by oxidative stress affecting normal electron flow in the cells, such as mitochondrial disorders, impaired energy processing disorders, metabolic disorders, neurodegenerative diseases, and diseases of aging, and can be treated or suppressed using the compounds disclosed herein. In some embodiments, including the foregoing embodiment, oxidative stress disorders include, in some embodiments, mitochondrial disorders (including inherited mitochondrial diseases) such as Alpers Disease, Barth syndrome, Beta-oxidation Defects, Carnitine-Acyl-Carnitine Deficiency, Carnitine Deficiency, Creatine Deficiency Syndromes, Co-Enzyme Q10 Deficiency, Complex I Deficiency, Complex II Deficiency, Complex III Deficiency, Complex IV Deficiency, Complex V Deficiency, COX Deficiency, chronic progressive external ophthalmoplegia (CPEO), CPT I Deficiency, CPT II Deficiency, Friedreich's Ataxia (FA), Glutaric Aciduria Type II, Kearns-Sayre Syndrome (KSS), Lactic Acidosis, Long-Chain Acyl-CoA Dehydrongenase Deficiency (LCAD), Long-chain 3-Hydroxyacyl-CoA Dehydrogenase Deficiency (LCHAD), Leigh Syndrome, Leigh-like Syndrome, Leber's Hereditary Optic Neuropathy (LHON, also referred to as Leber's Disease, Leber's Optic Atrophy (LOA), Leber's Optic Neuropathy (LON)), Lethal Infantile Cardiomyopathy (LIC), Luft Disease, Multiple Acyl-CoA Dehydrogenase Deficiency (MAD), Medium-Chain Acyl-CoA Dehydrongenase Deficiency (MCAD); Mitochondrial Myopathy, Encephalopathy, Lactacidosis, Stroke (MELAS); Myoclonic Epilepsy with Ragged Red Fibers (MERRF), Mitochondrial Recessive Ataxia Syndrome (MIRAS), Mitochondrial Cytopathy, Mitochondrial DNA Depletion, Mitochondrial Encephalopathy, Mitochondrial Myopathy; Myoneurogastointestinal Disorder and Encephalopathy (MNGIE); Neuropathy, Ataxia, and Retinitis Pigmentosa (NARP); Pearson Syndrome, Pyruvate Carboxylase Deficiency, Pyruvate Dehydrogenase Deficiency, disorder associated with POLG Mutations, Respiratory Chain Disorder, Short-Chain Acyl-CoA Dehydrogenase Deficiency (SCAD), Short-chain 3-Hydroxyacyl-CoA Dehydrogenase Deficiency (SCHAD), Very Long-Chain Acyl-CoA Dehydrongenase Deficiency (VLCAD); myopathies such as cardiomyopathy, encephalomyopathy, and mitochondrial myopathy; neurodegenerative diseases such as Parkinson's disease, Alzheimer's disease, Frontotemporal Dementia (FTD), Progressive Supranuclear Palsy (PSP), Pick disease, and amyotrophic lateral sclerosis (ALS, also known as Lou Gehrig's disease); motor neuron diseases; neurological diseases such as epilepsy; age-associated diseases, including diseases for which CoQ10 has been proposed for treatment, such as macular degeneration, diabetes (e.g. Type 2 diabetes mellitus), metabolic syndrome, and cancer (e.g. brain cancer); genetic diseases such as Huntington's Disease (which is also a neurological disease); mood disorders such as schizophrenia and bipolar disorder; pervasive developmental disorders such as autistic disorder, Asperger's syndrome, childhood disintegrative disorder (CDD), Rett's disorder, CDKL5 deficiency disorder, and PDD-not otherwise specified (PDD-NOS); cerebrovascular accidents such as stroke; vision impairments such as those caused by neurodegenerative diseases of the eye such as optic neuropathy, Leber's hereditary optic neuropathy, dominant inherited juvenile optic atrophy, Dominant optic atrophy (DOA), DOA plus, retinitis pigmentosa; optic neuropathy caused by toxic agents, glaucoma, age-related macular degeneration (both "dry" or non-exudative macular degeneration and "wet" or exudative macular degeneration), Stargardt's macular dystrophy, diabetic retinopathy, diabetic maculopathy, retinopathy of prematurity, or ischemic reperfusion-related retinal injury; muscular dystrophies such as Duchenne muscular dystrophy (DMD), Becker muscular dystrophy, congenital muscular dystrophy, Fukuyama type congenital muscular dystrophy, limb-girdle muscular dystrophy, or myotonic dystrophy; disorders caused by energy impairment include diseases due to deprivation, poisoning, or toxicity of oxygen, and qualitative or quantitative disruption in the transport of oxygen such as haemoglobinopathies, in some embodiments, thalassemia, sickle cell anemia, or other anemias such as those associated with G6PD deficiency; other diseases in which mitochondrial dysfunction is implicated such as excitoxic, neuronal injury, such as that associated with seizures, stroke and ischemia; and other disorders including renal tubular acidosis; attention deficit/hyperactivity disorder (ADHD); neurodegenerative disorders resulting in hearing or balance impairment; Maternally inherited diabetes and deafness (MIDD); chronic fatigue; contrast-induced kidney damage; contrast-induced retinopathy damage; Abetalipoproteinemia; Wolfram's disease; Tourette syndrome; cobalamin c defect; methylmalonic aciduria; glioblastoma; Down's syndrome; acute tubular necrosis; leukodystrophies; spinal muscular atrophy; hearing loss (e.g. noise induced hearing loss); traumatic brain injury; Juvenile Huntington's Disease; Multiple Sclerosis; NGLY1; Multisystem atrophy; Adrenoleukodystrophy; and Adrenomyeloneuropathy. It is to be understood that certain specific diseases or disorders may fall within more than one category; in some embodiments, Huntington's Disease is a genetic disease as well as a neurological disease. Furthermore, certain oxidative stress diseases and disorders may also be considered mitochondrial disorders.

Neurodegenerative disorders include, for example, α-synucleinopathies; Parkinson's disease; familial Parkinson's Disease; idiopathic Parkinson's Disease; Parkinson's Disease wherein the patient has a mutation in one or more of the following genes: MAPT (Microtubule-associated protein tau), PRKN (parkin), PINK1 (PINK1), LRRK2 (leucine-rich repeat kinase 2), GBA (glucocerebrosidase), SNCA (alpha synuclein), PARK7 (DJ-1), and/or UCHL1 (ubiquitin carboxyl-terminal esterase L1); Parkinson's Disease with dementia (PDD); multisystem atrophy (MSA); Frontotemporal Dementia (FTD); Dementia with Lewy Bodies (DLB); Gaucher's disease (GD); Neurodegeneration with Brain Iron Accumulation (NBIA); neuroaxonal dystrophies (PLA2G6-associated neurodegeneration); tauopathies; Alzheimer's disease; dementia pugilistica; Guam Amyotrophic lateral sclerosis-Parkinsonism-Dementia (Guam ALS/PD); Pick Disease; Argyrophilic grain dementia; Nieman-Pick type C; Subacute sclerosing panencephalitis (SSPE); Progressive supranuclear palsy (PSP); Corticobasoganglionic degeneration; Frontotemporal dementia with parkinsonism-17 (FTDP-17); Postencephalitic Parkinsonism (PEP); Autosomal recessive Parkinsonism; Huntington's Disease; Juvenile Huntington's Disease; amyotrophic lateral sclerosis (ALS); a motor neuron disease; a leukodystrophy; Adrenoleukodystrophy; Adrenomyeloneuropathy; Creutzfeldt-Jakob disease; Machado-Joseph disease; Spino-cerebellar ataxia; and Multiple sclerosis (MS).

For some disorders amenable to treatment with compounds and methods disclosed herein, the primary cause of the disorder is due to a defect in the respiratory chain or another defect preventing normal utilization of energy in mitochondria, cells, or tissue(s). In some embodiments, disorders falling in this category include inherited mitochondrial diseases, such as Myoclonic Epilepsy with Ragged Red Fibers (MERRF), Mitochondrial Myopathy, Encephalopathy, Lactacidosis, and Stroke (MELAS), Leber's Hereditary Optic Neuropathy (LHON, also referred to as Leber's Disease, Leber's Optic Atrophy (LOA), or Leber's Optic Neuropathy (LON)), Leigh Syndrome, Leigh-like Syndrome, Kearns-Sayre Syndrome (KSS), and Friedreich's Ataxia (FA). For some disorders amenable to treatment with compounds and methods disclosed herein, the primary cause of the disorder is not due to respiratory chain defects or other defects preventing normal utilization of energy in mitochondria, cells, or tissue(s); in some embodiments, disorders falling in this category include stroke, cancer, and diabetes. However, these latter disorders are particularly aggravated by energy impairments, and are particularly amenable to treatment with compounds disclosed herein in order to ameliorate the condition. In some embodiments, such disorders include ischemic stroke and hemorrhagic stroke, where the primary cause of the disorder is due to impaired blood supply to the brain. While an ischemic episode caused by a thrombosis or embolism, or a hemorrhagic episode caused by a ruptured blood vessel, is not primarily caused by a defect in the respiratory chain or another metabolic defect preventing normal utilization of energy, oxidative stress plays a role in the ischemic cascade due to oxygen reperfusion injury following hypoxia (this cascade occurs in heart attacks as well as in strokes). Accordingly, treatment with compounds and methods disclosed herein will mitigate the effects of the disease, disorder or condition. Modulating one or more energy biomarkers, normalizing one or more energy biomarkers, or enhancing one or more energy biomarkers can also prove beneficial in such disorders both as a therapeutic measure and a prophylactic measure. In some embodiments, for a patient scheduled to undergo non-emergency repair of an aneurysm, enhancing energy biomarkers before and during the pre-operative can improve the patient's prognosis should the aneurysm rupture before successful repair.

The term "oxidative stress disorder" or "oxidative stress disease" encompasses both diseases caused by oxidative stress and diseases aggravated by oxidative stress. The terms "oxidative stress disorder" and "oxidative stress disease" encompass (1) both diseases and disorders where the primary cause of the disease is due to a defect in the respiratory chain or another defect preventing normal utilization of energy in mitochondria, cells, or tissue(s), and (2) also diseases and disorders where the primary cause of the disease is not due to a defect in the respiratory chain or another defect preventing normal utilization of energy in mitochondria, cells, or tissue(s). The set of diseases in (1) can be referred to as "primary oxidative stress disorders," while set of diseases in (2) can be referred to as "secondary oxidative stress disorders." It should be noted that the distinction between "diseases caused by oxidative stress" and "diseases aggravated by oxidative stress" is not absolute; a disease may be both a disease caused by oxidative stress and a disease aggravated by oxidative stress. The boundary between "primary oxidative stress disorder" and a "secondary oxidative stress disorder" is more distinct, provided that there is only one primary cause of a disease or disorder and that primary cause is known.

Bearing in mind the somewhat fluid boundary between diseases caused by oxidative stress and diseases aggravated by oxidative stress, mitochondrial diseases or disorders and impaired energy processing diseases and disorders tend to fall into the category of diseases caused by oxidative stress, while neurodegenerative disorders and diseases of aging tend to fall into the category of diseases aggravated by oxidative stress. Mitochondrial diseases or disorders and impaired energy processing diseases and disorders are generally primary oxidative stress disorders, while neurodegenerative disorders and diseases of aging may be primary or secondary oxidative stress disorders.

### Clinical assessment of oxidative stress and efficacy of therapy

Several readily measurable clinical markers are used to assess the metabolic state of patients with oxidative stress disorders. These markers can also be used as indicators of the efficacy of a given therapy, as the level of a marker is moved from the pathological value to the healthy value. These clinical markers include, but are not limited to, energy biomarkers such as lactic acid (lactate) levels, either in whole blood, plasma, cerebrospinal fluid, or cerebral ventricular fluid; pyruvic acid (pyruvate) levels, either in whole blood, plasma, cerebrospinal fluid, or cerebral ventricular fluid; lactate/pyruvate ratios, either in whole blood, plasma, cerebrospinal fluid, or cerebral ventricular fluid; total, reduced or oxidized glutathione levels, or reduced/oxidized glutathione ratio either in whole blood, plasma, lymphocytes, cerebrospinal fluid, or cerebral ventricular fluid; total, reduced or oxidized cysteine levels, or reduced/oxidized cysteine ratio either in whole blood, plasma, lymphocytes, cerebrospinal fluid, or cerebral ventricular fluid; phosphocreatine levels, NADH (NADH+H+) or NADPH (NADPH+H+) levels; NAD+ or NADP+ levels; ATP levels; anaerobic threshold; reduced coenzyme Q (CoQred) levels; oxidized coenzyme Q (CoQox) levels; total coenzyme Q (CoQtot) levels; oxidized cytochrome C levels; reduced cytochrome C levels; oxidized cytochrome C/reduced cytochrome C ratio; acetoacetate levels, β-hydroxybutyrate levels, acetoacetate/β-hydroxybutyrate ratio, 8-hydroxy-2'-deoxyguanosine (8-OHdG) levels; levels of reactive oxygen species; and levels of oxygen consumption (VO₂), levels of carbon dioxide output (VCO₂), and respiratory quotient (VCO₂/VO₂). Several of these clinical markers are measured routinely in exercise physiology laboratories, and provide convenient assessments of the metabolic state of a subject. In one embodiment, the level of one or more energy biomarkers in a patient suffering from an oxidative stress disorder, such as Friedreich's ataxia, Leber's hereditary optic neuropathy, MELAS, KSS or CoQ10 deficiency, is improved to within two standard deviations of the average level in a healthy subject. In another embodiment, the level of one or more of these energy biomarkers in a patient suffering from an oxidative stress disorder, such as Friedreich's ataxia, Leber's hereditary optic neuropathy, MELAS, KSS or CoQ10 deficiency is improved to within one standard deviation of the average level in a healthy subject. Exercise intolerance can also be used as an indicator of the efficacy of a given therapy, where an improvement in exercise tolerance (i.e., a decrease in exercise intolerance) indicates efficacy of a given therapy.

Several metabolic biomarkers have already been used to evaluate efficacy of CoQ10, and these metabolic biomarkers can be monitored as energy biomarkers for use in the methods disclosed herein. Lactate, a product of the anaerobic metabolism of glucose, is removed by reduction to pyruvate in an aerobic setting or by oxidative metabolism, which is dependent on a functional mitochondrial respiratory chain. Dysfunction of the respiratory chain may lead to inadequate removal of lactate and pyruvate from the circulation and elevated lactate/pyruvate ratios are observed in mitochondrial cytopathies (see Scriver CR, The metabolic and molecular bases of inherited disease, 7th ed., New York: McGraw-Hill, Health Professions Division, 1995; and Munnich et al., J. Inherit. Metab. Dis. 15(4):448-55 (1992)). Blood lactate/pyruvate ratio (Chariot et al., Arch. Pathol. Lab. Med. 118(7):695-7 (1994)) is, therefore, widely used as a noninvasive test for detection of mitochondrial cytopathies (see again Scriver CR, The metabolic and molecular bases of inherited disease, 7th ed., New York: McGraw-Hill, Health Professions Division, 1995; and Munnich et al., J. Inherit. Metab. Dis. 15(4):448-55 (1992)) and toxic mitochondrial myopathies (Chariot et al., Arthritis Rheum. 37(4):583-6 (1994)). Changes in the redox state of liver mitochondria can be investigated by measuring the arterial ketone body ratio (acetoacetate/3-hydroxybutyrate: AKBR) (Ueda et al., J. Cardiol. 29(2):95-102 (1997)). Urinary excretion of 8-hydroxy-2'-deoxyguanosine (8-OHdG) often has been used as a biomarker to assess the extent of repair of ROS-induced DNA damage in both clinical and occupational settings (Erhola et al., FEBS Lett. 409(2):287-91 (1997); Honda et al., Leuk. Res. 24(6):461-8 (2000); Pilger et al., Free Radic. Res. 35(3):273-80 (2001); Kim et al. Environ Health Perspect 112(6):666-71 (2004)).

Magnetic resonance spectroscopy (MRS) has been useful in the diagnoses of mitochondrial cytopathy by demonstrating elevations in cerebrospinal fluid (CSF) and cortical white matter lactate using proton MRS (1H-MRS) (Kaufmann et al., Neurology 62(8):1297-302 (2004)). Phosphorous MRS (31P-MRS) has been used to demonstrate low levels of cortical phosphocreatine (PCr) (Matthews et al., Ann. Neurol. 29(4):435-8 (1991)), and a delay in PCr recovery kinetics following exercise in skeletal muscle (Matthews et al., Ann. Neurol. 29(4):435-8 (1991); Barbiroli et al., J. Neurol. 242(7):472-7 (1995); Fabrizi et al., J. Neurol. Sci. 137(1):20-7 (1996)). A low skeletal muscle PCr has also been confirmed in patients with mitochondrial cytopathy by direct biochemical measurements.

Exercise testing is particularly helpful as an evaluation and screening tool in mitochondrial myopathies. One of the hallmark characteristics of mitochondrial myopathies is a reduction in maximal whole body oxygen consumption (VO₂ₘₐₓ) (Taivassalo et al., Brain 126(Pt 2):413-23 (2003)). Given that VO₂ₘₐₓ is determined by cardiac output (Qc) and peripheral oxygen extraction (arterial-venous total oxygen content) difference, some mitochondrial cytopathies affect cardiac function where delivery can be altered; however, most mitochondrial myopathies show a characteristic deficit in peripheral oxygen extraction (A-V O2 difference) and an enhanced oxygen delivery (hyperkinetic circulation) (Taivassalo et al., Brain 126(Pt 2):413-23 (2003)). This can be demonstrated by a lack of exercise induced deoxygenation of venous blood with direct AV balance measurements (Taivassalo et al., Ann. Neurol. 51(1):38-44 (2002)) and non-invasively by near infrared spectroscopy (Lynch et al., Muscle Nerve 25(5):664-73 (2002); van Beekvelt et al., Ann. Neurol. 46(4):667-70 (1999)).

Several of these energy biomarkers are discussed in more detail as follows. It should be emphasized that, while certain energy biomarkers are discussed and enumerated herein, the invention is not limited to modulation, normalization or enhancement of only these enumerated energy biomarkers.

Lactic acid (lactate) levels: Mitochondrial dysfunction typically results in abnormal levels of lactic acid, as pyruvate levels increase and pyruvate is converted to lactate to maintain capacity for glycolysis. Mitochondrial dysfunction can also result in abnormal levels of NADH+H+, NADPH+H+, NAD+, or NADP+, as the reduced nicotinamide adenine dinucleotides are not efficiently processed by the respiratory chain. Lactate levels can be measured by taking samples of appropriate bodily fluids such as whole blood, plasma, or cerebrospinal fluid. Using magnetic resonance, lactate levels can be measured in virtually any volume of the body desired, such as the brain.

Measurement of cerebral lactic acidosis using magnetic resonance in MELAS patients is described in Kaufmann et al., Neurology 62(8): 1297 (2004). Values of the levels of lactic acid in the lateral ventricles of the brain are presented for two mutations resulting in MELAS, A3243G and A8344G. Whole blood, plasma, and cerebrospinal fluid lactate levels can be measured by commercially available equipment such as the YSI 2300 STAT Plus Glucose & Lactate Analyzer (YSI Life Sciences, Ohio).

NAD, NADP, NADH and NADPH levels: Measurement of NAD, NADP, NADH (NADH +H+) or NADPH (NADPH+H+) can be measured by a variety of fluorescent, enzymatic, or electrochemical techniques, e.g., the electrochemical assay described in US 2005/0067303.

GSH, GSSG, Cys, and CySS levels: Briefly, plasma levels of GSH, GSSG, Cys, and CySS are used to calculate the in vivo Eₕ values. Samples are collected using the procedure of Jones et al (2009 Free Radical Biology & Medicine 47(10) pp. 1329-1338), and bromobimane is used to alkylate free thiols and HPLC and either electrochemical or MSMS to separate, detect, and quantify the molecules. United States Patent Application Publication No. US 2015-0218079, describes in more detail a method for different experimental parameters to analyze the most common monothiols and disulfide (cystine, cysteine, reduced (GSH) and oxidized glutathione (GSSG)) present in human plasma, and using Bathophenanthroline disulfonic acid as the internal standard (IS). Complete separation of all the targets analytes and IS at 35°C on a C18 RP column (250mm×4.6mm, 3 micron) was achieved using 0.2% TFA:Acetonitrile as a mobile phase pumped at the rate of 0.6 ml min-1 using electrochemical detector in DC mode at the detector potential of 1475 mV.

Oxygen consumption (vO₂ or VO₂), carbon dioxide output (vCO₂ or VCO₂), and respiratory quotient (VCO₂/VO₂): vO₂ is usually measured either while resting (resting vO₂) or at maximal exercise intensity (vO₂ max). Optimally, both values will be measured. However, for severely disabled patients, measurement of vO₂ max may be impractical. Measurement of both forms of vO₂ is readily accomplished using standard equipment from a variety of vendors, e.g. Korr Medical Technologies, Inc. (Salt Lake City, Utah). VCO₂ can also be readily measured, and the ratio of VCO₂ to VO₂ under the same conditions (VCO₂/VO₂, either resting or at maximal exercise intensity) provides the respiratory quotient (RQ).

Oxidized Cytochrome C, reduced Cytochrome C, and ratio of oxidized Cytochrome C to reduced Cytochrome C: Cytochrome C parameters, such as oxidized cytochrome C levels (Cyt Cox), reduced cytochrome C levels (Cyt Cred), and the ratio of oxidized cytochrome C/reduced cytochrome C ratio (Cyt Cox)/(Cyt Cred), can be measured by in vivo near infrared spectroscopy. See, e.g., Rolfe, P., "In vivo near-infrared spectroscopy," Annu. Rev. Biomed. Eng. 2:715-54 (2000) and Strangman et al., "Non-invasive neuroimaging using near-infrared light" Biol. Psychiatry 52:679-93 (2002).

Exercise tolerance/Exercise intolerance: Exercise intolerance is defined as "the reduced ability to perform activities that involve dynamic movement of large skeletal muscles because of symptoms of dyspnea or fatigue" (Piña et al., Circulation 107:1210 (2003)). Exercise intolerance is often accompanied by myoglobinuria, due to breakdown of muscle tissue and subsequent excretion of muscle myoglobin in the urine. Various measures of exercise intolerance can be used, such as time spent walking or running on a treadmill before exhaustion, time spent on an exercise bicycle (stationary bicycle) before exhaustion, and the like. Treatment with the compounds or methods disclosed herein can result in about a 10% or greater improvement in exercise tolerance (in some embodiments, about a 10% or greater increase in time to exhaustion, in some embodiments, from 10 minutes to 11 minutes), about a 20% or greater improvement in exercise tolerance, about a 30% or greater improvement in exercise tolerance, about a 40% or greater improvement in exercise tolerance, about a 50% or greater improvement in exercise tolerance, about a 75% or greater improvement in exercise tolerance, or about a 100% or greater improvement in exercise tolerance. While exercise tolerance is not, strictly speaking, an energy biomarker, for the purposes disclosed herein, modulation, normalization, or enhancement of energy biomarkers includes modulation, normalization, or enhancement of exercise tolerance.

Similarly, tests for normal and abnormal values of pyruvic acid (pyruvate) levels, lactate/pyruvate ratio, ATP levels, anaerobic threshold, reduced coenzyme Q (CoQred) levels, oxidized coenzyme Q (CoQox) levels, total coenzyme Q (CoQtot) levels, oxidized cytochrome C levels, reduced cytochrome C levels, oxidized cytochrome C/reduced cytochrome C ratio, GSH and cysteine reduced, oxidized, total levels and ratio, acetoacetate levels, β-hydroxy butyrate levels, acetoacetate/β-hydroxy butyrate ratio, 8-hydroxy-2'-deoxyguanosine (8-OHdG) levels, and levels of reactive oxygen species are known in the art and can be used to evaluate efficacy of the compounds and methods disclosed herein. (For the purposes disclosed herein, modulation, normalization, or enhancement of energy biomarkers includes modulation, normalization, or enhancement of anaerobic threshold.)

Table A, following, illustrates the effect that various dysfunctions can have on biochemistry and energy biomarkers. It also indicates the physical effect (such as a disease symptom or other effect of the dysfunction) typically associated with a given dysfunction. It should be noted that any of the energy biomarkers listed in the table, in addition to energy biomarkers enumerated elsewhere, can also be modulated, enhanced, or normalized by the compounds and methods disclosed herein. RQ = respiratory quotient; BMR = basal metabolic rate; HR (CO) = heart rate (cardiac output); T = body temperature (preferably measured as core temperature); AT = anaerobic threshold; pH = blood pH (venous and/or arterial).

**Table A**

| **Site of Dysfunction** | **Biochemical Event** | **Measurable Energy Biomarker** | **Physical Effect** |
|---|---|---|---|
| Respiratory Chain | ↑ NADH | Δ lactate, Δ lactate: pyruvate ratio; and Δ acetoacetate: β-hydroxy butyrate ratio | Metabolic dyscrasia & fatigue |
| Respiratory Chain | ↓ H+ gradient | Δ ATP | Organ dependent dysfunction |
| Respiratory Chain | ↓ Electron flux | Δ VO₂, RQ, BMR, ΔT, AT, pH | Metabolic dyscrasia & fatigue |
| Mitochondria & cytosol | ↓ ATP, ↓ VO₂ | Δ Work, ΔHR (CO) | Exercise intolerance |
| Mitochondria & cytosol | ↓ ATP | Δ PCr | Exercise intolerance |
| Respiratory Chain | ↓ Cyt COx/Red | Δ λ ∼700 - 900 nm (Near Infrared Spectroscopy) | Exercise intolerance |
| Intermediary metabolism | ↓ Catabolism | Δ C14-Labeled substrates | Metabolic dyscrasia & fatigue |
| Respiratory Chain | ↓ Electron flux | Δ Mixed Venous VO₂ | Metabolic dyscrasia & fatigue |
| Mitochondria & cytosol | ↑ Oxidative stress | Δ Tocopherol & Tocotrienols, CoQ10, docosahexaenoic acid | Uncertain |
| Mitochondria & cytosol | ↑ Oxidative stress | Δ Glutathione_{red} | Uncertain |
| Mitochondria & cytosol | Nucleic acid oxidation | Δ 8-hydroxy 2-deoxy guanosine | Uncertain |
| Mitochondria & cytosol | Lipid oxidation | Δ Isoprostane(s), eicosanoids | Uncertain |
| Cell membranes | Lipid oxidation | Δ Ethane (breath) | Uncertain |
| Cell membranes | Lipid oxidation | Δ Malondialdehyde | Uncertain |

Treatment of a subject afflicted by an oxidative stress disorder in accordance with the methods disclosed herein may result in the inducement of a reduction or alleviation of symptoms in the subject, e.g., to halt the further progression of the disorder.

Partial or complete suppression of the oxidative stress disorder can result in a lessening of the severity of one or more of the symptoms that the subject would otherwise experience. In some embodiments, partial suppression of MELAS could result in reduction in the number of stroke-like or seizure episodes suffered.

Any one or any combination of the energy biomarkers described herein provide conveniently measurable benchmarks by which to gauge the effectiveness of treatment or suppressive therapy. Additionally, other energy biomarkers are known to those skilled in the art and can be monitored to evaluate the efficacy of treatment or suppressive therapy.

### Other uses of compounds, including in research applications, experimental systems, and assays

The compounds disclosed herein can also be used in research applications. They can be used in in vitro, in vivo, or ex vivo experiments to modulate one or more energy biomarkers in an experimental system. Such experimental systems can be cell samples, tissue samples, cell components or mixtures of cell components, partial organs, whole organs, or organisms. Any one or more of the compounds as described herein can be used in experimental systems or research applications. Such research applications can include, but are not limited to, use as assay reagents, elucidation of biochemical pathways, or evaluation of the effects of other agents on the metabolic state of the experimental system in the presence/absence of one or more compounds disclosed herein.

Additionally, the compounds disclosed herein can be used in biochemical tests or assays. Such tests can include incubation of one or more compounds disclosed herein with a tissue or cell sample from a subject to evaluate a subject's potential response (or the response of a specific subset of subjects) to administration of said one or more compounds, or to determine which compound disclosed herein produces the optimum effect in a specific subject or subset of subjects. One such test or assay would involve 1) obtaining a cell sample or tissue sample from a subject in which modulation of one or more energy biomarkers can be assayed; 2) administering one or more compounds disclosed herein to the cell sample or tissue sample; and 3) determining the amount of modulation of the one or more energy biomarkers after administration of the one or more compounds, compared to the status of the energy biomarker prior to administration of the one or more compounds. Another such test or assay would involve 1) obtaining a cell sample or tissue sample from a subject in which modulation of one or more energy biomarkers can be assayed; 2) administering at least two compounds disclosed herein to the cell sample or tissue sample; 3) determining the amount of modulation of the one or more energy biomarkers after administration of the at least two compounds, compared to the status of the energy biomarker prior to administration of the at least two compounds, and 4) selecting a compound or compounds for use in treatment, suppression, or modulation based on the amount of modulation determined in step 3.

Additionally, uses include *in vivo* uses where one or more of the compounds described herein forms *in vivo* after administration of another compound.

Also included is a method of contacting a cell with a compound, or any embodiment thereof, as described herein.

### Pharmaceutical compositions

The terms "pharmaceutical formulation" and "pharmaceutical composition" are used interchangeably herein.

The compounds described herein can be formulated as pharmaceutical compositions by formulation with additives such as pharmaceutically acceptable excipients, pharmaceutically acceptable carriers, and pharmaceutically acceptable vehicles. The terms "pharmaceutically acceptable excipients," "pharmaceutically acceptable carriers," and "pharmaceutically acceptable vehicles" are used interchangeably herein. Suitable pharmaceutically acceptable excipients, carriers and vehicles include processing agents and drug delivery modifiers and enhancers, such as, in some embodiments, calcium phosphate, magnesium stearate, talc, monosaccharides, disaccharides, starch, gelatin, cellulose, methyl cellulose, sodium carboxymethyl cellulose, dextrose, hydroxypropyl-β-cyclodextrin, polyvinylpyrrolidinone, low melting waxes, ion exchange resins, and the like, as well as combinations of any two or more thereof. Other suitable pharmaceutically acceptable excipients are described in "Remington's Pharmaceutical Sciences," Mack Pub. Co., New Jersey (1991), and "Remington: The Science and Practice of Pharmacy," Lippincott Williams & Wilkins, Philadelphia, 20th edition (2003) and 21st edition (2005).

A pharmaceutical composition can comprise a unit dose formulation, where the unit dose is a dose sufficient to have a therapeutic (including a suppressive) effect. The unit dose may be sufficient as a single dose to have a therapeutic (including a suppressive) effect. Alternatively, the unit dose may be a dose administered periodically in a course of treatment, prophylaxis, or suppression of a disorder.

Pharmaceutical compositions containing the compounds disclosed herein may be in any form suitable for the intended method of administration, including, in some embodiments, a solution, a suspension, or an emulsion. Liquid carriers are typically used in preparing solutions, suspensions, and emulsions. Liquid carriers contemplated for use in the practice include in some embodiments, water, saline, pharmaceutically acceptable organic solvent(s), pharmaceutically acceptable oils or fats, and the like, as well as mixtures of two or more thereof. The liquid carrier may contain other suitable pharmaceutically acceptable additives such as solubilizers, emulsifiers, nutrients, buffers, preservatives, suspending agents, thickening agents, viscosity regulators, stabilizers, and the like. Suitable organic solvents include, in some embodiments, monohydric alcohols, such as ethanol, and polyhydric alcohols, such as glycols. Suitable oils include, in some embodiments, soybean oil, coconut oil, olive oil, safflower oil, cottonseed oil, and the like. For parenteral administration, the carrier can also be an oily ester such as ethyl oleate, isopropyl myristate, and the like. Compositions disclosed herein may also be in the form of microparticles, microcapsules, liposomal encapsulates, and the like, as well as combinations of any two or more thereof.

Time-release or controlled release delivery systems may be used, such as a diffusion controlled matrix system or an erodible system, as described for example in: Lee, "Diffusion-Controlled Matrix Systems", pp. 155-198 and Ron and Langer, "Erodible Systems", pp. 199-224, in "Treatise on Controlled Drug Delivery", A. Kydonieus Ed., Marcel Dekker, Inc., New York 1992. The matrix may be, in some embodiments, a biodegradable material that can degrade spontaneously in situ and in vivo, in some embodiments, by hydrolysis or enzymatic cleavage, e.g., by proteases. The delivery system may be, in some embodiments, a naturally occurring or synthetic polymer or copolymer, in some embodiments, in the form of a hydrogel. Exemplary polymers with cleavable linkages include polyesters, polyorthoesters, polyanhydrides, polysaccharides, poly(phosphoesters), polyamides, polyurethanes, poly(imidocarbonates) and poly(phosphazenes).

The compounds disclosed herein may be administered enterally, orally, parenterally, sublingually, by inhalation (e.g. as mists or sprays), rectally, or topically in dosage unit formulations containing conventional nontoxic pharmaceutically acceptable carriers, adjuvants, and vehicles as desired. In some embodiments, suitable modes of administration include oral, subcutaneous, transdermal, transmucosal, iontophoretic, intravenous, intraarterial, intramuscular, intraperitoneal, intranasal (e.g. via nasal mucosa), subdural, rectal, gastrointestinal, and the like, and directly to a specific or affected organ or tissue. For delivery to the central nervous system, spinal and epidural administration, or administration to cerebral ventricles, can be used. Topical administration may also involve the use of transdermal administration such as transdermal patches or iontophoresis devices. The term parenteral as used herein includes subcutaneous injections, intravenous, intramuscular, intra-sternal injection, or infusion techniques. The compounds are mixed with pharmaceutically acceptable carriers, adjuvants, and vehicles appropriate for the desired route of administration. Oral administration is a preferred route of administration, and formulations suitable for oral administration are preferred formulations. The compounds described for use herein can be administered in solid form, in liquid form, in aerosol form, or in the form of tablets, pills, powder mixtures, capsules, granules, injectables, creams, solutions, suppositories, enemas, colonic irrigations, emulsions, dispersions, food premixes, and in other suitable forms. The compounds can also be administered in liposome formulations. Additional methods of administration are known in the art.

In some embodiments, especially those embodiments where a formulation is used for injection or other parenteral administration including the routes listed herein, but also including embodiments used for oral, gastric, gastrointestinal, or enteric administration, the formulations and preparations used in the methods disclosed herein are sterile. Sterile pharmaceutical compositions are compounded or manufactured according to pharmaceutical-grade sterilization standards (United States Pharmacopeia Chapters 797, 1072, and 1211; California Business & Professions Code 4127.7; 16 California Code of Regulations 1751, 21 Code of Federal Regulations 211) known to those of skill in the art.

Injectable preparations, in some embodiments, sterile injectable aqueous or oleaginous suspensions, may be formulated as would be appreciated by a person of skill in the art using suitable dispersing or wetting agents and suspending agents. The sterile injectable preparation may also be a sterile injectable solution or suspension in a nontoxic parenterally acceptable diluent or solvent, in some embodiments, as a solution in propylene glycol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of inj ectables.

Solid dosage forms for oral administration may include capsules, tablets, pills, powders, and granules. In such solid dosage forms, the active compound may be admixed with at least one inert diluent such as sucrose, lactose, or starch. Such dosage forms may also comprise additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents, such as water. Such compositions may also comprise adjuvants, such as wetting agents, emulsifying and suspending agents, cyclodextrins, and sweetening, flavoring, and perfuming agents.

The compounds disclosed herein can also be administered in the form of liposomes. As appreciated by a person of skill in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolizable lipid capable of forming liposomes can be used. The present compositions in liposome form can contain, in addition to a compound disclosed herein, stabilizers, preservatives, excipients, and the like. The preferred lipids are the phospholipids and phosphatidyl cholines (lecithins), both natural and synthetic. Methods to form liposomes will be appreciated by those of skill in the art. See, for example, Prescott, Ed., Methods in Cell Biology, Volume XIV, Academic Press, New York, N.W., p. 33 et seq (1976).

Also provided are articles of manufacture and kits containing materials. Also provided are kits which comprise any one or more of the compounds as described herein. In some embodiments, the kit disclosed herein comprises the container described herein.

In other aspects, the kits may be used for any of the methods described herein.

The amount of active ingredient that may be combined with the carrier materials to produce a single dosage form will vary depending upon the host to which the active ingredient is administered and the particular mode of administration. It will be understood, however, that the specific dose level for any particular patient will depend upon a variety of factors including the activity of the specific compound employed, the age, body weight, body area, body mass index (BMI), general health, sex, diet, time of administration, route of administration, rate of excretion, drug combination, and the type, progression, and severity of the particular disease undergoing therapy. The pharmaceutical unit dosage chosen is usually fabricated and administered to provide a defined final concentration of drug in the blood, tissues, organs, or other targeted region of the body. The therapeutically effective amount for a given situation can be readily determined by routine experimentation and is within the skill and judgment of the ordinary clinician.

In some embodiments, dosages which can be used are a therapeutically effective amount within the dosage range of about 0.1 mg/kg to about 300 mg/kg body weight, or within about 1.0 mg/kg to about 100 mg/kg body weight, or within about 1.0 mg/kg to about 50 mg/kg body weight, or within about 1.0 mg/kg to about 30 mg/kg body weight, or within about 1.0 mg/kg to about 10 mg/kg body weight, or within about 10 mg/kg to about 100 mg/kg body weight, or within about 50 mg/kg to about 150 mg/kg body weight, or within about 100 mg/kg to about 200 mg/kg body weight, or within about 150 mg/kg to about 250 mg/kg body weight, or within about 200 mg/kg to about 300 mg/kg body weight, or within about 250 mg/kg to about 300 mg/kg body weight. Compounds disclosed herein may be administered in a single daily dose, or the total daily dosage may be administered in divided dosage of two, three or four times daily.

While the compounds disclosed herein can be administered as the sole active pharmaceutical agent, they can also be used in combination with one or more other agents used in the treatment or suppression of disorders. Representative agents useful in combination with the compounds disclosed herein for the treatment or suppression of neurodegenerative disorders include, but are not limited to, Coenzyme Q, vitamin E, idebenone, MitoQ, vitamins, NAC, and antioxidant compounds.

When additional active agents are used in combination with the compounds disclosed herein, the additional active agents may generally be employed in therapeutic amounts as indicated in the Physicians' Desk Reference (PDR) 53rd Edition (1999), or such therapeutically useful amounts as would be known to one of ordinary skill in the art.

The compounds disclosed herein and the other therapeutically active agents can be administered at the recommended maximum clinical dosage or at lower doses. Dosage levels of the active compounds in the compositions disclosed herein may be varied so as to obtain a desired therapeutic response depending on the route of administration, severity of the disease and the response of the patient. When administered in combination with other therapeutic agents, the therapeutic agents can be formulated as separate compositions that are given at the same time or different times, or the therapeutic agents can be given as a single composition.

The disclosure will be further understood by the following non-limiting examples.

### Preparation of Compounds

The compounds disclosed herein can be prepared from readily available starting materials; non-limiting exemplary methods are described in the Examples. It will be appreciated by one of ordinary skill in the art that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvents used, but such conditions can be determined by one skilled in the art.

### Synthetic Reaction Parameters

The terms "solvent", "inert organic solvent" and "inert solvent" embrace a solvent that is inert under the conditions of the reaction being described in conjunction therewith. Solvents employed in synthesis of the compounds disclosed herein include, in some embodiments, methanol ("MeOH"), acetone, water, acetonitrile, 1,4-dioxane, dimethylformamide ("DMF"), benzene, toluene, xylene, tetrahydrofuran ("THF"), chloroform, methylene chloride (or dichloromethane, ("DCM")), diethyl ether, pyridine and the like, as well as mixtures thereof. Unless specified to the contrary, the solvents used in the reactions disclosed herein are inert organic solvents.

The term "q.s." means adding a quantity sufficient to achieve a stated function, e.g., to bring a solution to the desired volume (i.e., 100%).

The term "eq" means an equivalent quantity of one reagent with respect to another reagent.

The term "o/n" or "O/N" means overnight.

The compounds herein are synthesized by an appropriate combination of generally well-known synthetic methods. Techniques useful in synthesizing the compounds herein are both readily apparent and accessible to those of skill in the relevant art in light of the teachings described herein. While the Examples illustrate some of the diverse methods available for use in assembling the compounds herein, they are not intended to define the scope of reactions or reaction sequences that are useful in preparing the compounds herein. Synthetic methods for other compounds disclosed herein will be apparent to one skilled in the art in view of the illustrative examples.

For all of the compounds and methods described herein, the quinone form can also be used in its reduced (hydroquinone) form when desired. Likewise, the hydroquinone form can also be used in its oxidized (quinone) form when desired. The reduced (hydroxy) form may readily be converted to the oxidized (quinone) form using methods known in the art. See, e.g., air, silica Miller et al PCT Intl Appl 2006130775 7 Dec 2006. The oxidized (quinone) form may readily be converted to the reduced hydroxy form using methods known in the art. See, e.g., Zn, AcOH Fuchs et al EJOC 6 (2009) 833-40.

### Exemplary Synthetic Schemes for Preparation of Compounds Disclosed Herein

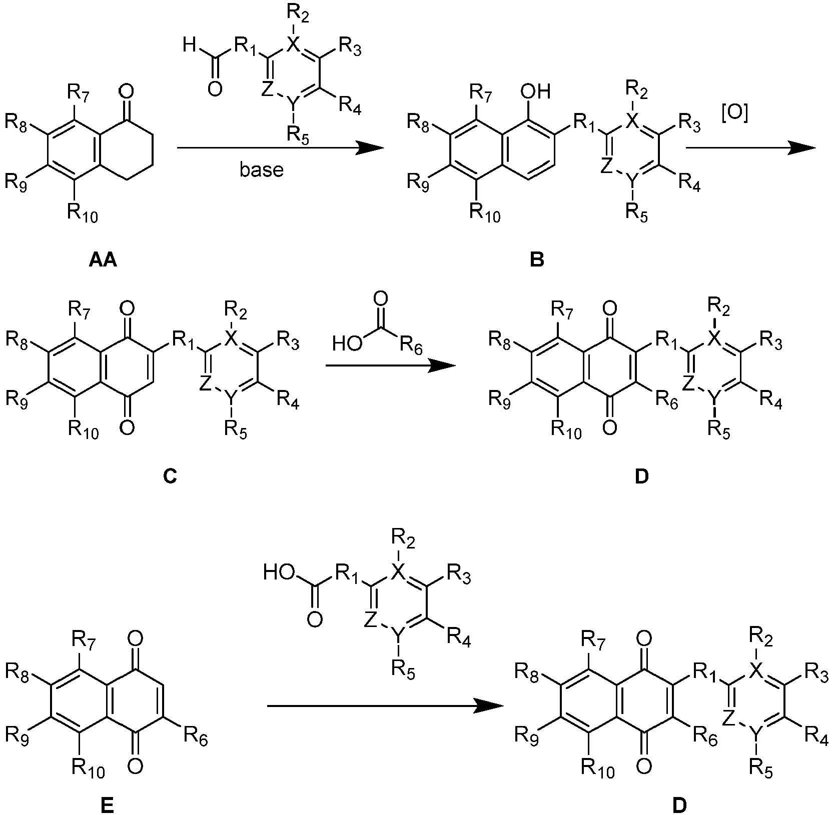

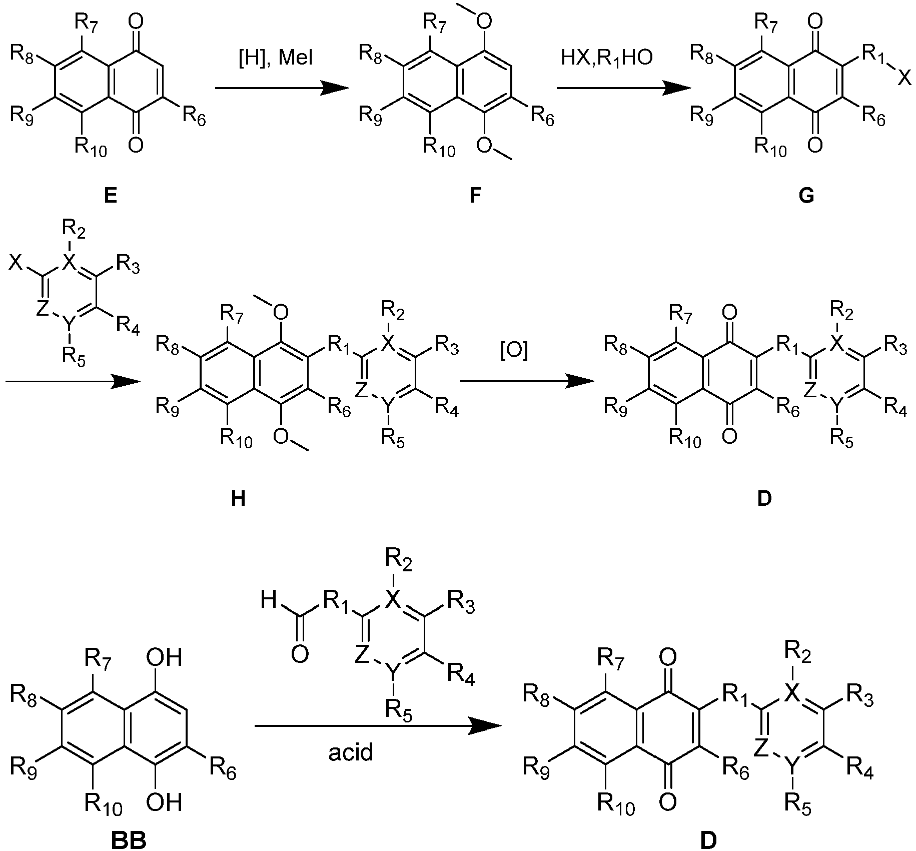

Tetralone **AA** is purchased commercially or prepared using methods known in the art. Aldol condensation followed by base-mediated isomerization provides **B**. Oxidation of **B** using hypervalent iodine or other oxidant leads to **C.** Intermediate **C** is trapped with a nucleophilic radical generated from an appropriately substituted carboxylic acid resulting in **D.** Alternatively, dione **E** is obtained commercially or prepared using methods known in the art. This compound is trapped with a nucleophilic radical generated from an appropriately substituted carboxylic acid resulting in **D.** Alternatively, **E** can be reduced and methylated to provide **F.** Halomethylation under acidic conditions leads to **G** which can be coupled under Negishi (or similar) conditions with an appropriately substituted halide resulting in **H.** Oxidative cleavage results in **D.** Alternatively, naphthalene diol **BB** reacts with aldehydes under acidic, aerobic conditions to form **D.**

### EXAMPLES

The following abbreviations were used in this section:

| | |
|---|---|
| AcOH | Acetic acid |
| ACN or MeCN | Acetonitrile |
| CAN | Cerric ammonium nitrate |
| DCE | Dichloroethane |
| DCM | Dichloromethane |
| DIEA | N,N-Diisopropylethylamine |
| DME | 1,2-dimethoxyethane |
| DMF | Dimethyl formamide |
| DMSO | Dimethyl sulfoxide |
| EtOAc or EA | Ethyl Acetate |
| EG | ethyleneglycol |
| IPA | isopropyl alcohol |
| LAH | Lithium aluminum hydride |
| LDA | Lithium diisopropylamide |
| MCPBA | meta Chloroperbenzoic acid |
| MOMCl | Methoxymethyl chloride |
| MsCl | Methanesulfonyl chloride |
| MTBE | Methyl tert butyl ether |
| M.W. | Microwave |
| PDC | Pyridinium dichromate |
| Pd(dppf)Cl₂ | [1,1'-Bis(diphenylphosphino)ferrocene]dichloropalladium(II) |
| PE | Petroleum ether |
| PIDA | Iodosobenzene diacetate |
| *p*-TsOH | para-toluene sulfonic acid |
| TEA | Triethylamine |
| TFA | Trifluoroacetic acid |
| TFAA | Trifluoroacetic anhydride |
| TMSCI | Trimethylsilyl chloride |
| THF | Tetrahydrofuran |
| TLC | Thin layer chromatography |
| UHP | urea hydrogen peroxide |

All reagents were obtained from commercial suppliers and used without further purification unless otherwise stated.

### Example 1 - 2-methyl-3-((5-(trifluoromethyl)pyridin-2-yl)methyl)naphthalene-1,4-dione

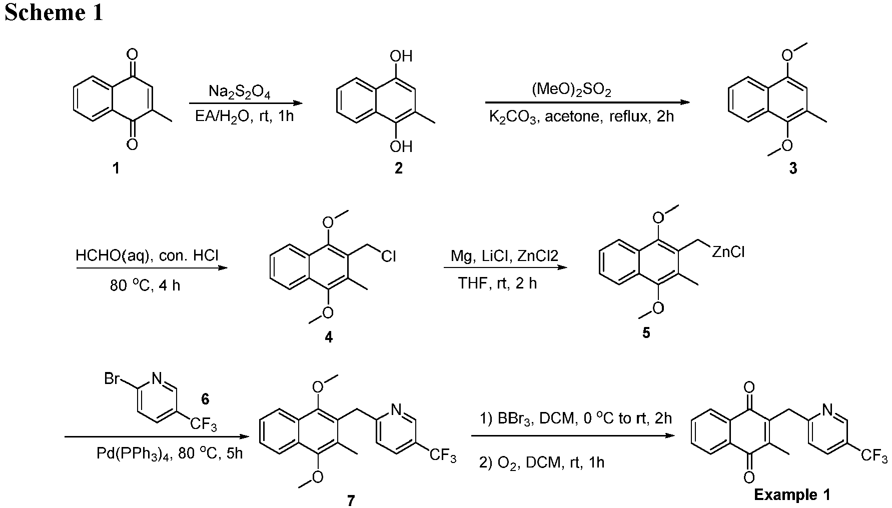

### Step 1 - 2-methylnaphthalene-1,4-diol (2)

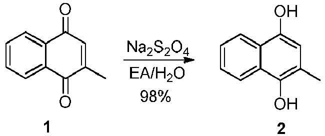

To a 250 mL solution of menadione (1) (20 g, 0.116 mol) in EtOAc was added a solution of sodium dithionite (61 g, 0.349 mol) in 250 mL water under nitrogen atmosphere. The reaction mixture was subjected to vigorous stirring. The discoloration of the organic layer indicated the completion of the reaction which was supported by TLC observation. The aqueous layer was separated and extracted with ethyl acetate (2 × 100 mL). The combined organic layers were washed with saturated sodium chloride solution and finally dried over Na₂SO₄ before evaporating the solvent in vacuum. A pale purple solid was recovered which was triturated with 300 mL of hexanes and the solid was filtered off to obtain 2-Methylnaphthalene-1,4-diol **2** (20 g, 98%) which was used in the next reaction without any purification.

### Step 2 - 1,4-dimethoxy-2-methylnaphthalene (3)

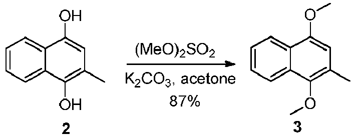

To a 250 mL solution of 2-methyl-naphthalene-1,4-diol (**2**) (20 g, 0.115 mol) in acetone was sequentially added K₂CO₃ (72 g, 0.575 mol) and dimethylsulfate (79 g, 0.575 mol). The resulting orange colored reaction mixture was refluxed for 2 h at which time TLC indicated the completion of the reaction. The reaction mixture was filtered and the filtrate was treated with 1N NaOH solution. The organic layer was separated and washed with brine before drying over Na₂SO₄. Solvent was removed under reduced pressure to provide crude 1,4-dimethoxy-2-methylnaphthalene (**3**). The residue was purified by column chromatography on silica gel (eluent: PE) to give 1,4-dimethoxy-2-methylnaphthalene (**3**) (20 g, 87%) as a yellow oil. ¹H NMR (400 MHz, CDCl₃): δ 8.15 (d, 1H), 8.03 (d, 1H), 7.50 (m, 1H), 7.49 (m, 1H), 6.61 (s, 1H), 3.97 (s, 3H), 3.87 (s, 3H), 2.46 (s, 3H).

### Step 3 - 2-(chloromethyl)-1,4-dimethoxy-3-methylnaphthalene (4)

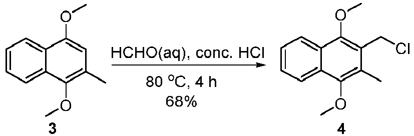

A mixture of 1,4-dimethoxy-2-methylnaphthalene (**3**) (20 g, 99 mmol), 37% aqueous formaldehyde (75 mL), and concentrated HCl (125 mL) was stirred for 4 h at 80 °C. The product was extracted with EA, and the extract was washed with water, dried over Na₂SO₄ and evaporated. The residue was purified by column chromatography on silica gel (eluent: PE) to give 2-(chloromethyl)-1,4-dimethoxy-3-methylnaphthalene (**4**) (17 g, 68%) as a white solid. ¹H NMR (400 MHz, CDCl₃): δ 8.08 (d, 2H), 7.54 (m, 2H), 4.93 (s, 2H), 4.04 (s, 3H), 3.88 (s, 3H), 2.54 (s, 3H).

### Step 4 - ((1,4-dimethoxy-3-methylnaphthalen-2-yl)methyl)zinc(II) chloride (5)

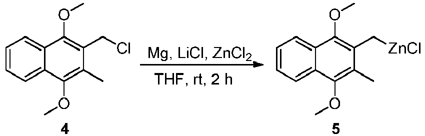

A dry and nitrogen-purged three-neck flask, equipped with a magnetic stirrer and a septum, was charged with magnesium turnings (2.4 g, 99.7 mmol) and LiCl (2.2 g, 51.9 mmol). Then ZnCl₂ (43.9 mL, 1.0 M in THF, 43.9 mmol) was added. The solution of 2-(chloromethyl)-1,4-dimethoxy-3-methylnaphthalene (**4**) (10 g, 39.9 mmol) in 100 mL dry THF was added at room temperature. The reaction mixture was stirred at room temperature for 2 h and used in the next reaction without any isolation or purification.

### Step 5 - 2-((1,4-dimethoxy-3-methylnaphthalen-2-yl)methyl)-5-(trifluoromethyl)pyridine (7)

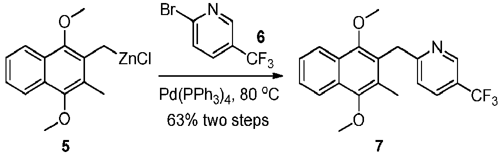

To a solution of 2-bromo-5-(trifluoromethyl)pyridine (**6**) (10.8 g, 47.9 mmol) in 50 mL THF at 25 °C was added successively ((1,4-dimethoxy-3-methylnaphthalen-2-yl)methyl)zinc(II) chloride (**5**) prepared in the previous step (144 mL, 39.9 mmol) and Pd(PPh₃)₄ (2.3 g, 2 mmol). The resulting reaction mixture was heated to 80 °C for 5 h. After cooling to 25 °C, the reaction mixture was diluted with EtOAc (100 mL) and quenched with sat. aqueous NH₄Cl solution. The phases were separated and the aqueous layer was extracted with EtOAc (3 × 50 mL). The combined extracts were dried over Na₂SO₄. Evaporation of the solvents *in vacuo* and purification by flash chromatography (silica gel, PE/EA =20/1) afforded 2-((1,4-dimethoxy-3-methylnaphthalen-2-yl)methyl)-5-(trifluoromethyl)pyridine (7) (9.1 g, 63%) as a light yellow oil. ¹H NMR (400 MHz, CDCl₃): δ 8.79 (s, 1H), 8.07 (m, 2H), 7.71 (m, 1H), 7.51 (m, 2H), 7.05 (d, 1H), 4.49 (s, 2H), 3.87 (s, 3H), 3.85 (s, 3H), 2.25 (s, 3H).

### Step 6 - 2-methyl-3-((5-(trifluoromethyl)pyridin-2-yl)methyl)naphthalene-1,4-dione (Example 1)

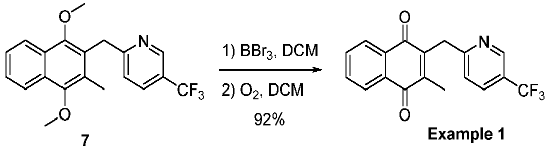

To a solution of 2-((1,4-dimethoxy-3-methylnaphthalen-2-yl)methyl)-5-(trifluoromethyl)pyridine (7) (8.5 g, 23.5 mmol) in DCM (120 mL) was added BBr₃ (59 mL, 2 M in DCM, 118 mmol) at 0 °C. The reaction mixture was stirred at room temperature for 2 h. MeOH was added dropwise to quench the reaction at 0 °C. Then saturated NaHCO₃ was added to adjust the pH to 8-9 and the aqueous mixture was extracted with DCM. The combined organic layer was washed with brine. The solution was stirred at room temperature under an O₂ balloon for 1 h. Solvent was removed to give a residue which was purified by column chromatography on silica gel (eluent: PE/EA =20/1) to give 2-methyl-3-((5-(trifluoromethyl)pyridin-2-yl)methyl)naphthalene-1,4-dione (**Example 1**) (7.2 g, 92%) as a yellow solid. ¹H NMR (400 MHz, DMSO-*d₆*): δ 8.79 (s, 1H), 8.11-8.07 (m, 1H), 8.02-7.98 (m, 1H), 7.97-7.93 (m, 1H), 7.85-7.79 (m, 2H), 7.60 (d, *J* = 8.0 Hz, 1H), 4.25 (s, 2H), 2.13 (s, 3H). MS (*m*/*z*) for C₁₈H₁₂F₃NO₂: found 332.05 (M+H).

The following compounds were prepared in a similar manner to **Example 1**:

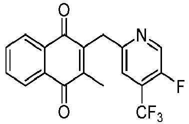

### Example 2 - 2-((5-fluoro-4-(trifluoromethyl)pyridin-2-yl)methyl)-3-methylnaphthalene-1,4-dione

¹H NMR (400 MHz, CDCl₃): δ 8.48 (s, 1H), 8.12-8.06 (m, 2H), 7.72-7.70 (m, 2H), 7.54 (d, J = 5.6 Hz, 1H), 4.23 (s, 2H), 2.31(s, 3H). MS (*m*/*z*) for C₁₈H₁₁F₄NO₂ found: 350.115 (M+H).

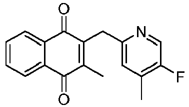

### Example 3 - 2-((5-fluoro-4-methylpyridin-2-yl)methyl)-3-methylnaphthalene-1,4-dione

¹H NMR (400 MHz, CDCl₃): δ 8.12 (s, 1H), 8.11-8.07 (m, 2H), 7.72-7.69 (m, 2H), 7.09 (d, J = 6.0 Hz, 1H), 4.14 (s, 2H), 2.28-2.26 (m, 6H). MS (*m*/*z*) for C₁₈H₁₄FNO₂: found 296.15 (M+H).

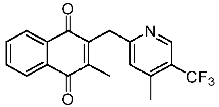

### Example 4 - 2-methyl-3-((4-methyl-5-(trifluoromethyl)pyridin-2-yl)methyl)naphthalene-1,4-dione

¹H NMR (400 MHz, CDCl₃): δ 8.63 (s, 1H), 8.12-8.07 (m, 2H), 7.72-7.70 (m, 2H), 7.19 (s, 1H), 4.21 (s, 2H), 2.45 (s, 3H), 2.29 (s, 3H). MS (*m*/*z*) for C₁₉H₁₄F₃NO₂: found 346.25 (M+H).

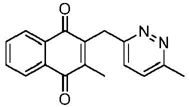

### Example 5 - 2-methyl-3-((6-methylpyridazin-3-yl)methyl)naphthalene-1,4-dione

¹H NMR (400 MHz, CDCl₃): δ 8.10-8.05 (m, 2H), 7.71-7.69 (m, 2H), 7.40 (d, J = 8.8 Hz, 1H), 7.23 (d, J = 8.8 Hz, 1H), 4.32 (s, 2H), 2.65 (s, 3H), 2.37 (s, 3H). MS (*m*/*z*) for C₁₇H₁₄N₂O₂: found 279.15 (M+H).

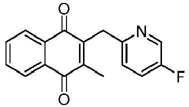

### Example 6 - 2-((5-fluoropyridin-2-yl)methyl)-3-methylnaphthalene-1,4-dione

¹H NMR (400 MHz, CDCl₃): δ 8.31 (d, J = 2.8 Hz, 1H), 8.08-8.06 (m, 2H), 7.71-7.68 (m, 2H), 7.29-7.26 (m, 2H), 4.18 (s, 2H), 2.29 (s, 3H). MS (*m*/*z*) for C₁₇H₁₂FNO₂: found 282.15 (M+H).

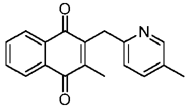

### Example 7 - 2-methyl-3-((5-methylpyridin-2-yl)methyl)naphthalene-1,4-dione

¹H NMR (400 MHz, CDCl₃): δ 8.30 (s, 1H), 8.11-8.07 (m, 2H), 7.71-7.67 (m, 2H), 7.38 (dd, J = 1.6, 8.0 Hz, 1H), 7.12 (d, J = 7.6 Hz, 1H), 4.18 (s, 2H), 2.27 (d, J = 4.0 Hz, 6H). MS (*m*/*z*) for C₁₈H₁₅NO₂: found 278.05 (M+H).

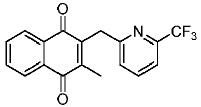

### Example 8 - 2-methyl-3-((6-(trifluoromethyl)pyridin-2-yl)methyl)naphthalene-1,4-dione

¹H NMR (400 MHz, CDCl₃): δ 8.11-8.08 (m, 2H), 7.75-7.70 (m, 3H), 7.49 (t, J = 7.2 Hz, 2H), 4.27 (s, 2H), 2.35 (s, 3H). MS (*m*/*z*) for C₁₈H₁₂F₃NO₂: found 332.25 (M+H).

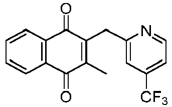

### Example 9 - 2-methyl-3-((4-(trifluoromethyl)pyridin-2-yl)methyl)naphthalene-1,4-dione

¹H NMR (400 MHz, CDCl₃): δ 8.63 (d, J = 4.4 Hz, 1H), 8.11-8.09 (m, 2H), 7.72-7.70 (m, 2H), 7.52 (s, 1H), 7.34-7.33 (m, 1H), 4.28 (s, 2H), 2.30 (s, 3H). MS (*m*/*z*) for C₁₈H₁₂F₃NO₂: found 332.15 (M+H).

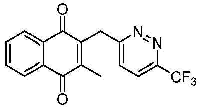

### Example 10 - 2-methyl-3-((6-(trifluoromethyl)pyridazin-3-yl)methyl)naphthalene-1,4-dione

¹H NMR (400 MHz, CDCl₃): δ 8.10-8.08 (m, 1H), 8.05-8.02 (m, 2H), 7.95-7.93 (m, 1H), 7.79-7.76 (m, 2H), 4.45 (s, 2H), 2.29 (s, 3H). MS (*m*/*z*) for C₁₇H₁₁F₃N₂O₂: found 333.05 (M+H).

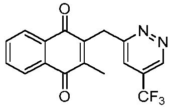

### Example 11 - 2-methyl-3-((5-(trifluoromethyl)pyridazin-3-yl)methyl)naphthalene-1,4-dione

¹H NMR (400 MHz, CDCl₃): δ 8.52 (s, 1H), 8.12-8.05 (m, 2H), 7.78(s, 1H), 7.75-7.69 (m, 2H), 4.46 (s, 2H), 2.40 (s, 3H). MS (*m*/*z*) for C₁₇H₁₁F₃N₂O₂: found 333.10 (M+H).

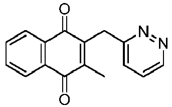

### Example 12 - 2-methyl-3-(pyridazin-3-ylmethyl)naphthalene-1,4-dione

¹H NMR (400 MHz, CDCl₃): δ 9.04 (dd, J = 5.2, 1.6 Hz, 1H), 8.11-8.05 (m, 2H), 7.73-7.69 (m, 2H), 7.52 (dd, J = 8.4, 1.2 Hz, 1H), 7.39 (m, 1H), 4.37 (s, 2H), 2.37 (s, 3H). MS (*m*/*z*) for C₁₆H₁₂N₂O₂: found 265.10 (M+H).

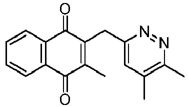

### Example 13 - 2-((5,6-dimethylpyridazin-3-yl)methyl)-3-methylnaphthalene-1,4-dione

¹H NMR (400 MHz, CDCl₃): δ 8.10-8.05 (m, 2H), 7.71-7.69 (m, 2H), 7.21 (s, 1H), 4.27 (s, 2H), 2.59 (s, 3H), 2.35 (s, 3H), 2.27 (s, 3H). MS (*m*/*z*) for C₁₈H₁₆N₂O₂: found 293.05 (M+H).

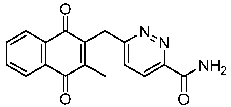

### Example 14 - 6-((3-methyl-1,4-dioxo-1,4-dihydronaphthalen-2-yl)methyl)pyridazine-3-carboxamide

¹H NMR (400 MHz, CDCl₃): δ 8.25 (d, J = 8.4 Hz, 1H), 8.12-8.05 (m, 2H), 7.89-7.88 (m, 1H), 7.76-7.71 (m, 3H), 5.67 (s, 1H), 4.43 (s, 2H), 2.38 (s, 3H). MS (*m*/*z*) for C₁₇H₁₃N₃O₃: found 308.05 (M+H).

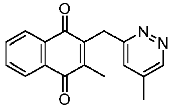

### Example 15 - 2-methyl-3-((5-methylpyridazin-3-yl)methyl)naphthalene-1,4-dione

¹H NMR (400 MHz, CDCl₃): δ 8.88 (s, 1H), 8.11-8.06 (m, 2H), 7.72-7.69 (m, 2H), 7.30 (s, 1H), 4.32 (s, 2H), 2.34 (d, J = 10.0 Hz, 6H). MS (*m*/*z*) for C₁₇H₁₄N₂O₂: found 279.10 (M+H).

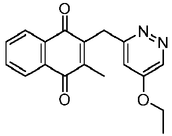

### Example 16 - 2-((5-ethoxypyridazin-3-yl)methyl)-3-methylnaphthalene-1,4-dione

¹H NMR (400 MHz, CDCl₃): δ 8.73 (s, 1H), 8.10-8.05 (m, 2H), 7.71-7.69 (m, 2H), 6.91 (s, 1H), 4.30 (s, 2H), 4.14-4.09 (m, 2H), 2.37 (s, 2H), 1.44 (t, J = 6.4 Hz, 3H); MS (*m*/*z*) for C₁₈H₁₆N₂O₃: found 309.05 (M+H).

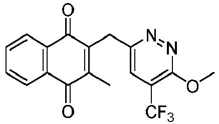

### Example 17 - 2-((6-methoxy-5-(trifluoromethyl)pyridazin-3-yl)methyl)-3-methylnaphthalene-1,4-dione

¹H NMR (400 MHz, CDCl₃): δ 8.11-8.05 (m, 2H), 7.74-7.70 (m, 3H), 4.32 (s, 2H), 4.19 (s, 3H), 2.38 (s, 3H). MS (*m*/*z*) for C₁₈H₁₃F₃N₂O₃: found 363.15 (M+H).

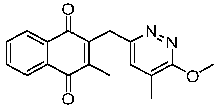

### Example 18 - 2-((6-methoxy-5-methylpyridazin-3-yl)methyl)-3-methylnaphthalene-1,4-dione

¹H NMR (400 MHz, CDCl₃): δ 8.10-8.06 (m, 2H), 7.71-7.69 (m, 2H), 7.24 (s, 1H), 4.21 (s, 2H), 4.08 (s, 3H), 2.35 (s, 3H), 2.19 (s, 3H). MS (*m*/*z*) for C₁₈H₁₆N₂O₃: found 309.10 (M+H).

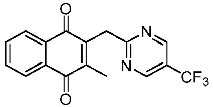

### Example 19 - 2-methyl-3-((5-(trifluoromethyl)pyrimidin-2-yl)methyl)naphthalene-1,4-dione

¹H NMR (400 MHz, CDCl₃): δ 8.86 (s, 2H), 8.14-8.07 (m, 2H), 7.73-7.71 (m, 2H), 4.47 (s, 2H), 2.22 (s, 3H). MS (m/z) for C₁₇H₁₁F₃N₂O₂: found 333.20 (M+H).

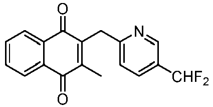

### Example 20 - 2-((5-(difluoromethyl)pyridin-2-yl)methyl)-3-methylnaphthalene-1,4-dione

¹H NMR (400 MHz, CDCl₃): δ 8.57 (s, 1H), 8.10-8.05 (m, 2H), 7.74-7.68 (m, 3H), 7.38-7.36 (d, *J* = 8.4 Hz, 1H), 6.78-6.50 (t, *J* = 55.8 Hz, 1H), 4.24 (s, 2H), 2.28 (s, 3H). MS (m/z) for C₁₈H₁₃F₂NO₂: found 312.05 (M-H).

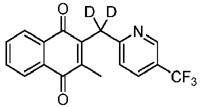

### Example 21 - 2-methyl-3-((5-(trifluoromethyl)pyridin-2-yl)methyl-d₂)naphthalene-1,4-dione

¹H NMR (CDCl₃, 400 MHz): δ 8.71 (s, 1H), 8.08-8.05 (m, 2H), 7.72 (m, 1H), 7.68 (m, 2H), 7.43-7.41 (m, 1H), 2.29 (s, 3H). MS (m/z) for C₁₈H₁₀D₂F₃NO₂: found 334.15 (M+H).

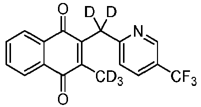

### Example 22 - 2-(methyl-d₃)-3-((5-(trifluoromethyl)pyridin-2-yl)methyl-d₂)naphthalene-1,4-dione

¹H NMR (400 MHz, CDCl₃): δ 8.71 (s, 1H), 8.08-8.04 (m, 2H), 7.78 (m, 1H), 7.68 (m, 2H), 7.43-7.41 (d, *J* = 8.4 Hz, 1H). MS (m/z) for C₁₈H₇D₅F₃NO₂: found 337.20 (M+H).

### Example 23 - 2-methyl-3-(pyridin-2-ylmethyl)naphthalene-1,4-dione

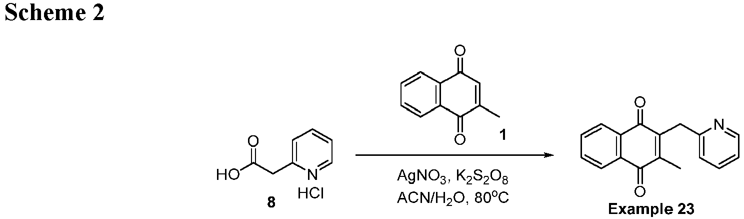

To a solution of 2-(pyridin-2-yl)acetic acid hydrochloride (**8**) (2 g, 11.63 mmol, 1.2 eq) in ACN (12 mL) was added menadione (**1**) (1.68 g, 9.69 mmol, 1.0 eq) and AgNO₃ (1.65 g, 9.69 mmol, 1.0 eq). The reaction mixture was stirred at 80 °C for 20 min. Then to the mixture was added K₂S₂O₈ (3.14 g, 11.63 mmol, 1.2 eq) in H₂O. The reaction mixture was stirred at 80 °C for another 2 h. The reaction was monitored by TLC. The mixture was extracted with EA (3 × 100 mL) and separated. The organic layer was washed by water, brine, dried over Na₂SO₄ and concentrated. The resultant residue was purified by flash chromatography on silica (PE: EA=50:1-20:1) to give the title compound (37 mg, 1%) as a yellow solid. ¹H NMR (400 MHz, DMSO-*d₆*): δ 8.39-8.38 (m, 1H), 8.16-8.06 (m, 3H), 8.03-7.97 (m, 2H), 7.84-7.82 (m, 2H), 7.68 (t, *J* = 7.0 Hz, 1H), 7.32 (d, *J* = 8.0 Hz, 1H), 7.18 (t, *J* = 6.6 Hz, 1H), 4.14 (s, 2H), 2.13 (s, 3H). MS (*m*/*z*) for C₁₇H₁₃NO₂: found 264.20 (M+H).

The following compound was prepared in a similar manner to **Example 23**:

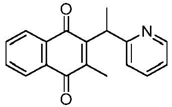

### Example 24 - 2-methyl-3-(1-(pyridin-2-yl)ethyl)naphthalene-1,4-dione

¹H NMR (400 MHz, CDCl₃): δ 8.47 (d, *J* = 4.8 Hz, 1H), 8.08-8.06 (m, 1H), 8.00-7.98 (m, 1H), 7.69-7.63 (m, 3H), 7.34 (d, *J* = 8.0 Hz, 1H), 7.12-7.09 (m, 1H), 4.74 (q, *J* = 7.2 Hz, 1H), 2.13 (s, 3H), 1.72 (d, *J* = 7.6 Hz, 3H). MS (*m*/*z*) for C₁₈H₁₅NO₂: found 278.10 (M+H).

### Example 25 - 2-((5-(trifluoromethyl)pyridin-2-yl)methyl)naphthalene-1,4-dione and Example 26 - 2-ethyl-3-((5-(trifluoromethyl)pyridin-2-yl)methyl)naphthalene-1,4-dione

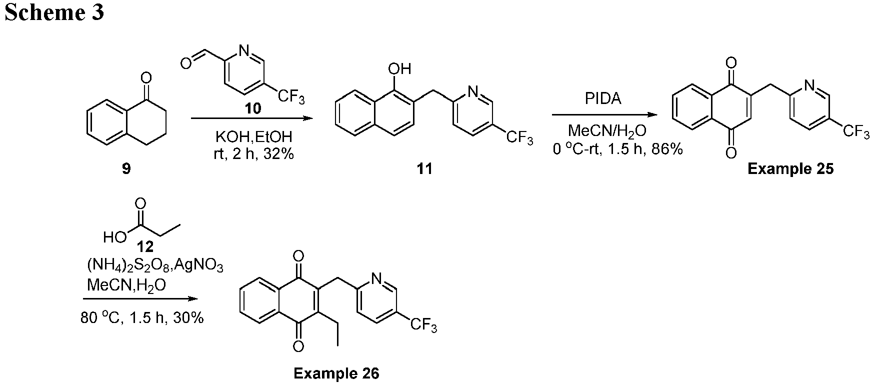

### Step 1 - 2-((5-(trifluoromethyl)pyridin-2-yl)methyl)naphthalen-1-ol (11)

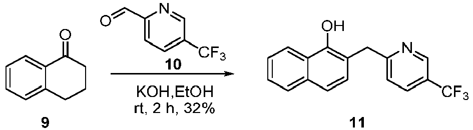

To a solution of 3,4-dihydronaphthalen-1(2H)-one (**9**) (6.4 g, 43.43 mmol, 1.0 eq) and 5-(trifluoromethyl)picolinaldehyde (**10**) (7.6 g, 43.43 mmol, 1.0 eq) in EtOH (100 mL) was added KOH (4.9 g, 86.86 mmol, 2.0 eq) under nitrogen atmosphere and stirred at room temperature for 2 h. The reaction was monitored by TLC. Then the mixture was concentrated under reduced pressure, and extracted with EA (3 × 100 mL). The organic layer was washed with H₂O (3 × 100 mL) and brine. The mixture was dried over Na₂SO₄ and concentrated under reduced pressure. The residue was purified by column chromatography on a silica gel to give 2-((5-(trifluoromethyl)pyridin-2-yl)methyl)naphthalen-1-ol (**11**)(4.27 g, 32%) as yellow solid.

### Step 2 - 2-((5-(trifluoromethyl)pyridin-2-yl)methyl)naphthalene-1,4-dione (Example 25)

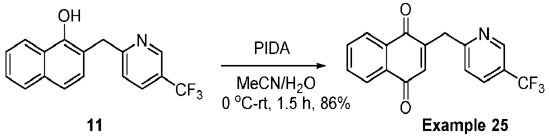

To a solution of 2-((5-(trifluoromethyl)pyridin-2-yl)methyl)naphthalen-1-ol (**11**) (2 g, 6.60 mmol, 1.0 eq) in ACN/H₂O (20 mL/ 6 mL) was added PIDA (4.46 g, 13.86 mmol, 2.1 eq) at 0 °C. After being stirred at 0 °C for 30 min, the mixture was allowed to warm to room temperature and stirred for 1 h. The reaction was monitored by TLC. To the mixture was added saturated aqueous of NaHCO₃ to adjust pH = 7 and extracted with EA (3 × 30 mL). The organic layer was washed with water, brine, dried over Na₂SO₄ and concentrated under reduced pressure. The resultant residue was purified by flash chromatography on silica to give the title compound (**Example 25**) (1.8 g, 86%) as a yellow solid. ¹H NMR (400 MHz, CDCl₃): δ 8.80 (s, 1H), 8.10-8.06 (m, 2H), 7.91-7.89 (m, 1H), 7.75-7.73 (m, 2H), 7.49 (d, J = 7.6 Hz, 1H), 6.85 (s, 1H), 4.16 (s, 2H). MS (*m*/*z*) for C₁₇H₁₀F₃NO₂: found 318.10 (M+H).

### Step 3 - 2-ethyl-3-((5-(trifluoromethyl)pyridin-2-yl)methyl)naphthalene-1,4-dione (Example 26)

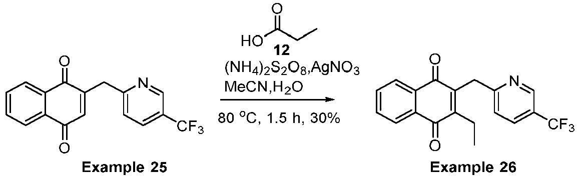

To a solution of 2-((5-(trifluoromethyl)pyridin-2-yl)methyl)naphthalene-1,4-dione (**Example 25**) (100 mg, 0.315 mmol, 1.0 eq) and propionic acid (**12**) (117 mg, 1.58 mmol, 5.0 eq) in ACN/H₂O (4 mL/ 1 mL) was added AgNO₃ (54 mg, 0.315 mmol, 1.0 eq). The reaction mixture was stirred at 80 °C for 30 min. Then to the mixture was added (NH₄)₂S₂O₈ (180 mg, 0.787 mmol, 2.5 eq) in ACN/H₂O (3 mL/ 1 mL). The reaction mixture was stirred at 80 °C for another 1 h. The reaction was monitored by TLC. The mixture was extracted with EA (3 × 10 mL) and separated. The organic layer was washed with water, brine, dried over Na₂SO₄ and concentrated. The resultant residue was purified by flash chromatography on silica to give the title compound (**Example 26**) (33 mg, 30%) as a yellow solid. ¹H NMR (400 MHz, CDCl₃): δ 8.71 (s, 1H), 8.09-8.04 (m, 2H), 7.83-7.81 (m, 1H), 7.70 (s, 2H), 7.44-7.42 (m, 1H), 4.26 (s, 2H), 2.80-2.78 (m, 2H), 1.10 (s, 3H). MS (*m*/*z*) for C₁₉H₁₄F₃NO₂: found 346.05 (M+H).

The following compounds were prepared in a similar manner to **Example 26**:

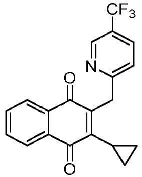

### Example 27 - 2-cyclopropyl-3-((5-(trifluoromethyl)pyridin-2-yl)methyl)naphthalene-1,4-dione

¹H NMR (400 MHz, CDCl₃): δ 8.72 (s, 1H), 8.02 (s, 2H), 7.83-7.81(m, 1H), 7.68 (m, 2H), 7.41-7.39 (m, 1H), 4.44 (s, 2H), 1.92 (s, 1H), 1.27 (m, 2H), 1.04 (m, 2H). MS (*m*/*z*) for C₂₀H₁₄F₃NO₂: found 358.15 (M+H).

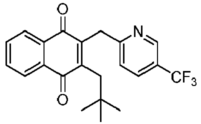

### Example 28 - 2-neopentyl-3-((5-(trifluoromethyl)pyridin-2-yl)methyl)naphthalene-1,4-dione

¹H NMR (400 MHz, CDCl₃): δ 8.69 (s, 1H), 8.09 (d, *J* = 5.6 Hz, 1H), 8.02-8.01(m, 1H), 7.81 (d, *J* = 7.6 Hz, 1H), 7.70 (m, 2H), 7.40 (d, *J* = 6.4 Hz, 1H), 4.31 (s, 2H), 2.87 (m, 2H), 0.99-0.98 (m, 9H). MS (*m*/*z*) for C₂₂H₂₀F₃NO₂: found 388.15 (M+H).

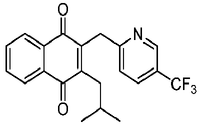

### Example 29 - 2-isobutyl-3-((5-(trifluoromethyl)pyridin-2-yl)methyl)naphthalene-1,4-dione

¹H NMR (400 MHz, CDCl₃): δ 8.71 (s, 1H), 8.10-8.04 (m, 2H), 7.83-7.81 (m, 1H), 7.71 (m, 2H), 7.39 (d, *J* = 7.6 Hz, 1H), 4.28 (s, 2H), 2.71-2.69 (m, 2H), 1.96-1.93 (m, 1H), 0.94-0.95 (m, 6H). MS (*m*/*z*) for C₂₁H₁₈F₃NO₂: found 374.15 (M+H).

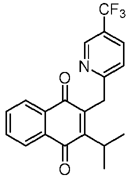

### Example 30 - 2-isopropyl-3-((5-(trifluoromethyl)pyridin-2-yl)methyl)naphthalene-1,4-dione

¹H NMR (400 MHz, CDCl₃): δ 8.73 (s, 1H), 8.06 (s, 2H), 7.82-7.81 (m, 1H), 7.70 (m, 2H), 7.39 (d, *J* = 5.6 Hz, 1H), 4.33 (s, 2H), 3.36-3.35 (m, 1H), 1.32-1.25 (m, 6H). MS (*m*/*z*) for C₂₀H₁₆F₃NO₂: found 360.20 (M+H).

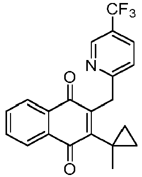

### Example 31 - 2-(1-methylcyclopropyl)-3-((5-(trifluoromethyl)pyridin-2-yl)methyl)naphthalene-1,4-dione

¹H NMR (400 MHz, CDCl₃): δ 8.66 (s, 1H), 8.11-8.10 (d, *J* = 6.8 Hz, 1H), 7.96(d, *J* = 7.6 Hz, 1H), 7.85 (d, *J* = 7.6 Hz, 1H), 7.71-7.66 (m, 2H), 7.42 (d, *J* = 7.6 Hz, 1H), 4.47 (s, 2H), 1.37-1.36 (m, 3H), 0.84-0.82 (m, 4H). MS (*m*/*z*) for C₂₁H₁₆F₃NO₂: found 372.20 (M+H).

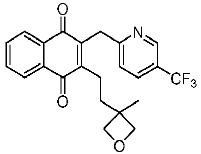

### Example 32 - 2-(2-(3-methyloxetan-3-yl)ethyl)-3-((5-(trifluoromethyl)pyridin-2-yl)methyl)naphthalene-1,4-dione

¹H NMR (400 MHz, DMSO-*d₆*): δ 8.80 (s, 1H), 8.12 (dd, *J* = 8.0, 1.6 Hz, 1H), 8.03 (dd, *J* = 8.8, 2.0 Hz, 1H), 7.97-7.96 (m, 1H), 7.85-7.84 (m, 2H), 7.64 (d, *J* = 8.4 Hz, 1H), 4.29-4.28 (m, 4H), 4.19 (d, *J* = 5.6 Hz, 2H), 2.58-2.54 (m, 2H), 1.58-1.54 (m, 2H), 1.22 (s, 3H). MS (*m*/*z*) for C₂₃H₂₀F₃NO₃: found 416.15 (M+H).

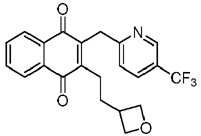

### Example 33 - 2-(2-(oxetan-3-yl)ethyl)-3-((5-(trifluoromethyl)pyridin-2-yl)methyl)naphthalene-1,4-dione

¹HNMR (400 MHz, CDCl₃): δ 8.71 (s, 1H), 8.11-8.04 (m, 2H), 7.83 (dd, *J* = 2.4, 8.0 Hz, 1H), 7.75-7.69 (m, 2H), 7.47 (d, *J* = 8.0 Hz, 1H), 4.83-4.79 (m, 2H), 4.34 (t, *J=* 6.0 Hz, 2H), 4.24 (s, 2H), 3.07-3.3 (m, 1H), 2.74-2.70 (m, 2H), 1.88 (q, *J* = 7.6 Hz, 2H). MS (*m*/*z*) for C₂₂H₁₈F₃NO₃: found 402.20 (M+H).

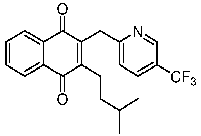

### Example 34 - 2-isopentyl-3-((5-(trifluoromethyl)pyridin-2-yl)methyl)naphthalene-1,4-dione

¹H NMR (400 MHz, CDCl₃): δ 8.73 (s, 1H), 8.11-8.04 (m, 2H), 7.82 (d, *J* = 8.4 Hz, 1H), 7.72-7.70 (m, 2H), 7.41 (d, *J* = 8.4 Hz, 1H), 4.26 (s, 2H), 2.75-2.71 (m, 2H), 1.66-1.61 (m, 1H), 1.30-1.25 (m, 2H), 0.93 (d, *J* = 6.4 Hz, 6H). MS *(m*/*z)* for C₂₂H₂₀F₃NO₂: found 388.30 (M+H).

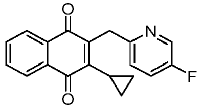

### Example 35 - 2-cyclopropyl-3-((5-fluoropyridin-2-yl)methyl)naphthalene-1,4-dione

¹H NMR (400 MHz, CDCl₃) δ 8.31 (d, *J* = 2.4 Hz, 1H), 8.05-7.99 (m, 2H), 7.70-7.65 (m, 2H), 7.33-7.24 (m, 2H), 4.36 (s, 2H), 1.96-1.92 (m, 1H), 1.29-1.25 (m, 2H), 1.04-0.99 (m, 2H). MS (*m*/*z*) for C₁₉H₁₄FNO₂: found 308.05 (M+H).

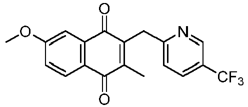

### Example 36 - 6-methoxy-2-methyl-3-((5-(trifluoromethyl)pyridin-2-yl)methyl)naphthalene-1,4-dione

¹H NMR (400 MHz, CDCl₃): δ 8.73 (s, 1H), 8.05 (d, *J* = 8 Hz, 1H), 7.81 (d, *J* = 8 Hz, 1H), 7.51 (s, 1H), 7.41 (d, *J* = 8 Hz, 1H), 7.18 (d, *J* = 8 Hz, 1H), 4.25 (s, 2H), 3.92 (s, 3H), 2.28 (s, 3H). MS (*m*/*z*) for C₁₉H₁₄F₃NO₃: found 362.05 (M+H).

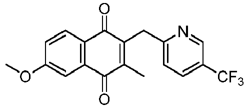

### Example 37 - 6-methoxy-3-methyl-2-((5-(trifluoromethyl)pyridin-2-yl)methyl)naphthalene-1,4-dione

¹H NMR (400 MHz, CDCl₃): δ 8.74 (s, 1H), 8.01 (d, *J* = 8.0 Hz, 1H), 7.81 (d, *J* = 8.0 Hz, 1H), 7.53 (s, 1H), 7.41 (d, *J* = 8.0 Hz, 1H), 7.16 (d, *J* = 8.0 Hz, 1H), 4.27 (s, 2H), 3.94 (s, 3H), 2.28 (s, 3H). MS (*m*/*z*) for C₁₉H₁₄F₃NO₃: found 362.05 (M+H).

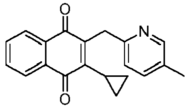

### Example 38 - 2-cyclopropyl-3-((5-methylpyridin-2-yl)methyl)naphthalene-1,4-dione

¹H NMR (400 MHz, CDCl₃): δ 8.29 (s, 1H), 8.05-7.98 (m, 2H), 7.67-7.65 (m, 2H), 7.38 (dd, *J* = 2.0, 7.6 Hz, 1H), 7.10 (d, *J* = 7.6 Hz, 1H), 4.35 (s, 2H), 2.27 (s, 3H), 1.96-1.92 (m, 1H), 1.28-1.24 (m, 2H), 1.02-0.97 (m, 2H). MS (*m*/*z*) for C₂₀H₁₇NO₂: found 304.15 (M+H).

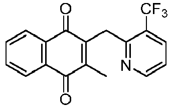

### Example 39 - 2-methyl-3-((3-(trifluoromethyl)pyridin-2-yl)methyl)naphthalene-1,4-dione

¹H NMR (400 MHz, CDCl₃): δ 8.50 (d, *J* = 4.4 Hz, 1H), 8.15-8.12 (m, 1H), 8.09-8.06 (m, 1H), 7.94 (d, *J* = 8.0 Hz, 1H), 7.72-7.70 (m, 2H), 7.24-7.21 (m, 1H), 4.38 (s, 2H), 2.12 (s, 3H). MS (*m*/*z*) for C₁₈H₁₂F₃NO₂: found 332.15 (M+H).

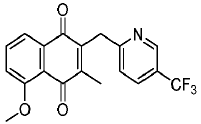

### Example 40 - 5-methoxy-3-methyl-2-((5-(trifluoromethyl)pyridin-2-yl)methyl)naphthalene-1,4-dione

¹H NMR (400 MHz, CDCl₃): δ 8.71 (s, 1H), 8.01 (d, *J* = 8.0 Hz, 1H), 7.75-7.68 (m, 2H), 7.54 (d, *J* = 8.0 Hz, 1H), 7.47 (d, *J* = 8.0 Hz, 1H), 4.27 (s, 2H), 3.97 (s, 3H), 2.18 (s, 3H). MS (*m*/*z*) for C₁₉H₁₄F₃NO₃: found 362.05 (M+H).

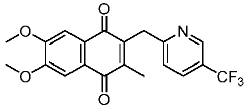

### Example 41 - 6,7-dimethoxy-2-methyl-3-((5-(trifluoromethyl)pyridin-2-yl)methyl)naphthalene-1,4-dione

¹H NMR (400 MHz, CDCl₃): δ 8.75 (s, 1H), 7.84 (d, *J* = 8.0 Hz, 1H), 7.52 (s, 1H), 7.49 (s, 1H), 7.42 (d, *J* = 8.0 Hz, 1H), 4.25 (s, 2H), 4.02 (s, 3H), 3.99 (s, 3H), 2.26 (s, 3H). MS (*m*/*z*) for C₁₅H₁₁NO₃: found 392.20 (M+H).

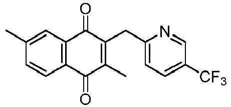

### Example 42 - 2,6-dimethyl-3-((5-(trifluoromethyl)pyridin-2-yl)methyl)naphthalene-1,4-dione

¹H NMR (400 MHz, CDCl₃): δ 8.77 (s, 1H), 7.97 (d, *J* = 8.0 Hz, 1H), 7.88-7.85 (m, 2H), 7.50 (d, *J* = 4.0 Hz, 1H), 7.42 (d, *J* = 8.0 Hz, 1H), 4.29 (s, 2H), 2.47 (s, 3H), 2.27 (s, 3H). MS (*m*/*z*) for C₁₉H₁₄F₃NO₂: found 346.10 (M+H).

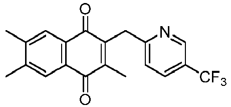

### Example 43 - 2,6,7-trimethyl-3-((5-(trifluoromethyl)pyridin-2-yl)methyl)naphthalene-1,4-dione

¹H NMR (400 MHz, CDCl₃): δ 8.75 (s, 1H), 7.87-7.82 (m, 3H), 7.46-7.42 (m, 1H), 4.27 (s, 2H), 2.41 (s, 3H), 2.40 (s, 3H), 2.29 (s, 3H).

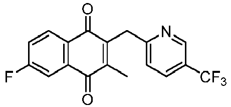

### Example 44 - 6-fluoro-3-methyl-2-((5-(trifluoromethyl)pyridin-2-yl)methyl)naphthalene-1,4-dione

¹H NMR (400 MHz, CDCl₃): δ 8.72 (s, 1H), 8.10 (d, *J* = 8.0 Hz, 1H), 7.81 (d, *J* = 8.0 Hz, 1H), 7.73 (d, *J* = 8.0 Hz, 1H), 7.45-7.35 (m, 2H), 4.27 (s, 2H), 2.30 (s, 3H). MS (*m*/*z*) for C₁₈H₁₁F₄NO₂: found 350.05 (M+H).

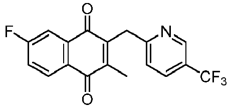

### Example 45 - 6-fluoro-2-methyl-3-((5-(trifluoromethyl)pyridin-2-yl)methyl)naphthalene-1,4-dione

¹H NMR (400 MHz, CDCl₃): δ 8.72 (s, 1H), 8.14 (d, *J* = 8.0 Hz, 1H), 7.82 (d, *J* = 8.0 Hz, 1H), 7.69 (d, *J* = 8.0 Hz, 1H), 7.45-7.35 (m, 2H), 4.26 (s, 2H), 2.30 (s, 3H). MS (*m*/*z*) for C₁₈H₁₁F₄NO₂: found 350.05 (M+H).

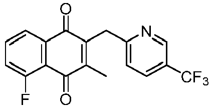

### Example 46 - 5-fluoro-3-methyl-2-((5-(trifluoromethyl)pyridin-2-yl)methyl)naphthalene-1,4-dione

¹H NMR (400 MHz, CDCl₃): δ 8.77 (s, 1H), 7.93 (d, *J* = 8.0 Hz, 1H), 7.81 (d, *J* = 8.0 Hz, 1H), 7.70-7.65 (m, 1H), 7.45-7.35 (m, 2H), 4.25 (s, 2H), 2.28 (s, 3H). MS (*m*/*z*) for C₁₈H₁₁F₄NO₂: found 350.00 (M+H).

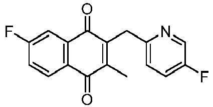

### Example 47 - 6-fluoro-3-((5-fluoropyridin-2-yl)methyl)-2-methylnaphthalene-1,4-dione

¹H NMR (400 MHz, CD₃OD): δ 8.27 (s, 1H), 8.13 (d, *J* = 8.0 Hz, 1H), 7.69 (d, *J* = 8.0 Hz, 1H), 7.55-7.45 (m, 2H), 7.40-7.36 (m, 1H), 4.19 (s, 2H), 2.18 (s, 3H). MS (*m*/*z*) for C₁₇H₁₁F₂NO₂: found 300.05 (M+H).

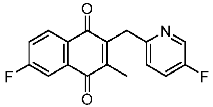

### Example 48 - 6-fluoro-2-((5-fluoropyridin-2-yl)methyl)-3-methylnaphthalene-1,4-dione

¹H NMR (400 MHz, CDCl₃): δ 8.31 (s, 1H), 8.11-8.08 (m, 1H), 7.73-7.71 (m, 1H), 7.35-7.25 (m, 3H), 4.17 (s, 2H), 2.27 (s, 3H). MS (*m*/*z*) for C₁₇H₁₁F₂NO₂: found 300.15 (M+H).

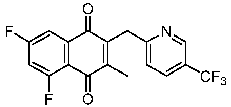

### Example 49 - 5,7-difluoro-3-methyl-2-((5-(trifluoromethyl)pyridin-2-yl)methyl)naphthalene-1,4-dione

¹H NMR (400 MHz, CDCl₃): δ 8.74 (s, 1H), 7.88-7.86 (m, 1H), 7.62-7.60 (m, 1H), 7.44-7.42 (m, 1H), 7.15-7.10 (m, 1H), 4.26 (s, 2H), 2.27 (s, 3H). MS (*m*/*z*) for C₁₈H₁₀F₅NO₂: found 368.05 (M+H).

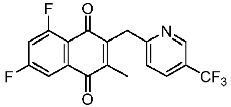

### Example 50 - 5,7-difluoro-2-methyl-3-((5-(trifluoromethyl)pyridin-2-yl)methyl)naphthalene-1,4-dione

¹H NMR (400 MHz, CDCl₃): δ 8.70 (s, 1H), 7.83 (d, *J* = 8.0 Hz, 1H), 7.66 (d, *J* = 8.0 Hz, 1H), 7.46 (d, *J* = 8.0 Hz, 1H), 7.15-7.06 (m, 1H), 4.24 (s, 2H), 2.31 (s, 3H). MS (*m*/*z*) for C₁₈H₁₀F₅NO₂: found 368.05 (M+H).

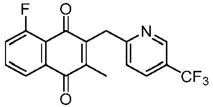

### Example 51 - 5-fluoro-2-methyl-3-((5-(trifluoromethyl)pyridin-2-yl)methyl)naphthalene-1,4-dione

¹H NMR (400 MHz, CDCl₃): δ 8.77 (s, 1H), 7.97 (d, *J* = 8.0 Hz, 1H), 7.90-7.85 (m, 1H), 7.75-7.67 (m, 1H), 7.49 (d, *J* = 8.0 Hz, 1H), 7.45-7.38 (m, 1H), 4.28 (s, 2H), 2.29 (s, 3H). MS (*m*/*z*) for C₁₈H₁₁F₄NO₂: found 350.10 (M+H).

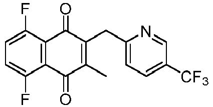

### Example 52 - 5,8-difluoro-2-methyl-3-((5-(trifluoromethyl)pyridin-2-yl)methyl)naphthalene-1,4-dione

¹H NMR (400 MHz, CDCl₃): δ 8.70 (s, 1H), 7.83 (d, *J* = 8.0 Hz, 1H), 7.50-7.38 (m, 3H), 4.22 (s, 2H), 2.29 (s, 3H). MS (*m*/*z*) for C₁₈H₁₀F₅NO₂: found 368.20 (M+H).

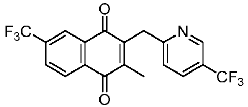

### Example 53 - 2-methyl-6-(trifluoromethyl)-3-((5-(trifluoromethyl)pyridin-2-yl)methyl)naphthalene-1,4-dione

¹H NMR (400 MHz, CDCl₃): δ 8.71 (s, 1H), 8.35 (s, 1H), 8.24 (d, *J* = 8.0 Hz, 1H), 7.99 (d, *J* = 8.0 Hz, 1H), 7.84 (d, *J* = 8.0 Hz, 1H), 7.45 (d, *J* = 8.0 Hz, 1H), 4.29 (s, 2H), 2.34 (s, 3H). MS (*m*/*z*) for C₁₉H₁₁F₆NO₂: found 398.05 (M-H).

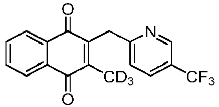

### Example 54 - 2-(methyl-d₃)-3-((5-(trifluoromethyl)pyridin-2-yl)methyl)naphthalene-1,4-dione

¹H NMR (400 MHz, CDCl₃): δ 8.66 (s, 1H), 8.05-7.99 (m, 2H), 7.77-7.74 (dd, *J* = 8 Hz,2 Hz, 1H), 7.65-7.63 (m, 2H), 7.37-7.35 (d, *J* = 8 Hz, 1H), 4.20 (s, 2H). MS (*m*/*z*) for C₁₈H₉D₃F₃NO₂: found .335 (M-H).

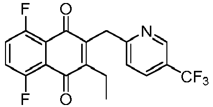

### Example 55 - 2-ethyl-5,8-difluoro-3-((5-(trifluoromethyl)pyridin-2-yl)methyl)naphthalene-1,4-dione

¹H NMR (400 MHz, CDCl₃): *δ* 8.68 (s, 1H), 7.82-7.80 (d, *J* = 8.0 Hz, 1H), 7.46-7.44 (d, *J* = 8.0 Hz, 1H), 7.39-7.35 (m, 2H), 4.20 (s, 2H), 2.79-2.73 (dd, *J* = 15.2 Hz, 7.6 Hz, 2H) and 1.12-1.08 (t, *J* = 7.6 Hz, 3H). MS (*m*/*z*) for C₁₉H₁₂F₅NO₂: found .382 (M+H).

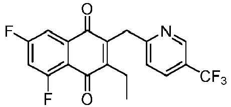

### Example 56 - 3-ethyl-5,7-difluoro-2-((5-(trifluoromethyl)pyridin-2-yl)methyl)naphthalene-1,4-dione

¹H NMR (400 MHz, DMSO-*d₆*): *δ* 8.77 (s, 1H), 8.11-8.09 (d, *J* = 8.4 Hz, 1H), 7.77-7.72 (d, *J* = 9.8 Hz, 2H), 7.62-7.57 (m, 2H), 4.22 (s, 2H), 2.60-2.58 (m, 2H) and 0.95-0.91 (t, *J* = 7.4 Hz, 3H). MS (*m*/*z*) for C₁₉H₁₂F₅NO₂: found .382 (M+H).

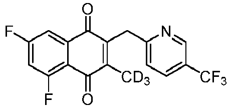

### Example 57 - 5,7-difluoro-3-(methyl-d₃)-2-((5-(trifluoromethyl)pyridin-2-yl)methyl)naphthalene-1,4-dione

¹H NMR (400 MHz, CDCl₃): *δ* 8.69 (s, 1H), 7.83-7.81 (d, *J* = 8.0 Hz, 1H), 7.61-7.59 (d, *J* = 8.4 Hz, 1H), 7.42-7.40 (d, *J* = 8.0 Hz, 1H), 7.12-7.08 (t, *J* = 8.8 Hz, 1H), and 4.22 (s, 2H). MS (*m*/*z*) for C₁₈H₇D₃F₅NO₂: found .371.05 (M+H).

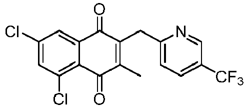

### Example 58 - 5,7-dichloro-3-methyl-2-[[5-(trifluoromethyl)-2-pyridyl]methyl]naphthalene-1,4-dione

¹H NMR (400 MHz, CDCl₃) δ 8.71 (s, 1H), 8.02 (d, *J* = 2.2 Hz, 1H), 7.84 (dd, *J* = 8.2, 2.4 Hz, 1H), 7.71 (d, *J* = 2.2 Hz, 1H), 7.42 (d, *J* = 8.2 Hz, 1H), 4.24 (s, 2H), 2.31 (s, 3H). MS (*m*/*z*) for C₁₈H₁₀Cl₂F₃NO₂: found 400.0 (M).

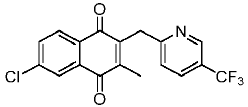

### Example 59 - 6-chloro-3-methyl-2-[[5-(trifluoromethyl)-2-pyridyl]methyl]naphthalene-1,4-dione

¹H NMR (400 MHz, CDCl₃) δ 8.72 (s, 1H), 8.06 (d, *J* = 2.2 Hz, 1H), 8.02 (d, *J* = 8.3 Hz, 1H), 7.85 (dd, *J* = 8.3, 2.3 Hz, 1H), 7.65 (dd, *J* = 8.3, 2.2 Hz, 1H), 7.44 (d, *J* = 8.2 Hz, 1H), 4.28 (s, 2H), 2.30 (s, 3H). MS (*m*/*z*) for C₁₈H₁₁ClF₃NO₂: found 366.2 (M+H).

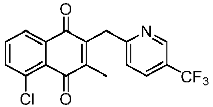

### Example 60 - 5-chloro-3-methyl-2-[[5-(trifluoromethyl)-2-pyridyl]methyl]naphthalene-1,4-dione

¹H NMR (400 MHz, CDCl₃) δ 8.72 (s, 1H), 8.07 (dd, *J* = 7.6, 1.4 Hz, 1H), 7.83 (dd, *J* = 8.2, 2.4 Hz, 1H), 7.72 (dd, *J* = 8.1, 1.4 Hz, 1H), 7.59 (t, *J* = 7.9 Hz, 1H), 7.42 (d, *J* = 8.2 Hz, 1H), 4.25 (s, 2H), 2.31 (s, 3H). MS (*m*/*z*) for C₁₈H₁₁ClF₃NO₂: found 366.2 (M+H).

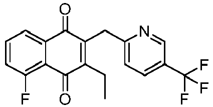

### Example 61 - 3-ethyl-5-fluoro-2-((5-(trifluoromethyl)pyridin-2-yl)methyl)naphthalene-1,4-dione

¹H NMR (400 MHz, CDCl₃) δ 8.69 (s, 1H), 7.89 (d, *J* = 7.6 Hz,1H), 7.81 (d, *J* = 7.6 Hz, 1H), 7.65 (dd, *J* = 12.4, 8.0 Hz, 1H), 7.45 - 7.33 (m, 2H), 4.22 (s, 2H), 2.75 (q, *J* = 7.6 Hz, 2H), 1.09 (t, *J* = 7.6 Hz, 3H). ¹⁹F NMR (400 MHz, CDCl₃) δ -62.38, -112.70. MS (*m*/*z*) for C₁₉H₁₃F₄NO₂: found 364.3 (M+H).

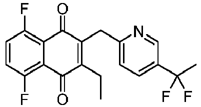

### Example 62 - 2-((5-(1,1-difluoroethyl)pyridin-2-yl)methyl)-3-ethyl-5,8-difluoronaphthalene-1,4-dione

¹H NMR (400 MHz, CDCl3) *δ* 8.56 (s, 1H), 7.70 (d, J = 8.0 Hz, 1H), 7.32-7.38 (m, 3H), 4.16 (s, 2H), 2.74 (q, J = 7.6 Hz, 2H), 1.89 (t, J = 18.4 Hz, 3H), 1.08 (t, J = 7.6 Hz, 3H). ¹⁹F NMR (400 MHz, CDCl3) δ -87.82, -116.16. MS (*m*/*z*) for C₂₀H₁₅F₄NO₂: found 378.3 (M+H).

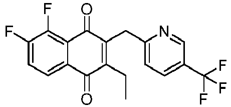

### Example 63 - 2-ethyl-5,6-difluoro-3-((5-(trifluoromethyl)pyridin-2-yl)methyl)naphthalene-1,4-dione

¹H NMR (400 MHz, CDCl₃) δ 8.71 (s, 1H), 7.98 (d, *J* = 4.2 Hz, 1H), 7.84 (d, *J* = 6.8 Hz, 1H), 7.48 (d, *J* = 7.6 Hz, 2H), 4.24 (s, 2H), 2.79 (q, *J* = 7.6 Hz, 2H), 1.11 (d, *J* = 7.6 Hz, 3H). ¹⁹F NMR (376 MHz, CDCl₃) δ -61.82, -126.84, -138.22. MS (*m*/*z*) for C₁₉H₁₂F₅NO₂: found 382.3 (M+H).

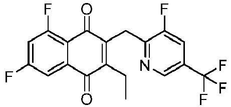

### Example 64 - 2-ethyl-5,7-difluoro-3-((3-fluoro-5-(trifluoromethyl)pyridin-2-yl)methyl)naphthalene-1,4-dione

¹H NMR (400 MHz, *d₆*-DMSO) δ 8.64 (s, 1H), 8.27 (d, *J* = 9.6 Hz, 1H), 7.71 (t, *J* =9.6 Hz, 1H), 7.64 (d, *J* =8.0 Hz 1H), 4.20 (s, 2H), 2.59 (d, *J* = 7.6 Hz, 2H), 0.96 (t, *J* = 7.6 Hz, 3H). ¹⁹F NMR (400MHz, CDCl₃) δ -55.75, -93.81, -117.74. MS (*m*/*z*) for C₁₉H₁₁F₆NO₂: found 400.2 (M+H).

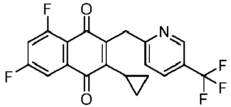

### Example 65 - 2-cyclopropyl-5,7-difluoro-3-((5-(trifluoromethyl)pyridin-2-yl)methyl)naphthalene-1,4-dione

¹H NMR (400 MHz, CDCl₃) δ 8.72 (s, 1H), 7.87 (dd, *J* = 8.4, 2.4 Hz, 1H), 7.58 (dd, *J* = 8.4, 1.5 Hz, 1H), 7.47 (d, *J* = 8.4 Hz, 1H), 7.09 (ddd, *J* = 10.6, 8.4, 2.4Hz, 1H), 4.44 (s, 2H), 1.92-1.95 (m, 1H), 1.25 - 1.20 (m, 2H), 1.09 - 1.03 (m, 2H). ¹⁹F NMR (400MHz, CDCl₃) δ -62.33, -97.482, -97.521, -106.548, -106.884. MS (*m*/*z*) for C₂₀H₁₂F₅NO₂: found 394.2 (M+H).

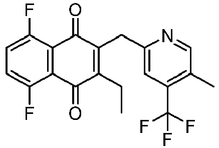

### Example 66 - 2-ethyl-5,8-difluoro-3-((5-methyl-4-(trifluoromethyl)pyridin-2-yl)methyl)naphthalene-1,4-dione

¹H NMR (400 MHz, *d₆*-DMSO) δ 8.50 (s, 1H), 7.64-7.70 (m, 3H), 4.16 (s, 2H), 2.58 (s, 1H), 2.35 (s, 1H), 0.93 (s, 3H). ¹⁹F NMR (400 MHz, *d₆*-DMSO) δ -62.58, -117.23. MS (*m*/*z*) for C₂₀H₁₄F₅NO₂: found 396.1 (M+H).

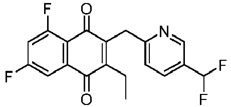

### Example 67 - 3-((5-(difluoromethyl)pyridin-2-yl)methyl)-2-ethyl-5,7-difluoronaphthalene-1,4-dione

¹H NMR (400 MHz, CDCl₃) δ 8.56 (s, 1H), 7.70 (dd, *J* = 9.2, 7.6 Hz, 2H), 7.42 (d, *J* = 8.0 Hz, 1H), 7.09 (s, 1H), 6.64 (t, *J* = 6.0 Hz, 1H), 4.21 (s, 2H), 2.77 (q, *J* = 7.6 Hz, 1H), 1.09 (t, *J* = 7.6 Hz, 3H). ¹⁹F NMR (400 MHz, CDCl₃) δ -97.576, -106.606, -111.903,-112.055. MS (*m*/*z*) for C₁₉H₁₃F₄NO₂: found 364.2 (M+H).

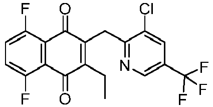

### Example 68 - 2-((3-chloro-5-(trifluoromethyl)pyridin-2-yl)methyl)-3-ethyl-5,8-difluoronaphthalene-1,4-dione

¹H NMR (400 MHz, CDCl₃) δ 8.52 (s, 1H), 7.92 (s, 1H), 7.40 (s, 2H), 4.29 (s, 2H), 2.62 (s, 2H), 1.08 (s, 3H). ¹⁹F NMR (400 MHz, CDCl₃) δ -62.34, -116.11. MS (*m*/*z*) for C₁₉H₁₁ClF₅NO₂: found 416.1 (M+H).

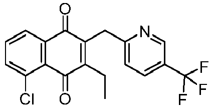

### Example 69 - 5-chloro-3-ethyl-2-((5-(trifluoromethyl)pyridin-2-yl)methyl)naphthalene-1,4-dione

¹H NMR (400 MHz,*d₆*-DMSO) δ 8.77 (s, 1H), 8.09 (d, *J* = 7.6 Hz, 1H), 7.95 (d, *J* = 7.2 Hz, 1H), 7.86 (d, *J* = 7.6 Hz, 1H), 7.75 (t, *J* = 7.6 Hz, 1H), 7.60 (d, *J* = 7.6 Hz, 1H), 4.22 (s, 2H), 2.61 (q, *J* = 7.2 Hz, 2H), 0.94 (t, *J* = 7.2 Hz, 3H). ¹⁹F NMR (400 MHz, *d₆*-DMSO) δ-60.77. MS (*m*/*z*) for C₁₉H₁₃ClF₃NO₂: found 380.2 (M+H).

### Example 70 - 6-((6-fluoro-3-methyl-1,4-dioxo-1,4-dihydronaphthalen-2-yl)methyl)-3-(trifluoromethyl)picolinonitrile

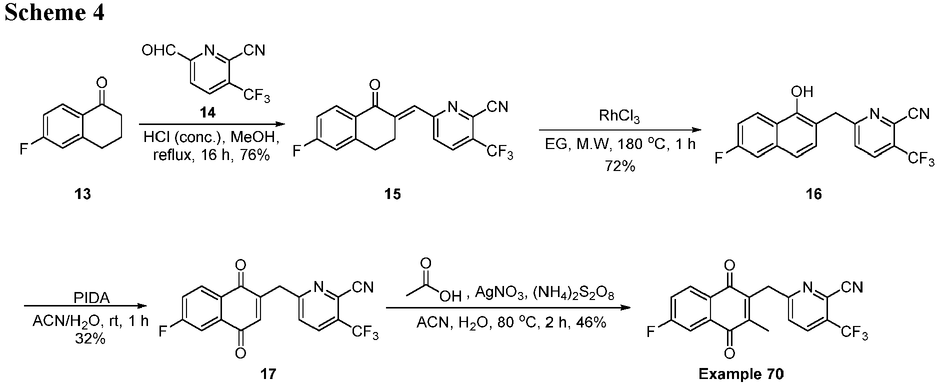

### Step 1 - (E)-6-((6-fluoro-1-oxo-3,4-dihydronaphthalen-2(1H)-ylidene)methyl)-3-(trifluoromethyl)picolinonitrile (15)

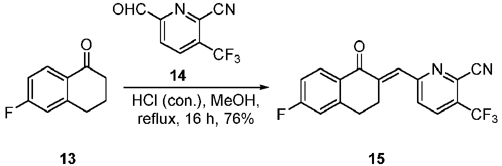

To the mixture of 6-fluoro-3,4-dihydronaphthalen-1(2H)-one (**13**) (1.0 g, 6.1 mmol, 1.0 eq) in methanol (12 mL) was added 6-formyl-3-(trifluoromethyl)picolinonitrile (**14**) (1.46 g, 7.3 mmol, 1.1 eq) and aq. HCl (16 mL). The mixture was stirred at reflux for 16 h. LCMS analysis of the reaction mixture showed full conversion to the desired product. Then the mixture was concentrated, extracted with ethyl acetate (20 mL × 2). The organic layer was dried over sodium sulfate and concentrated under reduced pressure. The residue was purified by column chromatography on a silica gel (petroleum ether: ethyl acetate, 90: 10) to afford (*E*)-6-((6-fluoro-l-oxo-3,4-dihydronaphthalen-2(1H)-ylidene)methyl)-3-(trifluoromethyl)picolinonitrile (**15**) (1.6 g, 76%).

### Step 2 - 6-((6-fluoro-1-hydroxynaphthalen-2-yl)methyl)-3-(trifluoromethyl)picolinonitrile (16)

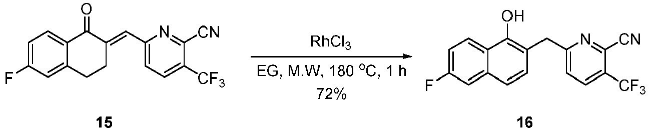

The mixture of (*E*)-6-((6-fluoro-1-oxo-3,4-dihydronaphthalen-2(1H)-ylidene)methyl)-3-(trifluoromethyl)picolinonitrile (**15**) (900 mg, 2.6 mmol, 1.0 eq) and rhodium trichloride (45 mg, 5% w/w) in ethylene glycol (10 mL) was stirred at 180 °C in microwave reactor for 1 h. LCMS analysis of the reaction mixture showed full conversion to the desired product. Then the mixture was concentrated, extracted with ethyl acetate (10 mL x 2). The organic layer was dried over sodium sulfate and concentrated under reduced pressure. The residue was purified by column chromatography on a silica gel (petroleum ether: ethyl acetate, 85:15) to afford 6-((6-fluoro-1-hydroxynaphthalen-2-yl)methyl)-3-(trifluoromethyl)picolinonitrile (**16**) (650 mg, 72%).

### Step 3 - 6-((6-fluoro-1,4-dioxo-1,4-dihydronaphthalen-2-yl)methyl)-3-(trifluoromethyl)picolinonitrile (17)

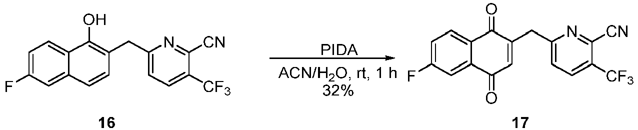

To the mixture of 6-((6-fluoro-1-hydroxynaphthalen-2-yl)methyl)-3-(trifluoromethyl)picolinonitrile (**16**) (300 mg, 0.87 mmol, 1.0 eq) in acetonitrile/water (4 mL/2 mL) was added (Diacetoxyiodo)benzene (558 mg, 1.73 mmol, 2.0 eq). The mixture was stirred at room temperature for 1 h. TLC analysis of the reaction mixture showed the reaction was complete. Then the mixture was diluted with water (10 mL) and extracted with ethyl acetate (10 mL × 2). The organic layer was dried over sodium sulfate, concentrated under reduced pressure and purified by column chromatography on a silica gel (petroleum ether: ethyl acetate, 85:15) to afford 6-((6-fluoro-1,4-dioxo-1,4-dihydronaphthalen-2-yl)methyl)-3-(trifluoromethyl)picolinonitrile (**17**) (100 mg, 32%).

### Step 4 - 6-((6-fluoro-3-methyl-1,4-dioxo-1,4-dihydronaphthalen-2-yl)methyl)-3-(trifluoromethyl)picolinonitrile (Example 70)

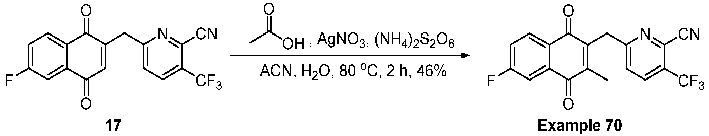

To a mixture of 6-((6-fluoro-1,4-dioxo-1,4-dihydronaphthalen-2-yl)methyl)-3-(trifluoromethyl)picolinonitrile (**17**) (125 mg, 0.35 mmol, 1.0 eq) in acetonitrile/water (4 mL/2 mL) was added acetic acid (41.67 mg, 0.70 mmol, 2.0 eq), ammonium persulfate (159.6 mg, 0.70 mmol, 2.0 eq) and silver nitrate (59.5 mg, 0.35 mmol, 1.0 eq). The mixture was stirred at 80 °C for 2 h until complete by TLC. Then the mixture was cooled to room temperature and extracted with ethyl acetate and water. The combined organic layer was dried over sodium sulfate and concentrated under reduced pressure. This residue was purified by prep-HPLC to afford 6-((6-fluoro-3-methyl-1,4-dioxo-1,4-dihydronaphthalen-2-yl)methyl)-3-(trifluoromethyl)picolinonitrile **(Example 70**) (60 mg, 46%) as yellow solid. ¹H NMR (400 MHz, CDCl₃): *δ* 8.08-8.05 (m, 1H), 8.01-7.99 (d, *J* = 8.0 Hz, 1H), 7.70 (m, 2H), 7.38-7.33 (m, 1H), 4.26 (s, 2H) and 2.34 (s, 3H). MS (*m*/*z*) for C₁₉H₁₀F₄N₂O₂: found 375.05 (M+H).

The following compounds were prepared in a similar manner to **Example 70**:

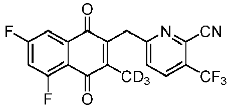

### Example 71 - 6-((5,7-difluoro-3-(methyl-d₃)-1,4-dioxo-1,4-dihydronaphthalen-2-yl)methyl)-3-(trifluoromethyl)picolinonitrile

¹H NMR (400 MHz, CDCl₃): *δ* 8.02-8.00 (d, *J* = 8.4 Hz, 1H), 7.69 (m, 1H), 7.57 (m, 1H), 7.15-7.10 (m, 1H) and 4.24 (s, 2H). MS (*m*/*z*) for C₁₉H₆D₃F₅N₂O₂: found 394.15 (M-H).

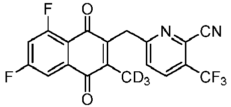

### Example 72 - 6-((6,8-difluoro-3-(methyl-d₃)-1,4-dioxo-1,4-dihydronaphthalen-2-yl)methyl)-3-(trifluoromethyl)picolinonitrile

¹H NMR (400 MHz, CDCl₃): *δ* 8.01-7.99 (d, *J* = 8.4 Hz, 1H), 7.66 (m, 1H), 7.57 (m, 1H), 7.13-7.08 (m, 1H) and 4.23 (s, 2H). MS (*m*/*z*) for C₁₉H₆D₃F₅N₂O₂: found 394.15 (M-H).

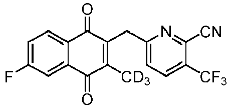

### Example 73 - 6-((6-fluoro-3-(methyl-d₃)-1,4-dioxo-1,4-dihydronaphthalen-2-yl)methyl)-3-(trifluoromethyl)picolinonitrile

¹H NMR (400 MHz, CDCl₃): *δ* 8.08-8.05 (dd, *J* = 8.8 Hz, 5.2 Hz, 1H), 8.01-7.99 (d, *J* = 8.4 Hz, 1H), 7.70 (m, 2H), 7.38-7.24 (m, 1H) and 4.26 (s, 2H). MS (*m*/*z*) for C₁₉H₇D₃F₄N₂O₂: found 378.10 (M+H).

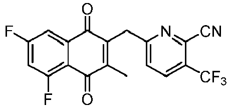

### Example 74 - 6-((5,7-difluoro-3-methyl-1,4-dioxo-1,4-dihydronaphthalen-2-yl)methyl)-3-(trifluoromethyl)picolinonitrile

¹H NMR (400 MHz, CDCl₃): *δ* 8.02-8.00 (d, *J* = 8.8 Hz, 1H), 7.70 (m, 1H), 7.57-7.56 (m, 1H), 7.15-7.10 (m, 1H), 4.24 (s, 2H) and 2.32 (s, 3H). MS (*m*/*z*) for C₁₉H₉F₅N₂O₂: found 391.10 (M-H).

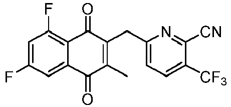

### Example 75 - 6-((6,8-difluoro-3-methyl-1,4-dioxo-1,4-dihydronaphthalen-2-yl)methyl)-3-(trifluoromethyl)picolinonitrile

¹H NMR (400 MHz, CDCl₃): *δ* 8.01-7.99 (d, *J* = 8.0 Hz, 1H), 7.74 (m, 1H), 7.66 (m, 1H), 7.11 (m, 1H), 4.23 (s, 2H) and 2.35 (s, 3H). MS (*m*/*z*) for C₁₉H₉F₅N₂O₂: found 393.40 (M+H).

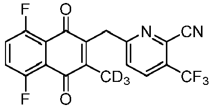

### Example 76 - 6-((5,8-difluoro-3-(methyl-d3)-1,4-dioxo-1,4-dihydronaphthalen-2-yl)methyl)-3-(trifluoromethyl)picolinonitrile

¹H NMR (400 MHz, CDCl₃): *δ* 8.01-7.99 (d, *J* = 8.4 Hz, 1H), 7.73 (m, 1H), 7.41-7.38 (m, 2H) and 4.21 (s, 2H). MS (*m*/*z*) for C₁₉H₆D₃F₅N₂O₂: found 396.05 (M+H).

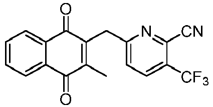

### Example 77 - 6-((3-methyl-1,4-dioxo-1,4-dihydronaphthalen-2-yl)methyl)-3-(trifluoromethyl)picolinonitrile

¹H NMR (400 MHz, CDCl₃): *δ* 8.11-7.98 (m, 3H), 7.70 (m, 1H), 7.72-7.70 (m, 3H), 4.27 (s, 2H) and 2.34 (s, 3H). MS (*m*/*z*) for C₁₉H₁₁F₃N₂O₂: found 357.10 (M+H).

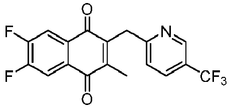

### Example 78 - 6,7-difluoro-2-methyl-3-((5-(trifluoromethyl)pyridin-2-yl)methyl)naphthalene-1,4-dione

¹H NMR (400 MHz, CDCl₃): *δ* 8.70 (s, 1H), 7.88-7.81 (m, 3H), 7.42-7.40 (d, *J* = 8.4 Hz, 1H), 4.24 (s, 2H) and 2.29 (s, 3H). MS (*m*/*z*) for C₁₈H₁₀F₅NO₂: found 368.05 (M+H).

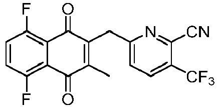

### Example 79 - 6-((5,8-difluoro-3-methyl-1,4-dioxo-1,4-dihydronaphthalen-2-yl)methyl)-3-(trifluoromethyl)picolinonitrile

¹H NMR (400 MHz, CDCl₃): *δ* 8.01-7.99 (d, *J* = 8.0 Hz, 1H), 7.73 (m, 1H), 7.42-7.37 (m, 2H), 4.23 (s, 2H) and 2.32 (s, 3H). MS (*m*/*z*) for C₁₉H₉F₅N₂O₂: found 393.05 (M+H).

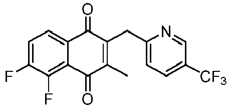

### Example 80 - 5,6-difluoro-3-methyl-2-((5-(trifluoromethyl)pyridin-2-yl)methyl)naphthalene-1,4-dione

¹H NMR (400 MHz, CDCl₃): *δ* 8.69 (s, 1H), 7.91-7.89 (m, 1H), 7.83-7.81 (m, 1H), 7.46-7.40 (m, 2H), 4.23 (s, 2H) and 2.27 (s, 3H). MS (*m*/*z*) for C₁₈H₁₀F₅NO₂: found 368.05 (M+H).

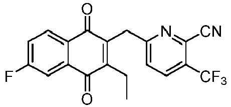

### Example 81 - 6-((3-ethyl-6-fluoro-1,4-dioxo-1,4-dihydronaphthalen-2-yl)methyl)-3-(trifluoromethyl)picolinonitrile

¹H NMR (400 MHz, CDCl₃): *δ* 8.01-8.00 (m, 2H), 7.67 (m, 2H), 7.32-7.27 (m, 1H), 4.20 (s, 2H), 2.79-2.73 (m, 2H) and 1.14-1.10 (t, *J* = 7.4 Hz, 3H). MS (*m*/*z*) for C₂₀H₁₂F₄N₂O₂: found 389.05 (M+H).

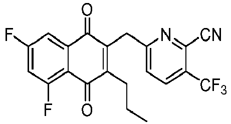

### Example 82 - 6-((5,7-difluoro-1,4-dioxo-3-propyl-1,4-dihydronaphthalen-2-yl)methyl)-3-(trifluoromethyl)picolinonitrile

¹H NMR (400 MHz, CDCl₃): *δ* 8.01-7.80 (d, *J* = 8.4 Hz, 1H), 7.74 (m, 1H), 7.54 (m, 1H), 7.12 (m, 1H), 4.23 (s, 2H), 2.76-2.72 (t, *J* = 7.4 Hz, 2H), 1.55 (s, 2H) and 1.04-1.00 (t, *J* = 7.2 Hz, 3H). MS (*m*/*z*) for C₂₁H₁₃F₅N₂O₂: found 421.10 (M+H).

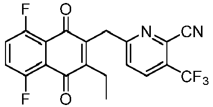

### Example 83 -6-((3-ethyl-5,8-difluoro-1,4-dioxo-1,4-dihydronaphthalen-2-yl)methyl)-3-(trifluoromethyl)picolinonitrile

¹H NMR (400 MHz, CDCl₃): *δ* 8.01-7.99 (d, *J* = 8.4 Hz, 1H), 7.74 (m, 1H), 7.42-7.35 (m, 2H), 4.20 (s, 2H), 2.82-2.76 (m, 2H) and 1.19-1.16 (m, 3H). MS (*m*/*z*) for C₂₀H₁₁F₅N₂O₂: found 407.10 (M+H).

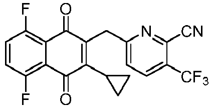

### Example 84 - 6-((3-cyclopropyl-5,8-difluoro-1,4-dioxo-1,4-dihydronaphthalen-2-yl)methyl)-3-(trifluoromethyl)picolinonitrile

¹H NMR (400 MHz, CDCl₃): *δ* 8.01-7.99 (d, *J* = 8.4 Hz, 1H), 7.71 (m, 1H), 7.38-7.34 (m, 2H), 4.38 (s, 2H), 1.93-1.91 (m, 1H), 1.27-1.23 (m, 2H) and 1.11-1.06 (m, 2H). MS (*m*/*z*) for C₂₀H₁₁F₅N₂O₂: found 407.10 (M+H). MS (*m*/*z*) for C₂₁H₁₁F₅N₂O₂: found 419.05 (M+H).

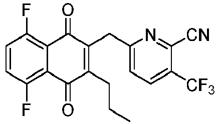

### Example 85 - 6-((5,8-difluoro-1,4-dioxo-3-propyl-1,4-dihydronaphthalen-2-yl)methyl)-3-(trifluoromethyl)picolinonitrile

¹H NMR (400 MHz, CDCl₃): *δ* 8.01-7.99 (d, *J* = 8.4 Hz, 1H), 7.73 (m, 1H), 7.41-7.36 (m, 2H), 4.20 (s, 2H), 2.75-2.71 (m, 2H), 1.56-1.51(m, 2H) and 1.04-1.00 (m, 3H). MS (*m*/*z*) for C₂₁H₁₁F₅N₂O₂: found 419.05 (M+H). MS (*m*/*z*) for C₂₁H₁₃F₅N₂O₂: found 421.10 (M+H).

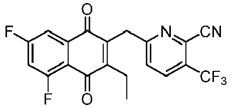

### Example 86 - 6-((3-ethyl-5,7-difluoro-1,4-dioxo-1,4-dihydronaphthalen-2-yl)methyl)-3-(trifluoromethyl)picolinonitrile

¹H NMR (400 MHz, DMSO-*d₆*): *δ* 8.040-8.37 (d, *J* = 8.4 Hz, 1H), 7.96-7.94 (d, *J* = 8.4 Hz, 1H), 7.75 (m, 1H), 7.58-7.56 (m, 1H), 4.26 (s, 2H), 2.65-2.59 (dd, *J* = 14.8 Hz, 7.4 Hz, 2H) and 1.02-0.98 (t, *J* = 7.6 Hz, 3H). MS (*m*/*z*) for C₂₀H₁₁F₅N₂O₂: found 407.10 (M+H).

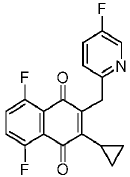

### Example 87 - 2-cyclopropyl-5,8-difluoro-3-((5-fluoropyridin-2-yl)methyl)naphthalene-1,4-dione

¹H NMR (400 MHz, CDCl₃): d 8.29 (d, *J* = 4 Hz, 1H), 7.37 - 7.26 (m, 4H), 4.30 (s, 2H), 1.96 - 1.89 (m, 1H), 1.24 - 1.21 (m, 2H), 1.05 - 1.00 (m, 2H). MS (*m*/*z*) for C₁₉H₁₂F₃NO₂: found 344.2 (M+H).

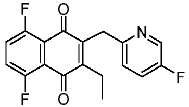

### Example 88 - 2-ethyl-5,8-difluoro-3-((5-fluoropyridin-2-yl)methyl)naphthalene-1,4-dione

¹H NMR (400 MHz, CDCl₃): d 8.29 (s, 1H), 7.39 - 7.35 (m, 2H), 7.32 - 7.30 (m, 2H), 4.13 (s, 2H), 2.77 (q, *J* = 8 Hz, 2H), 1.09 (t, *J* = 8 Hz, 3H). MS (*m*/*z*) for C₁₈H₁₂F₃NO₂: found 332 (M+H).

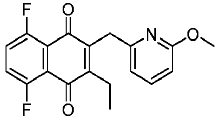

### Example 89 - 2-ethyl-5,8-difluoro-3-((6-methoxypyridin-2-yl)methyl)naphthalene-1,4-dione

¹H NMR (400 MHz, CDCl₃) δ 7.45 (t, *J* = 7.6 Hz, 1H), 7.36 (t, *J* = 6.8 Hz, 2H), 6.86 (d, *J* = 7.2 Hz, 1H), 6.52 (d, *J* = 8.0 Hz, 1H), 4.05 (s, 2H), 3.74 (s, 3H), 2.82 (q, *J* = 7.6 Hz, 2H), 1.09 (t, *J* = 7.6 Hz, 3H). MS (*m*/*z*) for C₁₉H₁₅F₂NO₃: found 344.3 (M+H).

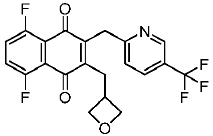

### Example 90 - 5,8-difluoro-2-(oxetan-3-ylmethyl)-3-((5-(trifluoromethyl)pyridin-2-yl)methyl)naphthalene-1,4-dione

¹H NMR (400 MHz, CDCl₃) δ 8.66 (s, 1H), 7.85 (d, *J* = 6.8 Hz, 1H), 7.50 (d, *J* = 8.0 Hz, 1H), 7.39 (s, 2H), 4.72 (t, *J* = 6.4 Hz, 2H), 4.52 (t, *J* = 6.4 Hz, 2H), 4.24 (s, 2H), 3.31 - 3.20 (m, 1H), 3.15 (d, *J* = 6.8 Hz, 2H). MS (m/z) for C₂₁H₁₄F₅NO₃: found 424.3 (M+H).

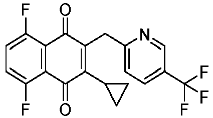

### Example 91 - 2-cyclopropyl-5,8-difluoro-3-((5-(trifluoromethyl)pyridin-2-yl)methyl)naphthalene-1,4-dione

¹H NMR (400 MHz, CDCl₃) δ 8.69 (s, 1H), 7.83 (d, *J* = 7.6 Hz, 1H), 7.43 (d, *J* = 7.6 Hz, 1H), 7.40 - 7.33 (m, 2H), 4.38 (s, 2H), 1.89 (s, 1H), 1.21 (d, *J* = 4.0 Hz, 2H), 1.03 (d, *J* = 4.0 Hz, 2H). MS (*m*/*z*) for C₂₀H₁₂F₅NO₂: found 394.1 (M+H).

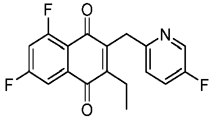

### Example 92 - 2-ethyl-5,7-difluoro-3-((5-fluoropyridin-2-yl)methyl)naphthalene-1,4-dione

¹H NMR (400 MHz, CDCl₃) δ 8.27 (s, 1H), 7.63 (d, *J* = 8.0 Hz, 1H), 7.31 (d, *J* = 5.6 Hz, 2H), 7.09 (dd, *J* = 13.6, 5.2 Hz, 1H), 4.14 (s, 2H), 2.77 (q, *J* = 7.6 Hz, 2H), 1.08 (t, *J* = 7.6 Hz, 3H). ¹⁹F NMR (376 MHz, CDCl₃) δ -97.64, -106.74, -130.30. MS (*m*/*z*) for C₁₈H₁₂F₃NO₂: found 332.3 (M+H).

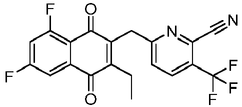

### Example 93 - 6-((3-ethyl-6,8-difluoro-1,4-dioxo-1,4-dihydronaphthalen-2-yl)methyl)-3-(trifluoromethyl)picolinonitrile

¹H NMR (400 MHz, CDCl₃) δ 8.01 (d, *J* = 8.4 Hz, 1H), 7.78 (d, *J* = 8.4 Hz, 1H), 7.67 (d, *J* = 8.0 Hz, 1H), 7.11 (t, *J* = 8.0 Hz, 1H), 4.22 (s, 2H), 2.82 (q, *J* = 7.6 Hz, 2H), 1.18 (t, *J* = 7.6 Hz, 3H). ¹⁹F NMR (400 MHz, CDCl₃) δ -61.75, -96.69, -106.57. MS (*m*/*z*) for C₂₀H₁₁F₅N₂O₂: found 407.3 (M+H).

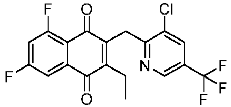

### Example 94 - 3-((3-chloro-5-(trifluoromethyl)pyridin-2-yl)methyl)-2-ethyl-5,7-difluoronaphthalene-1,4-dione

¹H NMR (400 MHz, CDCl₃) δ 8.52 (s, 1H), 7.92 (s, 1H), 7.70 (d, *J* = 8.4 Hz, 1H), 7.11 (t, *J* = 9.6 Hz, 1H), 4.31 (s, 2H), 2.63 (q, *J* = 7.6 Hz, 2H), 1.08 (t, *J* = 7.6 Hz, 3H). ¹⁹F NMR (400 MHz, CDCl₃) δ -62.33, -97.70, -106.62. MS (*m*/*z*) for C₁₉H₁₁ClF₅NO₂: found 416.3 (M+H).

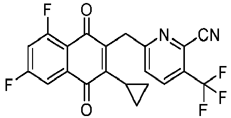

### Example 95 - 6-((3-cyclopropyl-6,8-difluoro-1,4-dioxo-1,4-dihydronaphthalen-2-yl)methyl)-3-(trifluoromethyl)picolinonitrile

¹H NMR (400 MHz, CDCl₃) δ 8.00 (d, *J* = 8.4 Hz, 1H), 7.72 (d, *J* = 8.4 Hz, 1H), 7.57 (d, *J* = 8.4 Hz, 1H), 7.08 (t, *J* = 9.6 Hz, 1H), 4.41 (s, 2H), 1.91-1.97 (m, 1H), 1.24 (d, *J* = 8.4 Hz, 2H), 1.10 (d, *J* = 8.4 Hz, 2H). ¹⁹F NMR (400MHz, CDCl₃) δ -61.76, -96.79, -106.54. MS (*m*/*z*) for C₂₁H₁₁F₅N₂O₂: found 419.2 (M+H).

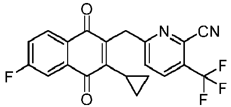

### Example 96 - 6-((3-cyclopropyl-6-fluoro-1,4-dioxo-1,4-dihydronaphthalen-2-yl)methyl)-3-(trifluoromethyl)picolinonitrile

¹H NMR (400 MHz, CDCl₃) δ 8.00-8.03 (m, 2H), 7.64-7.68 (m, 2H), 7.33 (t, *J* = 7.2 Hz, 1H), 4.43 (s, 2H), 1.92-1.96 (m, 1H), 1.28 (d, *J* = 7.2 Hz, 2H), 1.09 (d, *J* = 7.2 Hz, 2H). ¹⁹F NMR (400MHz, CDCl₃) δ -61.78, -102.06. MS (*m*/*z*) for C₂₁H₁₂F₄N₂O₂: found 399.3 (M-H).

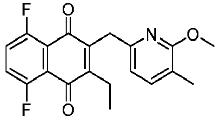

### Example 97 - 2-ethyl-5,8-difluoro-3-((6-methoxy-5-methylpyridin-2-yl)methyl)naphthalene-1,4-dione

¹H NMR (400 MHz, CDCl₃) δ 7.35 (t, *J* = 6.4 Hz, 2H), 7.2 (s, 1H), 6.8 (d, *J* = 7.2 Hz, 1H), 4.02 (s, 2H), 3. 6 (s, 3H), 2.84 (q, *J* = 7.6 Hz, 2H), 2.09 (s, 3H), 1.10 (t, *J* = 7.6 Hz, 3H). ¹⁹F NMR (400 MHz, CDCl₃) δ -116.50. MS (*m*/*z*) for C₂₀H₁₇F₂NO₃: found 358.1 (M-H).

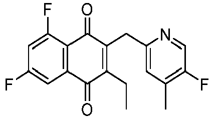

### Example 98 - 2-ethyl-5,7-difluoro-3-((5-fluoro-4-methylpyridin-2-yl)methyl)naphthalene-1,4-dione

¹H NMR (400 MHz, CDCl₃) δ 8.15 (s, 1H), 7.63 (d, *J* = 7.8 Hz, 1H), 7.10 (dd, *J* = 23.0, 7.2 Hz, 2H), 4.08 (s, 2H), 2.77 (q, *J* = 7.6 Hz, 2H), 2.25 (s, 3H), 1.07 (t, *J* = 7.6 Hz, 3H). ¹⁹F NMR (376 MHz, CDCl₃) δ -97.79, -106.77, -136.54. MS (*m*/*z*) for C₁₉H₁₄F₃NO₂: found 346.1 (M-H).

### Example 99: - 6-((3-methyl-1,4-dioxo-1,4-dihydronaphthalen-2-yl)methyl)nicotinic acid and Example 100: - N-cyclopentyl-6-((3-methyl-1,4-dioxo-1,4-dihydronaphthalen-2-yl)methyl)nicotinamide

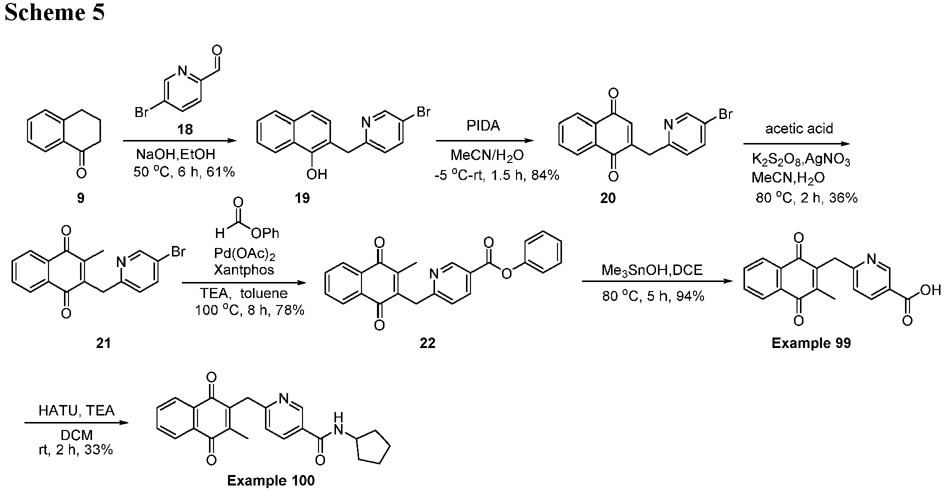

### Step 1 - 2-((5-bromopyridin-2-yl)methyl)naphthalen-1-ol (19)

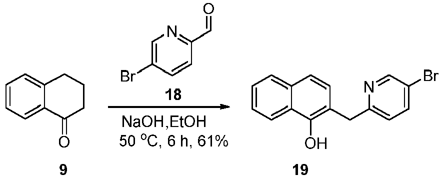

To a solution of 3,4-dihydronaphthalen-1(2H)-one (**9**) (3.19 g, 21.83 mmol, 1.0 eq) in EtOH (30 mL) was added NaOH (1.048 g, 26.20 mmol, 1.2 eq). After being stirred at room temperature for 30 min, 5-bromopicolinaldehyde (**18**) (4.06g, 21.83 mmol, 1.0 eq) was added. Then the mixture was stirred at 50 °C for another 6 h. The reaction was monitored by TLC. Upon completion, the mixture was concentrated under reduced pressure and extracted with EA (3 x 10 mL) and washed with water. The organic layer was washed with brine, dried over Na₂SO₄ and concentrated under reduced pressure. The resultant residue was purified by flash chromatography on silica to provide the title compound (**19**) (4.2 g, 61%) as a yellow solid. ¹H NMR (400 MHz, CDCl₃): δ 11.21 (s, 1H), 8.57 (s, 1H), 8.34(d, 1H), 7.78 (d, 1H), 7.72 (d, 1H), 7.46 (m, 2H), 7.40 (m, 1H), 7.28 (m, 1H), 7.12 (m, 1H), 4.13 (s, 2H).

### Step 2 - 2-((5-bromopyridin-2-yl)methyl)naphthalene-1,4-dione (20)

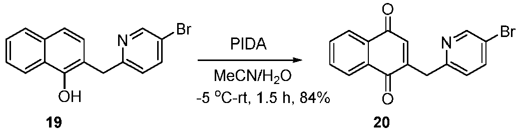

To a solution of 2-((5-bromopyridin-2-yl)methyl)naphthalen-1-ol (**19**) (4.2 g, 13.37 mmol, 1.0 eq) in ACN/H₂O (120 mL/40 mL) was slowly added PIDA (1.048 g, 26.20 mmol, 1.2 eq) at -5 °C. After being stirred at -5 °C for 30 min, the mixture was allowed to warm to room temperature and stirred for 1 h. The reaction was monitored by TLC. To the mixture was added saturated aqueous of NaHCO₃ to adjust to pH = 7 and extracted with EA (3 x 100 mL). The organic layer was washed with water, brine, dried over Na₂SO₄ and concentrated under reduced pressure. The resultant residue was purified by flash chromatography on silica to give 2-((5-bromopyridin-2-yl)methyl)naphthalene-1,4-dione (**20**) (3.7 g, 84%) as a yellow solid. ¹H NMR (400 MHz, CDCl₃): δ 8.59 (s, 1H), 8.06 (m, 2H), 7.77 (m, 3H), 7.24 (m, 1H), 6.78 (s, 1H), 4.03 (s, 2H).

### Step 3 - 2-((5-bromopyridin-2-yl)methyl)-3-methylnaphthalene-1,4-dione (21)

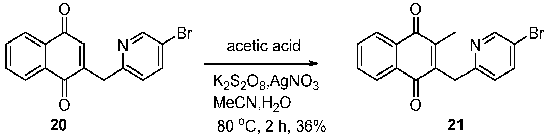

To a solution of 2-((5-bromopyridin-2-yl)methyl)naphthalene-1,4-dione (**20**) (3.7 g, 11.28 mmol, 1.0 eq) and AcOH (1.35 g, 22.56 mmol, 2.0 eq) in ACN (150 mL) was added AgNOs (1.92 g, 11.28 mmol, 1.0 eq). The reaction mixture was stirred at 80 °C for 30 min. Then to the mixture was added K₂S₂O₈ (6.1 g, 22.56 mmol, 2.0 eq) in H₂O (100 mL). The reaction mixture was stirred at 80 °C for another 2 h. The reaction was monitored by TLC. The mixture was extracted with EA (3 x 10 mL) and separated. The organic layer was washed with water, brine, dried over Na₂SO₄ and concentrated. The resultant residue was purified by flash chromatography on silica (PE: EA=100:1-60:1) to give the title compound (**21**) (1.4 g, 36%) as a yellow solid. ¹H NMR (400 MHz, CDCl₃): δ 8.51 (s, 1H), 8.07 (m, 2H), 7.71 (m, 3H), 7.17 (d, 1H), 4.16 (s, 2H), 2.28 (s, 3H).

### Step 4 - phenyl 6-((3-methyl-1,4-dioxo-1,4-dihydronaphthalen-2-yl)methyl)nicotinate (22)

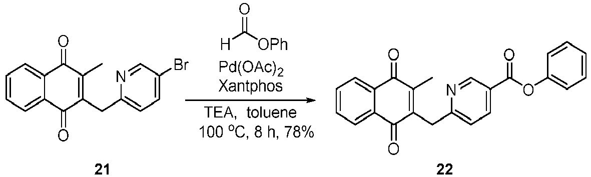

To a solution of 2-((5-bromopyridin-2-yl)methyl)-3-methylnaphthalene-1,4-dione (**21**) (0.8 g, 2.34 mmol, 1.0 eq), phenyl formate (572 mg, 4.68 mmol, 2.0 eq), Xantphos (135 mg, 0.234 mmol, 0.1 eq) and Et₃N (945 mg, 9.36 mmol, 4.0 eq) in toluene (10 mL) was added Pd(OAc)₂ (26.2 mg, 0.117 mmol, 0.05 eq). The mixture was stirred at 100 °C for 8 h under N₂. The reaction was monitored by TLC. Upon completion, the reaction was cooled to room temperature and was filtered through celite. The filtrate was diluted with ethyl acetate and washed with brine. The organic phase was separated, dried over Na₂SO₄ and concentrated. The residue was purified by flash chromatography on silica to give phenyl 6-((3-methyl-1,4-dioxo-1,4-dihydronaphthalen-2-yl)methyl)nicotinate (**22**) (708 mg, 78%) as a yellow solid. ¹H NMR (400 MHz, CDCl₃): δ 9.25 (s, 1H), 8.33 (m, 1H), 8.11 (m, 2H), 7.72 (m, 2H), 7.44 (m, 3H), 7.27 (m, 1H), 7.17 (m, 2H), 4.31 (s, 2H), 2.31 (s, 3H).

### Step 5 - 6-((3-methyl-1,4-dioxo-1,4-dihydronaphthalen-2-yl)methyl)nicotinic acid (Example 99)

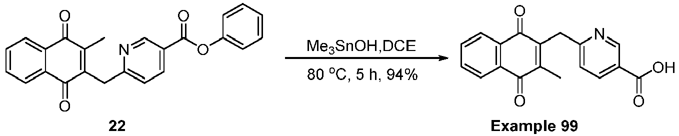

To a solution of phenyl 6-((3-methyl-1,4-dioxo-1,4-dihydronaphthalen-2-yl)methyl)nicotinate (**22**) (115 mg, 0.300 mmol, 1.0 eq) in DCE (2 mL) was added Me₃SnOH (271 mg, 1.50 mmol, 5.0 eq). The reaction mixture was stirred at 80 °C for 5 h. The reaction was monitored by TLC. The mixture was filtered, dried over Na₂SO₄ and concentrated under reduce pressure. The resultant residue was purified by flash chromatography on silica to give 6-((3-methyl-1,4-dioxo-1,4-dihydronaphthalen-2-yl)methyl)nicotinic acid (**Example 99**) (87 mg, 94%) as a yellow solid. ¹H NMR (400 MHz, CDCl₃): δ 9.13 (s, 1H), 8.23 (dd, *J* = 2.0, 8.4 Hz, 1H), 8.12-8.07 (m, 2H), 7.71 (t, *J* = 4.4 Hz, 2H), 7.39 (d, *J* = 8.0 Hz, 1H), 4.30 (s, 2H), 2.29 (s, 3H). MS (m/z) for C₁₈H₁₃NO₄: found 308.10 (M+H).

### Step 6 - N-cyclopentyl-6-((3-methyl-1,4-dioxo-1,4-dihydronaphthalen-2-yl)methyl)nicotinamide (Example 100)

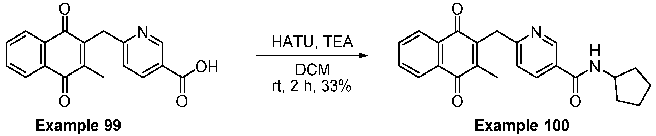

To a solution of 6-((3-methyl-1,4-dioxo-1,4-dihydronaphthalen-2-yl)methyl)nicotinic acid (**Example 99**) (100 mg, 0.326 mmol, 1.0 eq) in DCM (2 mL) was added cyclopentanamine (34 mg, 0.391 mmol, 1.2 eq), HATU (149 mg, 0.391 mmol, 1.2 eq) and TEA (66 mg, 0.652 mmol, 2.0 eq). The reaction mixture was stirred at rt for 2 h. The mixture was filtered and the solids were washed with EA (3 × 2 mL). The organic layer was washed with brine. The residue was dried over Na₂SO₄ and concentrated under reduced pressure. The resultant residue was purified by prep-TLC to give N-cyclopentyl-6-((3-methyl-1,4-dioxo-1,4-dihydronaphthalen-2-yl)methyl)nicotinamide (**Example 100**)(41 mg, 33%) as yellow solid. ¹H NMR (400 MHz, CDCl₃): δ 8.77 (d, *J* = 1.6 Hz, 1H), 8.11-8.06 (m, 2H), 7.98 (dd, *J* = 7.6, 2.4 Hz, 2H), 7.73-7.69 (m, 2H), 7.33 (d, *J* = 8.0 Hz, 1H), 5.95-5.94 (m, 1H), 4.37 (q, *J* = 7.2 Hz, 1H) , 4.25 (s, 24H), 2.27 (s, 3H), 2.11-2.04 (m, 2H), 1.74-1.62 (m, 4H), 1.50-144 (m, 2H). MS (m/z) for C₂₃H₂₂N₂O₃: found 375.15 (M+H).

The following compounds were prepared in a similar manner to **Example 100**:

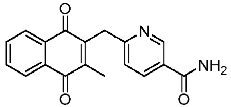

### Example 101 - 6-((3-methyl-1,4-dioxo-1,4-dihydronaphthalen-2-yl)methyl)nicotinamide

¹H NMR (400 MHz, CDCl₃): δ 8.83 (s, 1H), 8.10-8.08 (m, 1H), 8.03-8.00 (m, 2H), 7.98-7.96 (m, 1H), 7.84-7.82 (m, 2H), 7.49 (s, 1H), 7.41 (d, *J* = 8.4 Hz, 1H), 4.19 (s, 2H), 2.12 (s, 3H). MS (*m*/*z*) for C₁₈H₁₄N₂O₃: found 307.05 (M+H).

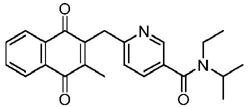

### Example 102 - N-ethyl-N-isopropyl-6-((3-methyl-1,4-dioxo-1,4-dihydronaphthalen-2-yl)methyl)nicotinamide

¹H NMR (400 MHz, CD₃OD): δ 8.40 (s, 1H), 8.09-8.03 (m, 2H), 7.78-7.72 (m, 3H), 7.44 (d, *J* = 8.4 Hz, 1H), 4.27 (s, 2H), 3.88 (s, 1H), 3.45-3.43(m, 2H), 2.21 (s, 3H), 1.26-1.17 (m, 9H). MS (*m*/*z*) for C₂₃H₂₄N₂O₃: found 377.30 (M+H).

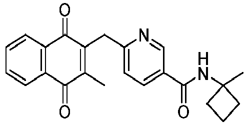

### Example 103 - 6-((3-methyl-1,4-dioxo-1,4-dihydronaphthalen-2-yl)methyl)-N-(1-methylcyclobutyl)nicotinamide

¹H NMR (400 MHz, DMSO-*d₆*): δ 8.77 (s, 1H), 8.44 (s, 1H), 8.05-7.96 (m, 3H), 7.84-7.82 (m, 2H), 7.39 (d, *J* = 8.0 Hz, 1H), 4.18 (s, 2H), 2.30-2.27 (m, 2H), 2.11 (s, 3H), 1.95-1.92 (m, 2H), 1.79-1.75 (m, 2H), 1.42 (s, 3H). MS (*m*/*z*) for C₂₃H₂₂N₂O₃: found 375.20 (M+H).

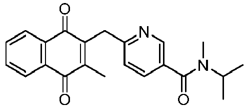

### Example 104 - N-isopropyl-N-methyl-6-((3-methyl-1,4-dioxo-1,4-dihydronaphthalen-2-yl)methyl)nicotinamide

¹H NMR (400 MHz, CD₃OD): *δ* 8.43 (s, 1H), 8.10-8.03 (m, 2H), 7.80-7.74 (m, 3H), 7.46-7.44 (m, 1H), 4.28 (s, 2H), 3.94-3.87 (m, 3H), 2.21 (s, 3H) and 1.23-1.17 (m, 6H). MS (*m*/*z*) for C₂₂H₂₂N₂O₃: found 363.15 (M+H).

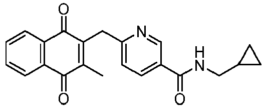

### Example 105 - N-(cyclopropylmethyl)-6-((3-methyl-1,4-dioxo-1,4-dihydronaphthalen-2-yl)methyl)nicotinamide

¹H NMR (400 MHz, CDCl₃): *δ* 8.82-8.81 (d, *J* = 1.2 Hz, 1H), 8.12-8.06 (m, 2H), 8.03-8.01 (dd, *J* = 7.6 Hz, 2.2 Hz, 1H), 7.73-7.69 (m, 2H), 7.36-7.34 (d, *J* = 8.0 Hz, 1H), 6.13 (m, 1H), 4.26 (s, 2H), 3.32-3.28 (m, 2H), 2.28 (s, 3H), 1.06-1.04 (m, 1H), 0.57-0.53 (m, 2H) and 0.28-0.25 (m, 2H). MS (*m*/*z*) for C₂₂H₂₀N₂O₃: found 361.25 (M+H).

### Example 106 - 2-((5-chloropyridin-2-yl)methyl)-3-propylnaphthalene-1,4-dione

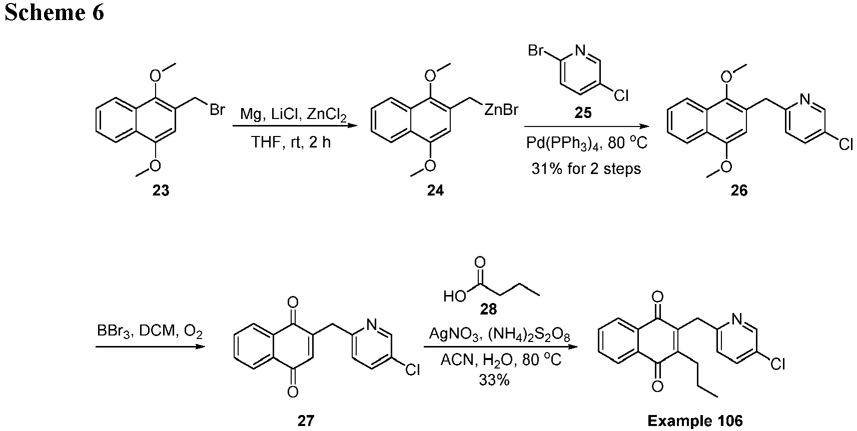

### Step 1 - ((1,4-dimethoxynaphthalen-2-yl)methyl)zinc(II) bromide (24)

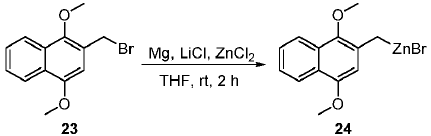

To a mixture of Mg (120 mg, 5.0 mmol, 2.5 eq) and LiCl (109 mg, 2.6 mmol, 1.3 eq) was added ZnCl₂ (0.5 M in THF, 4.4 mL, 2.2 mmol, 1.1 eq) under nitrogen atmosphere. Then a solution of 2-(bromomethyl)-1,4-dimethoxynaphthalene (**23**) in THF (560 mg, 2.0 mmol, 1.0 eq) was added dropwise. The reaction mixture was stirred at room temperature for 2 h. The reaction was monitored by ¹H NMR. The solution was used in next step directly without isolation or purification.

### Step 2 - 5-chloro-2-((1,4-dimethoxynaphthalen-2-yl)methyl)pyridine (26)

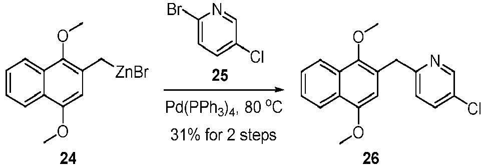

To a solution of 2-bromo-5-chloropyridine (**25**) (458 mg, 2.4 mmol, 1.2 eq) and Pd(PPh₃)₄ (116 mg, 0.1 mmol, 0.05 eq) in THF (3 mL) was slowly added the solution from the previous step (**24**). The mixture was stirred at 80 °C for 16 h under N₂. The reaction was monitored by TLC until completion. The mixture was concentrated, diluted with EA and H₂O, and extracted with EA (3 × 100 mL). The organic layer was separated, washed with brine, dried over Na₂SO₄ and concentrated. The resultant residue was purified by flash chromatography on silica to give 5-chloro-2-((1,4-dimethoxynaphthalen-2-yl)methyl)pyridine (**26**) (200 mg, 31%).

### Step 3 - 2-((5-chloropyridin-2-yl)methyl)naphthalene-1,4-dione (27)

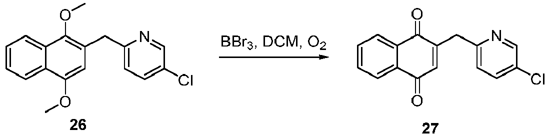

To a mixture of 5-chloro-2-((1,4-dimethoxynaphthalen-2-yl)methyl)pyridine (**26**) (180 mg, 0.575 mmol, 1.0 eq) in DCM (2 mL) was added BBr₃ (0.5 mL) at 0 °C. The mixture was stirred at rt for 2 h. Then the mixture was quenched with aq. NaHCO₃ (5 mL) and extracted with EA (2 × 5 mL). The organic layer was washed with brine, dried over Na₂SO₄, and filtered. This solution was stirred at rt for 24 h under O₂. The resulting mixture was concentrated. The residue was purified by Prep-TLC to give 2-((5-chloropyridin-2-yl)methyl)naphthalene-1,4-dione (**27**) (100 mg, 61%).

### Step 4 - 2-((5-chloropyridin-2-yl)methyl)-3-propylnaphthaline-1,4-dione (Example 106)

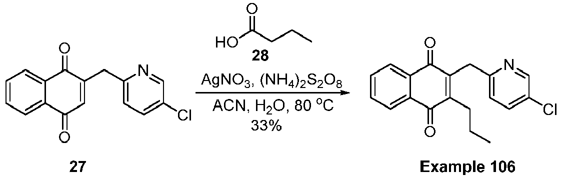

To a solution of 2-((5-chloropyridin-2-yl)methyl)naphthalene-1,4-dione (27) (100 mg, 0.353 mmol, 1.0 eq) and butyric acid (**28**) (155 mg, 1.77 mmol, 5.0 eq) in ACN/H₂O (4 mL/ 1 mL) was added AgNO₃ (60 mg, 0.353 mmol, 1.0 eq). The reaction mixture was stirred at 80 °C for 30 min. To this mixture was added (NH₄)₂S₂O₈ (201 mg, 0.883 mmol, 2.5 eq) in ACN/H₂O (3 mL/ 1 mL). The resulting mixture was stirred at 80 °C for another 1 h. Upon completion, the mixture was extracted with EA (3 × 10 mL) and separated. The organic layer was washed with water and brine. Then the organic layer was dried over Na₂SO₄ and concentrated. The resultant residue was purified by flash chromatography on silica to give compound 2-((5-chloropyridin-2-yl)methyl)-3-propylnaphthalene-1,4-dione (**Example 106**) (38 mg, 33%) as a yellow solid. ¹H NMR (400 MHz, CD₃OD): δ 8.71 (d, *J* = 2.4 Hz, 1H), 8.06-8.02 (m, 2H), 7.77-7.72 (m, 3H), 7.34 (d, *J* = 8.4 Hz, 1H), 4.20 (s, 2H), 2.66 (t, *J* = 8.0 Hz, 2H), 1.44-1.38 (m, 2H), 0.95(t, *J* = 7.2 Hz, 3H). MS (*m*/*z*) for C₁₉H₁₆ClNO₂: found 326.15 (M+H).

The following compounds were prepared in a similar manner to **Example 106**:

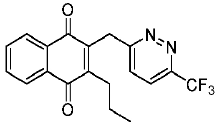

### Example 107 - 2-propyl-3-((6-(trifluoromethyl)pyridazin-3-yl)methyl)naphthalene-1,4-dione

¹H NMR (400 MHz, CD₃OD) δ 8.08 (d, *J* = 6.4 Hz, 1H), 8.04-8.01 (m, 1H), 7.95 (d, *J* = 8.4 Hz, 2H), 7.79-7.76 (m, 2H), 4.48 (s, 2H), 2.77-2.73 (m, 2H), 1.50-1.48 (m, 2H), 0.99 (t, *J* = 7.6 Hz, 3H). MS (*m*/*z*) for C₁₉H₁₅F₃N₂O₂: found 361.25 (M+H).

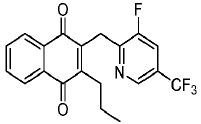

### Example 108 - 2-((3-fluoro-5-(trifluoromethyl)pyridin-2-yl)methyl)-3-propylnaphthalene-1,4-dione

¹H NMR (400 MHz, CDCl₃): δ 8.48 (s, 1H), 8.13-8.11 (m, 1H), 8.04-8.02 (m, 1H), 7.72-7.69 (m, 2H), 7.60 (d, *J* = 8.8 Hz, 1H), 4.26 (s, 2H), 2.68-2.64 (m, 2H), 1.52-1.46 (m, 2H), 0.98 (t, *J* = 7.6 Hz, 3H). MS (*m*/*z*) for C₂₀H₁₅F₄NO₂: found 378.10 (M+H).

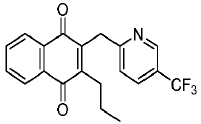

### Example 109 - 2-propyl-3-((5-(trifluoromethyl)pyridin-2-yl)methyl)naphthalene-1,4-dione

¹H NMR (400 MHz, CDCl₃): δ 8.72 (s, 1H), 8.08 (m, 2H), 7.82 (d, *J* = 8.0 Hz, 1H), 7.71-7.70 (m, 2H), 7.42 (d, *J* = 8.0 Hz, 1H), 4.27 (s, 2H), 2.76-2.72 (m, 2H), 1.52-1.42 (m, 2H), 1.00 (t, *J* = 6.8 Hz, 3H). MS (*m*/*z*) for C₂₀H₁₆F₃NO₂: found 360.20 (M+H).

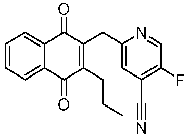

### Example 110 - 2-((1,4-dioxo-3-propyl-1,4-dihydronaphthalen-2-yl)methyl)-5-fluoroisonicotinonitrile

¹H NMR (400 MHz, CD₃OD): δ 8.56 (s, 1H), 8.09-8.06 (m, 1H), 8.03-8.01 (m, 1H), 7.82-7.75 (m, 3H), 4.25 (s, 2H), 2.73-2.69 (m, 2H), 1.49-1.47 (m, 2H), 0.98 (t, *J* = 7.6 Hz, 3H). MS (*m*/*z*) for C₂₀H₁₅FN₂O₂: found 333.05 (M-H).

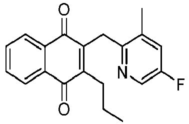

### Example 111 - 2-((5-fluoro-3-methylpyridin-2-yl)methyl)-3-propylnaphthalene-1,4-dione

¹H NMR (400 MHz, CDCl₃): δ 8.12-8.10 (m, 1H), 8.06-8.01 (m, 2H), 7.19 (dd, *J* = 2.4, 8.8 Hz, 1H), 4.05 (s, 2H), 2.64-2.60 (m, 2H), 2.48 (s, 3H), 1.49-1.43 (m, 2H), 0.95 (t, *J* = 7.2 Hz, 3H). MS (*m*/*z*) for C₂₀H₁₈FNO₂: found 324.20 (M+H).

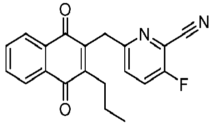

### Example 112 - 6-((1,4-dioxo-3-propyl-1,4-dihydronaphthalen-2-yl)methyl)-3-fluoropicolinonitrile

¹H NMR (400 MHz, CDCl₃): δ 8.16-8.11 (m, 1H), 8.10-8.02 (m, 1H), 7.74-7.70 (m, 2H), 7.62 (dd, *J* = 4.0, 8.8 Hz, 1H), 7.50 (t, *J* = 8.8 Hz, 1H), 4.19 (s, 2H), 2.78-2.74 (m, 2H), 1.54-1.48 (m, 2H), 1.04 (t, *J* = 7.2 Hz, 3H). MS (*m*/*z*) for C₂₀H₁₅FN₂O₂: found 335.15 (M+H).

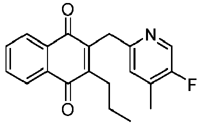

### Example 113 - 2-((5-fluoro-4-methylpyridin-2-yl)methyl)-3-propylnaphthalene-1,4-dione

¹H NMR (400 MHz, CDCl₃): δ 8.18 (s, 1H), 8.10-8.06 (m, 2H), 7.71-7.69 (m, 2H), 7.08 (d, *J* = 6.4 Hz, 1H), 4.14 (s, 2H), 2.75-2.71 (m, 2H), 2.25 (s, 3H) , 1.48-1.42 (m, 2H), 0.99 (t, *J* = 7.2 Hz, 3H). MS (*m*/*z*) for C₂₀H₁₈FNO₂: found 324.20 (M+H).

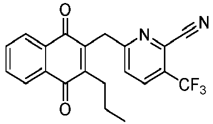

### Example 114 - 6-((1,4-dioxo-3-propyl-1,4-dihydronaphthalen-2-yl)methyl)-3-(trifluoromethyl)picolinonitrile

¹H NMR (400 MHz, CDCl₃): δ 8.11-8.08 (m, 1H), 8.02-7.99 (m, 2H), 7.74-7.68 (m, 3H), 4.27 (s, 2H), 2.79-2.75 (m, 2H), 1.57-1.51 (m, 2H), 1.04 (t, *J* = 7.2 Hz, 3H). MS (*m*/*z*) for C₂₁H₁₅F₃N₂O₂: found 385.10 (M+H).

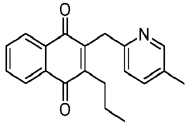

### Example 115 - 2-((5-methylpyridin-2-yl)methyl)-3-propylnaphthalene-1,4-dione

¹H NMR (400 MHz, CDCl₃): δ 8.29 (s, 1H), 8.10-8.05 (m, 2H), 7.69 (t, *J* = 4.0 Hz, 2H), 7.37 (dd, *J* = 8.0, 2.0 Hz, 1H), 7.09 (d, *J* = 8.0 Hz, 1H), 4.17 (s, 2H), 2.74-2.70 (m, 2H) , 2.26 (s, 3H), 1.44 (q, *J* = 8.0 Hz, 2H), 0.97 (t, *J* = 7.2 Hz, 3H). MS (*m*/*z*) for C₂₀H₁₉NO₂: found 306.20 (M+H).

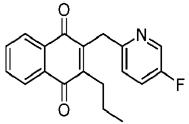

### Example 116 - 2-((5-fluoropyridin-2-yl)methyl)-3-propylnaphthalene-1,4-dione

¹H NMR (400 MHz, CDCl₃): δ 8.31 (d, *J* = 2.4 Hz, 1H), 8.10-8.05 (m, 2H), 7.71-7.69 (m, 2H), 7.31-7.28 (m, 2H), 4.19 (s, 2H), 2.73 (t, *J* = 8.0 Hz, 2H), 1.45 (q, *J* = 7.6 Hz, 2H), 0.99 (t, *J=* 7.2 Hz, 3H). MS *(m*/*z)* for C₁₉H₁₆FNO₂: found 310.20 (M+H).

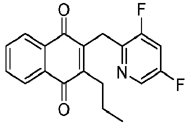

### Example 117 - 2-((3,5-difluoropyridin-2-yl)methyl)-3-propylnaphthalene-1,4-dione

¹H NMR (400 MHz, CDCl₃): δ 8.13 (d, *J* = 2.0 Hz, 1H), 8.11-8.09 (m, 1H), 8.05-8.03 (m, 1H), 7.72-7.66 (m, 2H), 7.18 (td, *J* = 8.4, 2.4 Hz, 1H), 4.18 (s, 2H), 2.64 (t, *J* = 8.0 Hz, 2H), 1.46 (q, *J* = 7.6 Hz, 2H), 0.96 (t, *J* = 7.2 Hz, 3H). MS (*m*/*z*)) for C₁₉H₁₅F₂NO₂: found 328.10 (M+H).

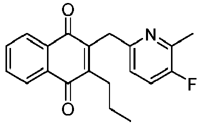

### Example 118 - 2-((5-fluoro-6-methylpyridin-2-yl)methyl)-3-propylnaphthalene-1,4-dione

¹H NMR (400 MHz, CDCl₃): δ 8.08-8.06 (m, 2H), 7.71-7.69 (m, 2H), 7.18 (t, *J* = 8.8 Hz, 1H), 7.02-7.01 (m, 1H), 4.15 (s, 2H), 2.74 (t, *J* = 8.0 Hz, 2H), 2.44 (s, 3H), 1.48-1.42 (m, 2H), 0.99 (t, *J* = 7.2 Hz, 3H). MS (*m*/*z*) for C₂₀H₁₈FNO₂: found 324.20 (M+H).

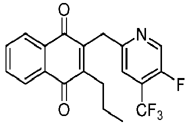

### Example 119 - 2-((5-fluoro-4-(trifluoromethyl)pyridin-2-yl)methyl)-3-propylnaphthalene-1,4-dione

¹H NMR (400 MHz, CDCl₃): δ 8.46 (s, 1H), 8.10-8.05 (m, 2H), 7.72-7.71 (m, 2H), 7.54 (d, *J* = 4.4 Hz, 1H), 4.23 (s, 2H), 2.76-2.73 (m, 2H), 1.52-1.46 (m, 2H), 1.03-0.99 (m, 3H). MS (*m*/*z*) for C₂₀H₁₅F₄NO₂: found 378.05 (M+H).

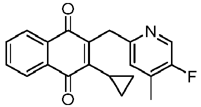

### Example 120 - 2-cyclopropyl-3-((5-fluoro-4-methylpyridin-2-yl)methyl)naphthalene-1,4-dione

¹H NMR (400 MHz, CDCl₃): δ 8.18 (s, 1H), 8.05-7.99 (m, 2H), 7.70-7.65 (m, 2H), 7.07 (d, *J* = 6.4 Hz, 1H), 4.30 (s, 2H), 2.25 (s, 3H), 1.99-1.91 (m, 1H), 1.30-1.26 (m, 2H), 1.04-0.99 (m, 2H). MS (*m*/*z*) for C₂₀H₁₆FNO₂: found 322.20 (M+H).

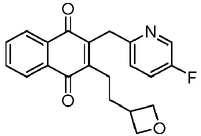

### Example 121 - 2-((5-fluoropyridin-2-yl)methyl)-3-(2-(oxetan-3-yl)ethyl)naphthalene-1,4-dione

¹H NMR (400 MHz, CDCl₃): *δ* 8.30-8.29 (t, *J* = 1.8 Hz, 1H), 8.10-8.05 (m, 2H), 7.73-7.68 (m, 2H), 7.32-7.26 (m, 2H), 4.82-4.78 (m, 2H), 4.44-4.41 (m, 2H), 4.16 (s, 2H), 3.06-3.02 (m, 1H), 2.73-2.69 (m, 2H) and 1.88-1.83 (m, 2H). MS (*m*/*z*) for C₂₁H₁₈FNO₃: found 352.15 (M+H).

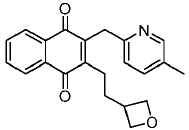

### Example 122 - 2-((5-methylpyridin-2-yl)methyl)-3-(2-(oxetan-3-yl)ethyl)naphthalene-1,4-dione

¹H NMR (400 MHz, CDCl₃): *δ* 8.28 (m, 1H), 8.09-8.06 (m, 2H), 7.71-7.69 (m, 2H), 7.40-7.38 (m, 1H), 7.15-7.13 (d, *J* = 8.0 Hz, 1H), 4.79-4.76 (m, 2H), 4.42-4.39 (t, *J* = 6.0 Hz, 1H), 4.15 (s, 2H), 3.04-3.01 (m, 1H), 2.71-2.67 (m, 2H), 2.62 (s, 3H) and 1.85-1.79 (m, 3H). MS (*m*/*z*) for C₂₂H₂₁NO₃: found 348.20 (M+H).

### Example 123 -6-((3-methyl-1,4-dioxo-1,4-dihydronaphthalen-2-yl)methyl)nicotinonitrile

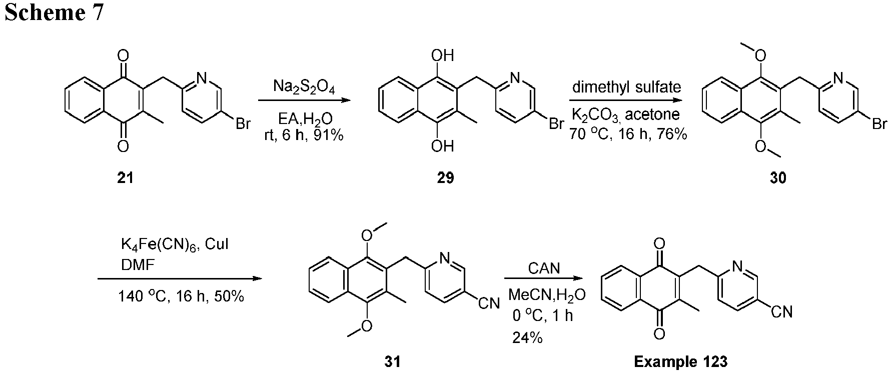

### Step 1 - 2-((5-bromopyridin-2-yl)methyl)-3-methylnaphthalene-1,4-diol (29)

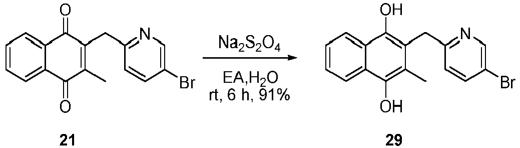

To a solution of 2-((5-bromopyridin-2-yl)methyl)-3-methylnaphthalene-1,4-dione (21) (500 mg, 11.63 mmol, 1.0 eq) in EtOAc (5 mL) was added a solution of Na₂S₂O₄ (763 mg, 4.38 mmol, 3.0 eq) in H₂O (5 mL) under nitrogen atmosphere. The reaction mixture was stirred at room temperature for 6 h. The reaction was monitored by TLC until completion. The mixture was washed with water and extracted with EA (3 × 30 mL). The organic layer was dried over anhydrous Na₂SO₄ and concentrated under reduced pressure to give crude 2-((5-bromopyridin-2-yl)methyl)-3-methylnaphthalene-1,4-diol (**29**)(460 mg, 91%) as a yellow oil. This material was used directly in the next step.

### Step 2 - 5-bromo-2-((1,4-dimethoxy-3-methylnaphthalen-2-yl)methyl)pyridine (30)

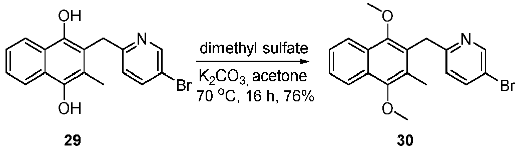

To a solution of 2-((5-bromopyridin-2-yl)methyl)-3-methylnaphthalene-1,4-diol (**29**) (460 mg, 1.16 mmol, 1.0 eq) and K₂CO₃ (641 mg, 4.64 mmol, 4.0 eq) in acetone (5 mL) was added dimethyl sulfate (366 mg, 2.90 mmol, 2.5 eq) under nitrogen atmosphere. The reaction mixture was stirred at 70 °C for 16 h. Upon completion, the mixture was quenched with saturated aqueous NaHCO₃ and extracted with EA (3 x 30 mL). The organic layer was dried over anhydrous Na₂SO₄ and concentrated under reduced pressure. The residue was purified by silica gel chromatography to give 5-bromo-2-((1,4-dimethoxy-3-methylnaphthalen-2-yl)methyl)pyridine (**30**) (330 mg, 76%) as a yellow oil. ¹H NMR (400 MHz, CDCl₃): δ 8.59 (d, 1H), 8.08 (m, 2H), 7.69 (d, 1H), 7.51 (m, 2H), 6.82 (d, 1H), 4.38 (s, 2H), 3.87 (s, 3H), 3.85 (s, 3H), 2.25 (s, 3H).

### Step 3 - 6-((1,4-dimethoxy-3-methylnaphthalen-2-yl)methyl)nicotinonitrile (31)

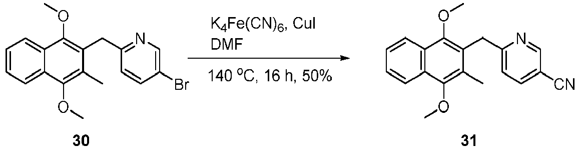

To a solution of 5-bromo-2-((1,4-dimethoxy-3-methylnaphthalen-2-yl)methyl)pyridine (**30**) (230 mg, 0.620 mmol, 1.0 eq) and CuI (59 mg, 0.311 mmol, 0.5 eq) in DMF (5 mL) was added K₄Fe(CN)₆ (262 mg, 0.620 mmol, 1.0 eq) under nitrogen atmosphere. The mixture was stirred at 140 °C for 16 h. TLC analysis of the reaction mixture showed disappearance of starting material. Then the mixture was filtered through a pad of celite and the filtrate was diluted with water and extracted with EA. The organic layer was dried over anhydrous Na₂SO₄ and concentrated under reduced pressure. The residue was purified by silica gel chromatography to afford 6-((1,4-dimethoxy-3-methylnaphthalen-2-yl)methyl)nicotinonitrile (**31**) (100 mg, 50%) as a yellow oil. ¹H NMR (400 MHz, CDCl₃): δ 8.81 (d, 1H), 8.08 (m, 2H), 7.77 (m, 1H), 7.52 (m, 2H), 7.11 (d, 1H), 4.49 (s, 2H), 3.88 (s, 3H), 3.86 (s, 3H), 2.26 (s, 3H).

### Step 4 - 6-((3-methyl-1,4-dioxo-1,4-dihydronaphthalen-2-yl)methyl)nicotinonitrile (Example 123)

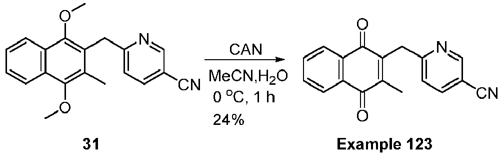

To a solution of 6-((1,4-dimethoxy-3-methylnaphthalen-2-yl)methyl)nicotinonitrile (**31**) (120 mg, 0.377 mmol, 1.0 eq) in ACN/H₂O (2 mL/ 1 mL) was added a solution of CAN (620 mg, 1.13 mmol, 3.0 eq) in H₂O at 0 °C under nitrogen atmosphere. The mixture was stirred at 0 °C for 1 h. TLC analysis of the reaction mixture showed full conversion to the desired product. Then the mixture was diluted with water and extracted with ethyl acetate. The organic layer was dried over anhydrous Na₂SO₄ and concentrated under reduced pressure. The residue was purified by prep-TLC to afford compound 6-((3-methyl-1,4-dioxo-1,4-dihydronaphthalen-2-yl)methyl)nicotinonitrile (**Example 123**) (26 mg, 24%) as a yellow solid. ¹H NMR (400 MHz, CDCl₃): δ 8.72 (s, 1H), 8.12-8.10 (m, 1H), 8.07-8.05 (m, 1H), 7.86 (dd, *J* = 2.0, 8.4 Hz, 1H), 7.71 (t, *J* = 3.8 Hz, 2H), 7.44 (d, *J* = 8.4 Hz, 1H), 4.26 (s, 2H), 2.3013 (s, 3H). MS (*m*/*z*) for C₁₈H₁₂N₂O₂: found 288.95 (M+H).

### Example 124 - 2-(methoxy(5-(trifluoromethyl)pyridin-2-yl)methyl)-3-methylnaphthalene-1,4-dione Scheme 8

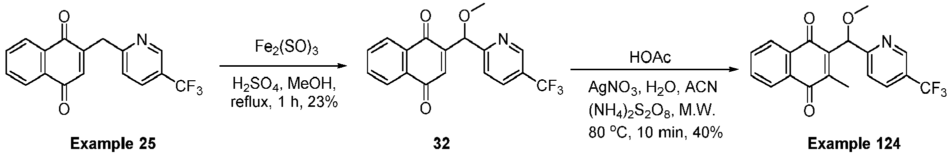

### Step 1 - 2-(methoxy(5-(trifluoromethyl)pyridin-2-yl)methyl)naphthalene-1,4-dione (32)

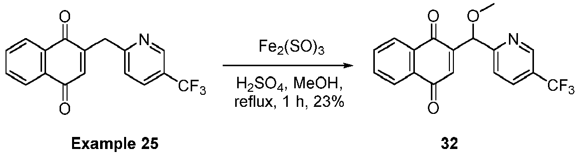

To a solution of 2-((5-(trifluoromethyl)pyridin-2-yl)methyl)naphthalene-1,4-dione (**Example 25**) (100 mg, 0.315 mmol, 1.0 eq) in MeOH (15 mL) was added H₂SO₄ (1 mL) and Fe₂(SO)₃ (252 mg, 0.63 mmol, 2.0 eq). The mixture was stirred at reflux for 1 h. The reaction was monitored by LCMS. Then the reaction mixture was poured into ice water (20 mL), and extracted with EA (2 × 5 mL). The organic layer was washed with brine. The mixture was dried over Na₂SO₄ and concentrated. The residue was purified by Prep-TLC (PE:EA = 6:1) to give 2-(methoxy(5-(trifluoromethyl)pyridin-2-yl)methyl)naphthalene-1,4-dione (**32**)(25 mg, 23%) as a brown oil.

### Step 2 - 2-(methoxy(5-(trifluoromethyl)pyridin-2-yl)methyl)-3-methylnaphthalene-1,4-dione (Example 124)

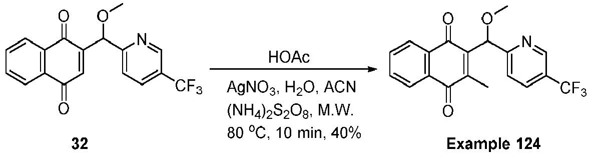

A mixture of 2-(methoxy(5-(trifluoromethyl)pyridin-2-yl)methyl)naphthalene-1,4-dione (**32**) (360 mg, 0.07 mmol, 1.0 eq), HOAc (42 mg, 0.7 mmol, 10.0 eq), AgNO₃ (12 mg, 0.07 mmol, 1.0 eq) and (NH₄)₂S₂O₈ (32 mg, 0.14 mmol, 2.0 eq) in ACN/ H₂O (1 mL /0.5 mL) was stirred at 80 °C for 10 min in the microwave under nitrogen atmosphere. The reaction was monitored by LCMS. Then the mixture was quenched with H₂O and extracted with EA (3 x 5 mL). The organic layer was washed with brine. The residue was dried over Na₂SO₄ and concentrated under reduced pressure. The residue was purified by Prep-TLC (PE/EA, 8:1) to give 2-(methoxy(5-(trifluoromethyl)pyridin-2-yl)methyl)-3-methylnaphthalene-1,4-dione (**Example 124**) (10 mg, 40%) as a brown oil. ¹H NMR (400 MHz, CDCl₃): δ 8.70 (s, 1H), 8.10-8.05 (m, 2H), 7.95 (d, *J* = 4.0 Hz, 1H), 7.86 (d, *J* = 4.0 Hz, 1H), 7.72 (d, *J* = 8.0 Hz, 1H), 6.15 (s, 1H), 3.54 (s, 3H), 2.15 (s, 3H). MS (m/z) for C₁₉H₁₄F₃NO₃: found 362.05 (M+H).

### Example 125 - 2-hydroxy-3-((5-(trifluoromethyl)pyridin-2-yl)methyl)naphthalene-1,4-dione

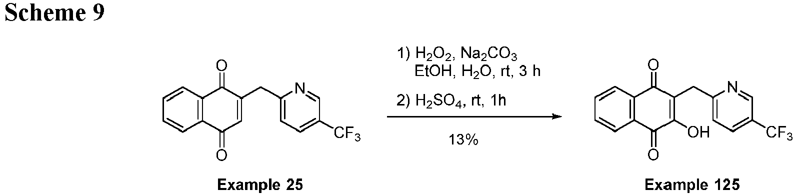

To a solution of 2-((5-(trifluoromethyl)pyridin-2-yl)methyl)naphthalene-1,4-dione (**Example 25**) (300 mg, 0.95 mmol, 1.0 eq) in EtOH (2 mL) was added Na₂CO₃ (13 mg, 0.17 mmol, 0.13 eq) in H₂O (0.2 mL). The mixture was stirred at rt for 3 h until complete by TLC. Then the mixture was diluted with aq. Na₂S₂O₃, and extracted with EA (3 × 2 mL). The organic layer was dried (Na₂SO₄) and concentrated *in vacuo.* To this residue was added H₂SO₄ (1 mL). This mixture was stirred at rt for 1 h. Then the mixture was poured into ice water and diluted with NaHCO₃ to pH 7. This solution was extracted with EA (3 × 2 mL) and the layers separated. The organic layer was washed with brine. The organics were dried over Na₂SO₄ and concentrated. The residue was purified by Prep-TLC (PE:EA, 1:1) to provide 2-hydroxy-3-((5-(trifluoromethyl)pyridin-2-yl)methyl)naphthalene-1,4-dione (**Example 125**) (40 mg, 13%) as a yellow solid. ¹H NMR (400 MHz, DMSO-*d₆*): δ 8.78 (s, 1H), 8.05-7.95 (m, 3H), 7.85-7.75 (m, 2H), 7.50 (d, *J* = 8.0 Hz, 1H), 4.07 (s, 2H). MS (m/z) for C₁₇H₁₀F₃NO₃: found 334.10 (M+H).

### Example 126 - 6-((5,8-difluoro-3-(methyl-d₃)-1,4-dioxo-1,4-dihydronaphthalen-2-yl)methyl)-3-(trifluoromethyl)picolinamide

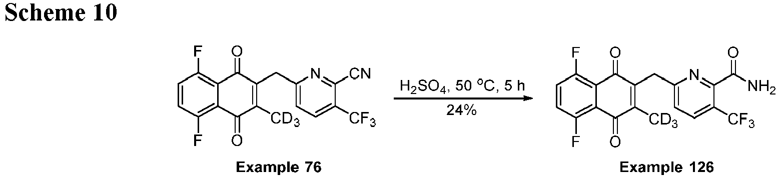

A mixture of 6-((5,8-difluoro-3-(methyl-*d₃*)-1,4-dioxo-1,4-dihydronaphthalen-2-yl)methyl)-3-(trifluoromethyl)picolinonitrile **(Example 76**) (200 mg, 0.51 mmol, 1.0 eq) was suspended in concentrated sulfuric acid (3 mL), warmed to 50 °C and stirred for 5 h. The reaction mixture was diluted with water and, extracted with ethyl acetate. The combined organic layer was washed with water, dried over sodium sulfate and concentrated under reduced pressure. The residue was purified by column chromatography on silica gel to afford 6-((5,8-difluoro-3-(methyl-*d₃*)-1,4-dioxo-1,4-dihydronaphthalen-2-yl)methyl)-3-(trifluoromethyl)picolinamide (**Example 126**) (50 mg, 24%) as yellow solid. ¹H NMR (400 MHz, DMSO-*d₆*): *δ* 8.19-8.17 (d, *J* = 8.4 Hz, 1H), 7.90 (s, 1H), 7.76-7.21 (m, 2H), 7.68 (s, 1H), 7.65-7.63 (d, *J* = 8.0 Hz, 1H) and 4.19 (s, 2H). MS (m/z) for C₁₉H₈D₃F₅N₂O₃: found 414.05 (M+H).

### Example 127 - 6-((5,8-difluoro-3-(methyl-d₃)-1,4-dioxo-1,4-dihydronaphthalen-2-yl)methyl)-3-(trifluoromethyl)picolinic acid

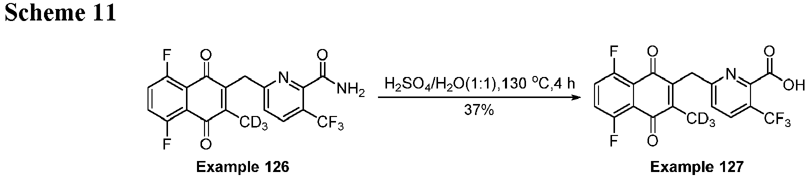

6-((5,8-difluoro-3-(methyl-*d₃*)-1,4-dioxo-1,4-dihydronaphthalen-2-yl)methyl)-3-(trifluoromethyl)picolinamide (**Example 126**) (300 mg, 0.73 mmol, 1.0 eq) was suspended in sulfuric acid/water (8 mL/8 mL), warmed to 130 °C and stirred for 4 h. The reaction mixture was diluted with water and extracted with ethyl acetate. The combined organic layer was washed with water, dried over sodium sulfate and concentrated under reduced pressure. The residue was purified by prep-HPLC to afford 6-((5,8-difluoro-3-(methyl-*d₃*)-1,4-dioxo-1,4-dihydronaphthalen-2-yl)methyl)-3-(trifluoromethyl)picolinic acid (**Example 127**) (50 mg, 37%) as yellow solid. ¹H NMR (400 MHz, DMSO-*d₆*): *δ* 8.22-8.20 (d, *J* = 8.4 Hz, 1H), 7.77-7.72 (m, 2H), 7.67-7.64 (d, *J* = 8.4 Hz, 1H) and 4.21 (s, 2H). MS (m/z) for C₁₉H₇D₃F₅NO₄: found 415.20 (M+H).

### Example 128 - 5,6-difluoro-2-methyl-3-((5-(trifluoromethyl)pyridin-2-yl)methyl)naphthalene-1,4-dione

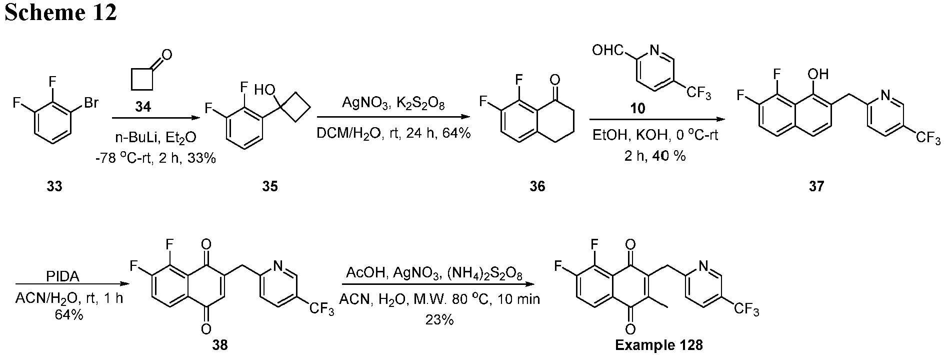

### Step 1 - 1-(2,3-difluorophenyl)cyclobutan-1-ol (35)

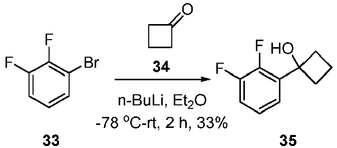

To the mixture of 1-bromo-2,3-difluorobenzene (**33**) (3.5 g, 18.13 mmol, 1.0 eq) in diethyl ether (20 mL) was added n-Butyllithium (8.7 mL, 21.76 mmol, 1.2 eq) dropwise at -78 °C. The mixture was stirred at -78 °C for 0.5 h. Cyclobutanone (**34**)(1.52 g, 21.76 mmol, 1.2 eq) was then added. The resulting mixture was stirred at room temperature for 2 h. TLC analysis of the reaction mixture showed full conversion to the desired product. Then the mixture was cooled to 0 °C and quenched with saturated aqueous ammonium chloride. The solution was diluted with water and extracted with ethyl acetate (20 mL × 2). The organic layer was dried over sodium sulfate and concentrated under reduced pressure. The residue was purified by column chromatography on silica gel (petroleum ether: ethyl acetate, 90:10) to afford 1-(2,3-difluorophenyl)cyclobutan-1-ol (**35**) (1.1 g, 33%).

### Step 2 - 7,8-difluoro-3,4-dihydronaphthalen-1(2H)-one (36)

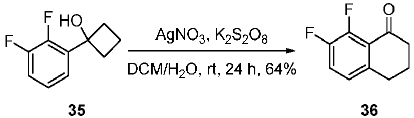

1-(2,3-difluorophenyl)cyclobutan-1-ol (**35**) (1.1 mg, 5.98 mmol, 1.0 eq) and silver nitrate (203 mg, 1.2 mmol, 0.2 eq) were dissolved in dichloromethane (10 mL) and stirred at room temperature. Potassium persulfate (4.84 g, 17.9 mmol, 3.0 eq) in water (10 mL) was then added dropwise. The reaction mixture was stirred at room temperature overnight. Then the mixture was diluted with water and extracted with dichloromethane (10 mL × 2). The organic layer was dried over sodium sulfate and concentrated under reduced pressure. The residue was purified by column chromatography on a silica gel (petroleum ether: ethyl acetate, 90:10) to afford 7,8-difluoro-3,4-dihydronaphthalen-1(2H)-one (**36**) (700 mg, 64%).

### Step 3 - 7,8-difluoro-2-((5-(trifluoromethyl)pyridin-2-yl)methyl)naphthalen-1-ol (37)

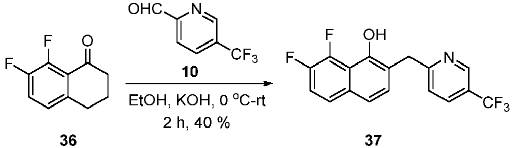

A mixture of 7,8-difluoro-3,4-dihydronaphthalen-1(2H)-one (**36**) (600 mg, 3.66 mmol, 1.0 eq) and 5-(trifluoromethyl)picolinaldehyde (**10**) (704 mg, 4.02 mmol, 1.1 eq) in ethanol (20 mL) was cooled to 0 °C. Potassium hydroxide (410 mg, 7.32 mmol, 2.0 eq) was then added and the solution was warmed to room temperature. The mixture was stirred at room temperature for 2 h under nitrogen atmosphere. Upon completion, the mixture was diluted with water, and extracted with ethyl acetate (10 mL × 2). The organic layer was dried over sodium sulfate and concentrated under reduced pressure. The residue was purified by column chromatography on a silica gel (petroleum ether: ethyl acetate, 90: 10) to afford 7,8-difluoro-2-((5-(trifluoromethyl)pyridin-2-yl)methyl)naphthalen-1-ol(**37**) (500 mg, 40%).

### Step 4 - 7,8-difluoro-2-((5-(trifluoromethyl)pyridin-2-yl)methyl)naphthalene-1,4-dione (38)

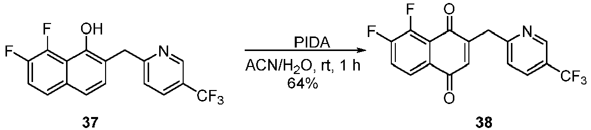

To the mixture of 7,8-difluoro-2-((5-(trifluoromethyl)pyridin-2-yl)methyl)naphthalen-l-ol(**37**) (500 mg, 1.47 mmol, 1.0 eq) in acetonitrile/water (8 mL/4 mL) was added (Diacetoxyiodo)benzene (950 mg, 2.95 mmol, 2.0 eq). The mixture was stirred at room temperature for 1 h. Then the mixture was diluted with water (10 mL) and extracted with ethyl acetate (10 mL × 2). The organic layer was dried over sodium sulfate and concentrated under reduced pressure. The residue was purified by column chromatography on silica gel (petroleum ether: ethyl acetate, 90:10) to afford 7,8-difluoro-2-((5-(trifluoromethyl)pyridin-2-yl)methyl)naphthalene-1,4-dione (**38**) (333 mg, 64%).

### Step 5 - 5,6-difluoro-2-methyl-3-((5-(trifluoromethyl)pyridin-2-yl)methyl)naphthalene-1,4-dione (Example 128)

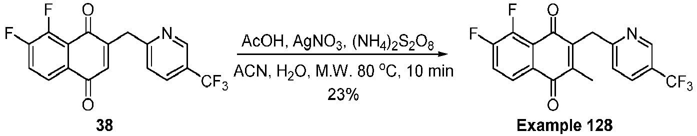

To a mixture of 7,8-difluoro-2-((5-(trifluoromethyl)pyridin-2-yl)methyl)naphthalene-1,4-dione(**38**)(170 mg, 0.48 mmol, 1.0 eq) in acetonitrile/water (3 mL/1.5 mL) was added acetic acid (58 mg, 0.96 mmol, 2.0 eq), ammonium persulfate (219 mg, 0.96 mmol, 2.0 eq) and silver nitrate (81.6 mg, 0.48 mmol, 1.0 eq). The mixture was stirred at 80 °C in a microwave for 10 min. Then the mixture was diluted with water and extracted with ethyl acetate. The combined organic layer was dried over sodium sulfate and concentrated under reduced pressure. The residue was purified by prep-HPLC to afford 5,6-difluoro-2-methyl-3-((5-(trifluoromethyl)pyridin-2-yl)methyl)naphthalene-1,4-dione (**Example 128**) (40 mg, 23%) as yellow solid. ¹H NMR (400 MHz, CDCl₃): *δ* 8.69 (s, 1H), 7.94 (m, 1H), 7.83-7.81 (m, 1H), 7.44 (m, 2H), 4.22 (s, 2H) and 2.29 (s, 3H). MS (m/z) for C₁₈H₁₀F₅NO₂: found 368.05 (M+H).

The following compound was made in a similar manner to **Example 128**:

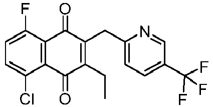

### Example 129 - 5-chloro-3-ethyl-8-fluoro-2-((5-(trifluoromethyl)pyridin-2-yl)methyl)naphthalene-1,4-dione

¹H NMR (400 MHz, CD₃OD) δ 8.68 (s, 1H), 8.01 (d, *J* = 7.3 Hz, 1H), 7.79 (dd, *J* = 8.8, 4.2 Hz, 1H), 7.56 (d, *J* = 8.0 Hz, 1H), 7.46 (t, *J* = 9.7 Hz, 1H), 4.25 (s, 2H), 2.69 (q, *J* = 7.2 Hz, 2H), 1.03 (t, *J* = 7.2 Hz, 3H). ¹⁹F NMR (400 MHz, CD₃OD) δ -63.92, -114.89. MS (m/z) for C₁₉H₁₂ClF₄NO₂: found 398.2 (M+H).

### Intermediate 1 - 5,7,8-trifluoro-3,4-dihydronaphthalen-1(2H)-one

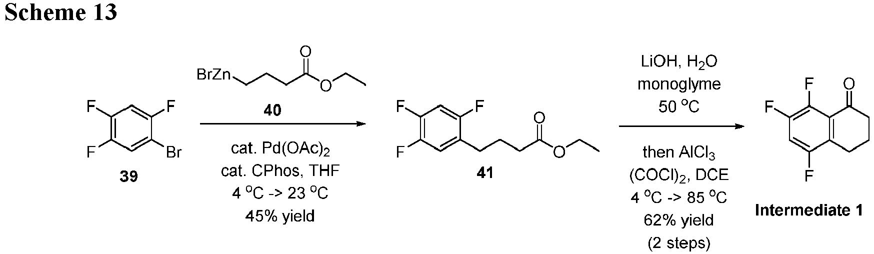

### Step 1 - ethyl 4-(2,4,5-trifluorophenyl)butanoate (41)

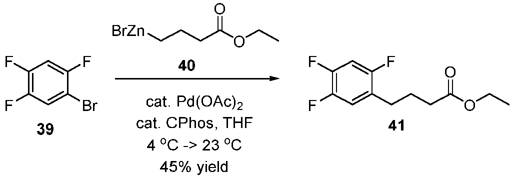

2-dicyclohexylphosphino-2',6'-bis(*N*,*N*-dimethylamino)biphenyl (Cphos) (0.45 g, 0.04 eq., 1.04 mmol), palladium(II) acetate (0.12 g, 0.02 eq., 0.53 mmol), and 1-bromo-2,4,5-trifluorobenzene (**39**, 5.3 g, 1 eq. 25.1 mmol) were weighed into an oven dried 250 mL round bottom flask fitted with a stir bar, septum, and nitrogen bubbler. Anhydrous THF (100 mL) was added and a clear ruby solution was obtained. This solution was cooled in an ice/water bath. To this solution was added 50 mL of a 0.56 M solution of (4-methoxy-2-methyl-4-oxobutyl)zinc bromide (**40**) in THF over 60 min via syringe pump. The ice bath was removed and the reaction mixture warmed to room temperature to stir overnight [18 h]. The reaction was quenched with 100 mL of 20 wt% NH₄Cl in water. 50 mL of MTBE was added and the phases were separated. The aqueous phase was back extracted with 100 mL of MTBE. The combined organic phases were washed with 200 mL of brine, dried over Na₂SO₄ and concentrated. The residue was purified by chromatography on a 220 g Isco Gold^{™} silica gel column eluted a 0-75% CH₂Cl₂/heptane gradient to obtain 2.9 g of ethyl 4-(2,4,5-trifluorophenyl)butanoate (**41**) as a colorless liquid (45% yield). ¹H NMR (400 MHz, CDCl₃) δ 7.03-6.96 (m, 1H), 6.91-6.84 (m, 1H), 4.13 (q, *J* = 7.2 Hz, 2H), 2.63 (t, *J* = 7.6 Hz, 2H), 2.32 (t, *J* = 7.6 Hz, 2H), 1.91 (quintet, *J* = 7.6 Hz, 2H), 1.26 (t, *J* = 7.2 Hz, 3H).

### Step 2 - 5,7,8-trifluoro-3,4-dihydronaphthalen-1(2H)-one (Intermediate 1)

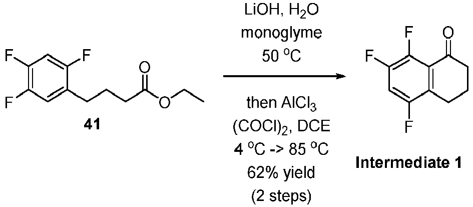

Ethyl 4-(2,4,5-trifluorophenyl)butanoate (**41**) (2.9 g, 1 eq., 11.78 mmol) was weighed into a 100 mL round bottom flask fitted with a stir bar and cap. To this was added 25 mL of 1,2-dimethoxyethane. A clear colorless solution was obtained. Lithium hydroxide (13 mL of 2.0 M) was added. The resulting cloudy colorless solution was warmed to 50 °C and stirred for 5 h. Upon reaction completion, 60 mL of 1.0 M hydrochloric acid was added and the solution was extracted with 2 × 100 mL of MTBE. The organic phase was washed with 2 × 100 mL of brine, dried over Na₂SO₄ and concentrated to obtain 2.45 g of the product as a clear pale yellow liquid. This material was used directly in the next step without further purification.

To a 20 mL scintillation vial containing 2.44 g crude product was added 10 mL dry DCE. The mixture was stirred at ambient temperature until the material dissolved. The solution was then cooled in an ice bath. Oxalyl chloride (2.13 g, 1.5 eq., 16.78 mmol) was added dropwise over 5 min and reaction was stirred at 4 °C for an additional 20 min. The reaction mixture was then warmed to room temperature and stirred for an additional 2 hours. Aluminum trichloride (0.5 eq.) was added in one portion at room temperature. The reaction mixture was warmed to 85 °C and stirred for 6 hours. Then another 0.6 eq of aluminum trichloride was added in one portion. The resulting suspension was stirred at 85 °C overnight. The reaction mixture was cooled to room temperature and filtered through a plug of celite and silica eluting with 300 mL ethyl acetate. The filtrate was washed with water and extracted with ethyl acetate (100 mL each). The aqueous layer was back extracted 4 times with 100 mL EA each. The combined organics were dried over Na₂SO₄ and concentrated to obtain a dark brown solid residue. This was purified by automated column chromatography on silica gel eluting with 0-20% ethyl acetate/heptane to obtain 1.65 g solid (62% yield over two steps) of 5,7,8-trifluoro-3,4-dihydronaphthalen-1(2H)-one (**Intermediate 1**). ¹H NMR (400 MHz, CDCl₃) δ 7.14-7.08 (m, 1H), 2.90 (t, *J* = 6.4 Hz, 2H), 2.67 (t, *J* = 6.4 Hz, 2H), 2.13 (quintet, *J* = 6.4 Hz, 2H).

### Example 130 - 6-((5,7,8-trifluoro-3-methyl-1,4-dioxo-1,4-dihydronaphthalen-2-yl)methyl)-3-(trifluoromethyl)picolinonitrile

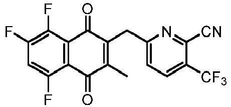

6-((5,7,8-trifluoro-3-methyl-1,4-dioxo-1,4-dihydronaphthalen-2-yl)methyl)-3-(trifluoromethyl)picolinonitrile (**Example 130**) was prepared as described in **Example 70**, using **Intermediate 1** instead of 6-fluoro-3,4-dihydronaphthalen-1(2H)-one. ¹H NMR (400 MHz, CDCl₃) δ 8.033 (d, *J* = 8.4 Hz, 1H), 7.75 (d, *J* = 8.4 Hz, 1H), 7.32-7.26 (m, 1H), 4.23 (s, 2H), 2.34 (s, 2H). MS (m/z) for C₁₉H₈F₆N₂O₂: found 409.20 (M-H).

### Example 131 - 2-methyl-3-((5-(trifluoromethyl)pyridin-2-yl)methyl)naphthalene-1,4-diol Scheme 14

### Step 1- 2-methyl-3-((5-(trifluoromethyl)pyridin-2-yl)methyl)naphthalene-1,4-diol (Example 131)

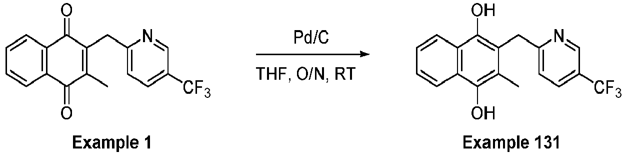

A solution comprising 2-methyl-3-((5-(trifluoromethyl)pyridin-2-yl)methyl)naphthalene-1,4-dione (**Example 1**) (1.0 eq) and Pd/C (10% w/w) in THF (0.5 M) was stirred at 25 °C for 16 h under H₂. The reaction was monitored by LCMS until completion. The reaction was filtered through a plug of silica gel and concentrated under vacuum to give 2-methyl-3-((5-(trifluoromethyl)pyridin-2-yl)methyl)naphthalene-1,4-diol (**Example 131**). ¹H NMR (400 MHz, DMSO-d₆) δ 8.91 (s, 1H), 8.85 (s, 1H), 8.37 (s, 1H), 8.11 (td, *J* = 7.0, 3.9 Hz, 2H), 8.04 (d, *J* = 8.3, 2.5 Hz, 1H), 7.39 (dq, *J* = 5.9, 3.3, 2.1 Hz, 2H), 7.29 (d, *J* = 8.3 Hz, 1H), 4.43 (s, 2H), 2.23 (s, 3H). MS (*m*/*z*) for C₁₈H₁₄F₃NO₂: found 333.31 (M+H).

### Example 132 - 6-((3-ethyl-5,8-difluoro-1,4-dioxo-1,4-dihydronaphthalen-2-yl)methyl)-3-fluoropicolinonitrile

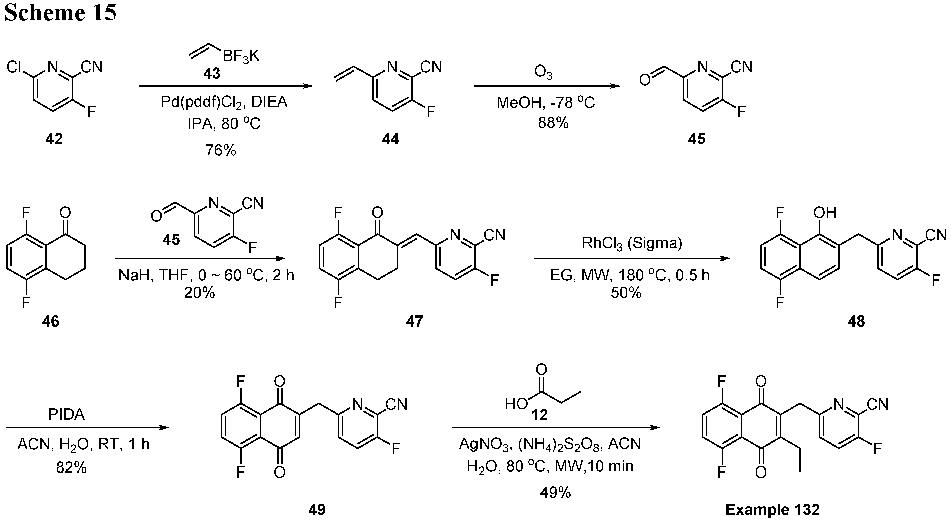

### Step 1 - 3-fluoro-6-vinylpicolinonitrile (44)

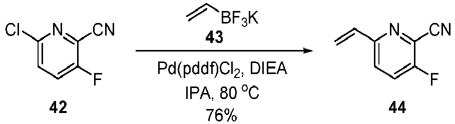

To the mixture of 6-chloro-3-fluoropicolinonitrile (**42**)(4.8 g, 30.6 mmol, 1.0 eq) in isopropyl alcohol (50 mL) was added vinyl potassium trifluoroborate (**43**) (4.9 g, 36.7 mmol, 1.2 eq), DIEA (3.9 g, 30.6 mmol, 1.0 eq) and Pd(dppf)Cl₂ (756 mg, 0.92 mmol, 0.03 eq). The reaction mixture was warmed to 80°C and stirred for 4 h under nitrogen. Upon reaction completion, the reaction mixture was diluted with water (100 mL) and extracted with ethyl acetate (50 mL × 3). The organic layer was washed with sat. NaCl aq. (50 mL × 2) and dried over anhydrous sodium sulfate. The suspension was filtered and concentrated under reduced pressure. The crude product was purified by column chromatography on silica gel (petroleum ether: ethyl acetate, 95:5) to afford 3-fluoro-6-vinylpicolinonitrile (**44**)(3.7 g, 76%) as white solid. ¹H NMR (400 MHz, CDCl₃) δ 7.63 - 7.47 (m, 2H), 6.77 (dd, *J* = 17.2, 10.8 Hz, 1H), 6.22 (d, *J* = 17.2 Hz, 1H), 5.60 (d, *J* = 10.8 Hz, 1H).

### Step 2- 3-fluoro-6-formylpicolinonitrile (45)

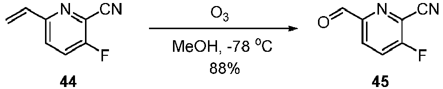

Ozone was bubbled into a solution of 3-fluoro-6-vinylpicolinonitrile (**44**)(3.8 g, 25.7 mmol, 1.0 eq) in methanol (50 mL) at -78 °C for 0.5 h. Excess O₃ was then purged by using N₂ and the resulting solution was warmed to 0 °C. Me₂S (4 mL) was added dropwise. Then the mixture was diluted with water (40 mL) and extracted with dichloromethane (40 mL × 3). The organic layer was washed with sat. aq. NaCl (40 mL × 2) and dried over anhydrous sodium sulfate. The suspension was filtered and concentrated under reduced pressure. The crude product was purified by column chromatography on silica gel (petroleum ether: ethyl acetate, 85:15) to afford 3-fluoro-6-formylpicolinonitrile (**45**)(3.4 g, 88%) as colorless oil. ¹H NMR (400 MHz, CDCl₃) δ 9.99 (s, 1H), 8.31 - 8.18 (m, 1H), 7.80 (t, *J* = 8.0 Hz, 1H).

### Step 3 - (E)-6-((5,8-difluoro-1-oxo-3,4-dihydronaphthalen-2(1H)-ylidene)methyl)-3-fluoropicolinonitrile (47)

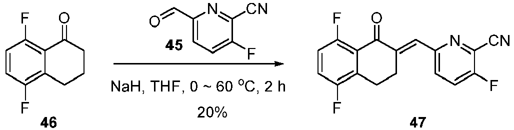

5,8-difluoro-3,4-dihydronaphthalen-1(2H)-one (**46**)(2.0 g, 11.0 mmol, 1.0 eq) was dissolved in tetrahydrofuran (30 mL) and cooled to 0 °C. Sodium hydride (1.1 g, 27.5 mmol, 2.5 eq) was then added portionwise at 0°C. The mixture was stirred at 0°C for 0.5 h under a nitrogen atmosphere. Then a solution of aldehyde **45** (2.5 g, 16.5 mmol, 1.5 eq) was added at the same temperature. Then the mixture was warmed to 60 °C and stirred for another 2 h. The reaction ran to completion and was cooled to 0 °C. It was quenched with water (20 mL) and extracted with ethyl acetate (20 mL × 2). The organic layer was washed with sat. aq. NaCl (20 mL × 2), dried over anhydrous sodium sulfate, filtered and concentrated under reduced pressure. The crude product was purified by column chromatography on silica gel (petroleum ether: ethyl acetate, 94:6) to afford (*E*)-6-((5,8-difluoro-1-oxo-3,4-dihydronaphthalen-2(1H)-ylidene)methyl)-3-fluoropicolinonitrile (**47**)(700 mg, 20%) as yellow solid. ¹H NMR (CDCl₃, 400 MHz): δ 7.74 - 7.58 (m, 3H), 7.24 - 7.20 (m, 1H), 7.02 (td, J = 9.6, 4.0 Hz, 1H), 3.53 (t, J = 6.0 Hz, 2H), 2.97 (t, J = 6.0 Hz, 2H).

### Step 4 - 6-((5,8-difluoro-1-hydroxynaphthalen-2-yl)methyl)-3-fluoropicolinonitrile (48)

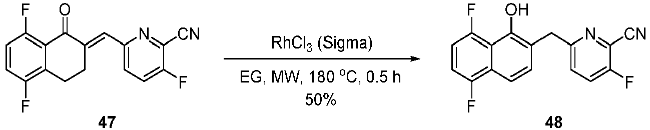

To a solution of (*E*)-6-((5,8-difluoro-1-oxo-3,4-dihydronaphthalen-2(1H)-ylidene)methyl)-3-fluoropicolinonitrile (**47**)(400 mg, 1.27 mmol, 1.0 eq) in ethylene glycol (15 mL) was added rhodium chloride trihydrate (20 mg). The mixture was stirred at 80 °C in a microwave reactor for 30 min. Then the mixture was diluted with water (20 mL) and extracted with ethyl acetate (20 mL × 2). The organic layer was washed with sat. aq. NaCl (20 mL × 2) and dried over anhydrous sodium sulfate. The suspension was filtered and concentrated under reduced pressure to afford the crude product. This was purified by column chromatography on silica gel (petroleum ether: ethyl acetate, 92:8) to afford 6-((5,8-difluoro-1-hydroxynaphthalen-2-yl)methyl)-3-fluoropicolinonitrile (**48**) (200 mg, 50%) as yellow solid. ¹H NMR (CDCl₃, 400 MHz): δ = 7.60 - 7.58 (d, J = 8.0 Hz, 1H), 7.50 - 7.49 (d, J = 4.4 Hz, 1H), 7.47 - 7.44 (m, 2H), 7.021 - 6.98 (m, 2H), 4.32 (s, 2H).

### Step 5 - 6-((5,8-difluoro-1,4-dioxo-1,4-dihydronaphthalen-2-yl)methyl)-3-fluoropicolinonitrile (49)

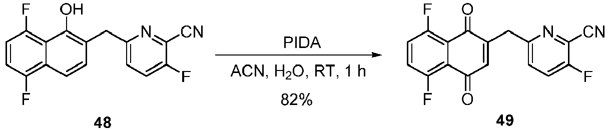

6-((5,8-difluoro-1-hydroxynaphthalen-2-yl)methyl)-3-fluoropicolinonitrile (**48**)(350 mg, 1.11 mmol, 1.0 eq) was dissolved in acetonitrile/water (10 mL/5 mL) and cooled to 0 °C. (Diacetoxyiodo)benzene (718 mg, 2.23 mmol, 2.0 eq) was added and the resulting mixture was stirred at 0 °C for 1 h. Then the mixture was diluted with water (20 mL) and extracted with ethyl acetate (20 mL × 2). The organic layer was washed with sat. aq. NaCl (20 mL × 2) and dried over anhydrous sodium sulfate. The suspension was filtered and concentrated under reduced pressure. The crude product was purified by column chromatography on silica gel (petroleum ether: ethyl acetate, 85:15) to afford 6-((5,8-difluoro-1,4-dioxo-1,4-dihydronaphthalen-2-yl)methyl)-3-fluoropicolinonitrile (**49**)(300 mg, 82%) as yellow solid. ¹H NMR (CDCl₃, 400 MHz):δ = 7.65 - 7.62 (q, J = 4.0 Hz, 1H), 7.57 - 7.55 (d, J = 8.0 Hz, 1H), 7.43-7.41 (m, 2H), 6.83 (s, 1H), 4.02 (s, 2H).

### Step 6 - 6-((3-ethyl-5,8-difluoro-1,4-dioxo-1,4-dihydronaphthalen-2-yl)methyl)-3-fluoropicolinonitrile

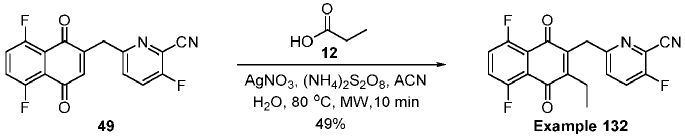

6-((5,8-difluoro-1,4-dioxo-1,4-dihydronaphthalen-2-yl)methyl)-3-fluoropicolinonitrile (**49**)(300 mg, 0.91 mmol, 1.0 eq) was dissolved in acetonitrile/water (10 mL/5 mL). Propionic acid (135 mg, 1.83 mmol, 2.0 eq), ammonium persulfate (417 mg, 1.83 mmol, 2.0 eq) and silver nitrate (155 mg, 0.91 mmol, 1.0 eq) were then added. The resulting mixture was stirred at 80 °C in a microwave reactor for 10 min. Then the mixture was extracted with ethyl acetate and water. The combined organic layer was dried over sodium sulfate and concentrated under reduced pressure. The residue was purified by prep-HPLC to afford 6-((3-ethyl-5,8-difluoro-1,4-dioxo-1,4-dihydronaphthalen-2-yl)methyl)-3-fluoropicolinonitrile (**Example 132**)(160 mg, 49%) as yellow solid. ¹H NMR (CDCl₃, 400 MHz): δ 7.74 - 7.59 (m, 1H), 7.51 (t, J = 8.4 Hz, 1H), 7.46 - 7.30 (m, 2H), 4.13 (s, 2H), 2.79 (q, J = 7.6 Hz, 2H), 1.15 (t, J = 7.6 Hz, 3H). MS (*m*/*z*) for C₁₉H₁₁F₃N₂O₂: found 357.2 (M+H).

The following compound was prepared in a similar manner to **Example 132**:

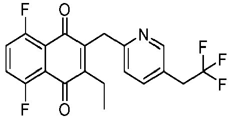

### Example 133 - 2-ethyl-5,8-difluoro-3-((5-(2,2,2-trifluoroethyl)pyridin-2-yl)methyl)naphthalene-1,4-dione

¹H NMR (CDCl₃, 400 MHz): δ 8.34 (s, 1H), 7.53 (d, J = 7.6 Hz, 1H), 7.47 - 7.18 (m, 3H), 4.14 (s, 2H), 3.30 (t, J = 10.8 Hz, 2H), 2.81 (q, J = 7.6 Hz, 2H), 1.07 (t, J = 7.6 Hz, 3H). MS (*m*/*z*) for C₂₀H₁₄F₅NO₂: found 396.3 (M+H).

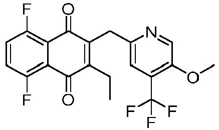

### Example 134 - 2-ethyl-5,8-difluoro-3-((5-methoxy-4-(trifluoromethyl)pyridin-2-yl)methyl)naphthalene-1,4-dione

**Example 134** was prepared in a similar manner to **Example 26** (**Scheme 3**). The aldehyde coupling partner was prepared as described in **Scheme 15**, compound **45**. ¹H NMR (400 MHz, CDCl₃) δ 8.26 (s, 1H), 7.45 (s, 1H), 7.38 (d, *J* = 6.4 Hz, 2H), 4.12 (s, 2H), 3.94 (s, 3H), 2.75 (q, *J* = 7.6 Hz, 2H), 1.10 (t, *J* = 7.6 Hz, 3H). MS *(m*/*z)* for C₂₀H₁₄F₅NO₃: found 412.3 (M+H).

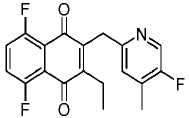

### Example 135 - 2-ethyl-5,8-difluoro-3-((5-fluoro-4-methylpyridin-2-yl)methyl)naphthalene-1,4-dione

**Example 135** was prepared in a similar manner to **Example 26** (**Scheme 3**). The aldehyde coupling partner was prepared as described in **Scheme 15**, compound **45**. ¹H NMR (CDCl₃, 400 MHz): *δ* 8.14 (s, 1H), 7.38-7.34 (t, J = 6.8 Hz, 2H), 7. 12-7.11 (d, J = 6.0 Hz, 1H), 4.06 (s, 2H), 2.74 (q, J = 7.6 Hz, 2H), 2.24 (s, 3H), 1.07 (t, J = 7.6 Hz, 3H) MS *(m*/*z)* for C₁₉H₁₄F₃NO₂: found 346.1 (M+H).

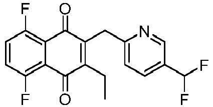

### Example 136 - 2-((5-(difluoromethyl)pyridin-2-yl)methyl)-3-ethyl-5,8-difluoronaphthalene-1,4-dione

**Example 136** was prepared in a similar manner to **Example 26** (**Scheme 3**). The aldehyde coupling partner was prepared as described in **Scheme 15**, compound **45**. ¹H NMR (CDCl₃, 400 MHz): δ 8.54 (s, 1H), 7.74 (d, J = 8.0 Hz, 1H), 7.44 - 7.31 (m, 3H), 6.64 (t, J = 6.0 Hz, 1H), 4.18 (s, 2H), 2.75 (q, J = 7.6 Hz, 2H), 1.08 (t, J = 7.6 Hz, 3H). MS *(m*/*z)* for C₁₉H₁₃F₄NO₂: found 364.3 (M+H).

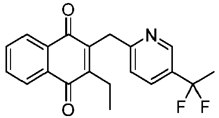

### Example 137 - 2-((5-(1,1-difluoroethyl)pyridin-2-yl)methyl)-3-ethylnaphthalene-1,4-dione

**Example 137** was prepared in a similar manner to **Example 26** (**Scheme 3**). The aldehyde coupling partner was prepared as described in **Scheme 15**, compound **45**. ¹H NMR (CDCl₃, 400 MHz): δ 8.59 (s, 1H), 8.12 - 8.00 (m, 2H), 7.68 (s, 3H), 7.32 (d, *J* = 8.4 Hz, 1H), 4.22 (s, 2H), 2.77 (q, *J* = 7.6 Hz, 2H), 1.89 (t, *J* = 18.4 Hz, 3H), 1.08 (t, *J* = 7.6 Hz, 3H). MS (*m*/*z*) for C₂₀H₁₇F₂NO₂: found 342.4 (M+H).

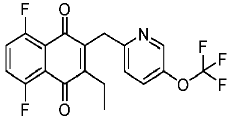

### Example 138 - 2-ethyl-5,8-difluoro-3-((5-(trifluoromethoxy)pyridin-2-yl)methyl)naphthalene-1,4-dione

**Example 138** was prepared in a similar manner to **Example 26** (**Scheme 3**). The aldehyde coupling partner was prepared as described in **Scheme 15**, compound **45**. ¹H NMR (400 MHz, CDCl₃) δ 8.37 (s, 1H), 7.48 (d, *J* = 8.4 Hz, 1H), 7.40 (d, *J* = 7.6 Hz, 3H), 4.17 (s, 2H), 2.79 (q, *J* = 7.6 Hz, 2H), 1.12 (t, *J* = 7.6 Hz, 3H). MS *(m*/*z)* for C₁₉H₁₂F₅NO₃: found 398.3 (M+H).

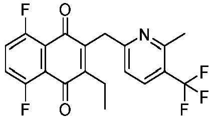

### Example 139 - 2-ethyl-5,8-difluoro-3-((6-methyl-5-(trifluoromethyl)pyridin-2-yl)methyl)naphthalene-1,4-dione

**Example 139** was prepared in a similar manner to **Example 26** (**Scheme 3**). The aldehyde coupling partner was prepared as described in **Scheme 15**, compound **45**. ¹H NMR (400 MHz, CDCl₃) δ 7.77 (d, *J* = 7.6 Hz, 1H), 7.39 (s, 2H), 7.23 (d, *J* = 7.6 Hz, 1H), 4.16 (s, 2H), 2.80 (q, *J* = 7.6 Hz, 2H), 2.60 (s, 3H), 1.15 (d, *J* = 7.6 Hz, 3H). ¹⁹F NMR (400 MHz, CDCl₃) δ -62.16, -116.15. MS (*m*/*z*) for C₂₀H₁₄F₅NO₂: found 396.2 (M+H).

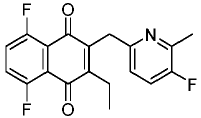

### Example 140 - 2-ethyl-5,8-difluoro-3-((5-fluoro-6-methylpyridin-2-yl)methyl)naphthalene-1,4-dione

**Example 140** was prepared in a similar manner to **Example 26** (**Scheme 3**). The aldehyde coupling partner was prepared as described in **Scheme 15**, compound **45**. ¹H NMR (400 MHz, CDCl₃) δ 7.36 (t, *J* = 6.4 Hz, 2H), 7.17 (t, *J* = 8.4 Hz, 1H), 7.08 (s, 1H), 4.07 (s, 2H), 2.77 (q, *J* = 7.6 Hz, 2H), 2.40 (s, 3H), 1.09 (t, *J* = 7.2 Hz, 3H). ¹⁹F NMR (400 MHz, CDCl₃) δ -116.29, -128.60. MS (*m*/*z*) for C₁₉H₁₄F₃NO₂: found 346.2 (M+H).

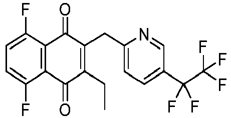

### Example 141 - 2-ethyl-5,8-difluoro-3-((5-(perfluoroethyl)pyridin-2-yl)methyl)naphthalene-1,4-dione

**Example 141** was prepared in a similar manner to **Example 26** (**Scheme 3**). The aldehyde coupling partner was prepared as described in **Scheme 15**, compound **45**. ¹H NMR (400 MHz, CDCl₃) δ 8.65 (s, 1H), 7.80 (d, *J* = 7.6 Hz, 1H), 7.48 (d, *J* = 7.6 Hz, 1H), 7.38 (t, *J* = 6.0 Hz, 2H), 4.21 (s, 2H), 2.77 (q, *J* = 7.6 Hz, 2H), 1.11 (t, *J* = 7.6 Hz, 3H). ¹⁹F NMR (400 MHz, CDCl₃) δ -84.95, -116.00. MS (*m*/*z*) for C₂₀H₁₂F₇NO₂: found 432.1 (M+H).

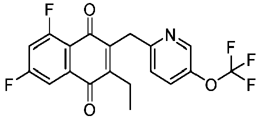

### Example 142 - 2-ethyl-5,7-difluoro-3-((5-(trifluoromethoxy)pyridin-2-yl)methyl)naphthalene-1,4-dione

**Example 142** was prepared in a similar manner to **Example 26** (**Scheme** 3). The aldehyde coupling partner was prepared as described in **Scheme 15**, compound **45**. ¹H NMR (400 MHz, CDCl₃) δ 8.37 (s, 1H), 7.65 (d, *J* = 7.6 Hz, 1H), 7.47 (d, *J* = 7.6 Hz, 2H), 7.40 (d, *J =* 8.0 Hz, 1H), 7.11 (t, *J* = 8.0 Hz, 1H), 4.18 (s, 2H), 2.80 (q, *J* = 7.6 Hz, 2H), 1.11 (t, *J* = 7.6 Hz, 3H). ¹⁹F NMR (400 MHz, CDCl₃) δ -58.30, -97.59, -106.66. MS (*m*/*z*) for C₁₉H₁₂F₅NO₃: found 398.2 (M+H).

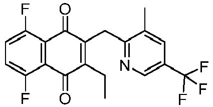

### Example 143 - 2-ethyl-5,8-difluoro-3-((3-methyl-5-(trifluoromethyl)pyridin-2-yl)methyl)naphthalene-1,4-dione

**Example 143** was prepared in a similar manner to **Example 26** (**Scheme 3**). The aldehyde coupling partner was prepared as described in **Scheme 15**, compound **45**. ¹H NMR (400 MHz, CDCl₃) δ 8.46 (s, 1H), 7.67 (s, 1H), 7.39 (m, 2H), 4.10 (s, 2H), 2.65 (q, *J* = 7.2 Hz, 2H), 2.54 (s, 3H), 1.10 (t, *J* = 7.2 Hz, 3H). ¹⁹F NMR (400 MHz, CDCl₃) δ -62.48, - 116.31. MS (*m*/*z*) for C₂₀H₁₄F₅NO₂: found 396.2 (M+H).

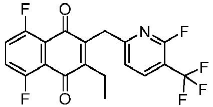

### Example 144 - 2-ethyl-5,8-difluoro-3-((6-fluoro-5-(trifluoromethyl)pyridin-2-yl)methyl)naphthalene-1,4-dione

**Example 144** was prepared in a similar manner to **Example 26** (**Scheme 3**). The aldehyde coupling partner was prepared as described in **Scheme 15**, compound **45**. ¹H NMR (400 MHz, CDCl₃) δ 7.93 (t, *J* = 8.4 Hz, 1H), 7.37 (m, 3H), 4.12 (s, 2H), 2.77 (q, *J* = 7.2 Hz, 2H), 1.15 (t, *J* = 7.2 Hz, 3H). ¹⁹F NMR (400 MHz, *d₆*-DMSO) δ -60.73, -68.578, -117.16.MS (*m*/*z*) for C₁₉H₁₁F₆NO₂: found 400.0 (M+H).

### Example 145 - 2-((5-methoxy-4-(trifluoromethyl)pyridin-2-yl)methyl)-3-methylnaphthalene-1,4-dione

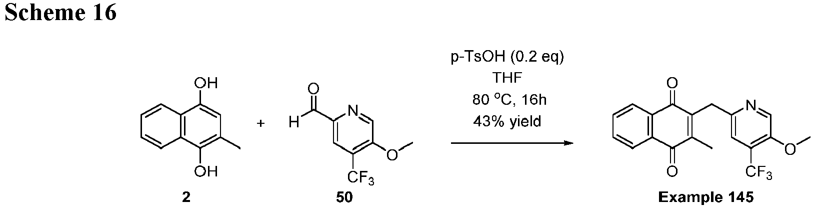

To the mixture of 5-methoxy-4-(trifluoromethyl)picolinaldehyde (**50**)(prepared as described in **Scheme 15,** compound **45**)(0.30 g, 1.7 mmol, 1.0 eq) in tetrahydrofuran (5 mL) was added 2-methylnaphthalene-1,4-diol (0.35 g, 1.7 mmol, 1.0 eq) and p-toluenesulfonic acid (0.33 g, 1.7 mmol, 1.0 eq). The mixture was degassed with nitrogen three times, warmed to 80 °C and stirred for 16 h. LCMS analysis of the reaction mixture showed full conversion to the desired product. The reaction mixture was concentrated under reduced pressure. The residue was triturated with ethanol (2 mL). The precipitated solid was filtered. The filter cake was dried under reduced pressure to afford 2-((5-methoxy-4-(trifluoromethyl)pyridin-2-yl)methyl)-3-methylnaphthalene-1,4-dione (**Example 145**)(269.1 mg, 43%) as yellow solid. ¹H NMR (CDCl₃, 400 MHz): δ 8.27 (s, 1H), 8.07 (t, J = 8.0 Hz, 2H), 7.72 - 7.65 (m, 2H), 7.42 (s, 1H), 4.17 (s, 2H), 3.93 (s, 3H), 2.27 (s, 3H). MS *(m*/*z)* for C₁₉H₁₄F₃NO₃: found 362.3 (M+H).

### Intermediate 2 - 2-ethylnaphthalene-1,4-diol

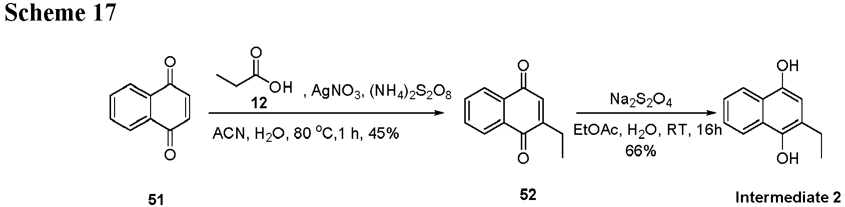

### Step 1 - 2-ethylnaphthalene-1,4-dione (52)

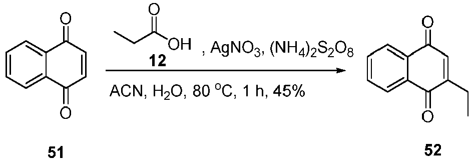

Naphthoquinone (**51**)(10.0 g, 63.3 mmol, 1.0 eq) was dissolved in acetonitrile/water (100 mL/50 mL). To this solution was added propionic acid (**12**)(4.68 g, 63.3 mmol, 1.0 eq), ammonium persulfate (28.1 g, 126.6 mmol, 2.0 eq) and silver nitrate (10.7, 63.3 mmol, 1.0 eq). The mixture was stirred at 80 °C for 1 h. Then the mixture was extracted with ethyl acetate (100 mL × 2). The combined organic layers were dried over anhydrous sodium sulfate, filtered and concentrated under reduced pressure. The residue was purified by column chromatography on silica gel (petroleum ether: ethyl acetate, 90:10) to afford 2-ethylnaphthalene-1,4-dione (**52**)(5.0 g, 45%) as yellow solid.

### Step 2 - 2-ethylnaphthalene-1,4-diol (Intermediate 2)

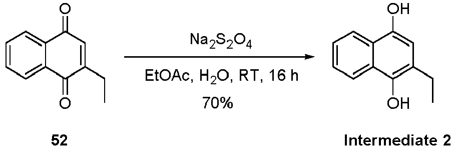

To a solution of Na₂S₂O₄ (11.7 g, 67.2 mol, 2.5 eq) in H₂O/EA (50 mL/50 mL) was added 2-ethylnaphthalene-1,4-dione (**52**)(5.0 g, 26.9 mol, 1.0 eq). The mixture was stirred at room temperature for 16 h under a nitrogen atmosphere. Reaction progress was monitored by TLC until the starting material disappeared. Then the mixture was filtered and extracted with EA (50 mL). The organic layer was washed with brine (50 mL). The residue was dried over anhydrous sodium sulfate, filtered and concentrated under reduced pressure to give crude 2-ethylnaphthalene-1,4-diol (**Intermediate 2**). This material was triturated with petroleum ether/ethyl acetate (15/1). The solid was collected and dried under reduced pressure to afford 2-ethylnaphthalene-1,4-diol **(Intermediate 2**)(3.5 g, 70%) as grey solid. ¹H NMR (400 MHz, *d₆*-DMSO) *δ* 9.33 (s, 1H), 8.19 (s, 1H), 8.00 (dd, J = 28.8, 8.2 Hz, 2H), 7.33 (dt, J = 26.8, 7.2 Hz, 2H), 6.61 (s, 1H), 2.65 (q, J = 7.6 Hz, 2H), 1.12 (t, J = 7.6 Hz, 3H).

The following compounds were prepared in a similar manner to **Example 145**:

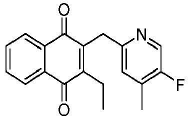

### Example 146 - 2-ethyl-3-((5-fluoro-4-methylpyridin-2-yl)methyl)naphthalene-1,4-dione

**Example 146** was prepared from **Intermediate 2.** The aldehyde coupling partner was prepared as described in **Scheme 15**, compound **45**. ¹H NMR (CDCl₃, 400 MHz): δ 8.16 (s, 1H), 8.10 - 8.02 (m, 2H), 7.72 - 7.64 (m, 2H), 7.07 (d, *J* = 6.0 Hz, 1H), 4.11 (s, 2H), 2.76 (q, *J* = 7.6 Hz, 2H), 2.23 (s, 3H), 1.05 (t, *J* = 7.6 Hz, 3H). MS (*m*/*z*) for C₁₉H₁₆FNO₂: found 310.1 (M+H).

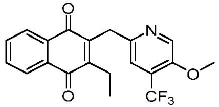

### Example 147 - 2-ethyl-3-((5-methoxy-4-(trifluoromethyl)139yridine-2-yl)methyl)naphthalene-1,4-dione

**Example 147** was prepared from **Intermediate 2**. ¹H NMR (CDCl₃, 400 MHz): δ 8.27 (s, 1H), 8.09-8.03 (m, 2H), 7.68 (t, *J* = 4.0 Hz, 2H), 7.43 (s, 1H), 4.16 (s, 2H), 3.93 (s, 3H), 2.77 (q, *J* = 7.6 Hz, 2H), 1.09 (t, *J* = 7.6 Hz, 3H). MS *(m*/*z)* for C₂₀H₁₆F₃NO₃: found 376.2 (M+H).

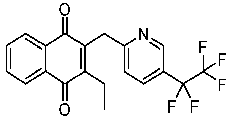

### Example 148 - 2-ethyl-3-((5-(perfluoroethyl)140yridine-2-yl)methyl)naphthalene-1,4-dione

**Example 148** was prepared from **Intermediate 2**. The aldehyde coupling partner was prepared as described in **Scheme 15**, compound **45**. **¹H NMR** (400 MHz, *d₆-*DMSO) δ 8.72 (s, 1H), 8.09 - 7.90 (m, 3H), 7.86 - 7.77 (m, 2H), 7.63 (d, *J* = 8.0 Hz, 1H), 4.25 (s, 2H), 2.60 (q, *J* = 7.6 Hz, 2H), 0.91 (t, *J* = 7.6 Hz, 3H) MS *(m*/*z)* for C₂₀H₁₄F₅NO₂: found 396.4 (M+H).

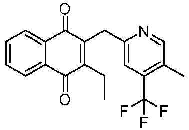

### Example 149 - 2-ethyl-3-((5-methyl-4-(trifluoromethyl)pyridin-2-yl)methyl)naphthalene-1,4-dione

**Example 149** was prepared from **Intermediate 2**. The aldehyde coupling partner was prepared as described in **Scheme 15**, compound **45**. ¹H NMR (400 MHz, CDCl₃) δ 8.42 (s, 1H), 8.15 - 8.03 (m, 2H), 7.76 - 7.66 (m, 2H), 7.47 (s, 1H), 4.23 (s, 2H), 2.79 (d, *J* = 7.6 Hz, 2H), 2.40 (s, 3H), 1.10 (t, *J* = 7.6 Hz, 3H). MS (*m*/*z*) for C₂₀H₁₆F₃NO₂: found 360.4 (M+H).

### Example 150 - 2-((3-methyl-1,4-dioxo-1,4-dihydronaphthalen-2-yl)methyl)-5-(trifluoromethyl)pyridine 1-oxide

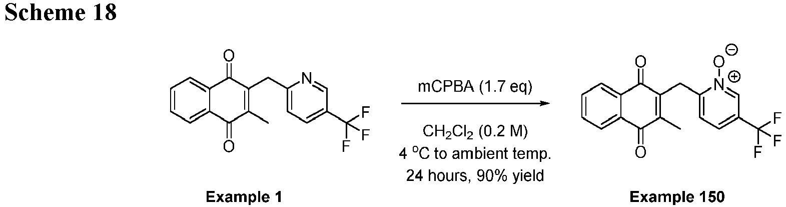

2-methyl-3-((5-(trifluoromethyl)pyridin-2-yl)methyl)naphthalene-1,4-dione (**Example 1**)(300 mg, 0.9 mmol, 1 eq.) was dissolved in methylene chloride (5 mL, 0.2 M) and cooled in an ice bath over 5 minutes. meta-Chloroperoxybenzoic acid (345 mg, 1.5 mmol, 1.7 eq.) was added in one portion. The reaction mixture was warmed to room temperature over 1 hour and then stirred at ambient temperature for a total of 24 hours. The homogeneous solution remained clear pale yellow throughout. The reaction solution was partitioned between water (75 mL) and ethyl acetate (75 mL) and extracted three times. The organic layers were combined, dried over sodium sulfate, filtered, and concentrated in vacuo. The crude residue was purified over flash chromatography (ethyl acetate/heptane gradient from 0 - 60%, on 24g silica cartridge; product elutes at 60%) and 2-((3-methyl-1,4-dioxo-1,4-dihydronaphthalen-2-yl)methyl)-5-(trifluoromethyl)pyridine 1-oxide (**Example 150**) was isolated as a pale yellow solid (286 mg, 90% yield). ¹H NMR (400 MHz, CDCl₃): δ 8.50 (s, 1H), 8.12 - 8.10 (m, 1H), 8.05 - 8.03 (m, 1H), 7.75 - 7.69 (m, 2H), 7.43 (d, *J* = 8 Hz, 1H), 7.37 (d, *J* = 8 Hz, 1H), 4.28 (s, 2H), 2.31 (s, 3H). MS (*m*/*z*) for C₁₈H₁₂F₃NO₃: found 348 (M+H).

The following compound was prepared in a similar manner to **Example 150**:

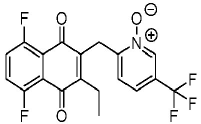

### Example 151 - 2-((3-ethyl-5,8-difluoro-1,4-dioxo-1,4-dihydronaphthalen-2-yl)methyl)-5-(trifluoromethyl)pyridine 1-oxide

¹H NMR (300 MHz, CDCl₃): δ 8.478 (s, 1H), 7.476 - 7.373 (m, 4H), 4.217 (s, 2H), 2.782 (q, *J* = 7 Hz, 2H), 1.116 (t, *J* = 7 Hz, 3H). ¹⁹F NMR (300 MHz, CDCl₃): d 63.181 (s, 3F), 115 (s, 2F). MS *(m*/*z)* for C₁₉H₁₂F₅NO₃: found 398.2 (M+H).

### Example 152 - 2-ethyl-5,8-difluoro-3-((6-methoxy-5-(trifluoromethyl)pyridin-2-yl)methyl)naphthalene-1,4-dione and Example 153 - 2-ethyl-5,8-difluoro-3-((6-hydroxy-5-(trifluoromethyl)pyridin-2-yl)methyl)naphthalene-1,4-dione

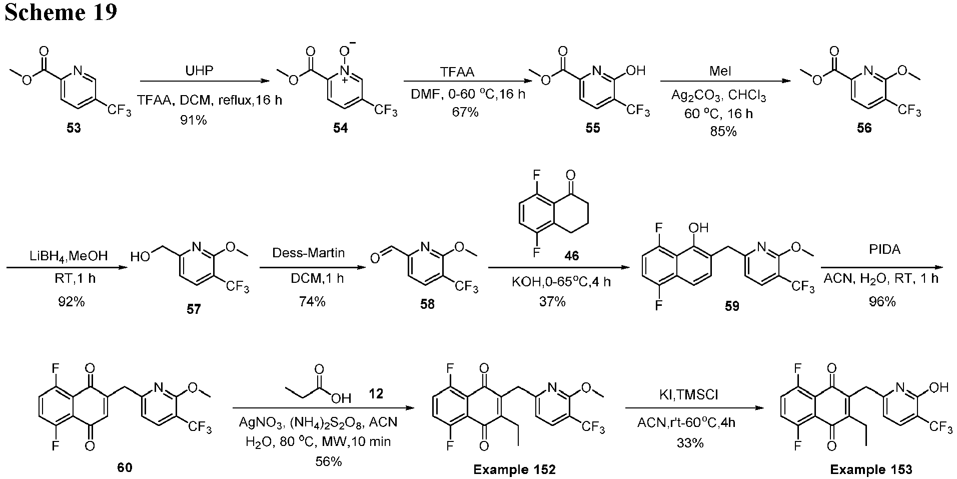

### Step 1 - 2-(methoxycarbonyl)-5-(trifluoromethyl)pyridine 1-oxide (54)

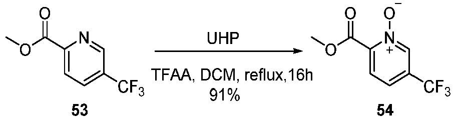

To the mixture of 2-(methoxycarbonyl)-5-(trifluoromethyl)pyridine 1-oxide (**53**) (10.0 g,48.8 mmol, 1.0 eq) in dichloromethane (500 mL) was added urea hydrogen peroxide (UHP)(16.0 g, 170.7 mmol, 3.5 eq). The resulting mixture was cooled to 0 °C. Then trifluoroacetic anhydride (25.6 g, 122 mmol, 2.5 eq) was added dropwise into the mixture at 0°C over 1 h. The mixture was stirred at room temperature for 16 h under a nitrogen atmosphere. The mixture was cooled to 0 °C and quenched via addition of saturated sodium bicarbonate aqueous solution. The resulting suspension was filtered and the filter cake was washed with 100 mL dichloromethane, 200 mL water, and dried under reduced pressure to afford 2-(methoxycarbonyl)-5-(trifluoromethyl)pyridine 1-oxide (**54**)(9.8 g, 91%) as white solid. ¹H NMR (CDCl₃, 400 MHz):δ 8.44 (s, 1H), 7.67 (d, J=7.6, 1H), 7.40 (d, J=7.6, 1H), 3.96 (s, 3H).

### Step 2 - methyl 6-hydroxy-5-(trifluoromethyl)picolinate (55)

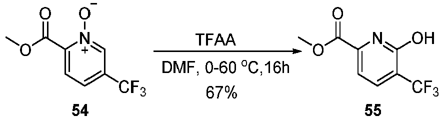

2-(methoxycarbonyl)-5-(trifluoromethyl)pyridine 1-oxide (**54**)(9.8 g,44.3 mmol, 1.0 eq) was dissolved in N,N-Dimethylformamide (200 mL) and cooled to 0 °C. To this solution was added trifluoroacetic anhydride (46.6 g, 222 mmol, 5.0 eq) dropwise at 0°C over 0.5 h. The mixture was warmed to 60 °C and stirred for 16 h under a nitrogen atmosphere. Upon completion, the reaction mixture was cooled to 0°C and quenched with saturated sodium bicarbonate aqueous solution. The mixture was extracted with ethyl acetate (200 mL × 2). The organic layer was washed with sat. NaCl a.q. (150 mL × 3), dried over anhydrous sodium sulfate, filtered and concentrated under reduced pressure to afford the crude product. This was purified by column chromatography on silica gel (petroleum ether: ethyl acetate, 85:15) to afford methyl 6-hydroxy-5-(trifluoromethyl)picolinate (**55**)(6.6 g, 67%) as white solid. ¹H NMR (CDCl₃, 400 MHz):δ 7.88 (d, J=7.6, 1H), 7.01 (d, J=7.6, 1H), 4.00 (s, 3H).

### Step 3 - methyl 6-methoxy-5-(trifluoromethyl)picolinate (56)

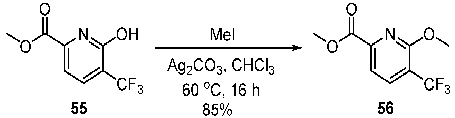

To the mixture of methyl 6-hydroxy-5-(trifluoromethyl)picolinate (**55**)(4.0 g, 18.1 mmol, 1.0 eq) in trichloromethane (200 mL) was added iodomethane (2.57 g, 54.3 mmol, 3.0 eq) and silver carbonate (6.0 g, 21.7 mmol, 1.2 eq). The mixture was stirred at 60 °C for 16 h under a nitrogen atmosphere. Upon reaction completion, the suspension was filtered and the filter cake was washed with 20 mL dichloromethane. The filtrate was concentrated under reduced pressure to afford the crude product which was purified by column chromatography on silica gel (petroleum ether: ethyl acetate, 96:4) to afford methyl 6-methoxy-5-(trifluoromethyl)picolinate (**56**)(3.7 g, 85%) as white solid. ¹H NMR (CDCl₃, 400 MHz):δ 7.98 (d, J=7.6, 1H), 7.75 (d, J=7.6, 1H), 4.11 (s, 3H), 3.98 (s, 3H).

### Step 4 - (6-methoxy-5-(trifluoromethyl)pyridin-2-yl)methanol (57)

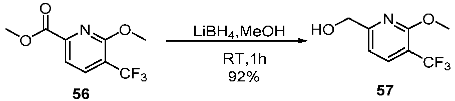

Methyl 6-methoxy-5-(trifluoromethyl)picolinate (**56**)(4.2 g, 17.9 mmol, 1.0 eq) was dissolved in methanol (40 mL) and cooled to 0 °C. LiBH₄ (793 mg, 35.7 mmol, 2.0 eq) was added portionwise at 0 °C. The mixture was warmed to room temperature and stirred for 1 h. The reaction mixture was cooled back to 0 °C and diluted with water (30 mL). This mixture was extracted with ethyl acetate (30 mL × 2). The organic layer was washed with sat. aq.NaCl (20 mL × 2), dried over anhydrous sodium sulfate, filtered and concentrated under reduced pressure. The crude product was purified by column chromatography on silica gel (petroleum ether: ethyl acetate, 94:6) to afford (6-methoxy-5-(trifluoromethyl)pyridin-2-yl)methanol (**57**) (3.4 g, 92%) as colorless oil. ¹H NMR (CD₃OD, 400 MHz):δ = 7.93 (d, J=7.6, 1H),7.17 (d, J=7.6, 1H), 4.63 (s, 2H), 3.98 (s, 3H).

### Step 5 - 6-methoxy-5-(trifluoromethyl)picolinaldehyde (58)

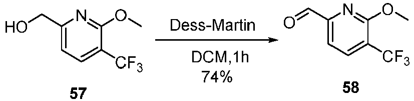

To the mixture of (6-methoxy-5-(trifluoromethyl)pyridin-2-yl)methanol (**57**)(3.4 g, 16.4 mmol, 1.0 eq) in dichloromethane (30 mL) was added Dess Martin periodinane (8.36 g, 19.7 mmol, 1.2 eq). The mixture was stirred at room temperature for 1 h under a nitrogen atmosphere. Upon completion, the reaction mixture was filtered. The filtrate was diluted with water (30 mL) and extracted with ethyl acetate (30 mL × 2). The organic layer was washed with sat. aq. NaCl (20 mL × 2), dried over anhydrous sodium sulfate, filtered and concentrated under reduced pressure. The crude product was purified by column chromatography on silica gel (petroleum ether: ethyl acetate, 96:4) to afford 6-methoxy-5-(trifluoromethyl)picolinaldehyde (**58**)(2.7 g, 74%) as white solid. ¹H NMR (CDCl₃, 400 MHz):δ = 9.98 (s, 1H), 8.03 (d, J=7.6, 1H), 7.61 (d, J=7.6, 1H), 4.13 (s, 3H).

### Step 6 - 5,8-difluoro-2-((6-methoxy-5-(trifluoromethyl)pyridin-2-yl)methyl)naphthalen-1-ol (59)

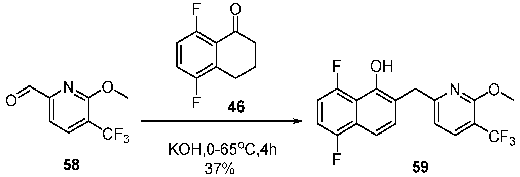

Methoxy-5-(trifluoromethyl)picolinaldehyde (**58**)(2.7 g, 13.2 mmol, 1.0 eq) and 5,8-difluoro-tetralone (**46**)(2.4 g, 13.2 mmol, 1.0 eq) were dissolved in ethanol (40 mL) and cooled to 0 °C. Potassium hydroxide (1.48 g, 26.3 mmol, 2.0 eq) in ethanol (10 mL) was added dropwise at 0 °C. The mixture was warmed to room temperature and stirred for 2 h under a nitrogen atmosphere. Then the mixture was warmed to 65 °C and stirred for another 2h. Upon reaction completion, the mixture was cooled to room temperature, diluted with water (30 mL), and extracted with ethyl acetate (30 mL × 2). The organic layer was dried over anhydrous sodium sulfate, filtered and concentrated under reduced pressure. The crude product was purified by column chromatography on silica gel (petroleum ether: ethyl acetate, 95:5) to afford 5,8-difluoro-2-((6-methoxy-5-(trifluoromethyl)pyridin-2-yl)methyl)naphthalen-1-ol (**59**)(1.8 g, 37%) as yellow solid. ¹H NMR (CDCl₃, 400 MHz): δ= 9.40 (s, 1H), 7.79 (d, J=7.6, 1H), 7.56 (d, J=8.4, 1H), 7.40 (d, J=8.8, 1H), 7.00-6.92 (m, 3H), 4.21 (s, 2H), 4.09 (s, 3H).

### Step 7 - 5,8-difluoro-2-((6-methoxy-5-(trifluoromethyl)pyridin-2-yl)methyl)naphthalene-1,4-dione (60)

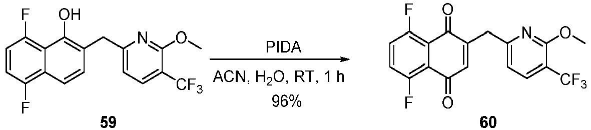

To the mixture of 5,8-difluoro-2-((6-methoxy-5-(trifluoromethyl)pyridin-2-yl)methyl)naphthalen-1-ol (**59**)(1.8 g, 4.88 mmol, 1.0 eq) in acetonitrile/water (20 mL/10 mL) was added (Diacetoxyiodo)benzene (3.1 g, 9.76 mmol, 2.0 eq). The reaction mixture was stirred at room temperature for 1 h. It was then diluted with water (10 mL) and extracted with ethyl acetate (20 mL × 2). The organic layer was washed with sat. aq. NaCl (20 mL × 2), dried over anhydrous sodium sulfate, filtered and concentrated under reduced pressure. The crude product was purified by column chromatography on silica gel (petroleum ether: ethyl acetate, 90:10) to afford 5,8-difluoro-2-((6-methoxy-5-(trifluoromethyl)pyridin-2-yl)methyl)naphthalene-1,4-dione (**60**)(1.8 g, 96%) as yellow solid. ¹H NMR (CDCl₃, 400 MHz):δ = 7.78 (d, J=7.6, 1H), 7.48 - 7.38 (m, 2H), 6.96 (d, J=7.6, 1H), 6.79 (s, 1H), 3.97 (s, 2H), 3.91 (s, 3H).

### Step 8 - 2-ethyl-5,8-difluoro-3-((6-methoxy-5-(trifluoromethyl)pyridin-2-yl)methyl)naphthalene-1,4-dione (Example 152)

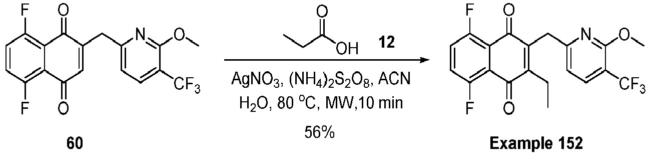

The mixture of 5,8-difluoro-2-((6-methoxy-5-(trifluoromethyl)pyridin-2-yl)methyl)naphthalene-1,4-dione (**60**)(500 mg, 1.3 mmol, 1.0 eq) in acetonitrile/water (10 mL/5 mL) was added propionic acid (**12**)(193 mg, 2.6 mmol, 2.0 eq), ammonium persulfate (595 mg, 2.6 mmol, 2.0 eq) and silver nitrate (222 mg, 2.6 mmol, 1.0 eq). The mixture was stirred at 80 °C in the microwave for 10 min. Then the mixture was diluted with water (10 mL) and extracted with ethyl acetate (20 mL × 2). The organic layer was washed with sat. aq. NaCl (20 mL × 2), dried over anhydrous sodium sulfate, filtered and concentrated under reduced pressure. The crude product was purified by column chromatography on silica gel (petroleum ether: ethyl acetate, 92:8) to afford 2-ethyl-5,8-difluoro-3-((6-methoxy-5-(trifluoromethyl)pyridin-2-yl)methyl)naphthalene-1,4-dione (**Example 152**) (300 mg, 56%) as yellow solid. ¹H NMR (CDCl₃, 400 MHz): δ 7.74 (d, J = 7.6 Hz, 1H), 7.42 - 7.30 (m, 2H), 6.98 (d, J = 7.6 Hz, 1H), 4.09 (s, 2H), 3.85 (s, 3H), 2.82 (6, J = 7.5 Hz, 2H), 1.13 (t, J = 7.6 Hz, 3H). MS (*m*/*z*) for C₂₀H₁₄F₅NO₃: found 412.0 (M+H).

### Step 9 - 2-ethyl-5,8-difluoro-3-((6-hydroxy-5-(trifluoromethyl)pyridin-2-yl)methyl)naphthalene-1,4-dione (Example 153)

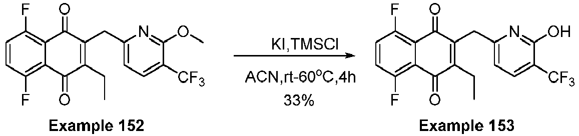

To the mixture of 2-ethyl-5,8-difluoro-3-((6-methoxy-5-(trifluoromethyl)pyridin-2-yl)methyl)naphthalene-1,4-dione (**Example 152**)(411 mg, 1.0 mmol, 1.0 eq) in acetonitrile (10 mL) was added trimethyl chlorosilane (436 mg, 4.0 mmol, 4.0 eq) and potassium iodide (332 mg, 2.0 mmol, 2.0 eq). The mixture was stirred at room temperature for 2 h under a nitrogen atmosphere. Then the mixture was warmed to 65 °C for and stirred for 2 h. Upon reaction completion, the solution was cooled to room temperature, diluted with water (20 mL) and extracted with ethyl acetate (20 mL × 2). The organic layer was dried over anhydrous sodium sulfate, filtered and concentrated under reduced pressure. The crude product was purified by column chromatography on silica gel (petroleum ether: ethyl acetate, 85:15) to afford 2-ethyl-5,8-difluoro-3-((6-hydroxy-5-(trifluoromethyl)pyridin-2-yl)methyl)naphthalene-1,4-dione (**Example 153**) (130 mg, 33%) as yellow solid. ¹H NMR (DMSO-*d*₆, 400 MHz): δ 7.72 (m, 3H), 6.01 (d, J = 8.0 Hz, 1H), 3.85 (s, 2H), 2.48 (q, J = 7.6 Hz, 2H), 0.96 (t, J = 7.6 Hz, 3H). MS (*m*/*z*) for C₁₉H₁₂F₅NO₃: found 398.3 (M+H).

### Example 154. Biological Data

### Example 154A. Cell Rescue in oxidative stress models.

In neuronal cells excess extracellular glutamate inhibits the cystine/glutamate antiporter leading to intracellular cysteine depletion, GSH depletion, reactive oxygen species (ROS) production and cell death, a phenomenon termed oxidative glutamate toxicity, oxytosis, or ferroptosis. Ferroptosis has been shown to lead to neurodegeneration. Q7 cells (ST HDH Q7/7; immortalized mouse striatal cells) challenged with cystine-free media or the selective, irreversible GPX4 inhibitor, methyl (1S,3R)-2-(2-chloroacetyl)-1-(4-(methoxycarbonyl)phenyl)-2,3,4,9-tetrahydro-1H-pyrido[3,4-b]indole-3-carboxylate (hereafter referred to as RSL3), recapitulate this phenotype, as do normal human fibroblasts (NHF) challenged with RSL3. An initial screen was performed to identify compounds effective in rescuing Q7 and/or NHF cells from death resulting from cystine deprivation or RSL3 challenge. This method is further described in Yonezawa et al., J. Neurochem 67, 566-573 (1996), Li et al., J Neurosci., 23, 5816-5826 (2003), Yang and Stockwell, Chem Biol., 15: 234-45 (2008), and Yang et al., Cell 156, 317-331 (2014). See also Hinman A, Holst CR, Latham JC, et al. Vitamin E hydroquinone is an endogenous regulator of ferroptosis via redox control of 15-lipoxygenase. PLoS One. 2018;13(8):e0201369. Published 2018 Aug 15. doi:10.1371/journal.pone.0201369; and Kahn-Kirby AH, Amagata A, Maeder CI, et al. Targeting ferroptosis: A novel therapeutic strategy for the treatment of mitochondrial disease-related epilepsy. PLoS One. 2019;14(3):e0214250. Published 2019 Mar 28. doi:10.1371/journal.pone.0214250.

DMEM (Catalog no. 11995-040), DMEM without Cystine (Catalog no. 21013-024), DMEM without Glucose (Catalog A1443001), PBS (Phosphate buffered saline), Penicillin-streptomycin mix, L-Glutamine, Glucose, Phenol Red, and Pyruvate were purchased from ThermoFisher Scientific (Gibco). Fetal Bovine Serum was obtained from Mediatech, Inc., or Sigma Aldrich. Mouse striatum derived ST HDH Q7/7 (Q7) and normal human skin fibroblast (NHF, product number GM21811) cells were obtained from the Coriell Institute. Methionine and Vitamin K2 were purchased from Sigma Aldrich. RSL3 was synthesized as previously described (see e.g. US Patent Publication US2010/0081654). Cell Titer Glo 2.0 was purchased from Promega. Q7 cell culture medium (Q7 growth medium) was made by combining 50 mL Fetal Bovine Serum, 100 U/mL penicillin, and 100 microgram/mL streptomycin; DMEM was added to make the volume up to 500 mL. Assay medium for the Cystine Deprivation assay was prepared by combining 50 mL Fetal Bovine Serum, 100 U/mL penicillin, 100 microgram/mL streptomycin, 4 mM L-Glutamine, 1 mM Pyruvate and 30 mg/L Methionine; DMEM without Cystine was added to make the volume up to 500 mL. NHF cell culture medium (NHF growth medium) was made by combining 50 mL Fetal Bovine Serum, 100 U/mL penicillin, 100 microgram/mL streptomycin, 2.8 mL 20% Glucose solution (final concentration 6 mM), L-glutamine (final concentration 2 mM), phenol red (final concentration 15 mg/L); DMEM without Glucose was added to make the volume up to 500 mL. Assay medium for the RSL3 challenge assay was identical to Growth medium. While not in use during the course of the experiments, these solutions were stored at 4 °C. The cells were grown in 150-cm² tissue culture-treated flasks. Every third or fourth day, the cells were sub-cultured by trypsinization and re-seeded at a cell density of 600,000 (Q7) or 300,000 (NHF) cells per flask.

Test samples were supplied in 1.5 mL glass vials. The compounds were diluted with an appropriate volume of DMSO to result in a 10 mM stock solution. Once dissolved, they were stored at -20 °C.

Test samples were screened according to the following protocol:

Q7 or NHF cells were cultured routinely as described herein. For 384-well cell survival assays, cells were seeded in clear bottom, black wall 384-well tissue culture-treated polystyrene plates by re-suspending a cell suspension at a density of 50,000 cells/mL in growth medium, then dispensing 60 microliters of cell suspension per well using either an electronic multichannel pipette or a Multidrop^{™} Combi Reagent Dispenser (ThermoFisher Scientific), corresponding to 3,000 cells/well. The cell-containing plates were incubated at room temperature for 15 minutes followed by 5 hours at 33 °C (Q7) or 37 °C (NHF) in an atmosphere with 95% humidity and 5% CO₂ to allow attachment of the cells to the culture plate.

For the Cystine Deprivation assay, 5 hours after cell seeding into the 384-well assay plate, the cell culture medium was replaced by washing 2 times with 70 microliters/well PBS (without Ca⁺⁺ and Mg⁺⁺) using a BioTek ELx405 plate washer. After the final aspiration, 60 microliters/well of Assay medium (without cystine) was added using the Multidrop^{™} Combi Reagent Dispenser. Within 45 minutes, test compounds were then added to varying final concentrations using the Tecan D300e Digital Dispenser, with subsequent back-filling with DMSO diluent to a final concentration of 0.3% (v/v). Cell plates were then incubated at 33 °C in an atmosphere of 5% CO₂ and 95% humidity.

For the RSL3 challenge assay, 5 hours after cell seeding into the 384-well assay plate, test compounds were added to varying final concentrations using the Tecan D300e Digital Dispenser, followed within 15 minutes by RSL3 (2 micromolar final concentration). DMSO diluent was back-filled to a final concentration of 0.3% (v/v). Cell plates were then incubated at 33°C in an atmosphere of 5% CO₂ and 95% humidity.

18 (Q7) or 48 hours (NHF) hours later, the plates were equilibrated to room temperature for 15 minutes. Then, 10 microliters/well of room temperature Cell Titer Glo 2.0 reagent was added using the Multidrop^{™} Combi Reagent Dispenser. After 15 minutes of incubation at room temperature, the luminescence (100 ms integration time) per well was determined using the BioTek Synergy plate reader. Data was imported into Microsoft Excel, then either analyzed using ACAS Curve Curator (John McNeil and Company) or Dotmatics Studies software suite to calculate the EC₅₀ values for each compound using standard four-parameter curve fitting algorithms.

For the Cystine Deprivation assay, the relative viability of test compound-treated cells were calculated relative to the average cystine-deprived, DMSO-treated cell viability (defined as 0% relative viability) and the average cystine-deprived, Vitamin K2 (1 micromolar) treated cell viability (defined as 100% relative viability).

For the RSL3 challenge assay, the relative viability of test compound-treated cells were calculated relative to the average RSL3-treated, DMSO-treated cell viability (defined as 0% relative viability) and the average RSL3-treated, Vitamin K2 (1 or 3 micromolar)-treated cell viability (defined as 100% relative viability).

EC₅₀ was the concentration corresponding to 50% relative viability. The assay performance was gauged by Z-prime calculations on each assay plate, with observed Z-prime values of >0.5.

### Example 154B Assay 1. CYP450 Inhibition Assay 1

The CYP450 inhibition assay provides in vitro assessment of the inhibitory potential of the test compounds against cytochrome P450 enzymes. Selective substrates were incubated with pooled human liver microsomes (HLM) as single substrates. The substrate, protein, and incubation time for each enzyme is summarized in Table 1 below. All assays employed 100 mM potassium phosphate buffer at pH 7.4.

The assays were performed in a 96-well PCR plate in a final volume of 140 µL at 37 °C. Test compounds were evaluated at seven concentrations to obtain IC₅₀ values, with a final concentration of 0.1% DMSO in the incubation (total organic solvent at 1% in the incubation). Reactions were initiated by the addition of NADPH. After the enzyme-specific incubation time had elapsed, an aliquot from that enzyme reaction was quenched in Acetonitrile with 0.2% Formic Acid. Supernatant from the quenched reactions was diluted with an equal volume of water, prior to analysis by LC-MS/MS.

**Table 1: Summarized Assay Conditions**

| **Enzyme** | **Substrate** | **Substrate (µM)** | **Metabolite (Mass Transition m/z)** | **HLM (mg/mL)** | **Time (min)** |
|---|---|---|---|---|---|
| CYP3A4 | Midazolam | 5 | 1'-Hydroxymidazolam (341.9 → 324.0) | 0.05 | 5 |

### LC-MS/MS Conditions

All samples were analyzed on LC-MS/MS using an AB Sciex 6500+ instrument equipped with electrospray ionization source, coupled to a Shimadzu Nexera X2 system. Samples were separated using a Waters UPLC HSS T3, 1.8 µm column, 1 × 50 mm (catalog # 186003535) at a flow rate of 200 µL/min. LC method consisted of mobile phases listed in Table 2. Injection volume was 2 µL.

**Table 2: Chromatography Solvents**

| **Solvent** | **Composition** |
|---|---|
| Mobile Phase A | 0.1% Formic Acid + 1mM Ammonium Formate in Water |
| Mobile Phase B | 0.1% Formic Acid + 1mM Ammonium Formate in (9:1) ACN:IPA |

### Example 154B Assay 2. CYP450 Inhibition Assay 2

In an alternative experimental design, test compounds were evaluated at 5 concentrations in a 96 well assay plate. Initial combination with pooled human liver microsomes (0.05 mg protein/mL) was followed by a 5 minute incubation. Then the substrate cocktail containing 1'-Hydroxymidazolam (2.5 µM) was added. The assay plate was incubated for an additional 30 minutes before the reaction was initiated with the addition of NADPH. After 10 minutes of reaction time, the reaction was quenched and samples were analyzed in a similar manner as described above.

### Example 154 Results.

Results are shown in Table 3 below.

**Table 3: Cell Rescue and CYP3A4 Inhibition Assays**

| **Compound** | **Synth. Ex. No.** | **Q7 Cystine Deprivation Survival EC50*** | **Q7 RSL3 Survival EC50*** | **NHF GM21811 RSL3 Survival EC50*** | **CYP3A4 IC₅₀ (µM) (Assay 1)** | **CYP3A4 IC₅₀ (µM) (Assay 2)** |
|---|---|---|---|---|---|---|
| 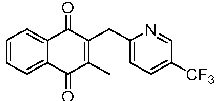 | 1 | ++++ | ++++ | ++++ | 5.782 | 35.7 |
| 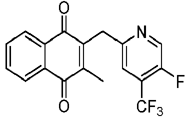 | 2 | ++++ | | | | |
| 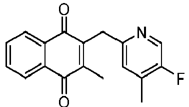 | 3 | ++++ | | | | |
| 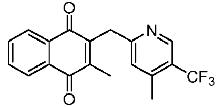 | 4 | ++++ | | | | |
| 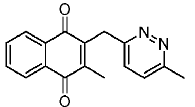 | 5 | ++++ | | | | |
| 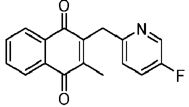 | 6 | ++++ | ++++ | ++++ | | |
| 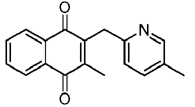 | 7 | ++++ | | | | |
| 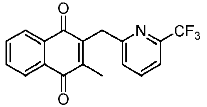 | 8 | ++++ | | | | |
| 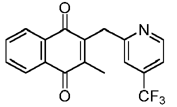 | 9 | ++++ | | | | 37.2 |
| 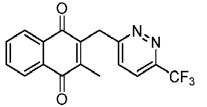 | 10 | ++++ | | | | |
| 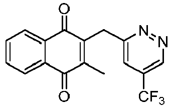 | 11 | ++++ | | | | |
| 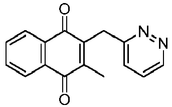 | 12 | ++++ | | | | |
| 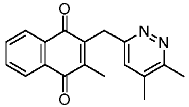 | 13 | ++++ | | | | |
| 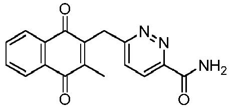 | 14 | ++++ | | | | |
| 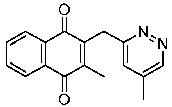 | 15 | ++++ | | | | |
| 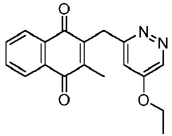 | 16 | ++++ | | | | |
| 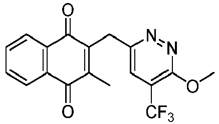 | 17 | ++++ | | | | |
| 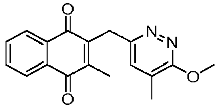 | 18 | + | | | | |
| 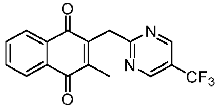 | 19 | ++++ | | | | |
| 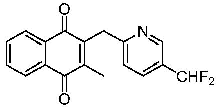 | 20 | ++++ | ++++ | ++++ | | |
| 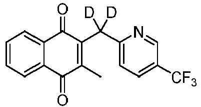 | 21 | | ++++ | ++++ | | |
| 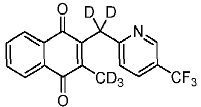 | 22 | | ++++ | | | |
| 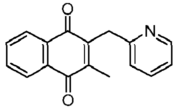 | 23 | ++++ | | | 22.75 | 12.7 |
| 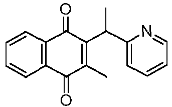 | 24 | ++++ | | | | 20.1 |
| 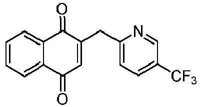 | 25 | ++ | | | | |
| 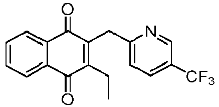 | 26 | ++++ | ++++ | ++++ | | 14.2 |
| 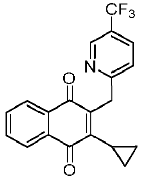 | 27 | ++++ | | | | 11.9 |
| 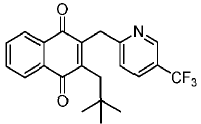 | 28 | +++ | | | | 50 |
| 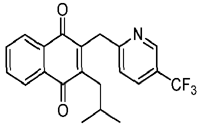 | 29 | ++++ | | | | 21.4 |
| 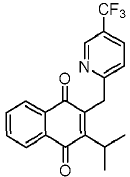 | 30 | ++++ | | ++++ | | 10.3 |
| 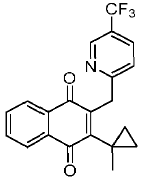 | 31 | ++++ | | | | 17.4 |
| 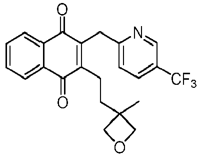 | 32 | ++++ | ++++ | +++ | | |
| 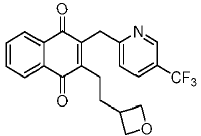 | 33 | ++++ | ++++ | +++ | | |
| 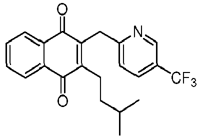 | 34 | +++ | | | | |
| 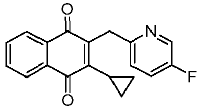 | 35 | ++++ | ++++ | ++++ | | |
| 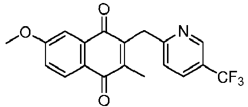 | 36 | ++++ | | | | |
| 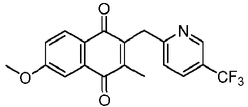 | 37 | ++++ | | | | |
| 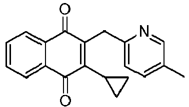 | 38 | ++++ | | | | |
| 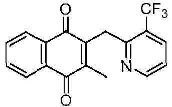 | 39 | ++++ | | | | 50 |
| 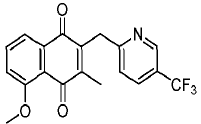 | 40 | ++++ | | | | |
| 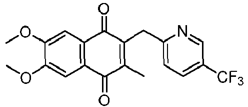 | 41 | ++++ | | | | |
| 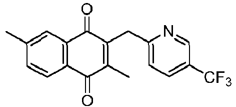 | 42 | ++++ | | ++++ | | |
| 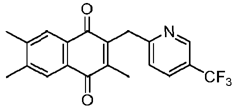 | 43 | +++ | ++ | | | |
| 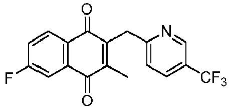 | 44 | ++++ | | ++++ | | |
| 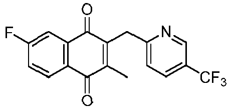 | 45 | ++++ | | | | |
| 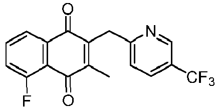 | 46 | ++++ | | | | |
| 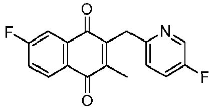 | 47 | ++++ | ++++ | | | |
| 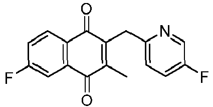 | 48 | ++++ | ++++ | ++++ | | |
| 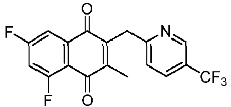 | 49 | ++++ | ++++ | | | |
| 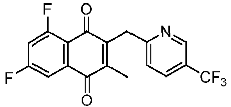 | 50 | ++++ | ++++ | | | |
| 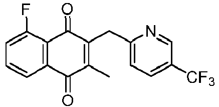 | 51 | ++++ | ++++ | ++++ | | |
| 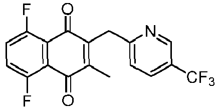 | 52 | ++++ | ++++ | ++++ | | |
| 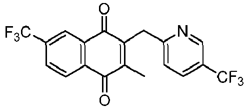 | 53 | +++ | +++ | | | |
| 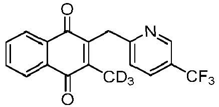 | 54 | ++++ | ++++ | | | |
| 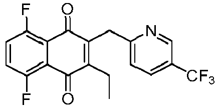 | 55 | | ++++ | ++++ | 3.678 | |
| 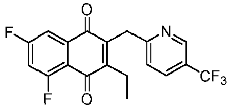 | 56 | | ++++ | ++++ | | |
| 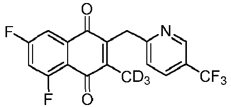 | 57 | | ++++ | | 1.488 | |
| 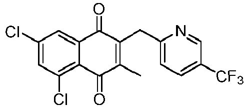 | 58 | | ++++ | | | |
| 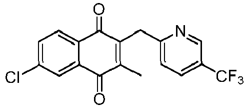 | 59 | | ++++ | | | |
| 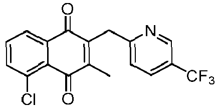 | 60 | | ++++ | ++++ | | |
| 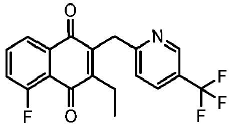 | 61 | | | ++++ | | |
| 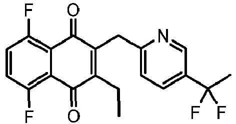 | 62 | | | ++++ | | |
| 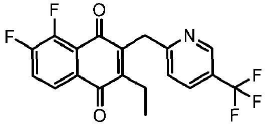 | 63 | | | ++++ | | |
| 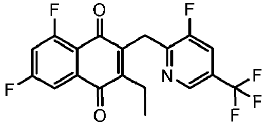 | 64 | | | ++++ | | |
| 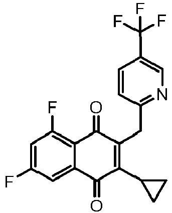 | 65 | | | ++++ | | |
| 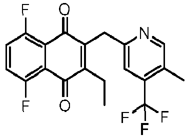 | 66 | | | ++++ | | |
| 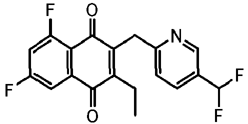 | 67 | | | ++++ | | |
| 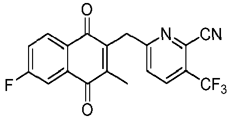 | 70 | | ++++ | ++++ | 4.14 | |
| 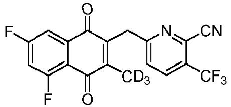 | 71 | | ++++ | ++++ | | |
| 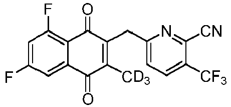 | 72 | | ++++ | ++++ | | |
| 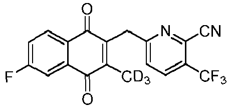 | 73 | | ++++ | ++++ | | |
| 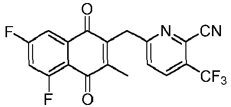 | 74 | | +++ | ++++ | | |
| 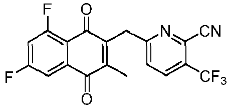 | 75 | | ++++ | ++++ | | |
| 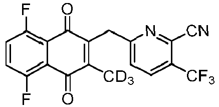 | 76 | | ++++ | ++++ | | |
| 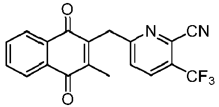 | 77 | | ++++ | ++++ | | |
| 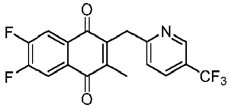 | 78 | | ++++ | | | |
| 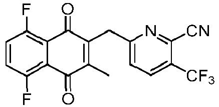 | 79 | | ++++ | ++++ | 2.399 | |
| 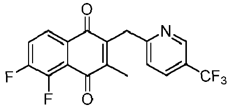 | 80 | | ++++ | | | |
| 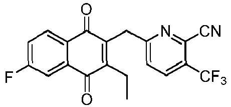 | 81 | | ++++ | ++++ | 10.99 | |
| 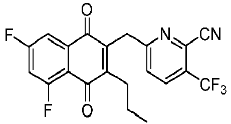 | 82 | | ++++ | ++++ | | |
| 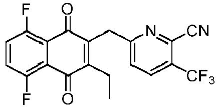 | 83 | | ++++ | | 2.727 | |
| 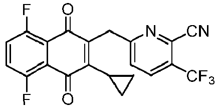 | 84 | | ++++ | ++++ | 1.559 | |
| 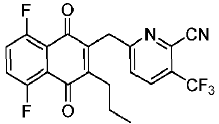 | 85 | | ++++ | | | |
| 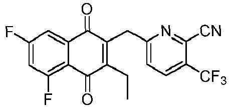 | 86 | | ++++ | ++++ | 6.656 | |
| 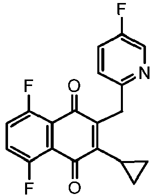 | 87 | | ++++ | ++++ | | |
| 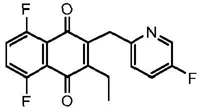 | 88 | | ++++ | ++++ | | |
| 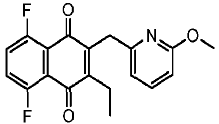 | 89 | | | ++++ | | |
| 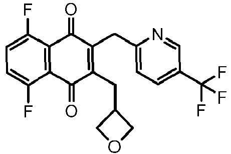 | 90 | | | ++++ | | |
| 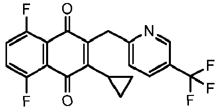 | 91 | | | ++++ | | |
| 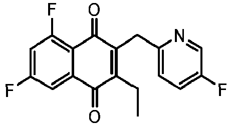 | 92 | | | ++++ | | |
| 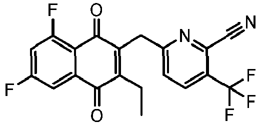 | 93 | | | ++++ | | |
| 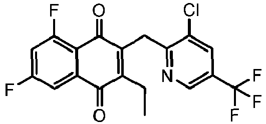 | 94 | | | ++++ | | |
| 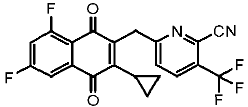 | 95 | | | ++++ | | |
| 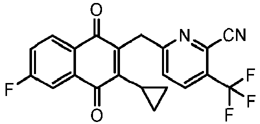 | 96 | | | ++++ | | |
| 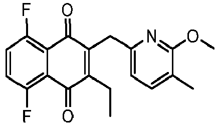 | 97 | | | ++++ | | |
| 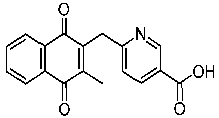 | 99 | + | | | | 50 |
| 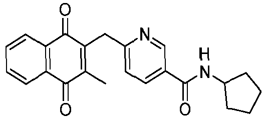 | 100 | +++ | | | | 50 |
| 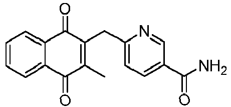 | 101 | ++++ | | | | 23 |
| 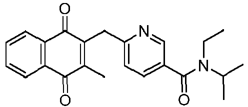 | 102 | ++++ | | | | 8.8 |
| 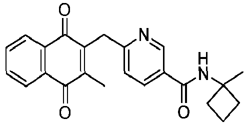 | 103 | ++ | | | | 41.5 |
| 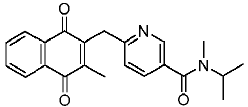 | 104 | ++++ | | | | 20.7 |
| 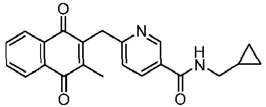 | 105 | +++ | | | | 50 |
| 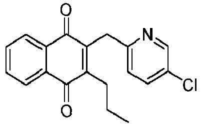 | 106 | ++++ | | | | |
| 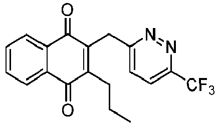 | 107 | ++++ | | | | |
| 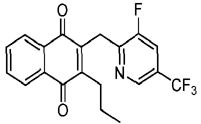 | 108 | +++ | | | | |
| 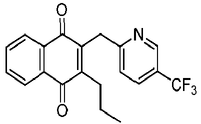 | 109 | ++++ | | | | 15.6 |
| 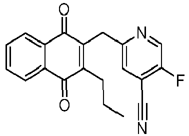 | 110 | ++++ | | | | |
| 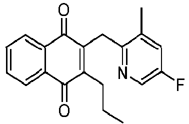 | 111 | +++ | | | | |
| 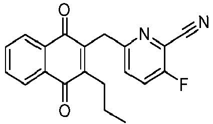 | 112 | ++++ | | | | |
| 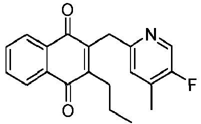 | 113 | ++++ | ++++ | ++++ | | |
| 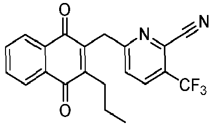 | 114 | ++++ | ++++ | | | |
| 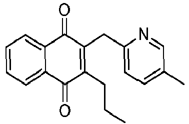 | 115 | ++++ | | | | |
| 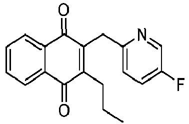 | 116 | ++++ | ++++ | ++++ | | |
| 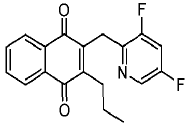 | 117 | ++++ | | | | |
| 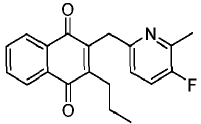 | 118 | ++++ | ++++ | ++++ | | |
| 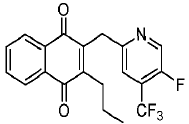 | 119 | ++++ | | | | |
| 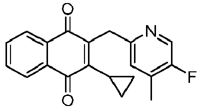 | 120 | ++++ | ++++ | ++++ | | |
| 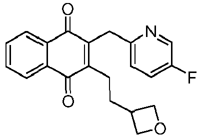 | 121 | ++++ | | | | |
| 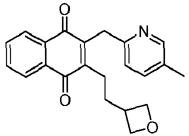 | 122 | ++++ | ++++ | +++ | | |
| 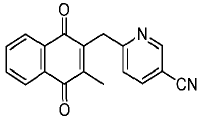 | 123 | ++++ | | | | 50 |
| 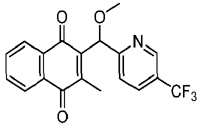 | 124 | ++++ | | | | |
| 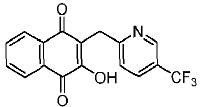 | 125 | + | | | | |
| 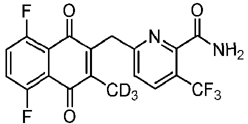 | 126 | | | ++++ | | |
| 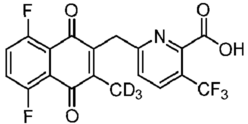 | 127 | | | +++ | | |
| 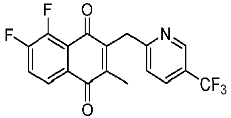 | 128 | | ++++ | | 1.304 | |
| 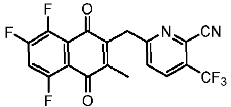 | 130 | | + | | | |
| 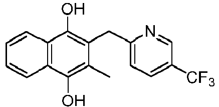 | 131 | ++++ | ++++ | | | |
| 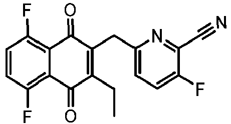 | 132 | | | ++++ | | |
| 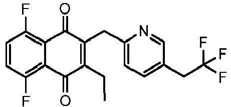 | 133 | | | ++++ | | |
| 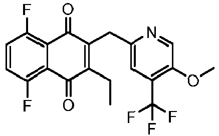 | 134 | | | ++++ | | |
| 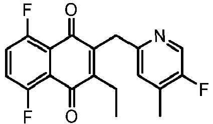 | 135 | | | ++++ | | |
| 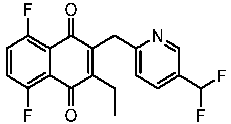 | 136 | | | ++++ | | |
| 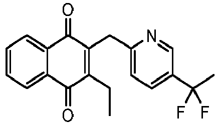 | 137 | | | ++++ | | |
| 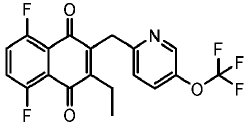 | 138 | | | ++++ | | |
| 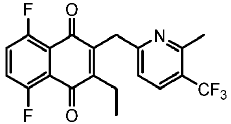 | 139 | | | ++++ | | |
| 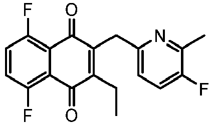 | 140 | | | ++++ | | |
| 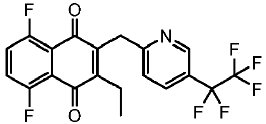 | 141 | | | ++++ | | |
| 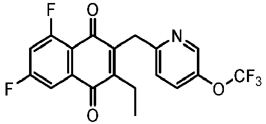 | 142 | | | ++++ | | |
| 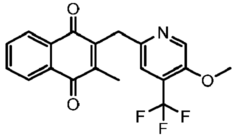 | 145 | | | ++++ | | |
| 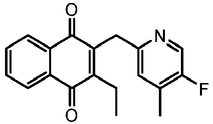 | 146 | | | ++++ | | |
| 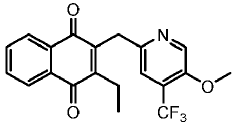 | 147 | | | ++++ | | |
| 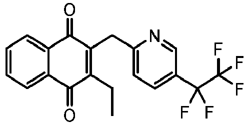 | 148 | | | ++++ | | |
| 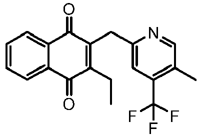 | 149 | | | ++++ | | |
| 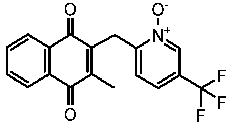 | 150 | | | ++++ | | |
| 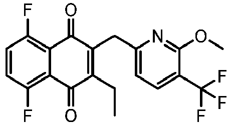 | 152 | | | ++++ | | |
| 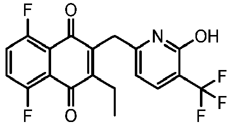 | 153 | | | ++++ | | |

| | | | | | | |
|---|---|---|---|---|---|---|
| *++++ is ≤50 nM; +++ is >50 to 250 nM; ++ is >250 to 1000 nM; + is >1000 nM | | | | | | |

Table 4 compares CYP3A4 IC₅₀ for a compound of the invention with two comparison compounds, wherein the orientation of the pyridine ring is different. Comparison compound 1 is a very potent inhibitor of CYP3A4, and Comparison compound 2 is a potent CYP3A4 inhibitor.

**Table 4: CYP3A4 Inhibition Comparison**

| **Compound** | **Synthetic Example No.** | **CYP3A4 IC₅₀ (uM) (Assay 1)** | **CYP3A4 IC₅₀ (uM) (Assay 2)** |
|---|---|---|---|
| 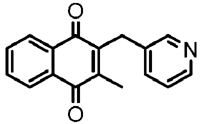 | Comparison Compound 1 | 0.4213 | |
| 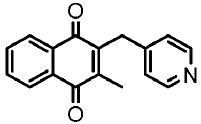 | Comparison Compound 2 | 1.068 | |
| 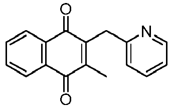 | 023 | 22.75 | 12.7 |

### Example 155. Administration of compounds disclosed herein

A compound disclosed herein is presented in a capsule containing 300 mg of compound in a pharmaceutically acceptable carrier. A capsule is taken orally, once a day, preferably during breakfast or lunch. In the case of very young children, the capsule is broken and its contents are mixed with food.

The invention is defined in the claims. Any aspects, embodiments and examples of the present disclosure which do not fall under the scope of the appended claims do not form part of the invention and are merely provided for illustrative purposes.

## Claims

1. A compound of the formula: 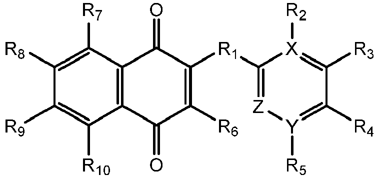 or the reduced form thereof;
wherein:
X and Y are C; or X is N, Y is C, and R₂ is not present; or X is C, Y is N, and R₅ is not present;
Z is N or N-oxide, wherein when Z is N-oxide then X and Y are C;
R₁ is -C(R₁₁)(R₁₂)-;
R₂, R₃, R₄, and R₅ are each independently selected from the group consisting of hydrogen, C₁-C₆ haloalkyl, halo, C₁-C₆ alkyl, C₂-C₆ alkenyl, C₂-C₆ alkynyl, cyano, C₁-C₆ alkoxy, -C(O)OH, -C(O)O(phenyl), -C(O)NR₁₃R₁₄, -OH, and C₁-C₆ haloalkoxy;
R₆ is C₁-C₆ alkyl, H, or OH; where the C₁-C₆ alkyl is optionally substituted with a 3-7 membered heterocyclic group which is optionally substituted with one C₁-C₆ alkyl or C₁-C₆ haloalkyl;
R₇, R₈, R₉, and R₁₀ are each independently selected from the group consisting of: hydrogen, halo, C₁-C₆ alkyl, C₁-C₆ haloalkyl, hydroxy, C₁-C₆ alkoxy, -NHSO₂CH₃, and 5-6 membered heteroaryl where the 5-6 membered heteroaryl is optionally substituted with one C₁-C₆ alkyl;
R₁₁ and R₁₂ are independently selected from the group consisting of hydrogen, methyl, and methoxy; with the proviso that R₁₁ and R₁₂ are not both methoxy; and
R₁₃ and R₁₄ are each independently H or C₁-C₆ alkyl; or wherein R₁₃ and R₁₄ together with the nitrogen atom to which they are attached form a 4-6 membered saturated heterocyclic ring in which one of the carbons is optionally replaced by an additional N, and wherein the 4-6 membered saturated heterocyclic ring is optionally substituted with one or more substituents independently selected from the group consisting of halo and C₁-C₆ alkyl;
or any salt(s), stereoisomer, mixture of stereoisomers, isotopologue(s), solvate(s), and/or hydrate(s) thereof.

2. The compound of claim 1, wherein R₁ is:
-CH₂-; or any salt(s), stereoisomer, mixture of stereoisomers, isotopologue(s), solvate(s), and/or hydrate(s) thereof; or
-CD₂-; or any salt(s), stereoisomer, mixture of stereoisomers, isotopologue(s), solvate(s), and/or hydrate(s) thereof; or
-CH(CH₃)-; or any salt(s), stereoisomer, mixture of stereoisomers, isotopologue(s), solvate(s), and/or hydrate(s) thereof; or
-CH(OCH₃)- ; or any salt(s), stereoisomer, mixture of stereoisomers, isotopologue(s), solvate(s), and/or hydrate(s) thereof.

3. The compound of claim 1 or claim 2, wherein R₁₃ and R₁₄ are each independently H or C₁-C₆ alkyl; or any salt(s), stereoisomer, mixture of stereoisomers, isotopologue(s), solvate(s), and/or hydrate(s) thereof.

4. The compound of claim 1 or claim 2, wherein R₂, R₃, R₄, and R₅ are each independently selected from the group consisting of: hydrogen, -CHF₂, -CF₃, -CF₂CF₃, -CF₂CH₃, -CH₂CF₃, -F, -Cl, -CH₃, -CH₂CH₃, -CH(CH₃)₂, -OCH₃, -OCF₃, and -OCHF₂, -C≡CH, -C≡CCH₃, -OH, -CN, -C(O)OH, -C(O)-NH₂, -C(O)-N(CH₂CH₃)(CH(CH₃)₂), -C(O)-N(H)(CH₂-cyclopropyl), -C(O)-N(H)(cyclopentyl), -C(O)-N(CH₃)(CH(CH₃)₂), and 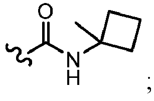 or any salt(s), stereoisomer, mixture of stereoisomers, isotopologue(s), solvate(s), and/or hydrate(s) thereof; or
wherein zero to two of R₂, R₃, R₄, and R₅ are independently selected from the group consisting of -CHF₂, -CF₃, -CF₂CF₃, -CF₂CH₃, -CH₂CF₃, -F, -Cl, -CH₃, -CH₂CH₃, - CH(CH₃)₂, -OCH₃, -OCF₃, and -OCHF₂, -C≡CH, -C≡CCH₃, -OH, -CN, -C(O)OH, -C(O)-NH₂, -C(O)-N(CH₂CH₃)(CH(CH₃)₂), -C(O)-N(H)(CH₂-cyclopropyl), -C(O)-N(H)(cyclopentyl), -C(O)-N(CH₃)(CH(CH₃)₂), and 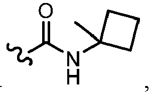 and wherein the others are hydrogen; or any salt(s), stereoisomer, mixture of stereoisomers, isotopologue(s), solvate(s), and/or hydrate(s) thereof; or
wherein zero to two of R₂, R₃, R₄, and R₅ are independently selected from the group consisting of -CF₃, -CHF₂, F, Cl, -CH₃, -OCF₃, and -CN, and wherein the others are hydrogen; or any salt(s), stereoisomer, mixture of stereoisomers, isotopologue(s), solvate(s), and/or hydrate(s) thereof.

5. The compound of any one of claims 1-4, wherein R₆ is selected from the group consisting of: -CH₃, -CD₃, -CH₂CH₃, -CH₂CH₂CH₃, -CH(CH₃)₂, -CH₂CH(CH₃)₂, -CH₂CH₂CH(CH₃)₂, -CH₂C(CH₃)₃, -cyclopropyl, 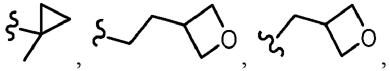 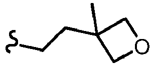 , -H, and -OH; or any salt(s), stereoisomer, mixture of stereoisomers, isotopologue(s), solvate(s), and/or hydrate(s) thereof; or
wherein R₆ is not methyl; or any salt(s), stereoisomer, mixture of stereoisomers, isotopologue(s), solvate(s), and/or hydrate(s) thereof.

6. The compound of any one of claims 1-5, wherein R₇, R₈, R₉, and R₁₀ are hydrogen; or any salt(s), stereoisomer, mixture of stereoisomers, isotopologue(s), solvate(s), and/or hydrate(s) thereof; or
wherein R₇, R₈, R₉, and R₁₀ are each independently selected from the group consisting of: H, -CH₃, -OCH₃, -F, -Cl, and -CF₃; or any salt(s), stereoisomer, mixture of stereoisomers, isotopologue(s), solvate(s), and/or hydrate(s) thereof; or
wherein R₇, R₈, R₉, and R₁₀ are each independently selected from the group consisting of: hydrogen and -F; or any salt(s), stereoisomer, mixture of stereoisomers, isotopologue(s), solvate(s), and/or hydrate(s) thereof; or
wherein R₇, R₈, R₉, and R₁₀ are each independently selected from the group consisting of: hydrogen and -Cl; or any salt(s), stereoisomer, mixture of stereoisomers, isotopologue(s), solvate(s), and/or hydrate(s) thereof.

7. The compound of any one of claims 1-5, wherein zero to three of R₇, R₈, R₉, and R₁₀ are each independently selected from the group consisting of: -CH₃, -OCH₃, -F, -Cl, and -CF₃, and wherein the others are hydrogen; or any salt(s), stereoisomer, mixture of stereoisomers, isotopologue(s), solvate(s), and/or hydrate(s) thereof; or
wherein zero to three of R₇, R₈, R₉, and R₁₀ are -F, and wherein the others are hydrogen; or any salt(s), stereoisomer, mixture of stereoisomers, isotopologue(s), solvate(s), and/or hydrate(s) thereof; or
wherein one or two of R₇, R₈, R₉, and R₁₀ are each independently selected from the group consisting of: -CH₃, -OCH₃, -F, -Cl, and -CF₃, and wherein the others are hydrogen; or any salt(s), stereoisomer, mixture of stereoisomers, isotopologue(s), solvate(s), and/or hydrate(s) thereof; or
wherein one or two of R₇, R₈, R₉, and R₁₀ are -F, and wherein the others are hydrogen; or any salt(s), stereoisomer, mixture of stereoisomers, isotopologue(s), solvate(s), and/or hydrate(s) thereof; or
wherein one or two of R₇, R₈, R₉, and R₁₀ are -Cl, and wherein the others are hydrogen; or any salt(s), stereoisomer, mixture of stereoisomers, isotopologue(s), solvate(s), and/or hydrate(s) thereof; or
wherein one or two of R₇, R₈, R₉, and R₁₀ are each independently selected from the group consisting of: -F and -Cl, and wherein the others are hydrogen; or any salt(s), stereoisomer, mixture of stereoisomers, isotopologue(s), solvate(s), and/or hydrate(s) thereof.

8. The compound of any one of claims 1-7, wherein Z is N; or any salt(s), stereoisomer, mixture of stereoisomers, isotopologue(s), solvate(s), and/or hydrate(s) thereof.

9. The compound of any one of claims 1-8, wherein X and Y are C; or any salt(s), stereoisomer, mixture of stereoisomers, isotopologue(s), solvate(s), and/or hydrate(s) thereof.

10. The compound of claim 1, wherein the compound is selected from the group consisting of: 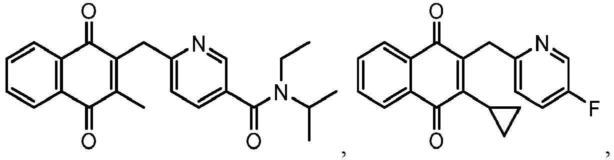 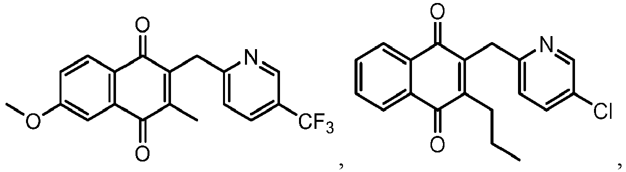 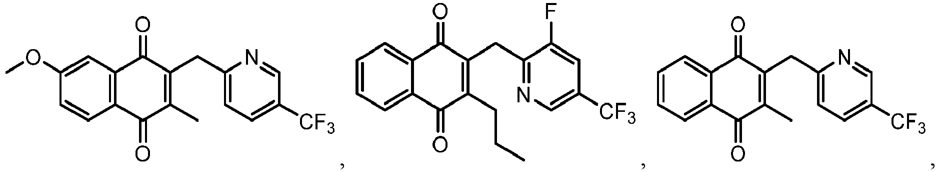 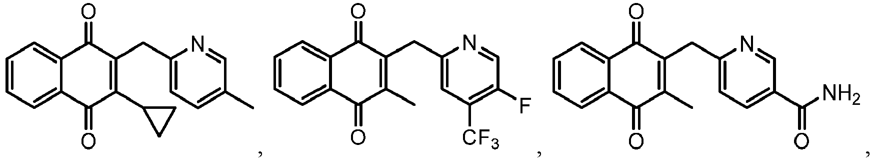 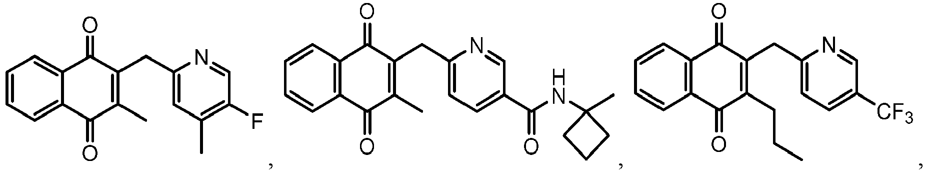 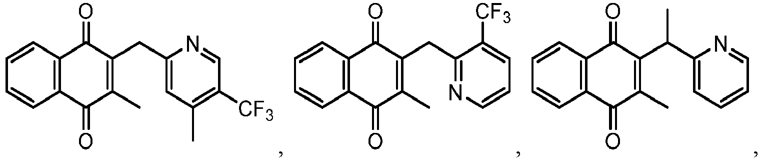 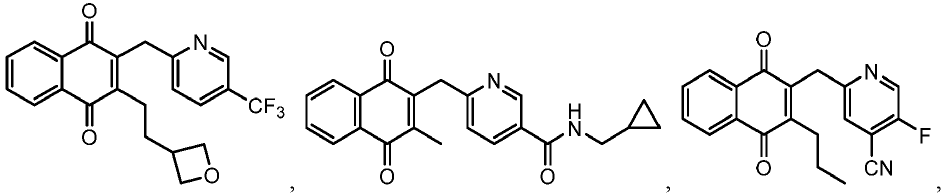 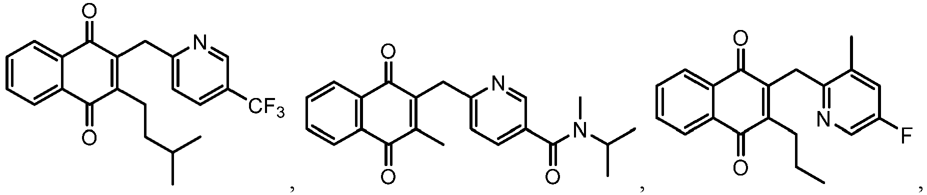 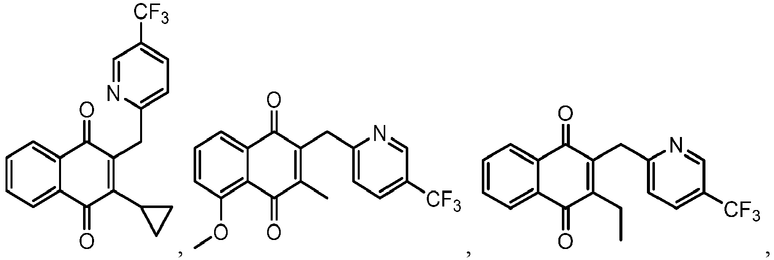 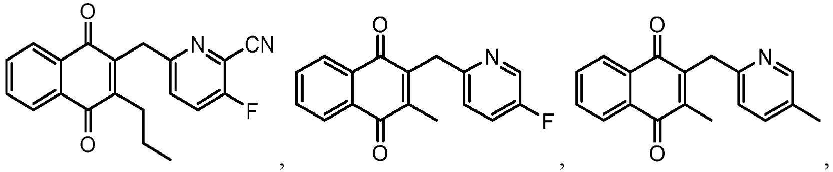 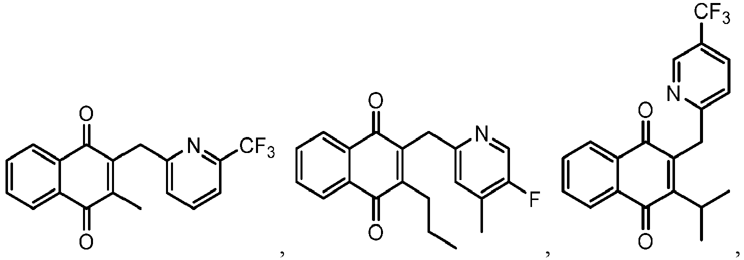 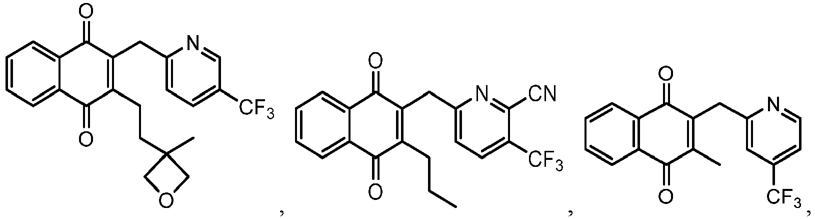 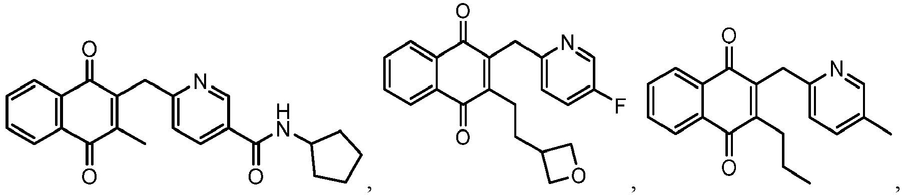 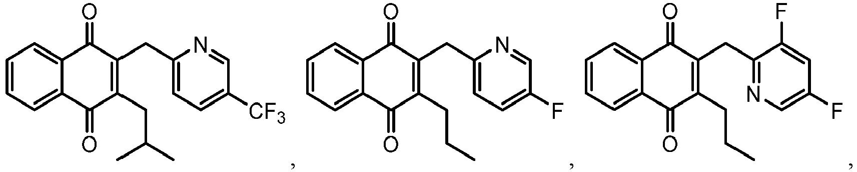 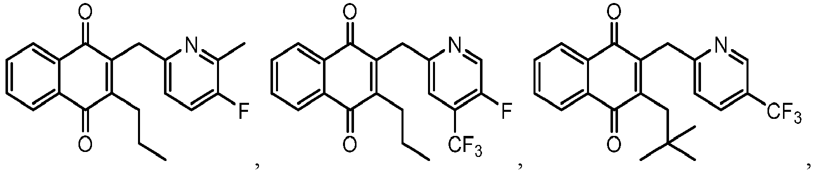 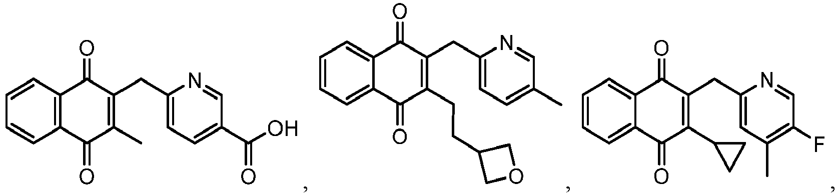 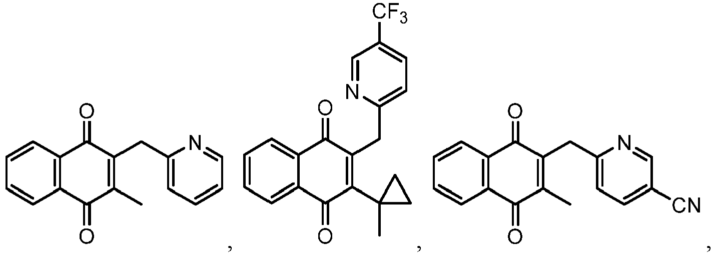 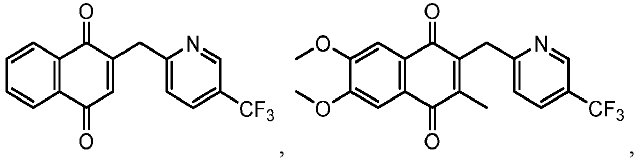 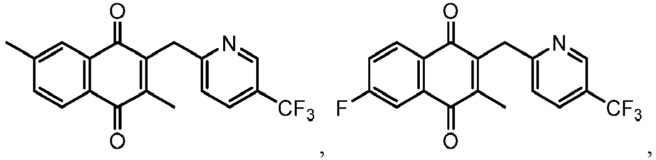 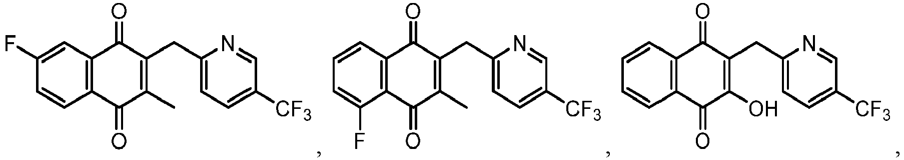 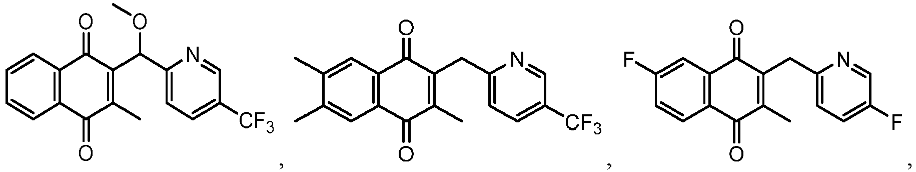 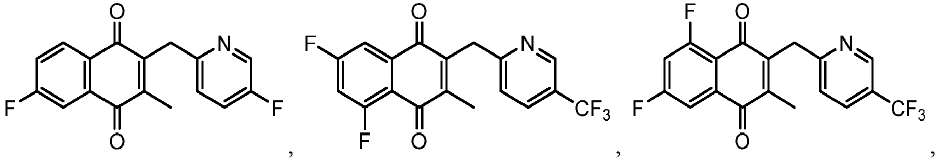 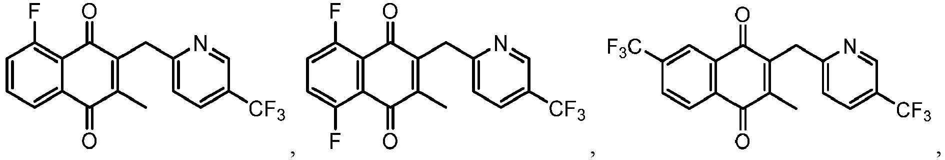 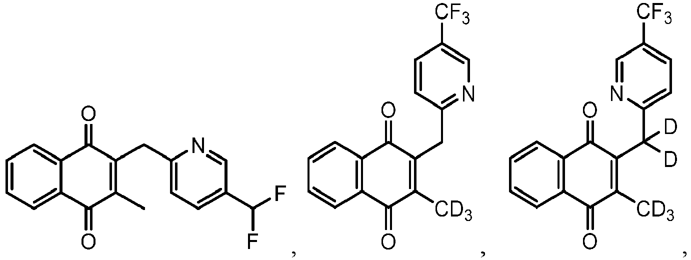 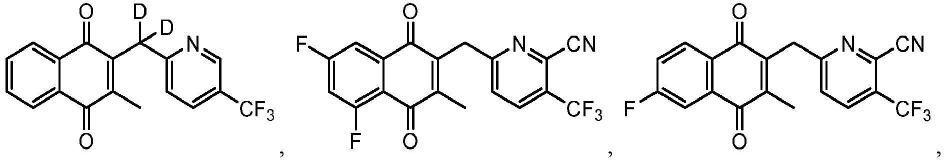 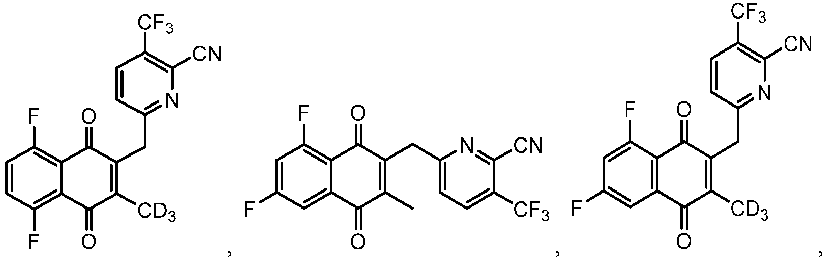 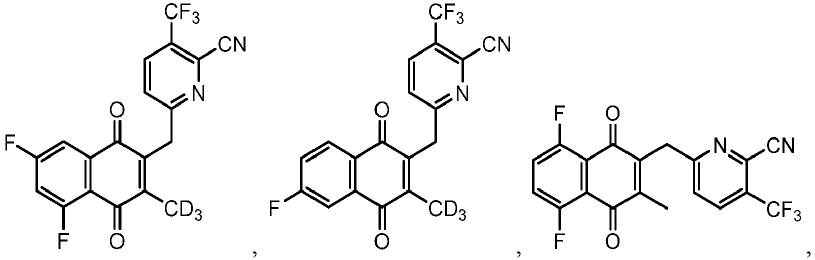 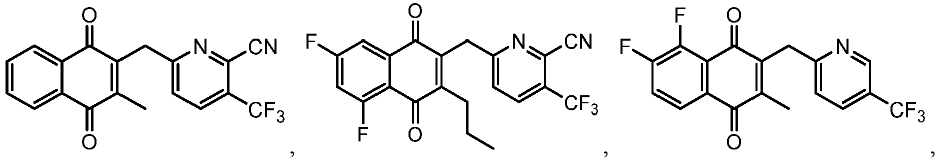 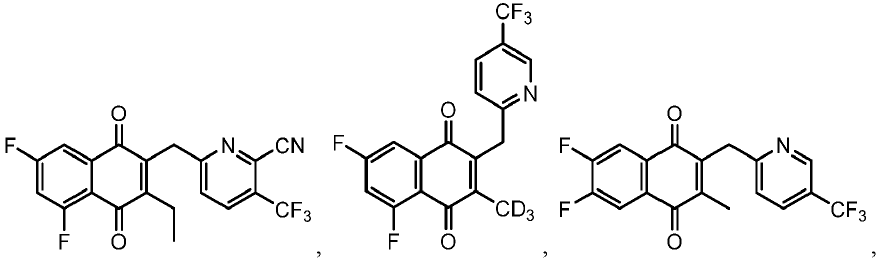 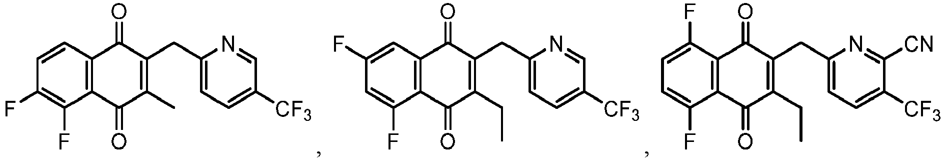 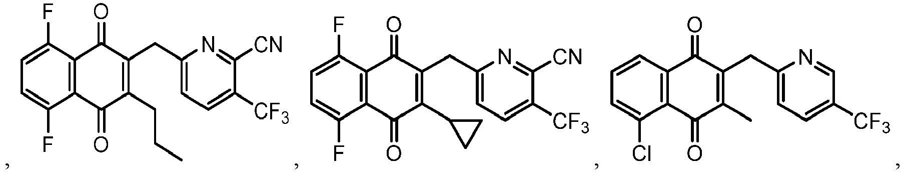 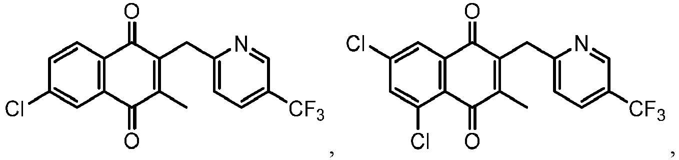 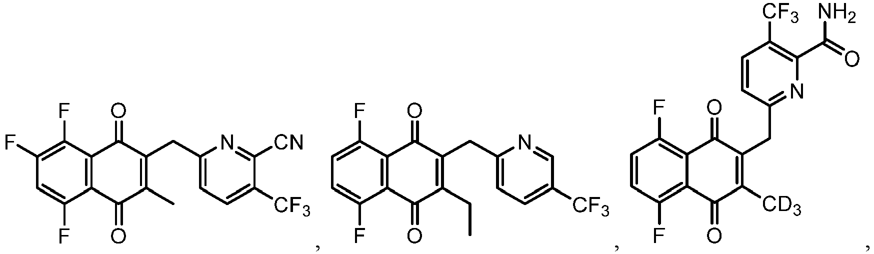 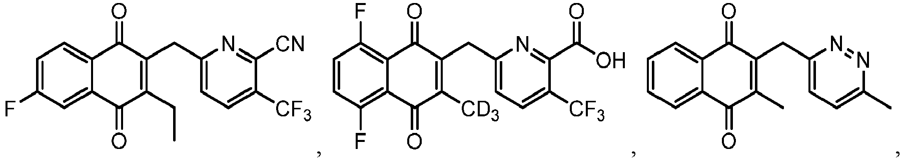 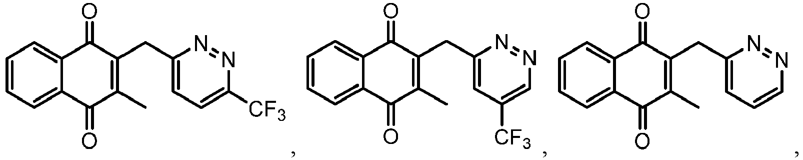 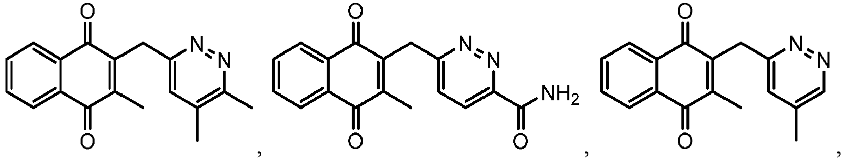 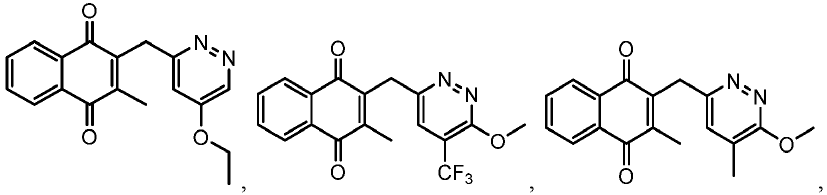 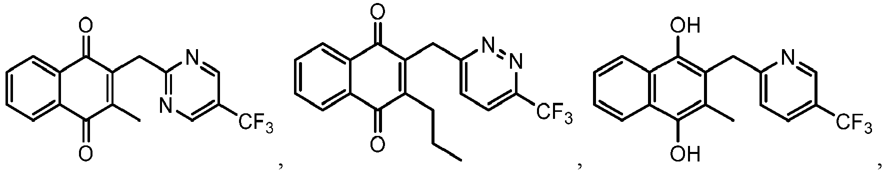 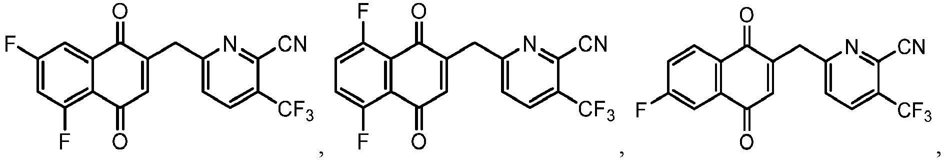 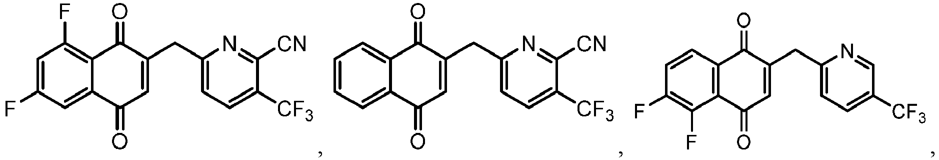 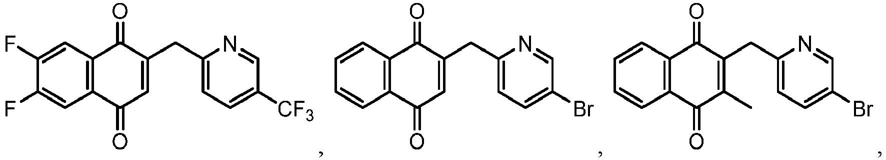 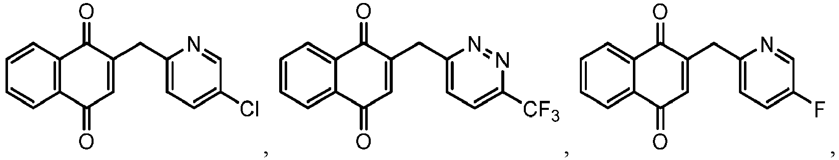 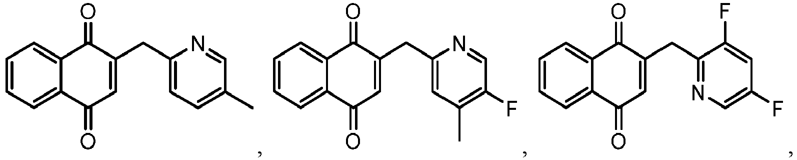 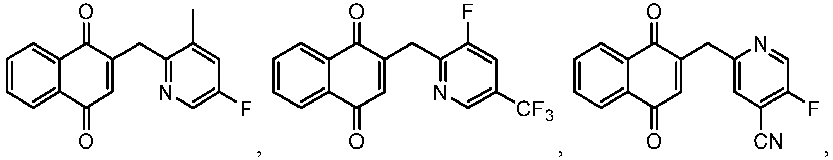 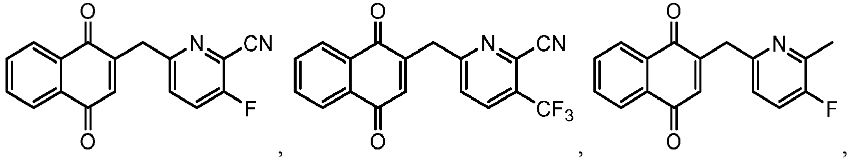 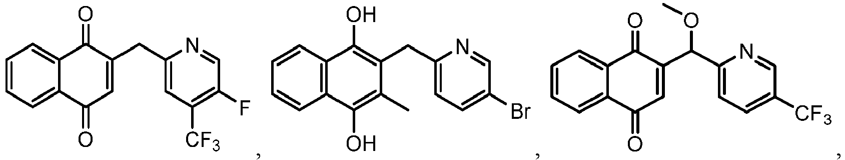 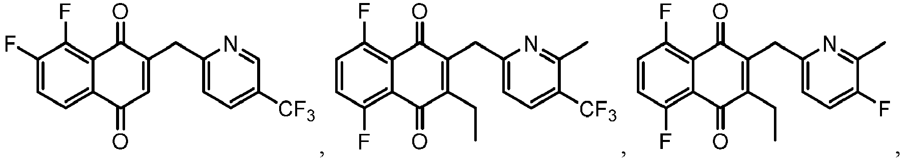 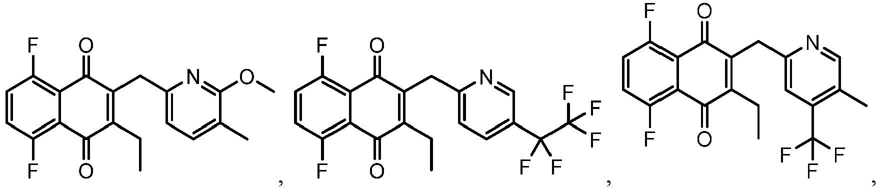 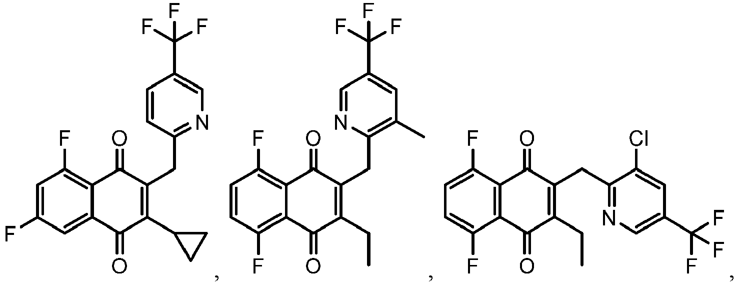 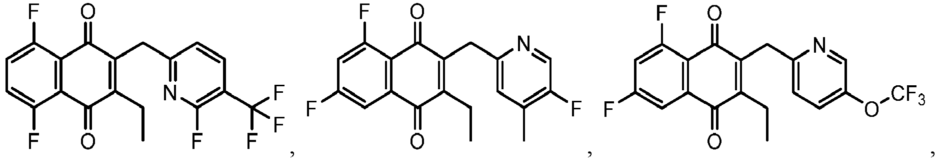 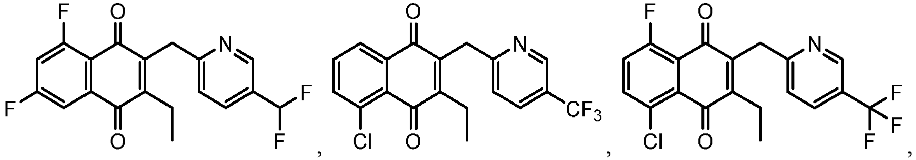 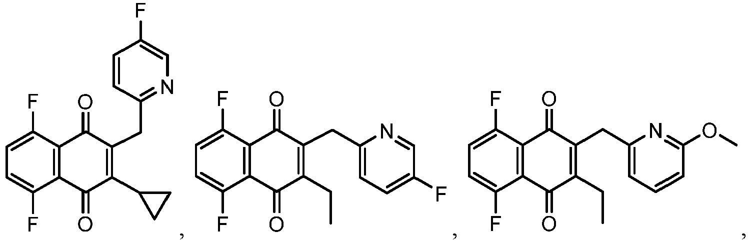 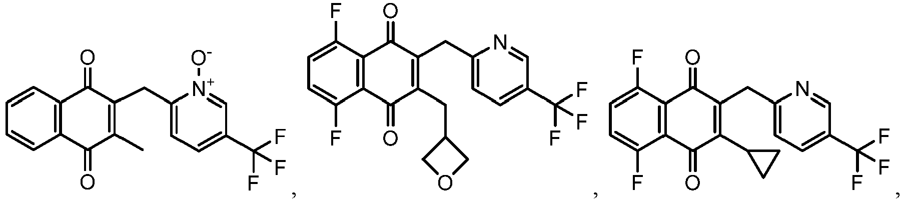 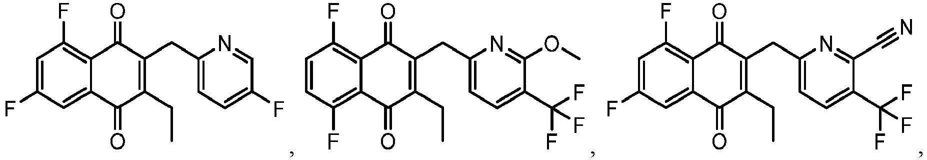 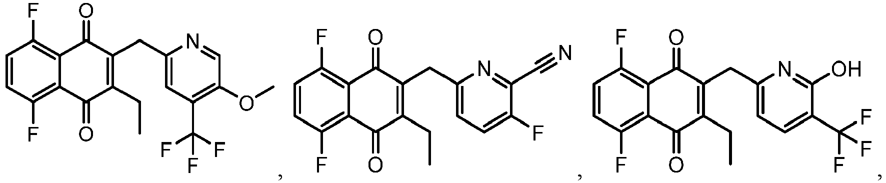 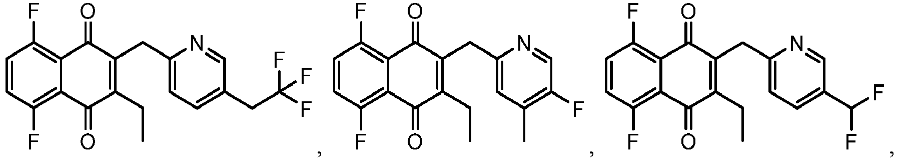 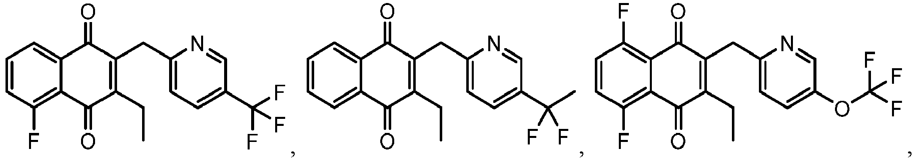 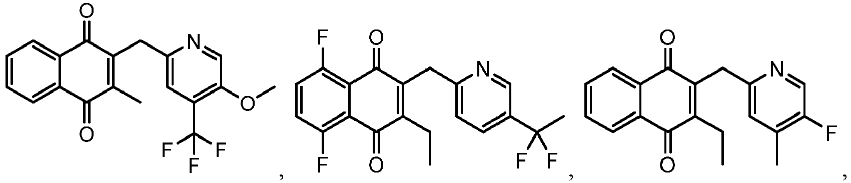 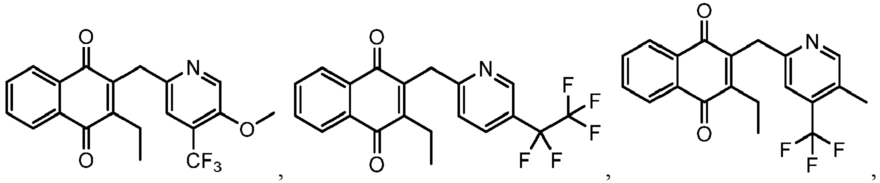 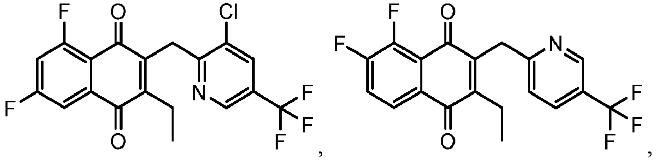 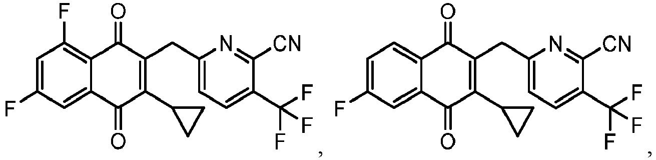 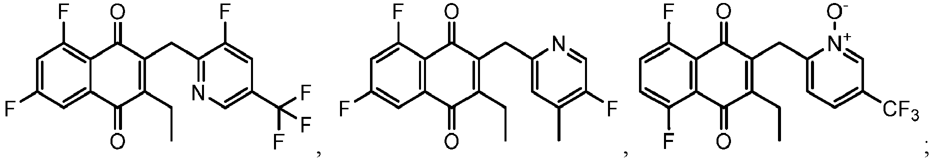 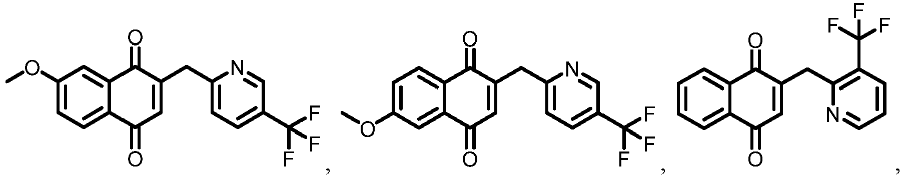 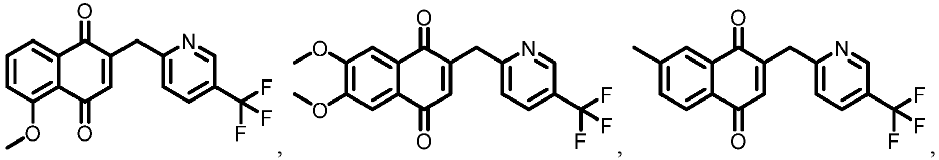 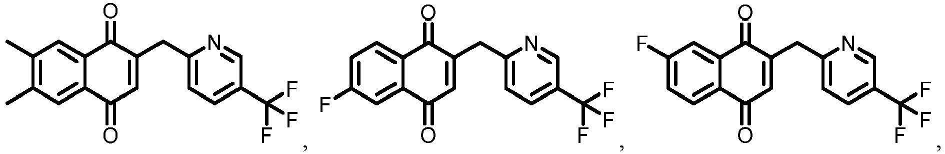 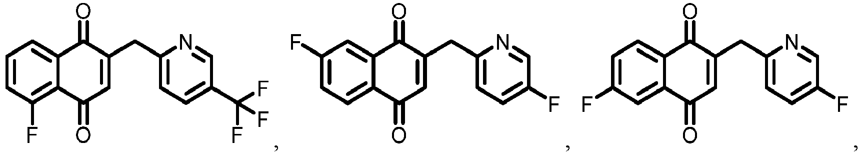 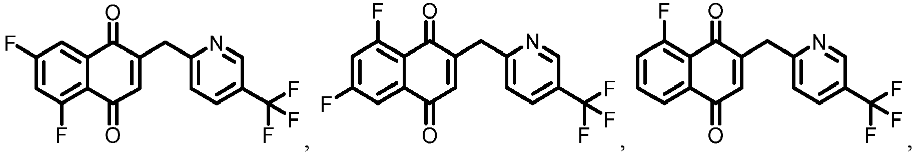 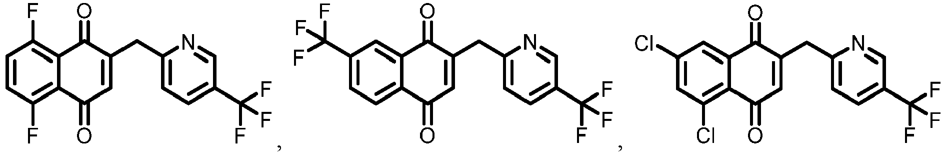 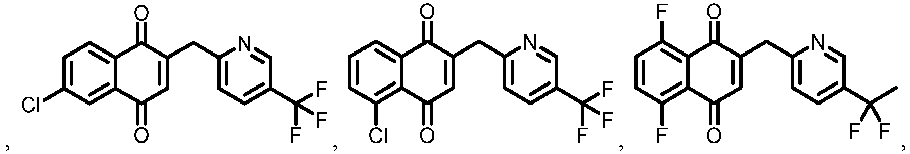 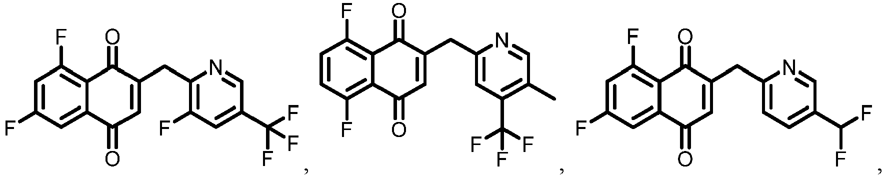 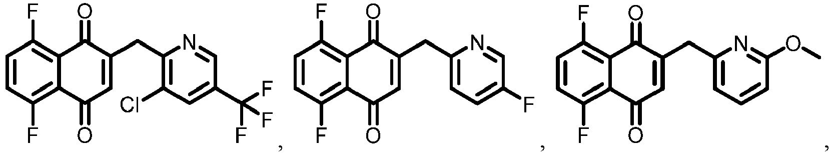 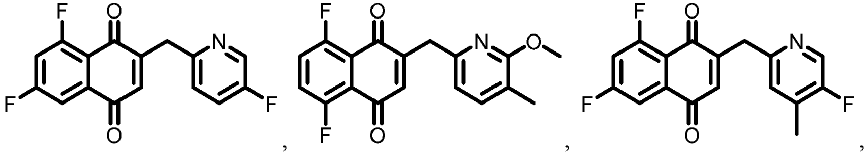 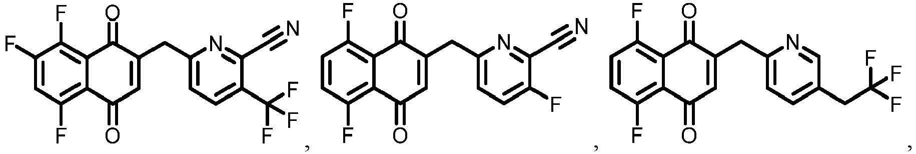 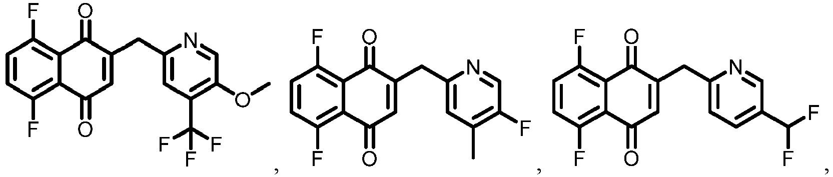 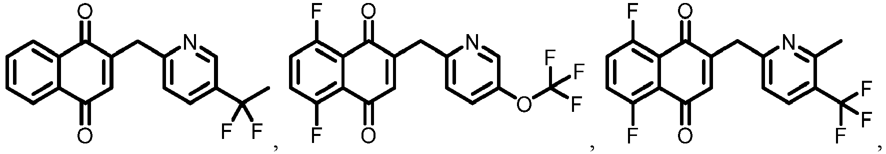 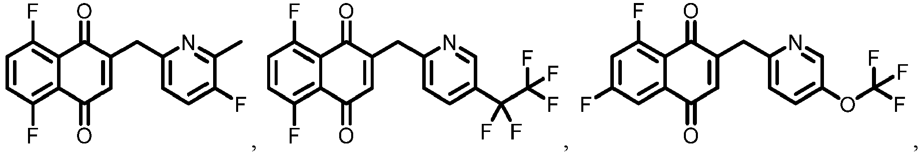 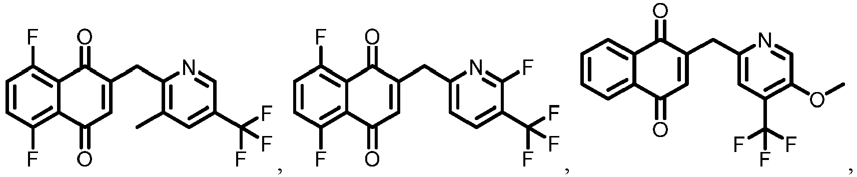 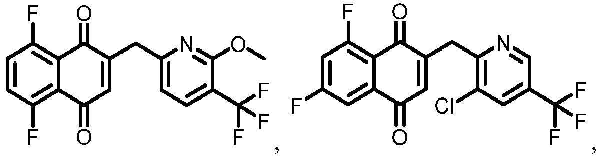 and 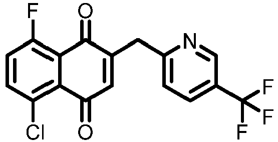 ; and the reduced forms thereof; and all salts, stereoisomers, mixtures of stereoisomers, isotopologues, solvates, and/or hydrates thereof.

11. The compound of any one of claims 1-10 wherein the compound is in the oxidized (quinone) form.

12. The compound of any one of claims 1-11 wherein the compound is not a salt.

13. The compound of any one of claims 1-11 wherein the compound is a pharmaceutically acceptable salt.

14. A pharmaceutical composition comprising a compound according to any one of claims 1-13 or a salt, stereoisomer, mixture of stereoisomers, isotopologue, solvate, and/or hydrate thereof; and a pharmaceutically acceptable carrier.

15. A compound according to any one of claims 1-13 or a salt, stereoisomer, mixture of stereoisomers, isotopologue, solvate, and/or hydrate thereof, or a pharmaceutical composition according to claim 14, for use in a method of treating or suppressing an oxidative stress disorder, the method comprising administering to a subject in need thereof a therapeutically effective amount of the compound, salt, stereoisomer, mixture of stereoisomers, isotopologue, solvate, and/or hydrate thereof, or pharmaceutical composition;
wherein when the compound is a salt, the salt is a pharmaceutically acceptable salt;
optionally wherein the oxidative stress disorder is selected from the group consisting of: a neurodegenerative disease; an α-synucleinopathy; Parkinson's disease; familial Parkinson's Disease; idiopathic Parkinson's Disease; Parkinson's Disease wherein the patient has a mutation in one or more of the following genes: MAPT (Microtubule-associated protein tau), PRKN (parkin), PINK1 (PINK1), LRRK2 (leucine-rich repeat kinase 2), GBA (glucocerebrosidase), SNCA (alpha synuclein), PARK7 (DJ-1), and/or UCHL1 (ubiquitin carboxyl-terminal esterase L1); Parkinson's Disease with dementia (PDD); multisystem atrophy (MSA); Frontotemporal Dementia (FTD); Dementia with Lewy Bodies (DLB); Gaucher's disease (GD); Neurodegeneration with Brain Iron Accumulation (NBIA); neuroaxonal dystrophies (PLA2G6-associated neurodegeneration); a tauopathy; Alzheimer's disease; dementia pugilistica; Guam Amyotrophic lateral sclerosis-Parkinsonism-Dementia (Guam ALS/PD); Pick Disease; Argyrophilic grain dementia; Nieman-Pick type C; Subacute sclerosing panencephalitis (SSPE); Progressive supranuclear palsy (PSP); Corticobasoganglionic degeneration; Frontotemporal dementia with parkinsonism-17 (FTDP-17); Postencephalitic Parkinsonism (PEP); Autosomal recessive Parkinsonism; Huntington's Disease; Juvenile Huntington's Disease; amyotrophic lateral sclerosis (ALS); a motor neuron disease; a leukodystrophy; Adrenoleukodystrophy; Adrenomyeloneuropathy; traumatic brain injury; chronic traumatic encephalopathy (CTE); spinal muscular atrophy (SMA); a neurological disease; epilepsy; seizures; a mood disorder; schizophrenia; bipolar disorder; attention deficit/hyperactivity disorder (ADHD); Tourette syndrome; a pervasive developmental disorder; Down's syndrome; autistic disorder; Asperger's syndrome; childhood disintegrative disorder (CDD); Rett syndrome; CDKL5 deficiency disorder; PDD-not otherwise specified (PDD-NOS); NGLY1-congenital disorder of deglycosylation; a mitochondrial disorder; an inherited mitochondrial disease; Alpers Disease; Barth syndrome; a Beta-oxidation Defect; Carnitine-Acyl-Carnitine Deficiency; Carnitine Deficiency; a Creatine Deficiency Syndrome; Co-Enzyme Q10 Deficiency; Complex I Deficiency; Complex II Deficiency; Complex III Deficiency; Complex IV Deficiency; Complex V Deficiency; COX Deficiency; chronic progressive external ophthalmoplegia (CPEO); CPT I Deficiency; CPT II deficiency; Friedreich's Ataxia (FA); Glutaric Aciduria Type II; Kearns-Sayre Syndrome (KSS); Lactic Acidosis; Long-Chain Acyl-CoA Dehydrongenase Deficiency (LCAD); Long-chain 3-Hydroxyacyl-CoA Dehydrogenase Deficiency (LCHAD); Leigh Syndrome; Leigh-like Syndrome; Leber's Hereditary Optic Neuropathy (LHON); Lethal Infantile Cardiomyopathy (LIC); Luft Disease; Multiple Acyl-CoA Dehydrogenase Deficiency (MAD); Medium-Chain Acyl-CoA Dehydrongenase Deficiency (MCAD); Mitochondrial Myopathy, Encephalopathy, Lactacidosis, Stroke (MELAS); Myoclonic Epilepsy with Ragged Red Fibers (MERRF); Mitochondrial Recessive Ataxia Syndrome (MIRAS); Mitochondrial Cytopathy, Mitochondrial DNA Depletion; Mitochondrial Encephalopathy; Mitochondrial Myopathy; Myoneurogastointestinal Disorder and Encephalopathy (MNGIE); Neuropathy, Ataxia, and Retinitis Pigmentosa (NARP); Pearson Syndrome; Pyruvate Carboxylase Deficiency; Pyruvate Dehydrogenase Deficiency; disorder associated with a POLG Mutation; a Respiratory Chain Disorder; Short-Chain Acyl-CoA Dehydrogenase Deficiency (SCAD); Short-chain 3-Hydroxyacyl-CoA Dehydrogenase Deficiency (SCHAD); Very Long-Chain Acyl-CoA Dehydrongenase Deficiency (VLCAD); a myopathy; cardiomyopathy; encephalomyopathy; a mitochondrial myopathy; a primary mitochondrial myopathy; a muscular dystrophy; Duchenne muscular dystrophy (DMD); Becker muscular dystrophy; congenital muscular dystrophy; Fukuyama type congenital muscular dystrophy; limb-girdle muscular dystrophy; myotonic dystrophy; a cerebrovascular accident; stroke; a vision impairment; vision disorders; ocular disease; optic neuropathy; dominant inherited juvenile optic atrophy; optic neuropathy caused by a toxic agent; Leber's hereditary optic neuropathy (LHON); Dominant Optic Atrophy (DOA); DOA plus; glaucoma; retinitis pigmentosa; macular degeneration; age-related macular degeneration (AMD); dry AMD; wet AMD; Stargardt's macular dystrophy; diabetic retinopathy; diabetic maculopathy; retinopathy of prematurity; ischemic reperfusion related retinal injury; ischemia; ischemia reperfusion injury; ischemia reperfusion injury due to transplant; ischemia reperfusion injury due to surgery; oxygen poisoning; an age-associated disease; diabetes; metabolic syndrome; Wolfram's disease; cancer; brain cancer; glioblastoma; chronic fatigue; a genetic disease; a haemoglobionopathy; thalassemia; sickle cell anemia; G6PD deficiency; Multiple Sclerosis; a neurodegenerative disorder resulting in hearing or balance impairment; hearing loss; noise induced hearing loss; Maternally inherited diabetes and deafness (MIDD); renal tubular acidosis; acute tubular necrosis; contrast-induced kidney damage; contrast-induced retinopathy damage; Abetalipoproteinemia; cobalamin c defect; methylmalonic aciduria; and radiation damage or injury.

16. The compound or pharmaceutical composition for use according to claim 15, wherein the oxidative stress disorder is selected from the group consisting of Parkinson's Disease, Duchenne muscular dystrophy, Leigh Syndrome, Complex I Deficiency, Huntington's Disease, Alzheimer's Disease, Juvenile Huntington's Disease, amyotrophic lateral sclerosis, frontotemporal dementia (FTD), and Friedreich's Ataxia.

## Patentansprüche

1. Verbindung der folgenden Formel: 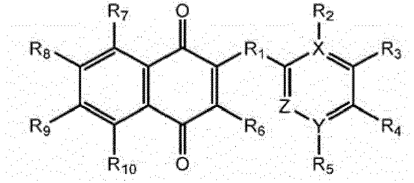 oder die reduzierte Form davon;
wobei:
X und Y C sind; oder X N ist, Y C ist und R₂ nicht vorhanden ist; oder X C ist, Y N ist und R₅ nicht vorhanden ist;
Z N oder N-Oxid ist, wobei, wenn Z N-Oxid ist, dann X und Y C sind;
R₁ -C(R₁₁)(R₁₂)- ist;
R₂, R₃, R₄ und R₅ jeweils unabhängig ausgewählt sind aus der Gruppe bestehend aus Wasserstoff, C₁-C₆-Haloalkyl, Halo, C₁-C₆-Alkyl, C₂-C₆-Alkenyl, C₂-C₆-Alkynyl, Cyan, C₁-C₆-Alkoxy, -C(O)OH, -C(O)O(Phenyl), -C(O)NR₁₃R₁₄, -OH und C₁-C₆-Haloalkoxy;
R₆ C₁-C₆-Alkyl, H oder OH ist; wobei das C₁-C₆-Alkyl optional substituiert ist mit einer 3-7-gliedrigen heterocyclischen Gruppe, die optional substituiert ist mit einem C₁-C₆-Alkyl oder C₁-C₆-Haloalkyl;
R₇, Rs, R₉ und R₁₀ jeweils unabhängig ausgewählt sind aus der Gruppe bestehend aus: Wasserstoff, Halo, C₁-C₆-Alkyl, C₁-C₆-Haloalkyl, Hydroxy, C₁-C₆-Alkoxy, -NHSO₂CH₃ und 5-6-gliedrigem Heteroaryl, wobei das 5-6-gliedrige Heteroaryl optional substituiert ist mit einem C₁-C₆-Alkyl;
R₁₁ und R₁₂ unabhängig ausgewählt sind aus der Gruppe bestehend aus Wasserstoff, Methyl und Methoxy; unter der Bedingung, dass R₁₁ und R₁₂ nicht beide Methoxy sind; und
R₁₃ und R₁₄ jeweils unabhängig H oder C₁-C₆-Alkyl sind; oder wobei R₁₃ und R₁₄ zusammen mit dem Stickstoffatom, an dem sie befestigt sind, einen 4-6-gliedrigen, gesättigten heterocyclischen Ring bilden, in dem einer der Kohlenstoffe optional ersetzt ist durch ein zusätzliches N, und wobei der 4-6-gliedrige, gesättigte heterocyclische Ring optional substituiert ist mit einem oder mehreren Substituenten, unabhängig ausgewählt aus der Gruppe bestehend aus Halo und C₁-C₆-Alkyl;
oder jegliche(s) Salz(e), Stereoisomer, Gemisch aus Stereoisomeren, Isotopolog(e), Solvat(e) und/oder Hydrat(e) davon.

2. Verbindung nach Anspruch 1, wobei Ri Folgendes ist:
-CH₂-; oder jegliche(s) Salz(e), Stereoisomer, Gemisch aus Stereoisomeren, Isotopolog(e), Solvat(e) und/oder Hydrat(e) davon; oder
-CD₂-; oder jegliche(s) Salz(e), Stereoisomer, Gemisch aus Stereoisomeren, Isotopolog(e), Solvat(e) und/oder Hydrat(e) davon; oder
-CH(CH₃)-; oder jegliche(s) Salz(e), Stereoisomer, Gemisch aus Stereoisomeren, Isotopolog(e), Solvat(e) und/oder Hydrat(e) davon; oder
-CH(OCH₃)-; oder jegliche(s) Salz(e), Stereoisomer, Gemisch aus Stereoisomeren, Isotopolog(e), Solvat(e) und/oder Hydrat(e) davon.

3. Verbindung nach Anspruch 1 oder Anspruch 2, wobei R₁₃ und R₁₄ jeweils unabhängig H oder C₁-C₆-Alkyl sind; oder jegliche(s) Salz(e), Stereoisomer, Gemisch aus Stereoisomeren, Isotopolog(e), Solvat(e) und/oder Hydrat(e) davon.

4. Verbindung nach Anspruch 1 oder Anspruch 2, wobei R₂, R₃, R₄ und R₅ jeweils unabhängig ausgewählt sind aus der Gruppe bestehend aus: Wasserstoff, -CHF₂, -CF₃, - CF₂CF₃, -CF₂CH₃, -CH₂CF₃, -F, -Cl, -CH₃, -CH₂CH₃, -CH(CH₃)₂, -OCH₃, -OCF₃ und -OCHF₂, -C=CH, -C≡CCH₃, -OH, -CN, -C(O)OH, -C(O)-NH₂, -C(O)-N(CH₂CH₃)(CH(CH₃)₂), -C(O)-N(H)(CH₂-Cyclopropyl), -C(O)-N(H)(Cyclopentyl), -C(O)-N(CH₃)(CH(CH₃)₂) und 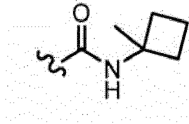 ; oder jegliche(s) Salz(e), Stereoisomer, Gemisch aus Stereoisomeren, Isotopolog(e), Solvat(e) und/oder Hydrat(e) davon; oder
wobei null bis zwei aus R₂, R₃, R₄ und R₅ unabhängig ausgewählt sind aus der Gruppe bestehend aus -CHF₂, -CF₃, -CF₂CF₃, -CF₂CH₃, -CH₂CF₃, -F, -Cl, -CH₃, -CH₂CH₃, -CH(CH₃)₂, -OCH₃, -OCF₃ und -OCHF₂, -C=CH, -C≡CCH₃, -OH, -CN, -C(O)OH, -C(O)-NH₂, -C(O)-N(CH₂CH₃)(CH(CH₃)₂), -C(O)-N(H)(CH₂-Cyclopropyl), -C(O)-N(H)(Cyclopentyl), -C(O)-N(CH₃)(CH(CH₃)₂) und 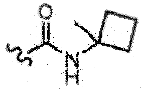 ; und wobei die anderen Wasserstoff sind; oder jegliche(s) Salz(e), Stereoisomer, Gemisch aus Stereoisomeren, Isotopolog(e), Solvat(e) und/oder Hydrat(e) davon; oder
wobei null bis zwei aus R₂, R₃, R₄ und R₅ unabhängig ausgewählt sind aus der Gruppe bestehend aus -CF₃, -CHF₂, F, Cl, -CH₃, -OCF₃ und -CN, und wobei die anderen Wasserstoff sind; oder jegliche(s) Salz(e), Stereoisomer, Gemisch aus Stereoisomeren, Isotopolog(e), Solvat(e) und/oder Hydrat(e) davon.

5. Verbindung nach einem der Ansprüche 1-4, wobei R₆ ausgewählt ist aus der Gruppe bestehend aus: -CH₃, -CD₃, -CH₂CH₃, -CH₂CH₂CH₃, -CH(CH₃)₂, -CH₂CH(CH₃)₂, - CH₂CH₂CH(CH₃)₂, -CH₂C(CH₃)₃, -Cyclopropyl, 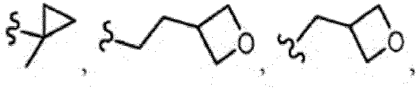 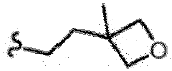 , -H und -OH; oder jegliche(s) Salz(e), Stereoisomer, Gemisch aus Stereoisomeren, Isotopolog(e), Solvat(e) und/oder Hydrat(e) davon; oder
wobei R₆ nicht Methyl ist; oder jegliche(s) Salz(e), Stereoisomer, Gemisch aus Stereoisomeren, Isotopolog(e), Solvat(e) und/oder Hydrat(e) davon.

6. Verbindung nach einem der Ansprüche 1-5, wobei R₇, Rs, R₉ und R₁₀ Wasserstoff sind; oder jegliche(s) Salz(e), Stereoisomer, Gemisch aus Stereoisomeren, Isotopolog(e), Solvat(e) und/oder Hydrat(e) davon; oder
wobei R₇, R₈, R₉ und R₁₀ jeweils unabhängig ausgewählt sind aus der Gruppe bestehend aus: H, -CH₃, -OCH₃, -F, -Cl und -CF₃; oder jegliche(s) Salz(e), Stereoisomer, Gemisch aus Stereoisomeren, Isotopolog(e), Solvat(e) und/oder Hydrat(e) davon;
wobei R₇, R₈, R₉ und R₁₀ jeweils unabhängig ausgewählt sind aus der Gruppe bestehend aus: Wasserstoff und -F; oder jegliche(s) Salz(e), Stereoisomer, Gemisch aus Stereoisomeren, Isotopolog(e), Solvat(e) und/oder Hydrat(e) davon; oder
wobei R₇, R₈, R₉ und R₁₀ jeweils unabhängig ausgewählt sind aus der Gruppe bestehend aus: Wasserstoff und -Cl; oder jegliche(s) Salz(e), Stereoisomer, Gemisch aus Stereoisomeren, Isotopolog(e), Solvat(e) und/oder Hydrat(e) davon.

7. Verbindung nach einem der Ansprüche 1-5, wobei null bis drei aus R₇, R₈, R₉ und R₁₀ jeweils unabhängig ausgewählt sind aus der Gruppe bestehend aus: -CH₃, -OCH₃, -F, -Cl und - CF₃, und wobei die anderen Wasserstoff sind; oder jegliche(s) Salz(e), Stereoisomer, Gemisch aus Stereoisomeren, Isotopolog(e), Solvat(e) und/oder Hydrat(e) davon; oder
wobei null bis drei aus R₇, R₈, R₉ und R₁₀ -F sind, und wobei die anderen Wasserstoff sind; oder jegliche(s) Salz(e), Stereoisomer, Gemisch aus Stereoisomeren, Isotopolog(e), Solvat(e) und/oder Hydrat(e) davon; oder
wobei ein oder zwei aus R₇, R₈, R₉ und R₁₀ jeweils unabhängig ausgewählt sind aus der Gruppe bestehend aus: -CH₃, -OCH₃, -F, -Cl und -CF₃, und wobei die anderen Wasserstoff sind; oder jegliche(s) Salz(e), Stereoisomer, Gemisch aus Stereoisomeren, Isotopolog(e), Solvat(e) und/oder Hydrat(e) davon; oder
wobei ein oder zwei aus R₇, R₈, R₉ und R₁₀ -F sind, und wobei die anderen Wasserstoff sind; oder jegliche(s) Salz(e), Stereoisomer, Gemisch aus Stereoisomeren, Isotopolog(e), Solvat(e) und/oder Hydrat(e) davon; oder
wobei ein oder zwei aus R₇, R₈, R₉ und R₁₀ -Cl sind, und wobei die anderen Wasserstoff sind; oder jegliche(s) Salz(e), Stereoisomer, Gemisch aus Stereoisomeren, Isotopolog(e), Solvat(e) und/oder Hydrat(e) davon; oder
wobei ein oder zwei aus R₇, R₈, R₉ und R₁₀ jeweils unabhängig ausgewählt sind aus der Gruppe bestehend aus: -F und -Cl, und wobei die anderen Wasserstoff sind; oder jegliche(s) Salz(e), Stereoisomer, Gemisch aus Stereoisomeren, Isotopolog(e), Solvat(e) und/oder Hydrat(e) davon.

8. Verbindung nach einem der Ansprüche 1-7, wobei Z N ist; oder jegliche(s) Salz(e), Stereoisomer, Gemisch aus Stereoisomeren, Isotopolog(e), Solvat(e) und/oder Hydrat(e) davon.

9. Verbindung nach einem der Ansprüche 1-8, wobei X und Y C sind; oder jegliche(s) Salz(e), Stereoisomer, Gemisch aus Stereoisomeren, Isotopolog(e), Solvat(e) und/oder Hydrat(e) davon.

10. Verbindung nach Anspruch 1, wobei die Verbindung ausgewählt ist aus der Gruppe bestehend aus: 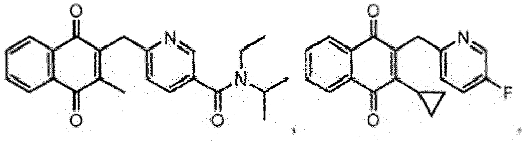 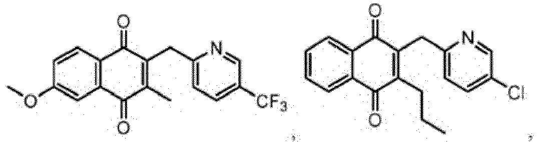 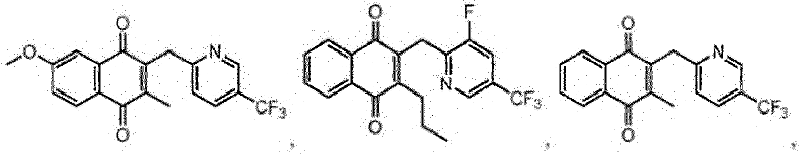 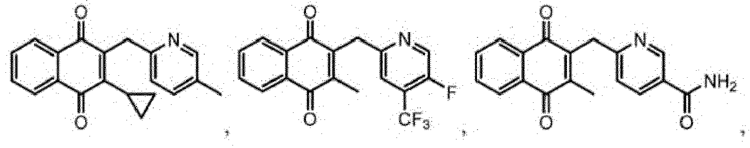 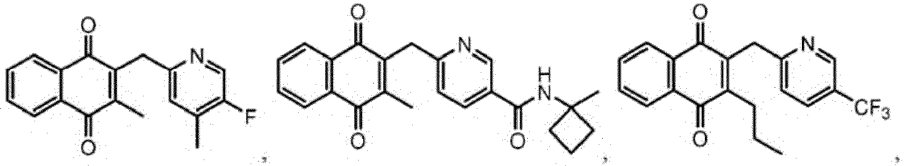 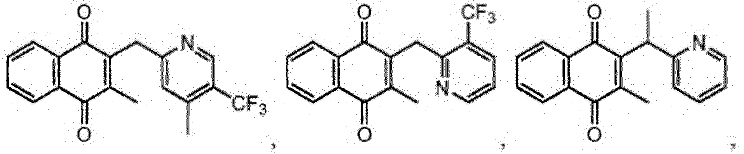 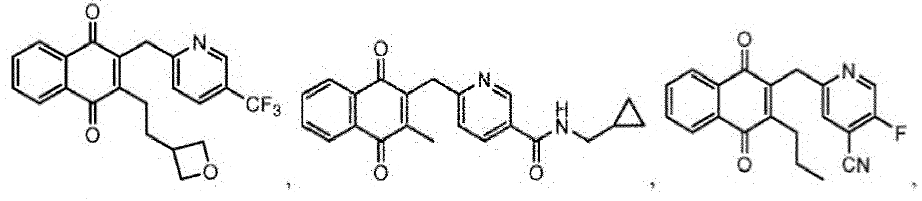 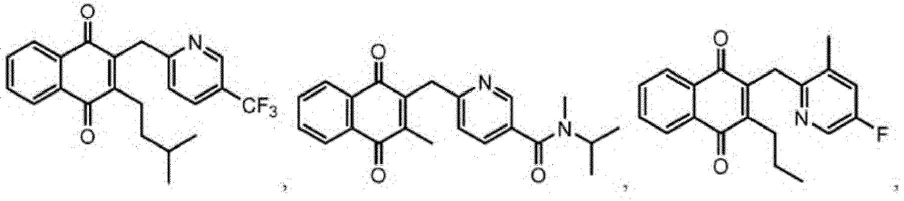 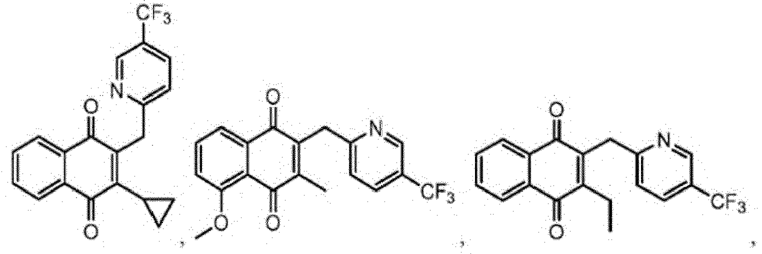 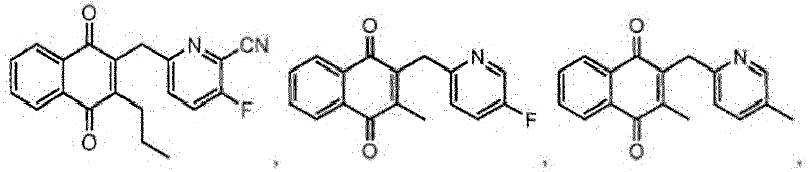 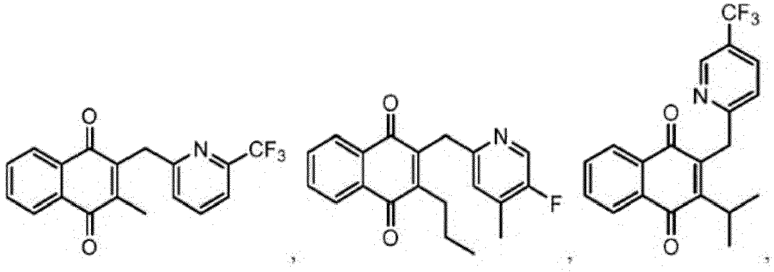 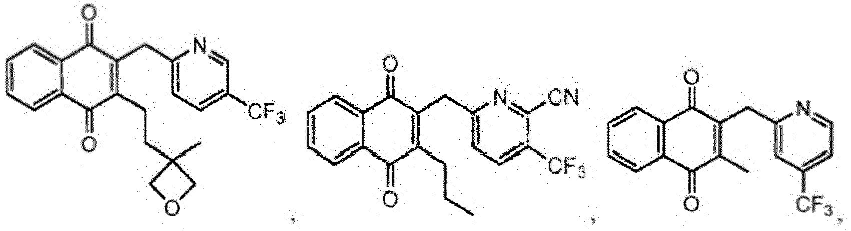 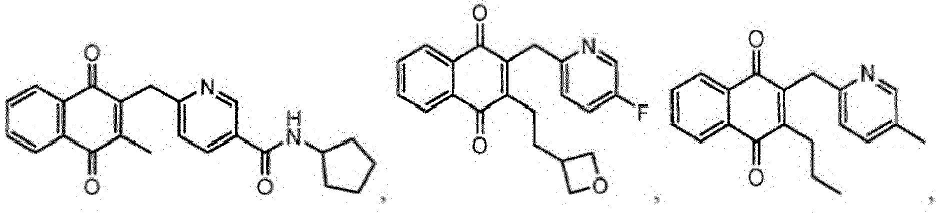 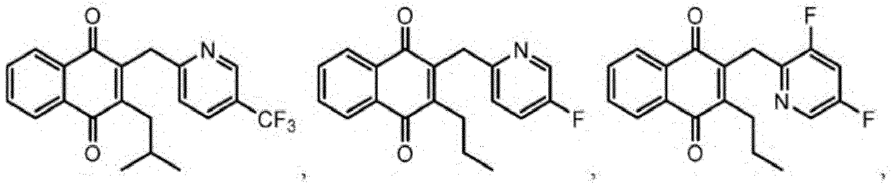 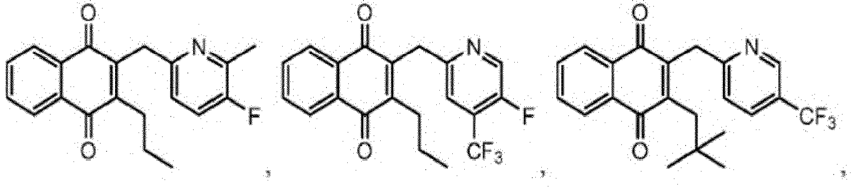 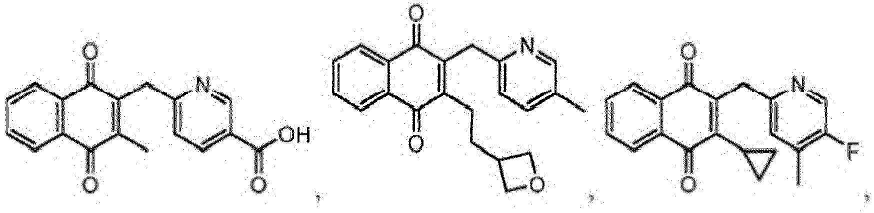 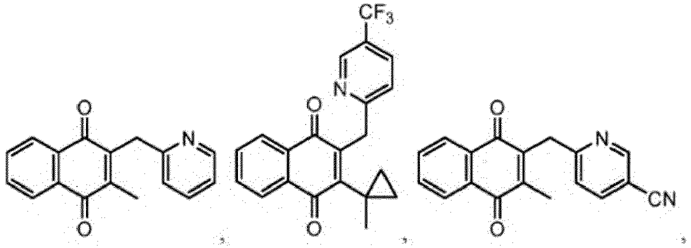 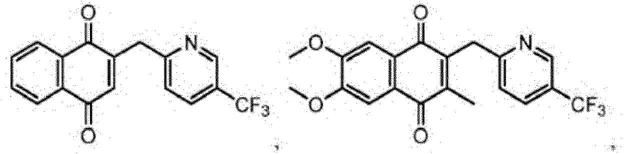 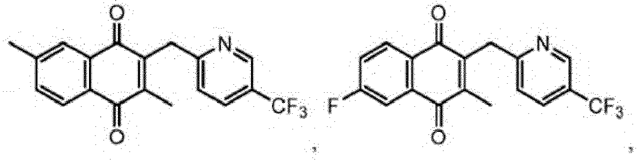 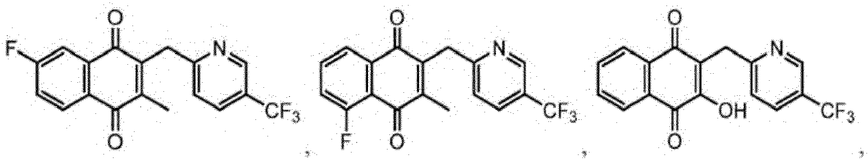 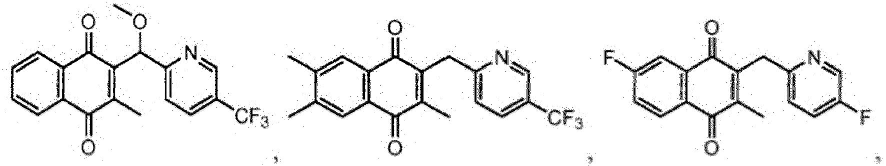 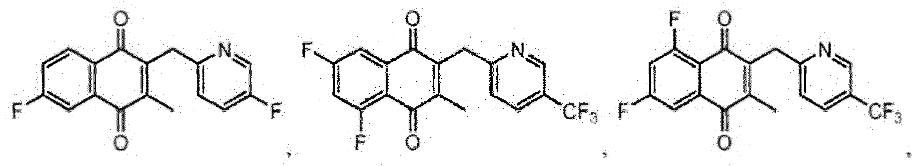 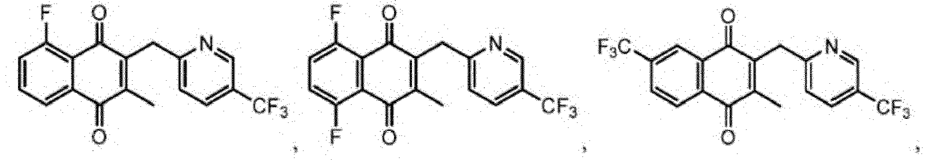 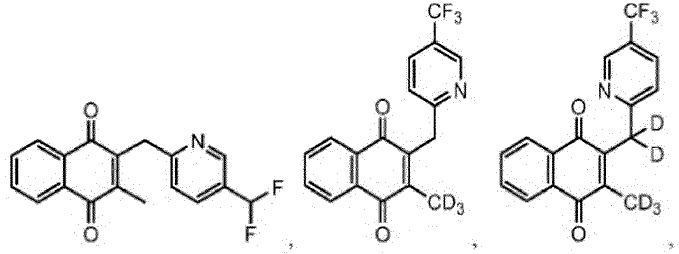 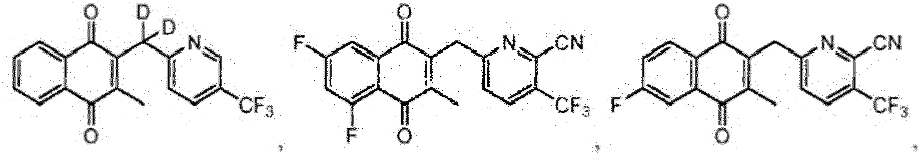 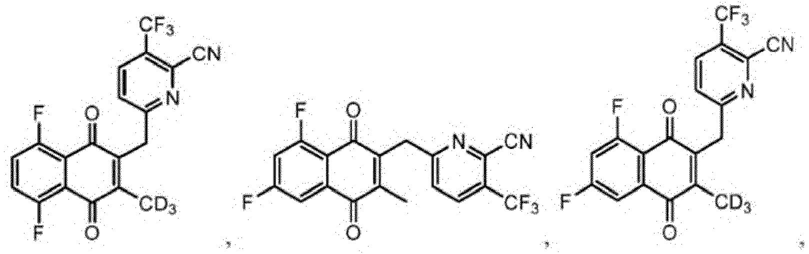 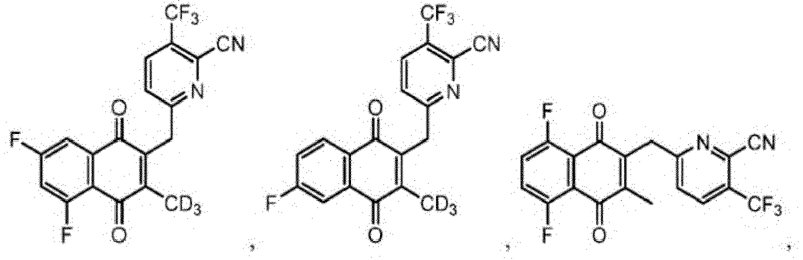 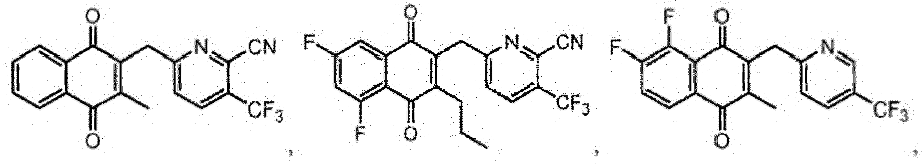 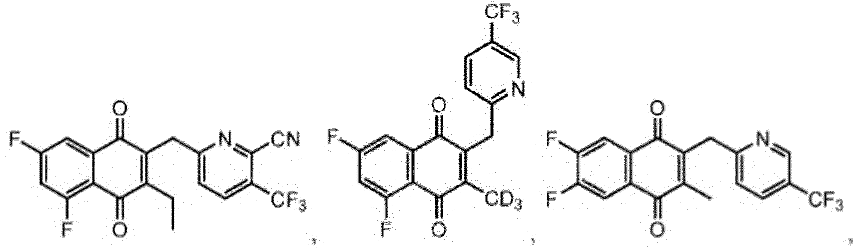 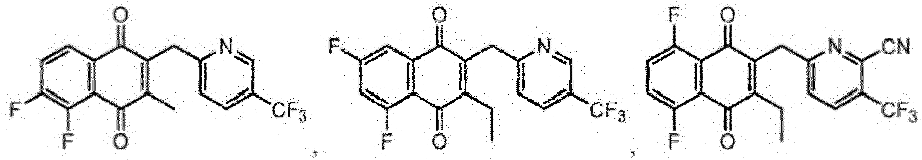 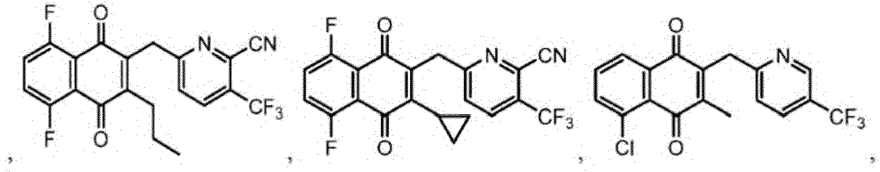 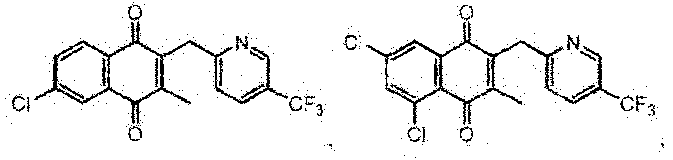 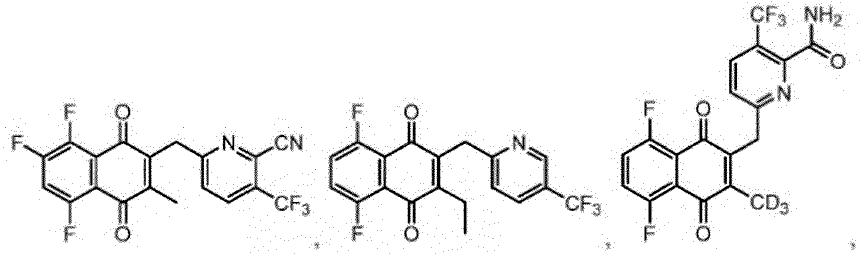 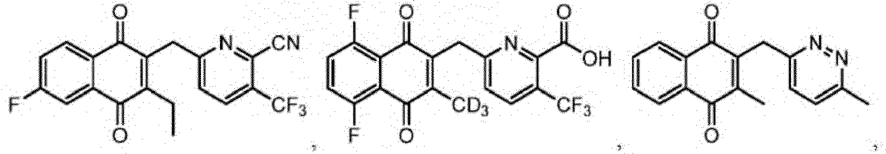 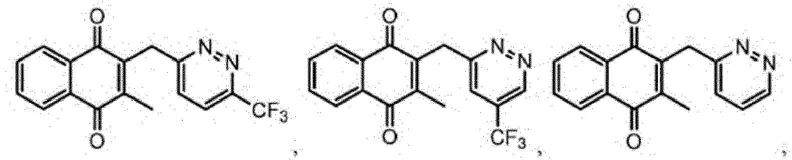 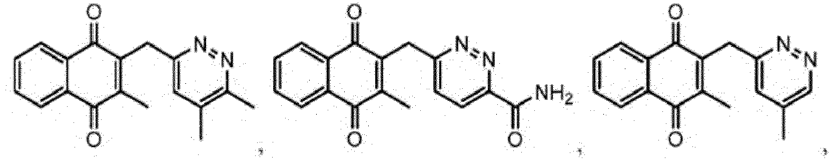 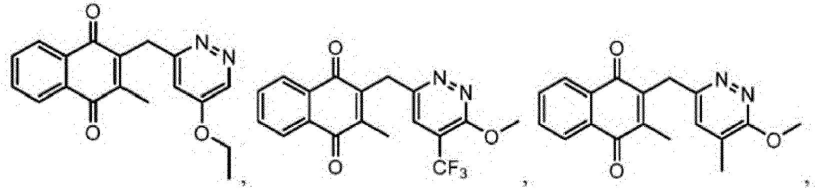 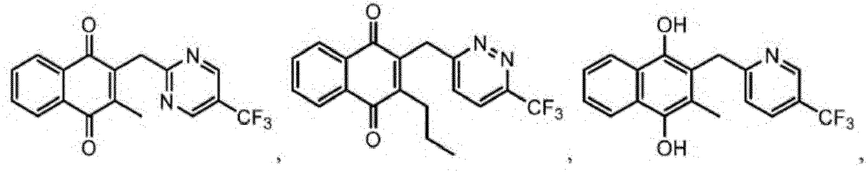 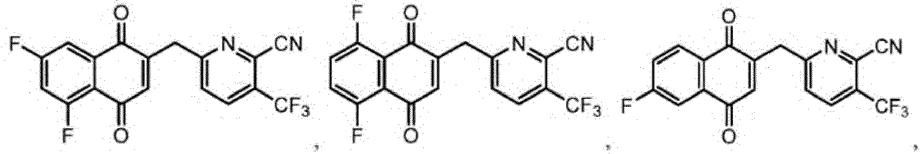 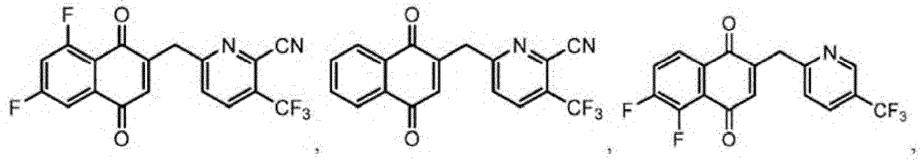 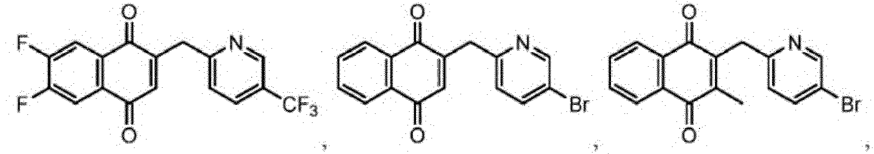 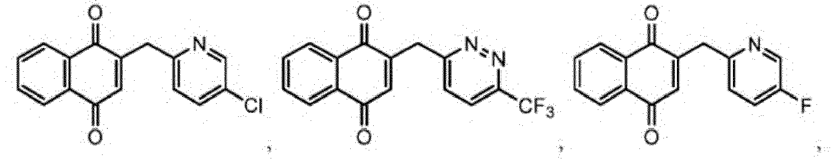 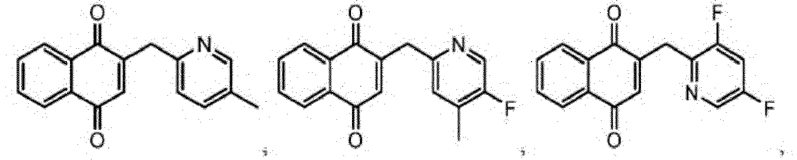 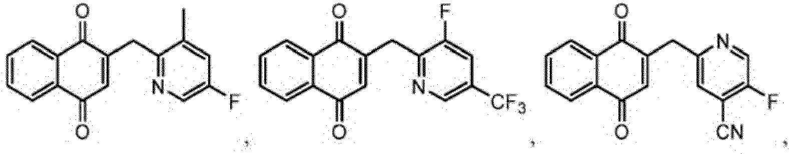 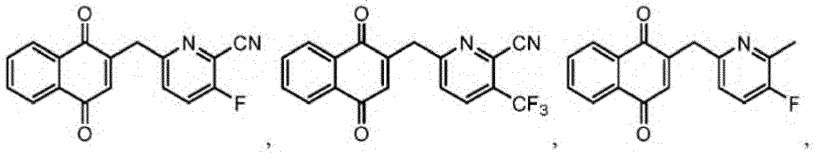 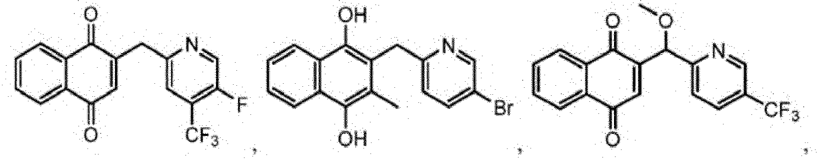 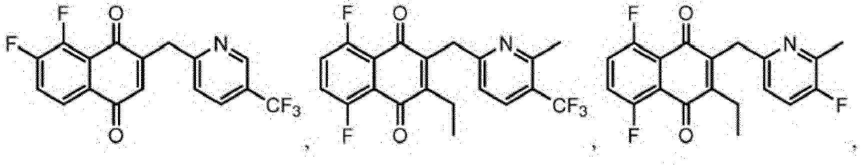 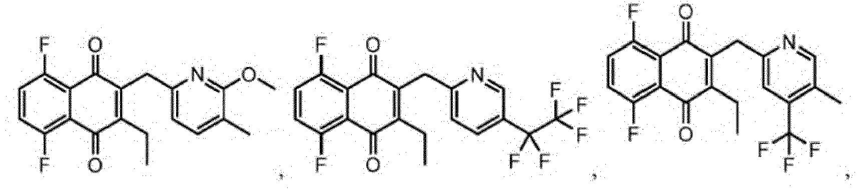 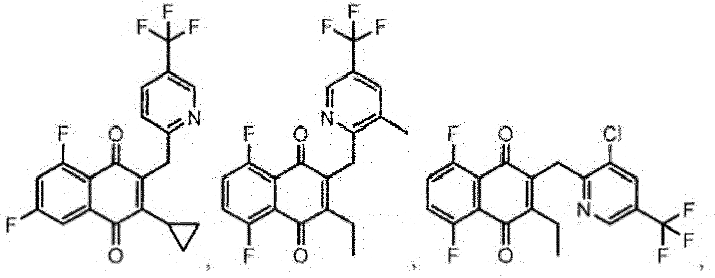 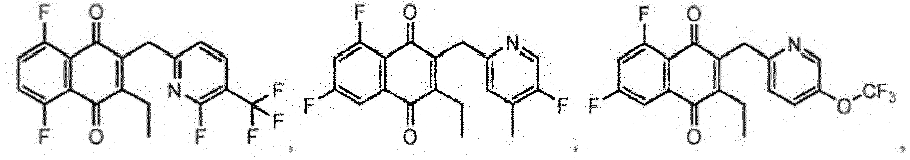 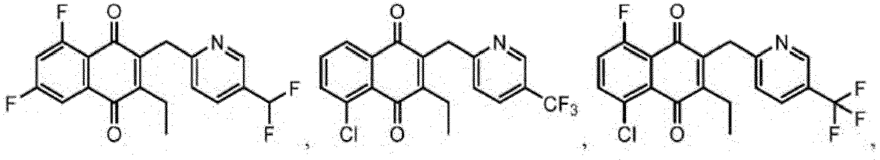 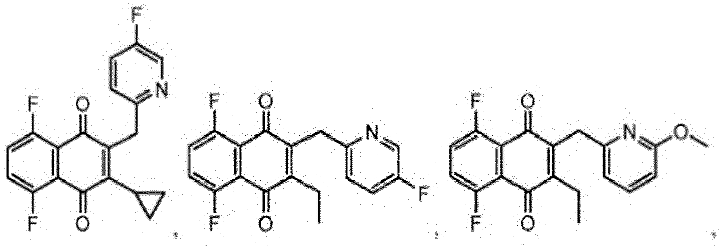 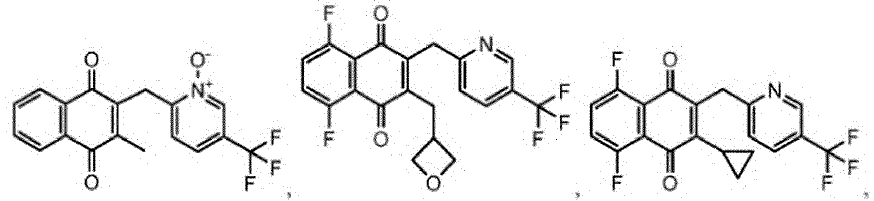 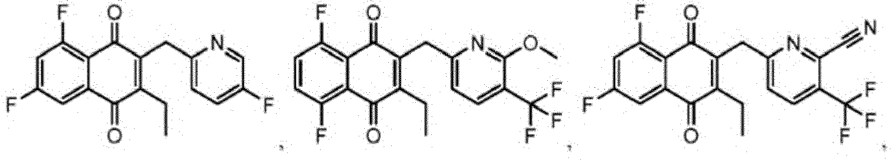 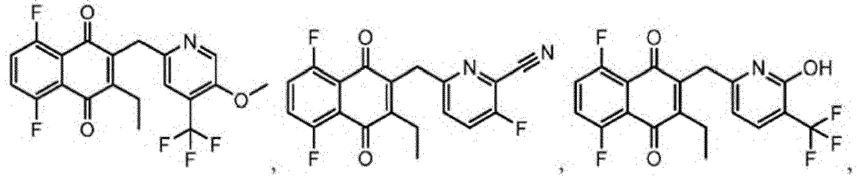 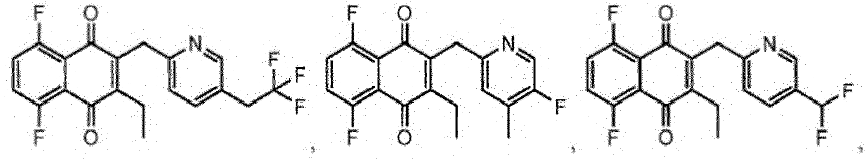 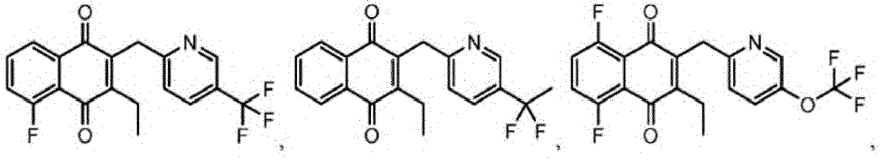 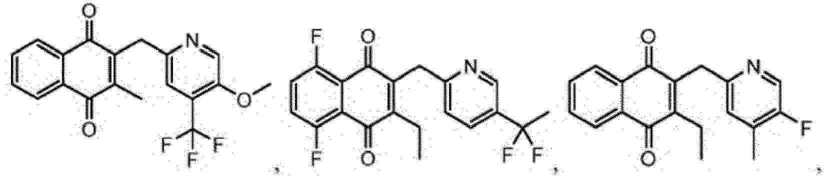 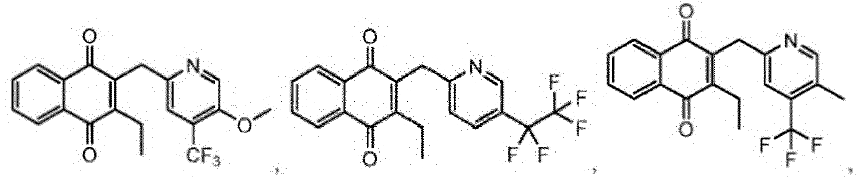 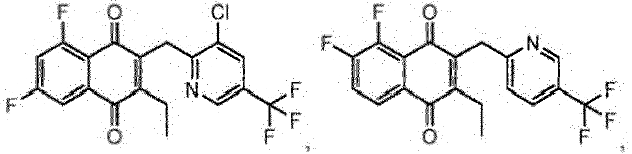 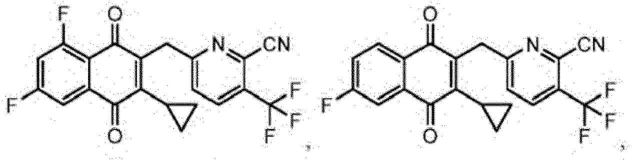 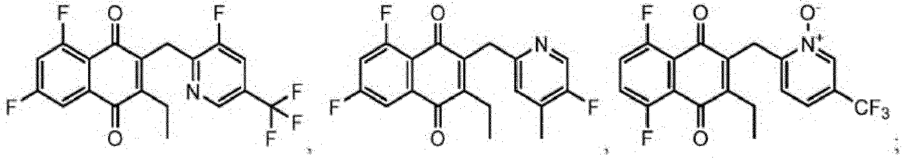 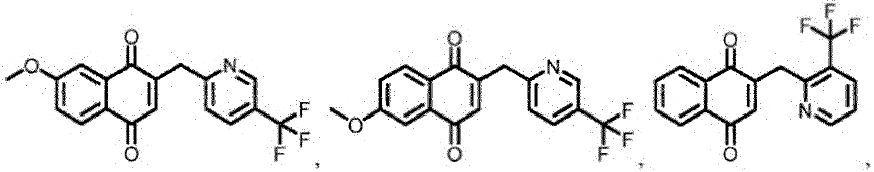 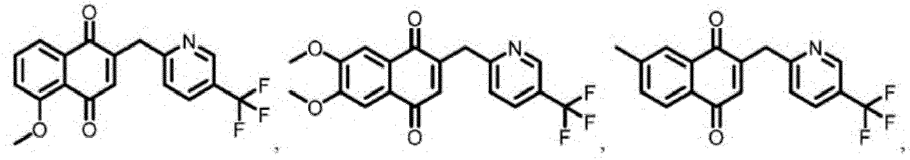 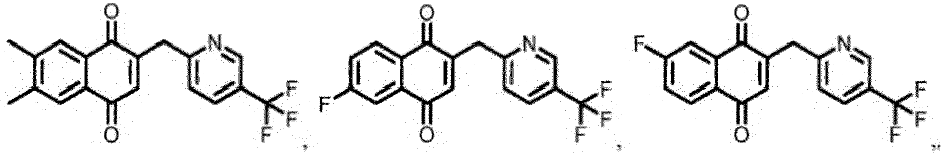 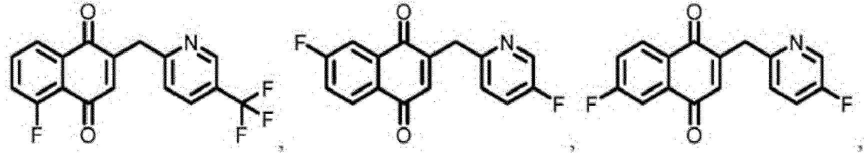 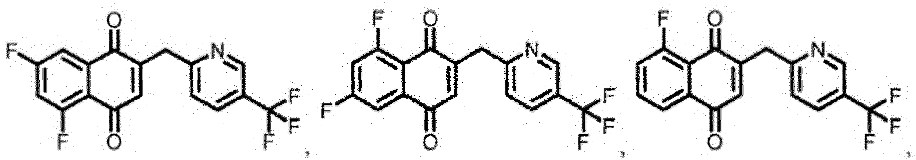 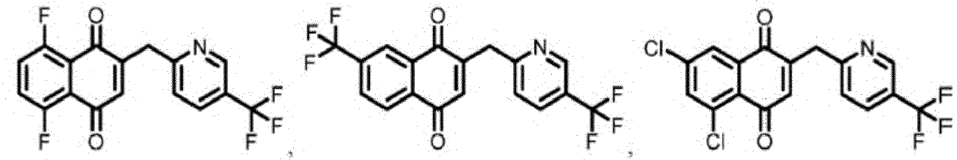 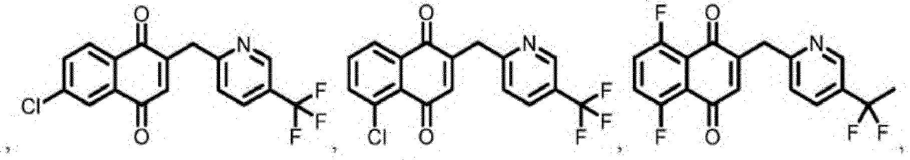 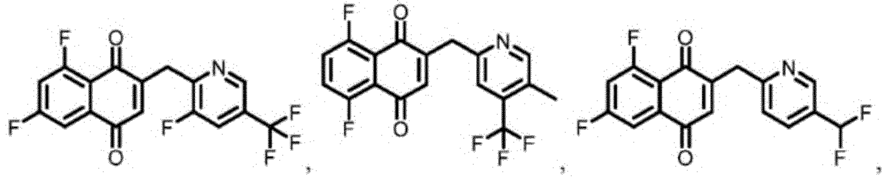 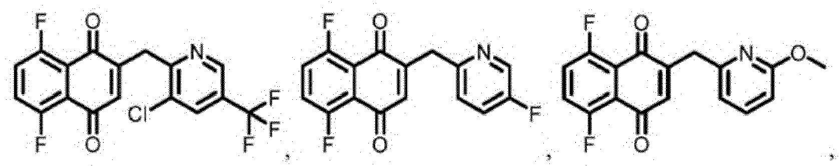 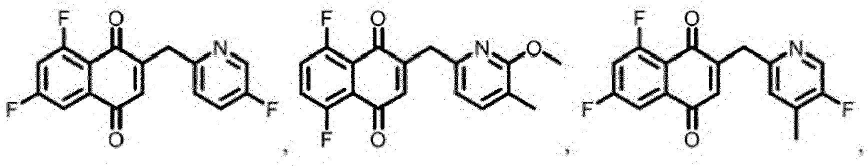 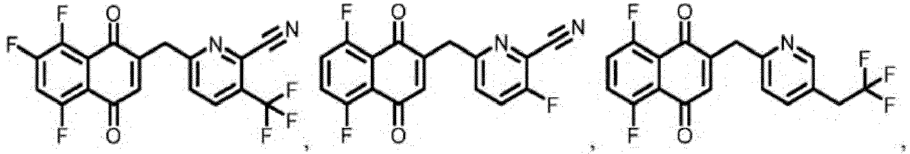 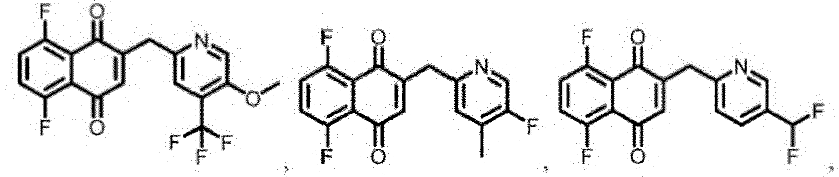 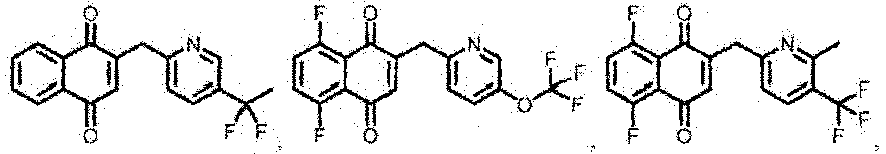 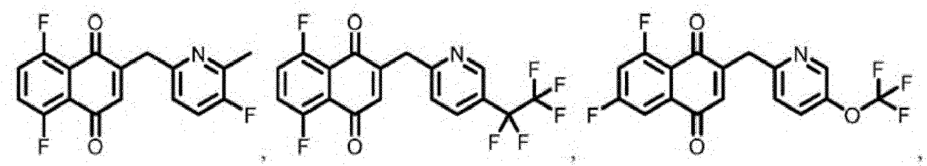 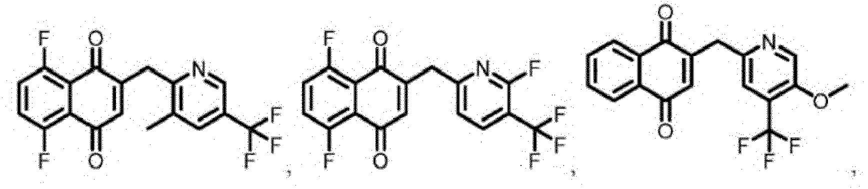 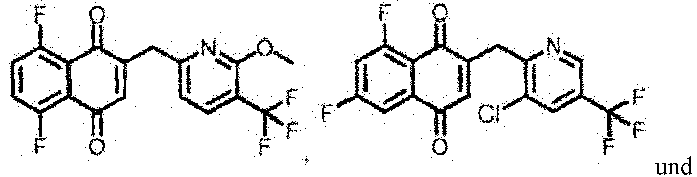 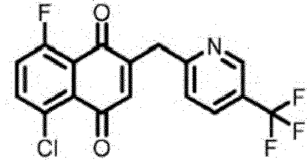 ; und den reduzierten Formen davon; und allen Salzen, Stereoisomeren, Gemischen aus Stereoisomeren, Isotopologen, Solvaten und/oder Hydraten davon.

11. Verbindung nach einem der Ansprüche 1-10, wobei die Verbindung in der oxidierten (Chinon-) Form ist.

12. Verbindung nach einem der Ansprüche 1-11, wobei die Verbindung kein Salz ist.

13. Verbindung nach einem der Ansprüche 1-11, wobei die Verbindung ein pharmazeutisch annehmbares Salz ist.

14. Pharmazeutische Zusammensetzung, umfassend eine Verbindung nach einem der Ansprüche 1-13 oder ein Salz, Stereoisomer, Gemisch aus Stereoisomeren, Isotopolog, Solvat und/oder Hydrat davon; und einen pharmazeutisch annehmbaren Träger.

15. Verbindung nach einem der Ansprüche 1-13 oder ein Salz, Stereoisomer, Gemisch aus Stereoisomeren, Isotopolog, Solvat und/oder Hydrat davon, oder eine pharmazeutische Zusammensetzung nach Anspruch 14, zur Verwendung in einem Verfahren des Behandelns oder Unterdrückens einer oxidativen Stressstörung, wobei das Verfahren das Verabreichen einer therapeutisch wirksamen Menge der Verbindung, des Salzes, des Stereoisomers, des Gemischs von Stereoisomeren, des Isotopologs, des Solvats und/oder des Hydrats davon oder einer pharmazeutischen Zusammensetzung an ein Subjekt, das dessen bedarf, umfasst;
wobei, wenn die Verbindung ein Salz ist, das Salz ein pharmazeutisch annehmbares Salz ist;
wobei optional die oxidative Stressstörung ausgewählt ist aus der Gruppe bestehend aus: einer neurodegenerativen Erkrankung; einer α-Synucleinopathie; Parkinson-Krankheit; familiärer Parkinson-Krankheit; idiopathischer Parkinson-Krankheit; Parkinson-Krankheit wobei der Patient eine Mutation in einem oder mehreren der folgenden Genen aufweist: MAPT (Mikrotubuli-assoziiertes Protein Tau), PRKN (Parkin), PINK1 (PINK1), LRRK2 (leucinreiche Repeat-Kinase 2), GBA (Glukozerebrosidase), SNCA (Alpha-Synuklein), PARK7 (DJ-1) und/oder UCHL1 (Ubiquitin-Carboxyl-Terminal-Hydrolase L1); Parkinson-Krankheit mit Demenz (PDD); Multisystematrophie (MSA); frontotemporaler Demenz (FTD); Demenz mit Lewy-Körperchen (DLB); Gaucher-Krankheit (GD); Neurodegeneration mit Eisenablagerung im Gehirn (NBIA); neuroaxonaler Dystrophie (PLA2G6-assoziierter Neurodegeneration); einer Tauopathie; Alzheimer-Krankheit; Dementia pugilistica; Guam-amyotropher-Lateralsklerose-Parkinsonismus-Demenz (Guam ALS/PD); Pick-Krankheit; Demenz mit argyrophilen Granula; Niemann-Pick-Krankheit Typ C; subakuter sklerosierender Panenzephalitis (SSPE); progressiver supranukleärer Blickparese (PSP); kortikobasaler Degeneration; frontotemporaler Demenz mit Parkinsonismus-17 (FTDP-17); postenzephalitischer Parkinsonismus (PEP); autosomal rezessiver Parkinsonismus; Huntington-Krankheit; juveniler Huntington-Krankheit; amyotropher Lateralsklerose (ALS); einer Motoneuronerkrankung; einer Leukodystrophie; Adrenoleukodystrophie; Adrenomyeloneuropathie; Schädel-Hirn-Trauma; chronischer traumatischer Enzephalopathie (CTE); spinaler Muskelatrophie (SMA); einer neurologischen Erkrankung; Epilepsie; Krampfanfällen; einer Gemütsstörung; Schizophrenie; bipolarer Störung; Aufmerksamkeitsdefizit-/Hyperaktivitätsstörung (ADHD); Tourette-Syndrom; einer tiefgreifenden Entwicklungsstörung; Down-Syndrom; autistischer Störung; Asperger-Syndrom; desintegrativen Störungen des Kindesalters (CDD); Ret-Syndrom; CDKL5-Defizit-Störung; PDD-nicht näher bezeichnet (PDD-NOS); NGLY1-kongenitale Störung der Deglykosylierung; einer mitochondrialer Störung; einer vererbten mitochondrialen Störung; Alpers-Syndrom; Barth-Syndrom; einem Beta-Oxidationsdefekt; Carnitin-Acylcarnitin-Mangel; Carnitin-Mangel; einem Kreatin-Mangel-Syndrom; Coenzym-Q10-Mangel; Komplex-I-Mangel; Komplex-II-Mangel; Komplex-III-Mangel; Komplex-IV-Mangel; Komplex-V-Mangel; COX-Mangel; chronisch progressiver externer Ophthalmoplegie (CPEO); CPT-I-Mangel; CPT-II-Mangel; Friedreich-Ataxie (FA); Glutarazidurie Typ II; Kearns-Sayre-Syndrom (KSS); Laktatazidose; Langketten-Acyl-CoA-Dehydrogenase-Mangel (LCAD); Langketten-3-Hydroxyacyl-CoA-Dehydrogenase-Mangel (LCHAD); Leigh-Syndrom; Leigh-artigem Syndrom; Leberscher Optikusatrophie (LHON); tödlicher infantiler Kardiomyopathie (LIC); Luft-Krankheit; Multiplem-Acyl-CoA-Dehydrogenase-Mangel (MAD); Mittelketten-Acyl-CoA-Dehydrongenase-Mangel (MCAD); mitochondrialer Myopathie, Enzephalopathie, Laktatazidose, Schlaganfall (MELAS); Myoklonusepilepsie mit Ragged Red Fasern (MERRF); mitochondrialem rezessivem Ataxiesyndrom (MIRAS); mitochondrialer Zytopathie, mitochondrialer DNA-Depletion; mitochondrialer Enzephalopathie; mitochondrialer Myopathie; mitochondrialer neurogastrointestinaler Enzephalopathie (MNGIE); Neuropathie, Ataxie und Retinitis Pigmentosa (NARP); Pearson-Syndrom; Pyruvat-Carboxylase-Mangel; Pyruvat-Dehydrogenase-Mangel; Störung in Verbindung mit einer POLG-Mutation; einer Atmungskettenstörung; Kurzketten-Acyl-CoA-Dehydrogenase-Mangel (SCAD); Kurzketten-3-Hydroxyacyl-CoA-Dehydrogenase-Mangel (SCHAD); Sehr-Langketten-Acyl-CoA-Dehydrongenase-Mangel (VLCAD); einer Myopathie; Kardiomyopathie; Enzephalomyopathie; einer mitochondrialen Myopathie; einer primären mitochondrialen Myopathie; einer Muskeldystrophie; Duchenne-Muskeldystrophie (DMD); Becker-Muskeldystrophie; kongenitaler Muskeldystrophie; kongenitaler Muskeldystrophie Typ Fukuyama; Gliedergürtel-Muskeldystrophie; myotoner Dystrophie; einem Apoplex; Schlaganfall; einer Sehbehinderung; Sehstörungen; Augenerkrankung; optischer Neuroathie; dominanter, vererbter, juveniler Optikusatrophie; optischer Neuropathie verursacht durch einen Giftstoff; Leberscher hereditärer Optikus-Neuropathie (LHON); dominanter Optikusatrophie (DOA); DOA plus; Glaukom; Retinitis pigmentosa; Makuladegeneration; altersbedingter Makuladegeneration (AMD); trockener AMD; feuchter AMD; Morbus Stargardt; diabetischer Retinopathie; diabetischer Makulopathie; Frühgeborenen-Retinopathie; ischämischer Reperfusion in Bezug auf eine Verletzung der Netzhaut; Ischämie; Ischämie-Reperfusionsschaden; Ischämie-Reperfusionsschaden aufgrund eines Transplantats; Ischämie-Reperfusionsschaden aufgrund einer Operation; Sauerstoffvergiftung; einer altersassoziierten Krankheit; Diabetes; metabolischem Syndrom; Wolfram-Syndrom; Krebs; Hirnkrebs; Glioblastom; chronischer Müdigkeit; einer Erbkrankheit; einer Hämoglobinopathie; Thalassämie; Sichelzellenanämie; G6PD-Mangel; Multipler Sklerose; einer neurodegenerativen Erkrankung, die zu einer Hör- oder Gleichgewichtsstörung führt; Hörverlust; Lärmschwerhörigkeit; maternal vererbtem Diabetes mit Schwerhörigkeit (MIDD); renaler tubulärer Azidose; akuter tubulärer Nekrose; kontrastmittelinduzierter Nierenschädigung; kontrastmittelinduzierter Retinopathiestörung; Abetalipoproteinämie; Cobalamin-C-Defekt; methylmalonischer Azidurie; und Strahlenschäden.

16. Verbindung oder pharmazeutische Zusammensetzung zur Verwendung nach Anspruch 15, wobei die oxidative Stressstörung ausgewählt ist aus der Gruppe bestehend aus Parkinson-Krankheit, Duchenne-Muskeldystrophie, Leigh-Syndrom, Komplex-I-Mangel, Huntington-Krankheit, Alzheimer-Krankheit, juveniler Huntington-Krankheit, amyotropher Lateralsklerose, frontotemporaler Demenz (FTD) und Friedreich-Ataxie.

## Revendications

1. Composé de formule : 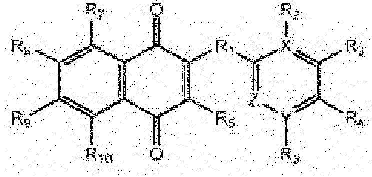 ou sa forme réduite;
dans lequel :
X et Y sont C ; ou X est N, Y est C et R₂ n'est pas présent ; ou X est C, Y est N et R₅ n'est pas présent ;
Z est N ou N-oxyde, dans lequel lorsque Z est N-oxyde alors X et Y sont C ;
R₁ est -C(R₁₁)(R₁₂)- ;
R₂, R₃, R₄ et R₅ sont chacun indépendamment choisis dans le groupe constitué par haloalkyle C₁-C₆, halo, alkyle C₁-C₆, alcényle C₂-C₆, alcynyle C₂-C₆, cyano, alcoxy C₁-C₆, -C(O)OH, - C(O)O(phényle), -C(O)NR₁₃R₁₄, -OH et haloalcoxy C₁-C₆;
R₆ est alkyle C₁-C₆, H ou OH ; où l'alkyle C₁-C₆ est éventuellement substitué par un groupe hétérocyclique à 3 à 7 chaînons qui est éventuellement substitué par un alkyle C₁-C₆ ou un haloalkyle C₁-C₆ ;
R₇, R₈, R₉ et R₁₀ sont chacun indépendamment choisis dans le groupe constitué par : hydrogène, halo, alkyle C₁-C₆, haloalkyle C₁-C₆, hydroxy, alcoxy C₁-C₆, -NHSO₂CH₃ et 5-6 chaînons hétéroaryle dans lequel les 5-6 chaînons hétéroaryle est éventuellement substitué par un alkyle C₁-C₆ ;
R₁₁ et R₁₂ sont choisis indépendamment dans le groupe constitué par hydrogène, méthyle et méthoxy ; à condition que R₁₁ et R₁₂ ne soient pas tous les deux méthoxy ; et
R₁₃ et R₁₄ sont chacun indépendamment H ou alkyle C₁-C₆ ; ou dans lequel R₁₃ et R₁₄ ensemble avec l'atome d'azote auquel ils sont liés forment un cycle hétérocyclique saturé à 4 à 6 chaînons dans lequel l'un des carbones est éventuellement remplacé par un N supplémentaire, et dans lequel le cycle hétérocyclique saturé à 4 à 6 chaînons est éventuellement substitué par un ou plusieurs substituants choisis indépendamment dans le groupe constitué par halo et alkyle C₁-C₆ ; ou tout sel(s), stéréoisomère, mélange de stéréoisomères, isotopologue(s), solvate(s) et/ou hydrate(s) de celui-ci.

2. Composé selon la revendication 1, dans lequel R₁ est :
-CH₂- ; ou tout sel(s), stéréoisomère, mélange de stéréoisomères, isotopologue(s), solvate(s) et/ou hydrate(s) de celui-ci ; ou
-CD₂- ; ou tout sel(s), stéréoisomère, mélange de stéréoisomères, isotopologue(s), solvate(s) et/ou hydrate(s) de celui-ci ; ou
-CH(CH₃)- ; ou tout sel(s), stéréoisomère, mélange de stéréoisomères, isotopologue(s), solvate(s) et/ou hydrate(s) de celui-ci ; ou
-CH(OCH₃)- ; ou tout sel(s), stéréoisomère, mélange de stéréoisomères, isotopologue(s), solvate(s) et/ou hydrate(s) de celui-ci.

3. Composé selon la revendication 1 ou la revendication 2, dans lequel R₁₃ et R₁₄ sont chacun indépendamment H ou alkyle C₁-C₆ ; ou tout sel(s), stéréoisomère, mélange de stéréoisomères, isotopologue(s), solvate(s) et/ou hydrate(s) de celui-ci.

4. Composé selon la revendication 1 ou la revendication 2, dans lequel R₂, R₃, R₄ et R₅ sont chacun indépendamment choisis dans le groupe constitué par : hydrogène, -CHF₂, -CF₃, - CF₂CF₃, -CF₂CH₃, -CH₂CF₃, -F, -Cl, -CH₃, -CH₂CH₃, -CH(CH₃)₂, -OCH₃, -OCF₃ et -OCHF₂, - C=CH, -C≡CCH₃, -OH, -CN, -C(O)OH, -C(O)-NH₂, -C(O)-N(CH₂CH₃)(CH(CH₃)₂), -C(O)-N(H)(CH₂-cyclopropyle), -C(O)-N(H)(cyclopentyle), -C(O)-N(CH₃)(CH(CH₃)₂), et 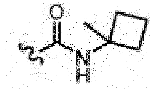 ; ou tout sel(s), stéréoisomère, mélange de stéréoisomères, isotopologue(s), solvate(s) et/ou hydrate(s) de celui-ci ; ou
dans lequel zéro à deux parmi R₂, R₃, R₄ et R₅ sont choisis indépendamment dans le groupe constitué par -CHF₂, -CF₃, -CF₂CF₃, -CF₂CH₃, -CH₂CF₃, -F, -Cl, -CH₃, -CH₂CH₃, -CH(CH₃)₂, - OCH₃, -OCF₃ et -OCHF₂, -C=CH, -C≡CCH₃, -OH, -CN, -C(O)OH, -C(O)-NH₂, -C(O)-N(CH₂CH₃)(CH(CH₃)₂), -C(O)-N(H)(CH₂-cyclopropyle), -C(O)-N(H)(cyclopentyle), -C(O)-N(CH₃)(CH(CH₃)₂), et 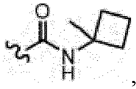 et dans lequel les autres sont l'hydrogène ; ou tout sel(s), stéréoisomère, mélange de stéréoisomères, isotopologue(s), solvate(s) et/ou hydrate(s) de celui-ci ; ou
dans lequel zéro à deux parmi R₂, R₃, R₄ et R₅ sont choisis indépendamment dans le groupe constitué par -CF₃, -CHF₂, F, Cl, -CH₃, -OCF₃ et -CN, et dans lequel les autres sont hydrogène ; ou tout sel(s), stéréoisomère, mélange de stéréoisomères, isotopologue(s), solvate(s) et/ou hydrate(s) de celui-ci.

5. Composé selon l'une quelconque des revendications 1 à 4, dans lequel R₆ est choisi dans le groupe constitué par : -CH₃, -CD₃, -CH₂CH₃, -CH₂CH₂CH₃, -CH(CH₃)₂, -CH₂CH(CH₃)₂, - CH₂CH₂CH(CH₃)₂, -CH₂C(CH₃)₃, -cyclopropyle, 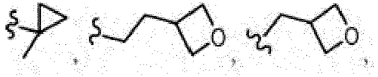 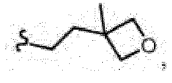 -H et -OH; ou tout sel(s), stéréoisomère, mélange de stéréoisomères, isotopologue(s), solvate(s) et/ou hydrate(s) de celui-ci ; ou
dans lequel R₆ n'est pas méthyle ; ou tout sel(s), stéréoisomère, mélange de stéréoisomères, isotopologue(s), solvate(s) et/ou hydrate(s) de celui-ci.

6. Composé selon l'une quelconque des revendications 1 à 5, dans lequel R₇, R₈, R₉ et R₁₀ sont l'hydrogène ; ou tout sel(s), stéréoisomère, mélange de stéréoisomères, isotopologue (s), solvate(s) et/ou hydrate(s) de celui-ci ; ou
dans lequel R₇, R₈, R₉ et R₁₀ sont chacun indépendamment choisis dans le groupe constitué par : H, -CH₃, -OCH₃, -F, -Cl et -CF₃ ; ou tout sel(s), stéréoisomère, mélange de stéréoisomères, isotopologue (s), solvate(s) et/ou hydrate(s) de celui-ci ; ou
dans lequel R₇, R₈, R₉ et R₁₀ sont chacun indépendamment choisis dans le groupe constitué par : hydrogène et -F ; ou tout sel(s), stéréoisomère, mélange de stéréoisomères, isotopologue(s), solvate(s) et/ou hydrate(s) de celui-ci ; ou
dans lequel R₇, R₈, R₉ et R₁₀ sont chacun indépendamment choisis dans le groupe constitué par : hydrogène et -Cl ; ou tout sel(s), stéréoisomère, mélange de stéréoisomères, isotopologue(s), solvate(s) et/ou hydrate(s) de celui-ci.

7. Composé selon l'une quelconque des revendications 1 à 5, dans lequel zéro à trois parmi R₇, R₈, R₉ et R₁₀ sont chacun indépendamment choisis dans le groupe constitué par : -CH₃, - OCH, -F, -Cl et -CF₃, et dans lequel les autres sont hydrogène ; ou tout sel(s), stéréoisomère, mélange de stéréoisomères, isotopologue(s), solvate(s) et/ou hydrate(s) de celui-ci ; ou
dans lequel zéro à trois parmi R₇, R₈, R₉ et R₁₀ sont -F, et dans lequel les autres sont hydrogène ; ou tout sel(s), stéréoisomère, mélange de stéréoisomères, isotopologue(s), solvate(s) et/ou hydrate(s) de celui-ci ; ou
dans lequel un ou deux parmi R₇, R₈, R₉ et R₁₀ sont chacun indépendamment choisis dans le groupe constitué par : -CH₃, -OCH₃, -F, -Cl et -CF₃, et dans lequel les autres sont hydrogène ; ou tout sel(s), stéréoisomère, mélange de stéréoisomères, isotopologue(s), solvate(s) et/ou hydrate(s) de celui-ci ; ou
dans lequel un ou deux parmi R₇, R₈, R₉ et R₁₀ sont -F, et dans lequel les autres sont hydrogène ; ou tout sel(s), stéréoisomère, mélange de stéréoisomères, isotopologue(s), solvate(s) et/ou hydrate(s) de celui-ci ; ou
dans lequel un ou deux parmi R₇, R₈, R₉ et R₁₀ sont -Cl, et dans lequel les autres sont hydrogène ; ou tout sel(s), stéréoisomère, mélange de stéréoisomères, isotopologue(s), solvate(s) et/ou hydrate(s) de celui-ci ; ou
dans lequel un ou deux parmi R₇, R₈, R₉ et R₁₀ sont chacun indépendamment choisis dans le groupe constitué par : -F et -Cl, et dans lequel les autres sont hydrogène ; ou tout sel(s), stéréoisomère, mélange de stéréoisomères, isotopologue(s), solvate(s) et/ou hydrate(s) de celui-ci.

8. Composé selon l'une quelconque des revendications 1 à 7, dans lequel Z est N ; ou tout sel(s), stéréoisomère, mélange de stéréoisomères, isotopologue(s), solvate(s) et/ou hydrate(s) de celui-ci.

9. Composé selon l'une quelconque des revendications 1 à 8, dans lequel X et Y sont C ; ou tout sel(s), stéréoisomère, mélange de stéréoisomères, isotopologue(s), solvate(s) et/ou hydrate(s) de celui-ci.

10. Composé selon la revendication 1, dans lequel le composé est choisi dans le groupe constitué par : 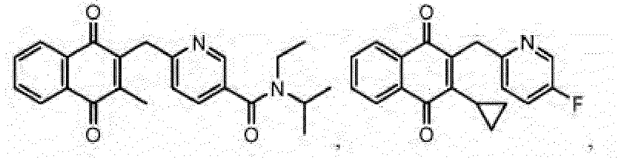 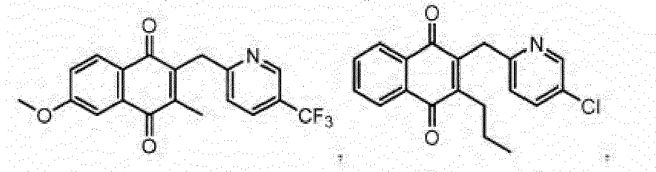 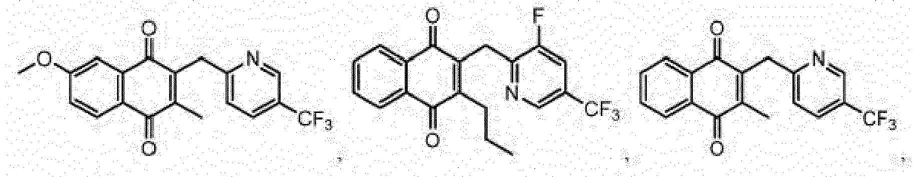 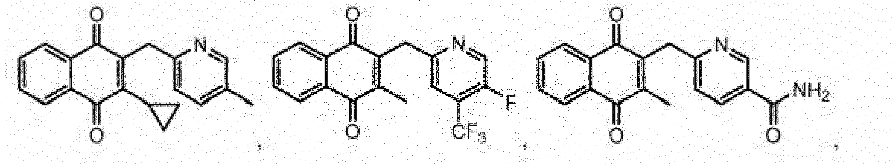 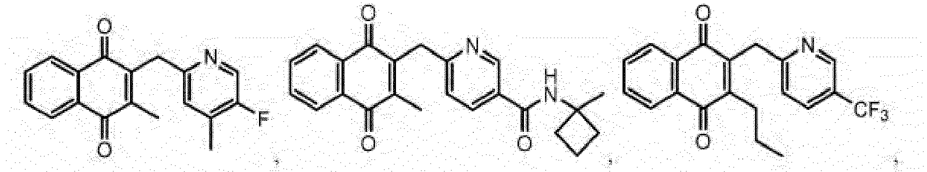 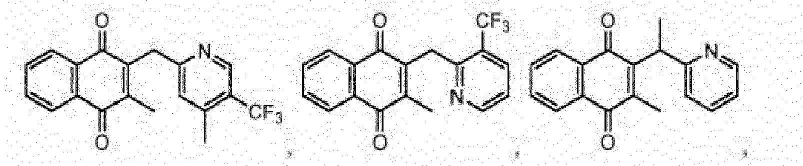 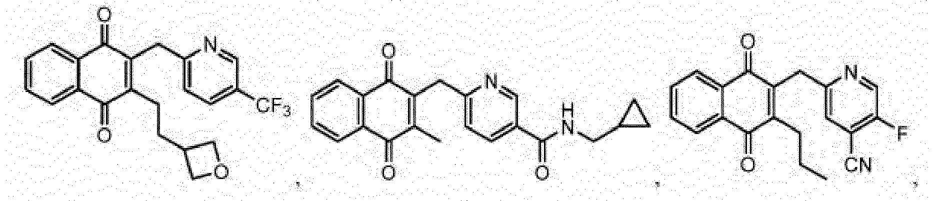 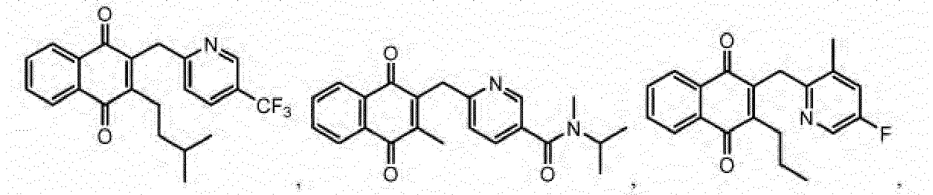 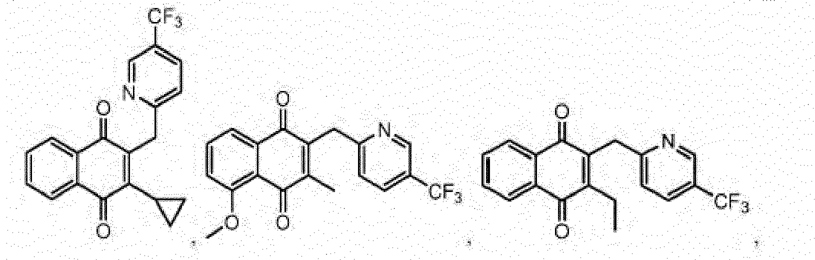 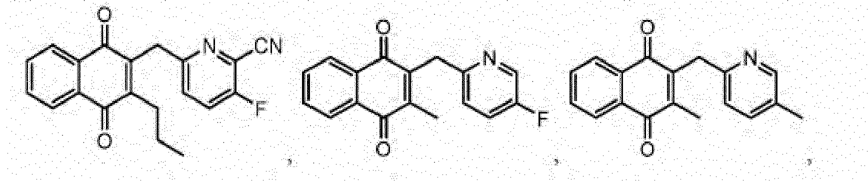 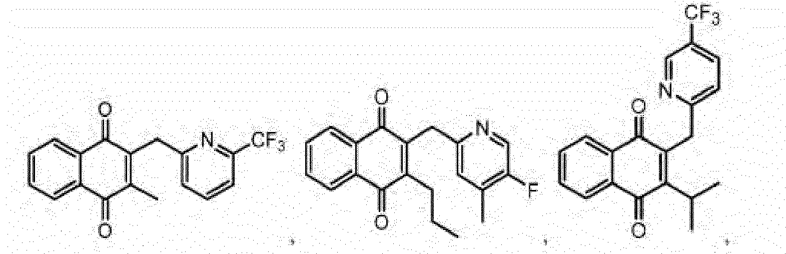 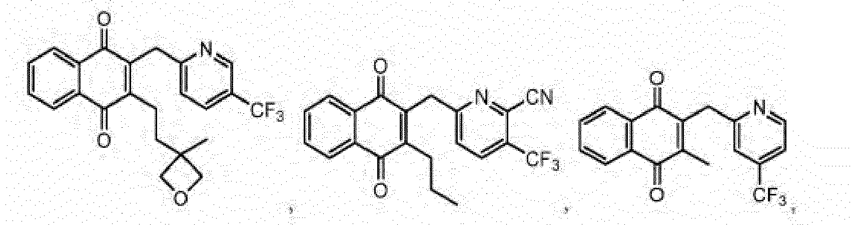 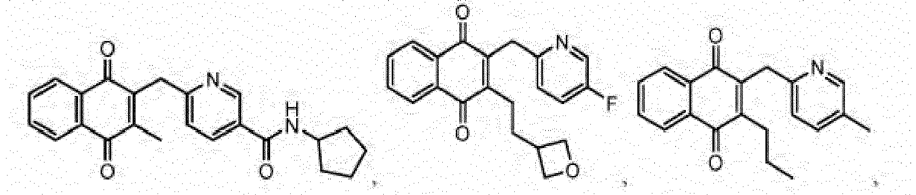 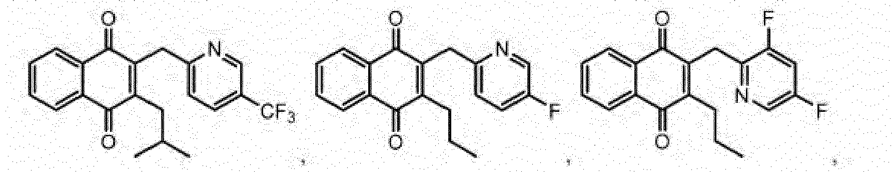 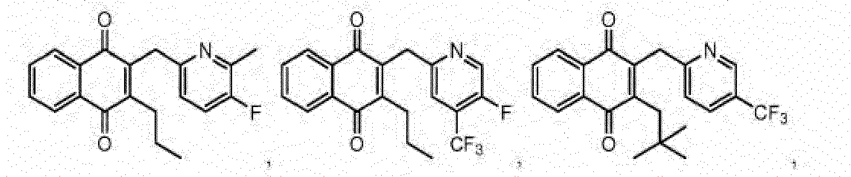 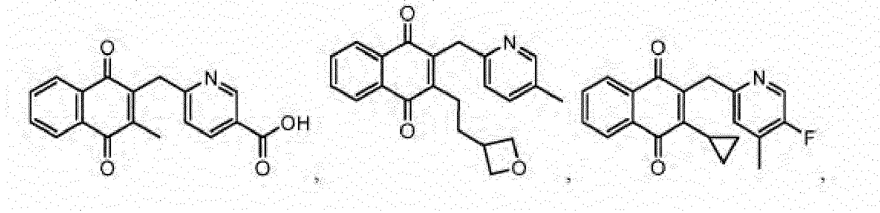 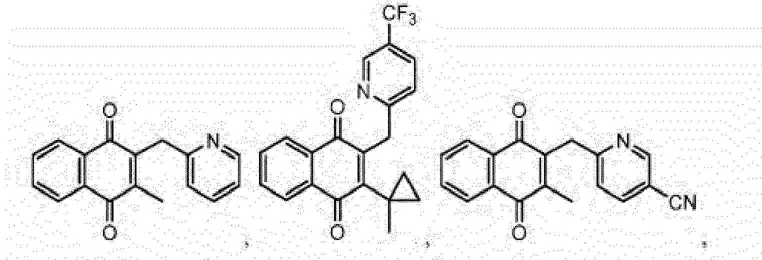 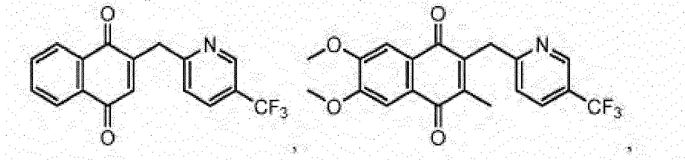 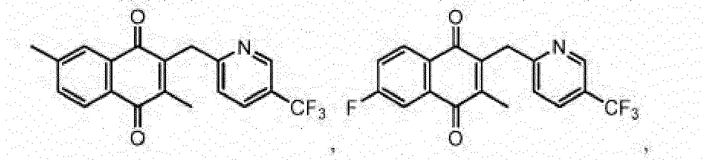 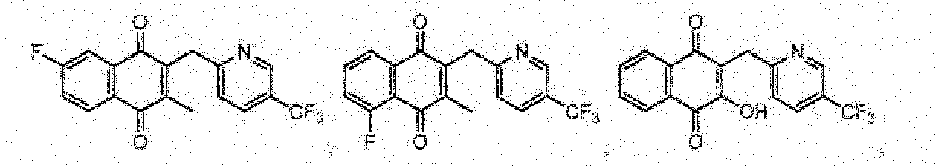 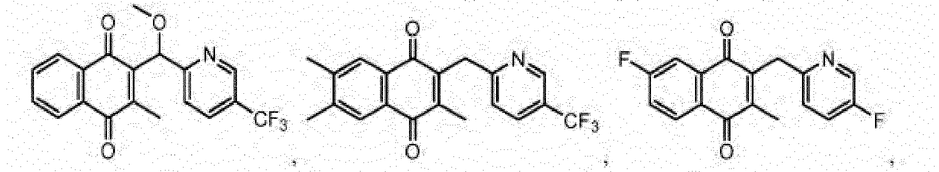 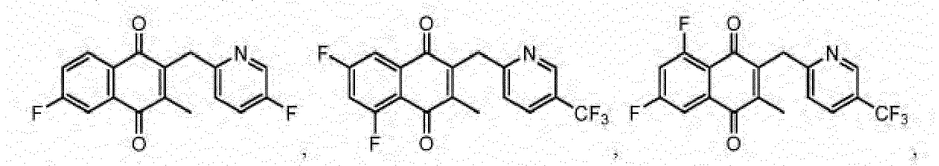 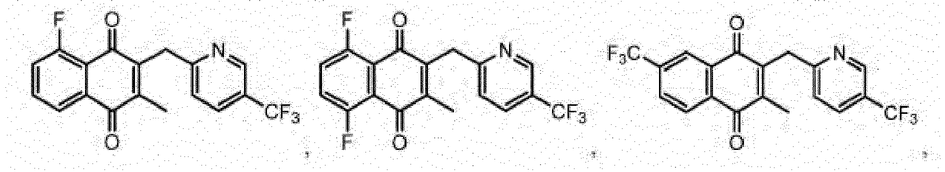 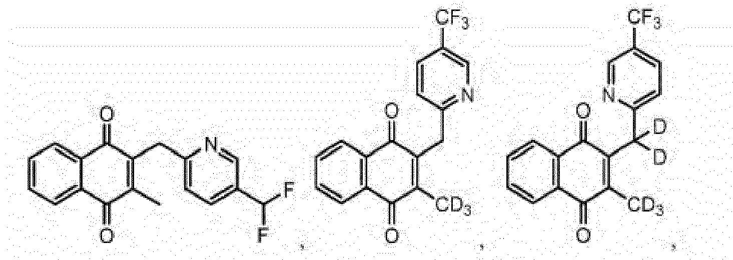 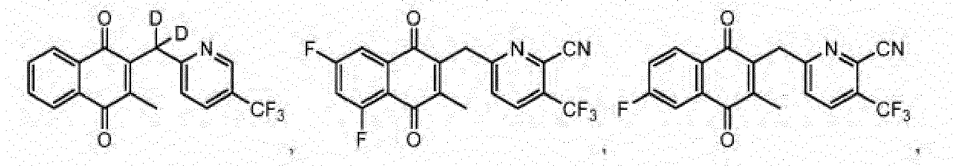 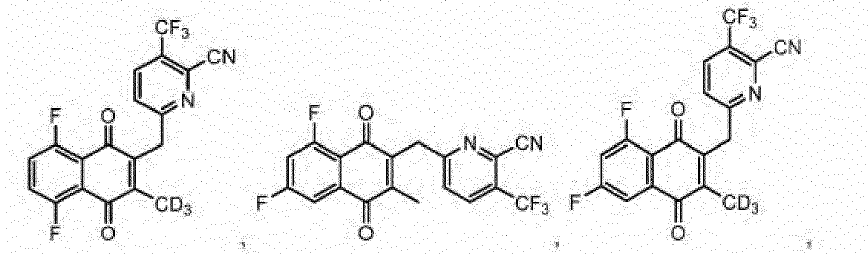 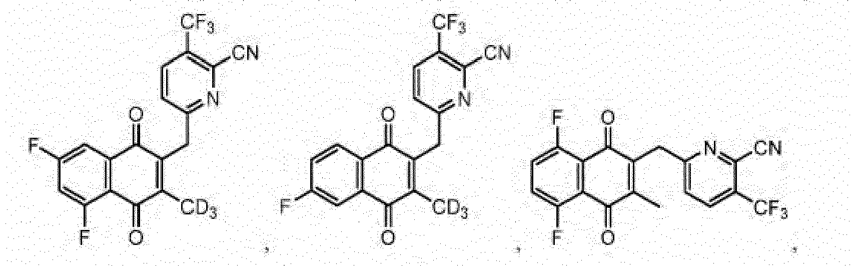 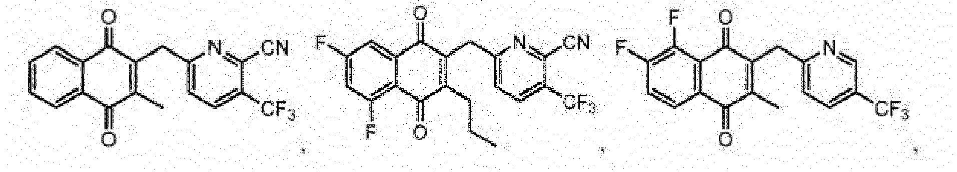 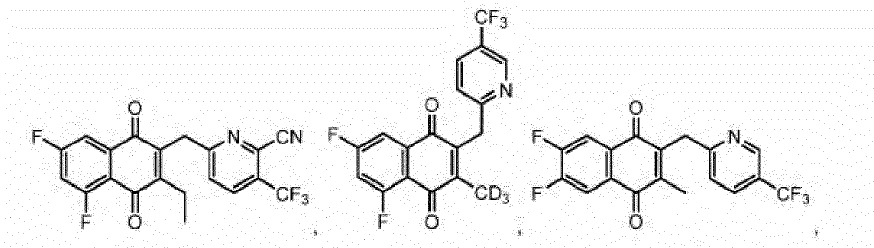 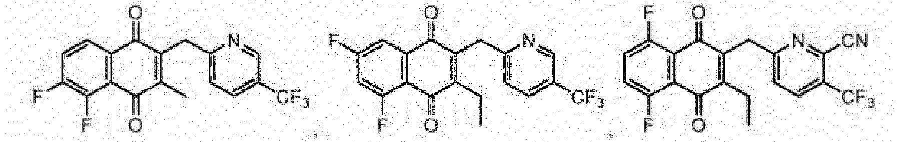 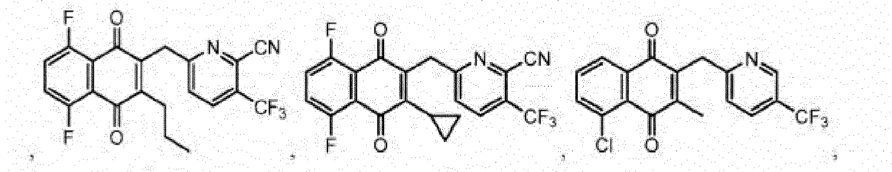 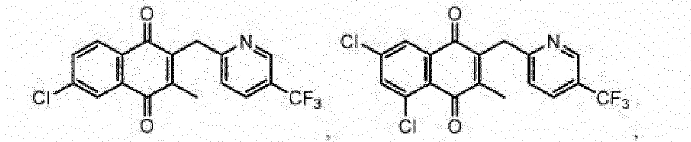 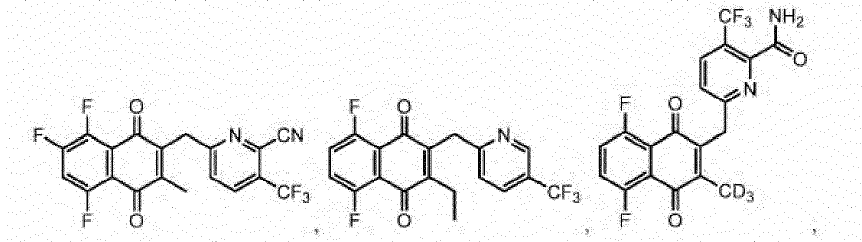 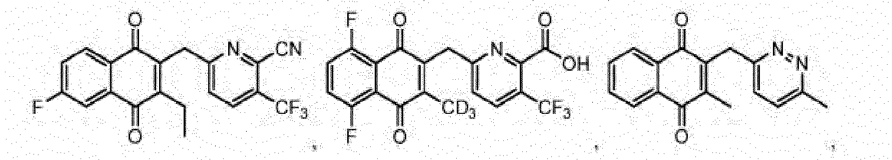 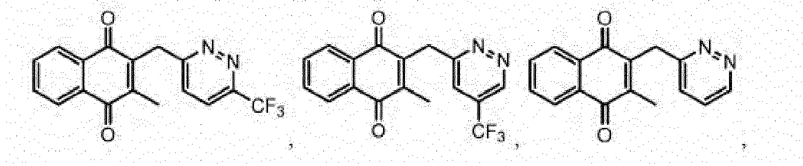 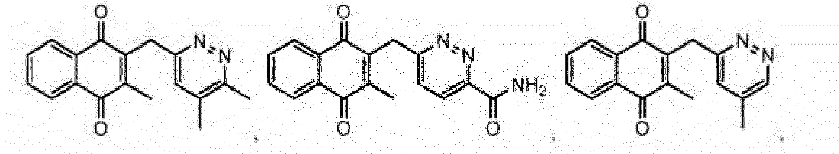 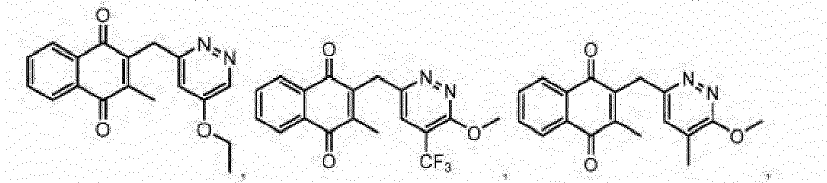 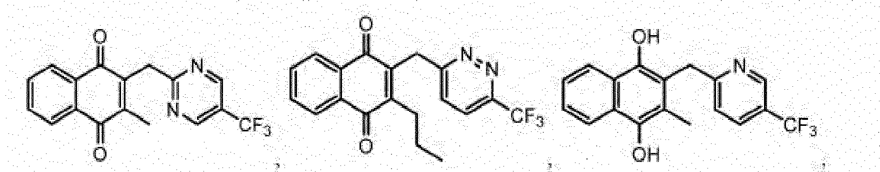 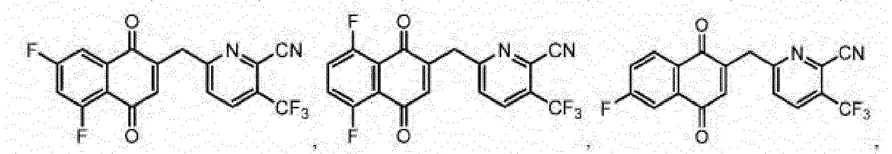 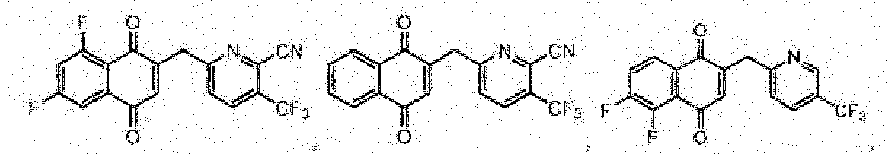 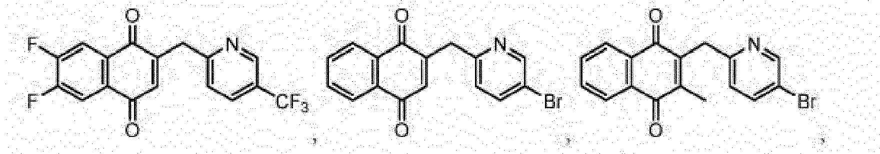 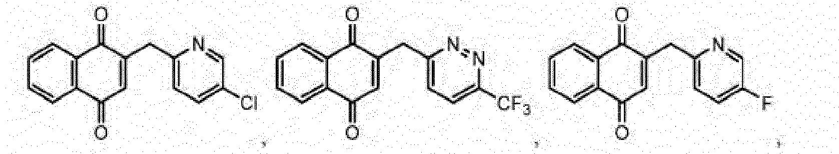 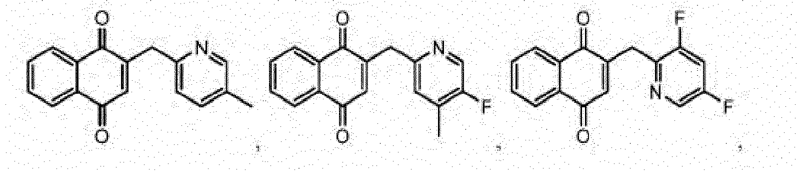 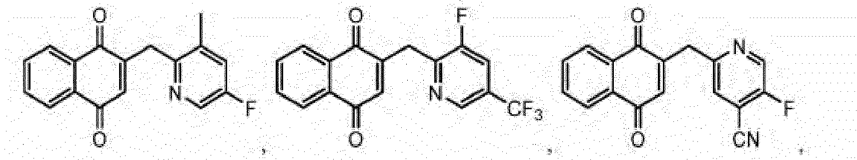 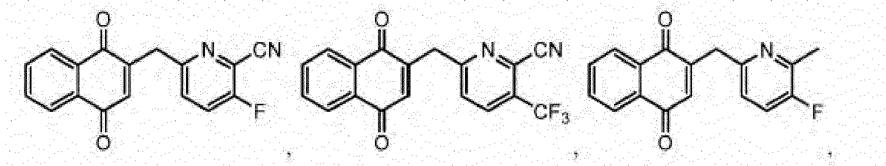 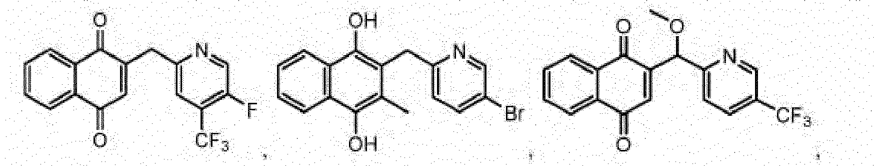 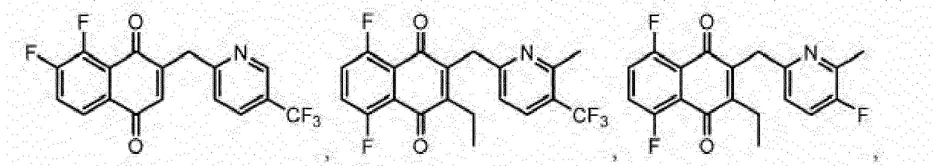 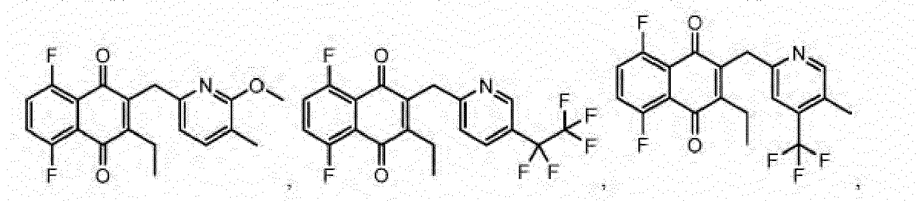 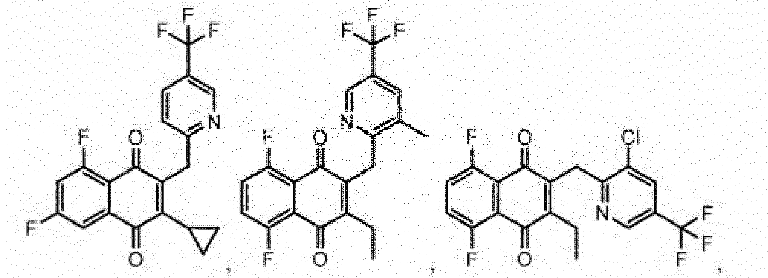 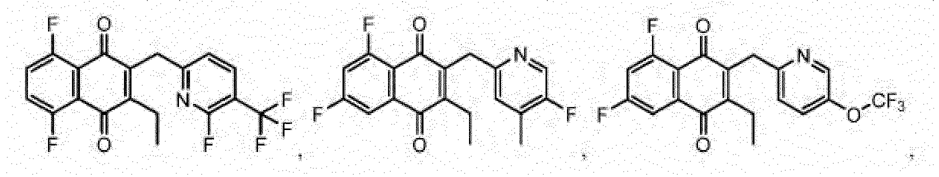 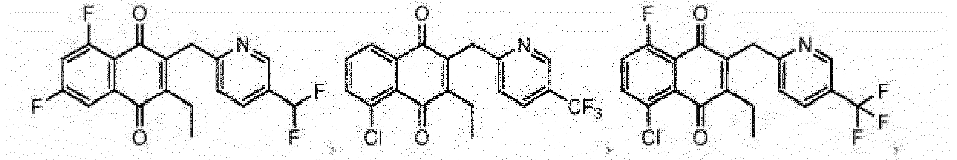 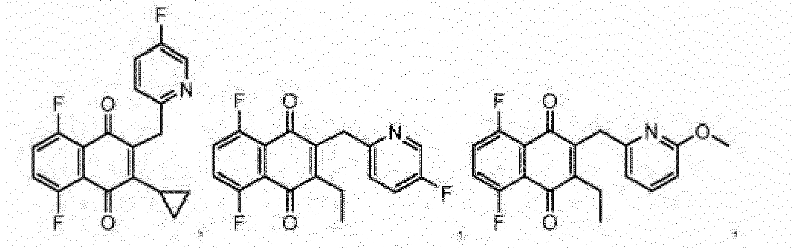 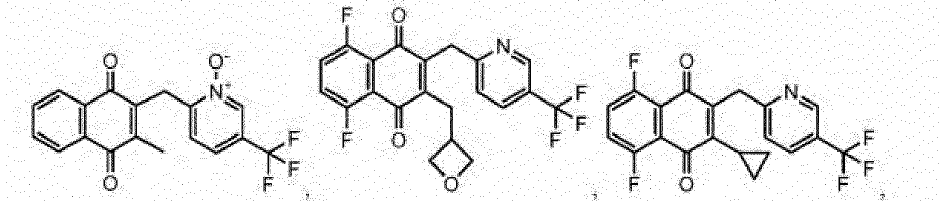 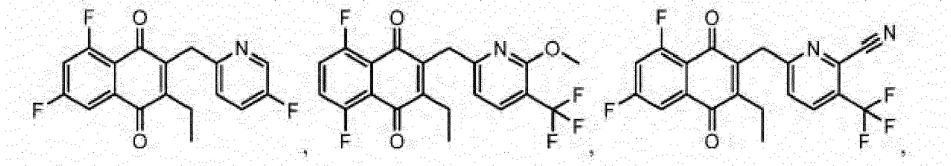 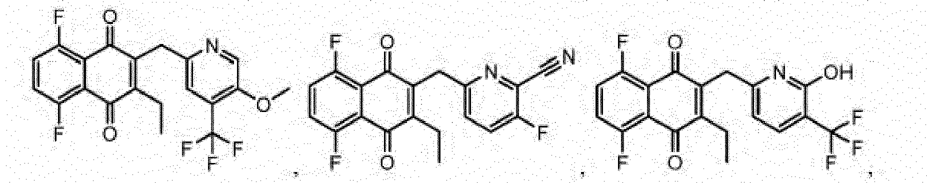 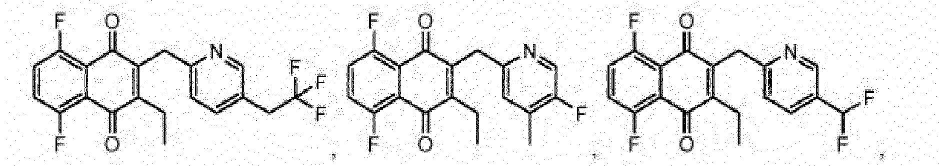 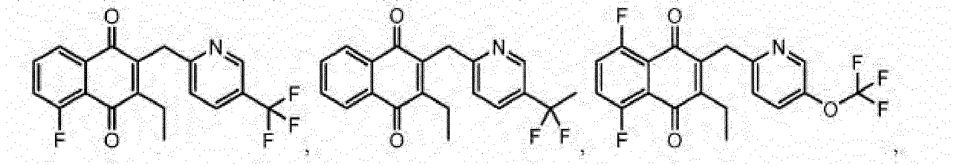 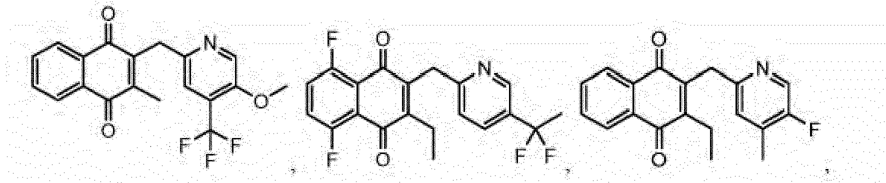 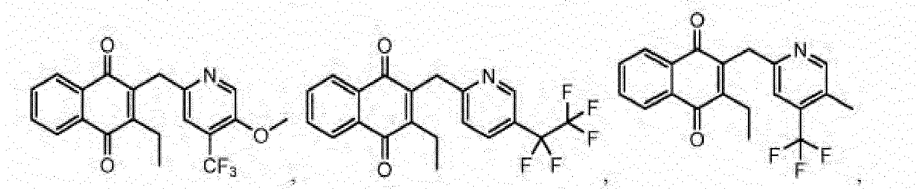 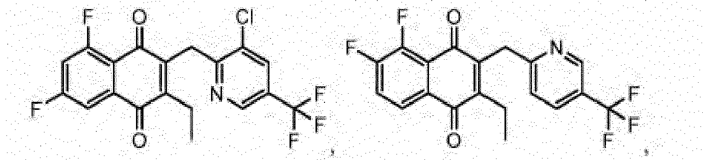 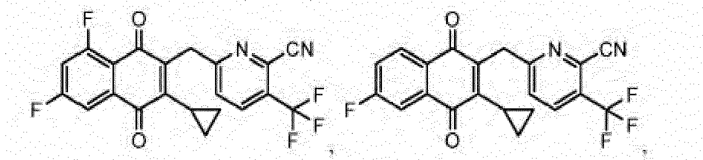 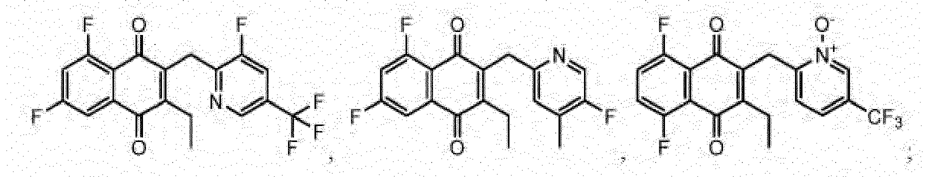 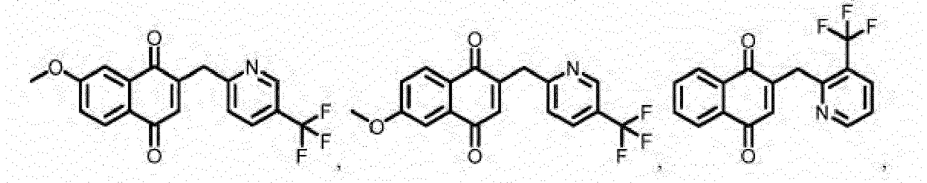 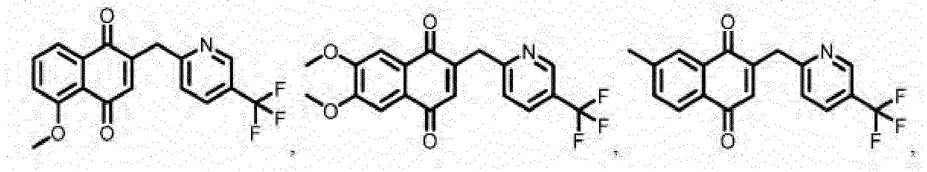 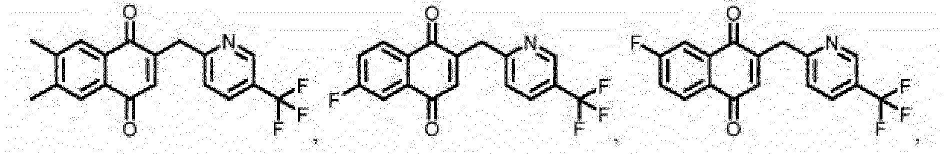 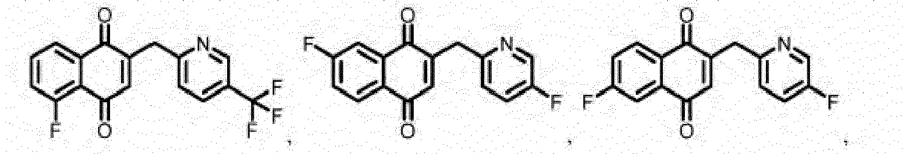 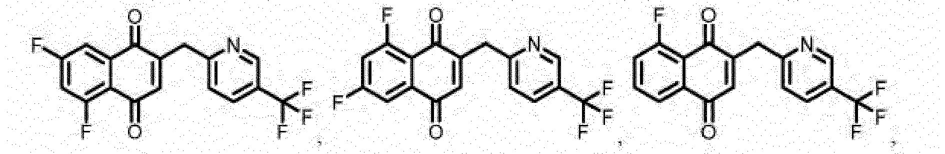 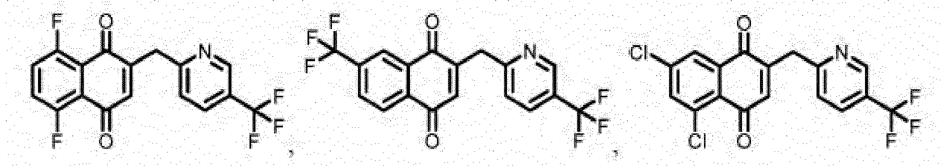 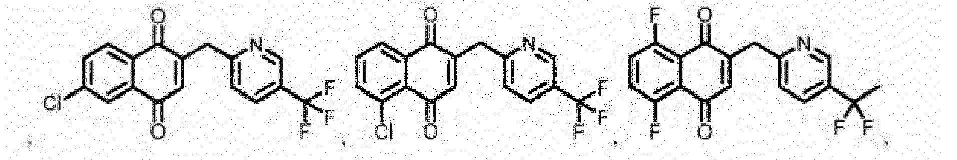 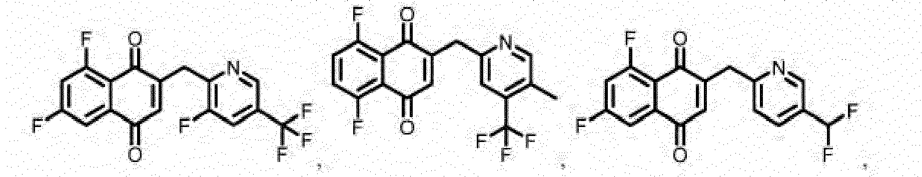 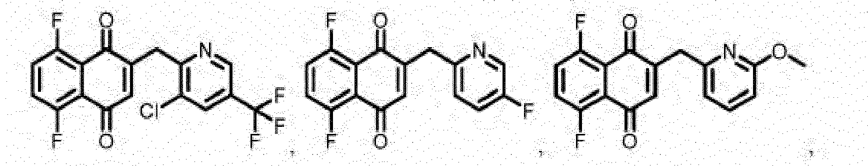 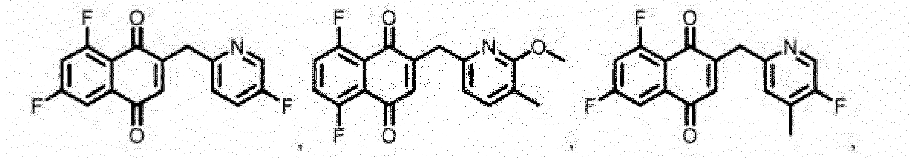 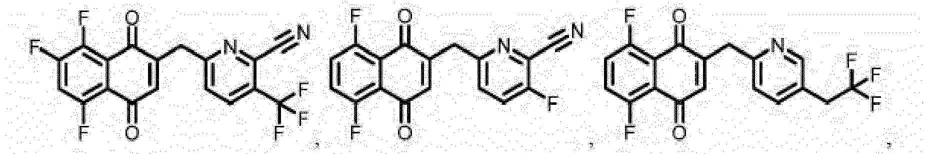 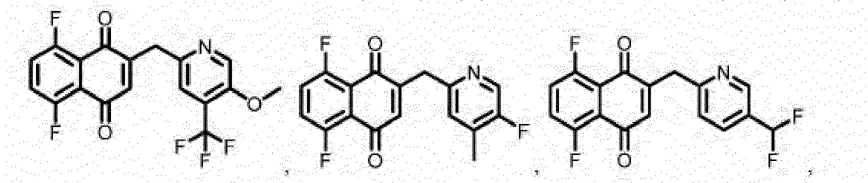 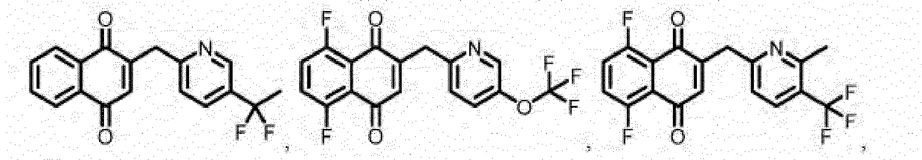 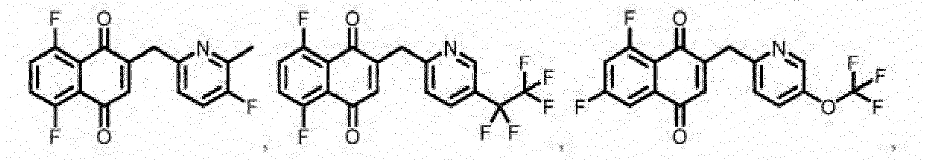 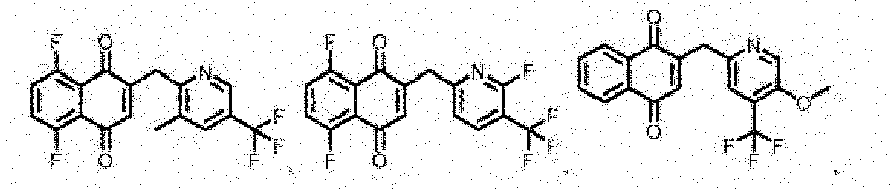 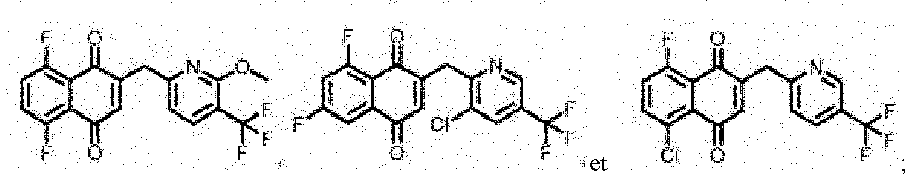 et leurs formes réduites : et tous les sels, stéréoisomères, mélanges de stéréoisomères, isotopologues, solvates et/ou hydrates de ceux-ci.

11. Composé selon l'une quelconque des revendications 1 à 10, dans lequel le composé est sous la forme oxydée (quinone).

12. Composé selon l'une quelconque des revendications 1 à 11, dans lequel le composé n'est pas un sel.

13. Composé selon l'une quelconque des revendications 1 à 11, dans lequel le composé est un sel pharmaceutiquement acceptable.

14. Composition pharmaceutique comprenant un composé selon l'une quelconque des revendications 1 à 13 ou un sel, stéréoisomère, mélange de stéréoisomères, isotopologue, solvate et/ou hydrate de celui-ci ; et un support pharmaceutiquement acceptable.

15. Composé selon l'une quelconque des revendications 1 à 13 ou un sel, un stéréoisomère, un mélange de stéréoisomères, un isotopologue, un solvate et/ou un hydrate de celui-ci, ou une composition pharmaceutique selon la revendication 14, à utiliser dans un procédé de traitement ou de suppression d'un trouble de stress oxydatif, le procédé comprenant l'administration à un sujet en ayant besoin d'une quantité thérapeutiquement efficace du composé, sel, stéréoisomère, mélange de stéréoisomères, isotopologue, solvate et/ou hydrate de celui-ci, ou composition pharmaceutique ;
dans lequel lorsque le composé est un sel, le sel est un sel pharmaceutiquement acceptable;
éventuellement dans lequel le trouble de stress oxydatif est choisi dans le groupe constitué par : une maladie neurodégénérative ; une α- synucléinopathie ; la maladie de Parkinson; la maladie de Parkinson familiale ; la maladie de Parkinson idiopathique; la maladie de Parkinson dans laquelle le patient présente une mutation dans un ou plusieurs des gènes suivants : MAPT (protéine tau associée aux microtubules), PRKN (parkine), PINK 1 (PINK1), LRRK2 (kinase à répétition riche en leucine 2), GBA (glucocérébrosidase), SNCA (alpha synucléine), PARK7 (DJ-1) et/ou UCHL1 (ubiquitine carboxylestérase terminale L1) ; la maladie de Parkinson avec démence (PDD) ; l'atrophie multisystémique (MSA) ; la démence frontotemporale (FTD) ; la démence à corps de Lewy (DLB) ; la maladie de Gaucher (MG) ; la neurodégénérescence avec accumulation de fer dans le cerveau (NBIA) ; les dystrophies neuroaxonales (neurodégénérescence associée au PLA2G6 ) ; une tauopathie ; la maladie d'Alzheimer; la démence pugilistique ; sclérose latérale amyotrophique-Parkinsonisme-démence Guam (Guam ALS/PD) ; la maladie de Pick ; la démence à grains argyrophiles ; Nieman-Pick type C; la panencéphalite sclérosante subaiguë (SSPE) ; la paralysie supranucléaire progressive (PSP) ; la dégénérescence corticobasoganglionnaire ; la démence frontotemporale avec parkinsonisme-17 (FTDP-17); Parkinsonisme postencéphalitique (PEP) ; Parkinsonisme autosomique récessif ; la maladie de Huntington ; la maladie de Huntington juvénile ; la sclérose latérale amyotrophique (SLA) ; une maladie du motoneurone ; une leucodystrophie ; l'adrénoleucodystrophie ; l'adrénomyéloneuropathie ; une lésion cérébrale traumatique; l'encéphalopathie traumatique chronique (CTE) ; l'amyotrophie musculaire spinale (SMA) ; une maladie neurologique ; l'épilepsie ; les convulsions ; un trouble de l'humeur ; la schizophrénie ; le trouble bipolaire; le trouble déficitaire de l'attention/hyperactivité (TDAH) ; le syndrome de Tourette ; un trouble envahissant du développement ; le syndrome de Down ; un trouble autistique ; le syndrome d'Asperger ; trouble désintégratif de l'enfance (CDD) ; le syndrome de Rett ; le trouble déficitaire en CDKL5 ; PDD non spécifié ailleurs (PDD-NOS) ; le trouble congénital de la déglycosylation NGLY1 ; un trouble mitochondrial ; une maladie mitochondriale héréditaire ; la maladie d'Alpers ; le syndrome de Barth ; un défaut de bêta-oxydation ; la carence en carnitine-acyl-carnitine ; la carence en carnitine ; un syndrome de déficience en créatine ; la carence en coenzyme Q10 ; le déficit en Complexe I; le déficit en Complexe II ; le déficit en Complexe III ; le déficit en Complexe IV; le déficit en complexe V ; le déficit en COX ; l'ophtalmoplégie externe progressive chronique (OPEC) ; le déficit en CPT I ; le déficit en CPT II ; l'ataxie de Friedreich (FA) ; la Glutarique acidurie de type II ; le syndrome de Kearns-Sayre (KSS) ; l'acidose lactique ; le déficit en acyl-CoA déshydrogénase à longue chaîne (LCAD); le déficit en 3-hydroxyacyl-CoA déshydrogénase à longue chaîne (LCHAD) le syndrome de Leigh ; le syndrome de type Leigh ; la neuropathie optique héréditaire de Leber (NOHL) ; la cardiomyopathie infantile létale (LIC); la maladie de Luft ; le déficit en acyl-CoA déshydrogénase multiple (MAD) ; le déficit en Acyl-CoA déshydrogénase à chaîne moyenne (MCAD) ; la myopathie mitochondriale, l'encéphalopathie, la lactacidose, l'accident vasculaire cérébral (MELAS) ; l'épilepsie myoclonique avec fibres rouges déchiquetées (MERRF); le syndrome d'ataxie récessive mitochondriale (MIRAS) ; la cytopathie mitochondriale, l'épuisement de l'ADN mitochondrial ; l'encéphalopathie mitochondriale ; la myopathie mitochondriale ; le trouble myoneuro-gasto-intestinal et l'encéphalopathie (MNGIE); la neuropathie, l'ataxie et rétinite pigmentaire (NARP) ; le syndrome de Pearson ; le déficit en pyruvate carboxylase ; le déficit en pyruvate déshydrogénase ; le trouble associé à une mutation POLG ; un trouble de la chaîne respiratoire ; le déficit en acyl-CoA déshydrogénase à chaîne courte (SCAD) ; le déficit en 3-hydroxyacyl-CoA déshydrogénase à chaîne courte (SCHAD) ; le déficit en Acyl-CoA déshydrogénase à très longue chaîne (VLCAD) ; une myopathie ; la cardiomyopathie ; l'encéphalomyopathie ; une myopathie mitochondriale ; une myopathie mitochondriale primitive ; une dystrophie musculaire; une dystrophie musculaire de Duchenne (DMD) ; une dystrophie musculaire de Becker ; une dystrophie musculaire congénitale ; une dystrophie musculaire congénitale de type Fukuyama ; une dystrophie musculaire des ceintures ; une dystrophie myotonique ; un accident vasculaire cérébral ; une attaque ; une déficience visuelle ; des troubles de la vision ; une maladie oculaire ; une neuropathie optique ; une atrophie optique juvénile héréditaire dominante ; la neuropathie optique causée par un agent toxique ; la neuropathie optique héréditaire de Leber (LHON) ; l'atrophie optique dominante (DOA) ; la DOA plus ; le glaucome; la rétinite pigmentaire ; la dégénérescence maculaire ; la dégénérescence maculaire liée à l'âge (DMLA) ; la DMLA sèche ; la DMLA humide ; la dystrophie maculaire de Stargardt ; la rétinopathie diabétique ; la maculopathie diabétique ; la rétinopathie du prématuré ; la lésion rétinienne liée à la reperfusion ischémique ; l'ischémie ; la lésion de reperfusion d'ischémie ; la lésion d'ischémie-reperfusion due à une greffe ; la lésion d'ischémie-reperfusion due à une intervention chirurgicale ; l'empoisonnement à l'oxygène ; une maladie liée à l'âge ; le diabète ; lesyndrome métabolique ; la maladie de Wolfram ; le cancer ; le cancer du cerveau ; le glioblastome ; la fatigue chronique ; une maladie génétique ; une hémoglobionopathie ; la thalassémie ; l'anémie falciforme ; le déficit en G6PD ; la sclérose en plaques ; un trouble neurodégénératif entraînant des troubles de l'ouïe ou de l'équilibre ; la perte auditive ; la perte auditive induite par le bruit ; le diabète et surdité hérités de la mère (MIDD) ; l'acidose tubulaire rénale ; la nécrose tubulaire aiguë ; les lésions rénales induites par le contraste ; lésions de rétinopathie induites par le contraste ; l'abêtalipoprotéinémie ; le défaut de cobalamine c ; l'acidurie méthylmalonique ; et les dommages ou blessures causés par les radiations.

16. Composé ou composition pharmaceutique à utiliser selon la revendication 15, dans lequel le trouble de stress oxydatif est choisi dans le groupe constitué par la maladie de Parkinson, la dystrophie musculaire de Duchenne, le syndrome de Leigh, le déficit en complexe I, la maladie de Huntington, la maladie d'Alzheimer, la maladie de Huntington juvénile, la sclérose amyotrophique latérale, la démence frontotemporale (DFT) et l'ataxie de Friedreich.
